# DEMANDE DE BREVET EUROPEEN

(11) **EP 4 671 300 A2**
(43) Date de publication de la demande: **31.12.2025**
(21) Numéro de dépôt: 25168993.1
(22) Date de dépôt: 07.12.2018
(51) Int. Cl.: C08G 69/10

(54) **SOLUTION INJECTABLE A PH 7 COMPRENANT AU MOINS UNE INSULINE BASALE DONT LE PI EST COMPRIS ENTRE 5,8 ET 8,5 ET UN CO-POLYAMINOACIDE PORTEUR DE CHARGES CARBOXYLATES ET DE RADICAUX HYDROPHOBES**

(30) Priorité: 07.12.2017 FR 1761807; 07.12.2017 US 201762606138 P; 29.06.2018 FR 1855934
(62) Demande divisionnaire de: 18814899.3
(71) Demandeur: Adocia, 69003 Lyon (FR)
(72) Inventeur: CHAN, You-Ping, 69360 TERNAY (FR); GEISSLER, Alexandre, 69330 MEYZIEU (FR); NOEL, Romain, 69100 VILLEURBANNE (FR); CHARVET, Richard, 69140 RILLIEUX LA PAPE (FR); LAURENT, Nicolas, 01700 MIRIBEL (FR)
(74) Mandataire: Tripoz, Inès

(57) **Abrégé**

L'invention concerne des compositions stables physiquement sous forme d'une solution aqueuse injectable, dont le pH est compris entre 6,0 et 8,0, comprenant au moins :
a) une insuline basale dont le point isoélectrique (pI) est compris entre 5,8 et 8,5 et
b) un co-polyaminoacide porteur de charges carboxylates et d'au moins un radical hydrophobe.

## Description

L'invention concerne les thérapies par injection d'insuline(s) pour traiter le diabète.

L'invention concerne des compositions stables physiquement sous forme d'une solution aqueuse injectable, dont le pH est compris entre 6,0 et 8,0, comprenant au moins une insuline basale dont le point isoélectrique (pI) est compris entre 5,8 et 8,5 et un co-polyaminoacide porteur de charges carboxylates et de radicaux hydrophobes.

L'insulinothérapie, ou thérapie du diabète par injection d'insuline, a connu ces dernières années des progrès remarquables grâce notamment à la mise au point de nouvelles insulines offrant une meilleure correction de la glycémie des patients en comparaison de l'insuline humaine et qui permettent de mieux simuler l'activité physiologique du pancréas.

Lorsqu'un diabète de type II est diagnostiqué chez un patient, un traitement graduel est mis en place. Le patient prend en premier lieu des antidiabétiques oraux (OAD) comme la Metformine. Lorsque les OAD seuls ne suffisent plus à réguler le niveau de glucose dans le sang, un changement dans le traitement doit être fait et, en fonction des spécificités des patients, différentes associations de traitements peuvent être mises en place. Le patient peut par exemple avoir un traitement à base d'une insuline basale de type insuline glargine ou insuline detemir en complément des OAD, puis ensuite en fonction de l'évolution de la pathologie un traitement à base d'insuline basale et d'insuline prandiale.

Par ailleurs, aujourd'hui, pour assurer la transition des traitements par les OAD, lorsque ceux-ci ne sont plus en mesure de contrôler le niveau de glucose dans le sang, vers un traitement insuline basale/insuline prandiale, l'injection d'analogues de GLP-1 RA est préconisée.

Les GLP-1 RA pour agonistes du récepteur Glucagon-Like Peptide-1, sont des peptides insulinotropiques ou incrétines, et appartiennent à la famille des hormones gastro-intestinales (ou Gut Hormones) qui stimulent la sécrétion d'insuline lorsque la glycémie est trop élevée, par exemple après un repas.

Les hormones gastro-intestinales (Gut hormones) sont aussi appelées hormones de satiété. Elles comprennent notamment le GLP-1 RA (Glucagon like peptide-1 receptor agonist) et le GIP (Glucose-dependent insulinotropic peptide), l'oxyntomoduline (un dérivé du proglucagon), le peptide YY, l'amyline, la cholecystokinine, le polypeptide pancréatique (PP), la ghreline et l'entérostatine qui ont des structures peptidiques ou protéiques. Elles stimulent également la sécrétion d'insuline, en réponse au glucose et aux acides gras et sont donc à ce titre des candidats potentiels pour le traitement du diabète.

Parmi celles-ci, les GLP-1 RA sont celles qui ont apporté à ce jour les meilleurs résultats dans le développement de médicaments. Elles ont permis à des patients atteints de diabète de type II de perdre du poids tout en ayant un meilleur contrôle de leur glycémie.

Des analogues ou des dérivés de GLP-1 RA ont ainsi été développés notamment pour améliorer leur stabilité.

D'autre part, pour couvrir ses besoins journaliers en insuline, un patient diabétique dispose, actuellement, de façon schématisée, de deux types d'insulines ayant des actions complémentaires : les insulines prandiales (ou insulines dites à action rapide) et les insulines basales (ou insulines dites à action lente).

Les insulines prandiales permettent une prise en charge rapide (métabolisation et/ou stockage) du glucose apporté lors des repas et collations. Le patient doit s'injecter une insuline prandiale avant chaque prise alimentaire, soit environ 2 à 3 injections par jour. Les insulines prandiales les plus utilisées sont : l'insuline humaine recombinante, NovoLog^{®} (insuline aspart de NOVO NORDISK), Humalog^{®} (insuline lispro de ELI LILLY) et Apidra^{®} (insuline glulisine de SANOFI).

Les insulines basales assurent le maintien de l'homéostasie glycémique du patient, en dehors des périodes de prise alimentaire. Elles agissent essentiellement pour bloquer la production endogène de glucose (glucose hépatique). La dose journalière d'insuline basale correspond généralement à 40-50 % des besoins totaux journaliers en insuline. Selon l'insuline basale utilisée, cette dose est dispensée en 1 ou 2 injections, régulièrement réparties au cours de la journée. Les insulines basales les plus utilisées sont Levemir^{®} (insuline detemir de NOVO NORDISK) et Lantus^{®} (insuline glargine de SANOFI).

On notera pour être exhaustif que la NPH (insuline NPH pour Neutral Protamine Hagedorn ; Humuline NPH^{®}, Insulatard^{®}) est la plus ancienne insuline basale. Cette formulation est le résultat d'une précipitation de l'insuline humaine (anionique à pH neutre) par une protéine cationique, la protamine. Les microcristaux ainsi formés sont dispersés dans une suspension aqueuse et se dissolvent lentement après injection sous-cutanée. Cette dissolution lente assure une libération prolongée de l'insuline. Cependant cette libération n'assure pas une concentration constante d'insuline au cours du temps. Le profil de libération est en forme de cloche et dure seulement entre 12 et 16 heures. Elle est donc injectée deux fois par jour. Cette insuline basale NPH est bien moins performante que les insulines basales modernes, Levemir^{®} et Lantus^{®}. La NPH est une insuline basale à action intermédiaire.

Le principe de la NPH a évolué avec l'apparition des insulines analogues rapides pour donner des produits appelés « Premix » offrant à la fois une action rapide et une action intermédiaire. NovoLog Mix^{®} (NOVO NORDISK) et Humalog Mix^{®} (ELI LILLY) sont des formulations comprenant une insuline analogue rapide, Novolog^{®} et Humalog^{®}, complexée partiellement par la protamine. Ces formulations contiennent ainsi des microcristaux d'insuline analogue dont l'action est dite intermédiaire et une partie d'insuline restée soluble dont l'action est rapide. Ces formulations offrent bien l'avantage d'une insuline rapide mais elles ont aussi le défaut de la NPH, c.-à-d. une durée d'action limitée entre 12 et 16 heures et une insuline libérée en « cloche ». Cependant, ces produits permettent au patient de s'injecter en une seule fois une insuline basale à action intermédiaire avec une insuline prandiale à action rapide. Or nombreux sont les patients soucieux de réduire leur nombre d'injections.

Les insulines basales actuellement commercialisées peuvent être classées en fonction de la solution technique qui permet d'obtenir l'action prolongée et à ce jour deux approches sont utilisées.

La première, celle de l'insuline detemir est la liaison à l'albumine *in vivo.* Il s'agit d'un analogue, soluble à pH 7, qui comprend une chaine latérale d'acide gras (tetradecanoyl) fixée à la position B29 qui, *in vivo,* permet à cette insuline de s'associer à l'albumine. Son action prolongée est principalement due à cette affinité pour l'albumine après injection sous-cutanée.

Cependant son profil pharmacocinétique ne permet pas de couvrir une journée, ce qui fait qu'elle est le plus souvent utilisée en deux injections par jour.

Une autre insuline soluble à pH 7, est l'insuline degludec commercialisée sous le nom de Tresiba^{®d}. Elle comprend également une chaîne latérale d'acide gras fixée sur l'insuline (hexadecandioyl-γ-L-Glu).

La seconde, celle de l'insuline glargine, est la précipitation à pH physiologique. L'insuline glargine est un analogue de l'insuline humaine obtenu par élongation de la partie C-terminale de la chaine B de l'insuline humaine par deux résidus arginine, et par substitution du résidu d'asparagine A21, par un résidu de glycine (US 5,656,722). L'addition de deux résidus d'arginine a été pensée pour ajuster le pI (point isoélectrique) d'insuline glargine au pH physiologique, et ainsi rendre cet analogue de l'insuline humaine insoluble en milieu physiologique.

Aussi, la substitution de l'A21 a été pensée afin de rendre l'insuline glargine stable à pH acide et pouvoir ainsi la formuler sous forme de solution injectable à pH acide. Lors de l'injection sous-cutanée, le passage de l'insuline glargine d'un pH acide (pH 4-4,5) à un pH physiologique (pH neutre) provoque sa précipitation sous la peau. La redissolution lente des micro-particules d'insuline glargine assure une action lente et prolongée.

L'effet hypoglycémiant de l'insuline glargine est quasi-constant sur une durée de 24 heures ce qui permet à la plupart des patients de se limiter à une seule injection par jour.

L'insuline glargine est considérée aujourd'hui comme l'insuline basale la plus utilisée.

Cependant le pH nécessairement acide des formulations d'insulines basales, dont le point isoélectrique est compris entre 5,8 et 8,5, de type insuline glargine, peut être un réel inconvénient, car ce pH acide de la formulation d'insuline glargine entraîne parfois chez les patients des douleurs à l'injection et surtout empêche toute formulation avec d'autres protéines et en particulier avec les insulines prandiales car ces dernières ne sont pas stables à pH acide. L'impossibilité de formuler une insuline prandiale, à pH acide, tient au fait qu'une insuline prandiale subit, dans ces conditions, une réaction secondaire de déamidation en position A21, ce qui ne permet pas de répondre aux exigences de stabilité applicables aux médicaments injectables.

A ce jour, dans les demandes WO 2013/021143 A1, WO 2013/104861 A1, WO 2014/124994 A1 et WO 2014/124993 A1 il a été démontré qu'il était possible de solubiliser ces insulines basales, de type insuline glargine dont le point isoélectrique est compris entre 5,8 et 8,5, à pH neutre, tout en maintenant une différence de solubilité entre le milieu *in-vitro* (le contenant) et le milieu *in-vivo* (sous la peau), indépendamment du pH.

La demande WO 2013/104861 A1, en particulier, décrit des compositions sous forme d'une solution aqueuse injectable, dont le pH est compris entre 6,0 et 8,0, comprenant au moins (a) une insuline basale dont le point isoélectrique pI est compris entre 5,8 et 8,5 et (b) un co-polyaminoacide porteur de charges carboxylates substitué par des radicaux hydrophobes.

Ces compositions de l'art antérieur ont l'inconvénient majeur de ne pas être suffisamment stables pour répondre aux cahiers des charges applicables aux formulations pharmaceutiques.

Dans les exemples de la partie expérimentale de la présente demande de brevet il est démontré que les compositions décrites en particulier dans WO 2013/104861 A1 présentent une stabilité insatisfaisante dans le temps.

Il existe donc un besoin de trouver une solution qui permet de solubiliser une insuline basale dont le point isoélectrique (pI) est compris entre 5,8 et 8,5 tout en conservant son profil basal après injection mais qui permettent également de satisfaire à des conditions de stabilité physique standard pour les produits pharmaceutiques à base d'insuline.

De manière surprenante, la demanderesse a trouvé que les co-polyaminoacides porteurs de charges carboxylates et de radicaux hydrophobes selon l'invention permettent d'obtenir des compositions sous forme de solutions qui non seulement répondent aux exigences décrites dans WO 2013/104861 A1 mais qui de plus sont en mesure de conférer une stabilité physique améliorée auxdites compositions sans avoir à augmenter la quantité d'excipients utilisée.

Ces performances a priori jamais atteintes sont de plus conservées lorsque l'insuline basale dont le point isoélectrique est compris entre 5,8 et 8,5 est associée dans la composition avec une insuline prandiale et/ou une hormone gastro-intestinale.

Ainsi, de façon surprenante, l'affinité des co-polyaminoacides selon l'invention pour l'insuline glargine a été augmentée en ce qu'elle permet d'obtenir une solubilisation et une stabilisation des solutions d'insuline glargine à un ratio [Hy]/[insuline basale] inférieur à celui de l'art antérieur ; ces résultats sont de plus obtenus sans altérer, voire en améliorant, la propension de l'insuline glargine à précipiter comme cela est démontré dans la partie expérimentale.

Cette amélioration de l'affinité permet en outre dans le cadre de traitements chroniques de limiter le niveau d'exposition auxdits excipients.

Les co-polyaminoacides porteurs de charges carboxylates et de radicaux hydrophobes Hy selon l'invention présentent une excellente résistance à l'hydrolyse. Ceci peut notamment être vérifié en conditions accélérées, par exemple par des tests d'hydrolyse à pH basique (pH 12).

En outre des tests d'oxydation forcée, par exemple du type oxydation de Fenton, montrent que les co-polyaminoacides porteurs de charges carboxylates et de radicaux hydrophobes Hy présentent une bonne résistance à l'oxydation.

L'invention concerne ainsi des compositions stables physiquement sous forme d'une solution aqueuse injectable, dont le pH est compris entre 6,0 et 8,0, comprenant au moins :
a) une insuline basale dont le point isoélectrique (pI) est compris entre 5,8 et 8,5 et
b) un co-polyaminoacide porteur de charges carboxylates et d'au moins un radical hydrophobe de formule X.

Dans un mode de réalisation, l'invention concerne une composition sous forme d'une solution aqueuse injectable, dont le pH est compris entre 6,0 et 8,0, comprenant au moins :
a) une insuline basale dont le point isoélectrique pI est compris entre 5,8 et 8,5 ;
b) un co-polyaminoacide porteur de charges carboxylates et de radicaux hydrophobes -Hy, ledit co-polyaminoacide étant constitué d'unités glutamiques ou aspartiques et lesdits radicaux hydrophobes Hy étant de formule X suivante : 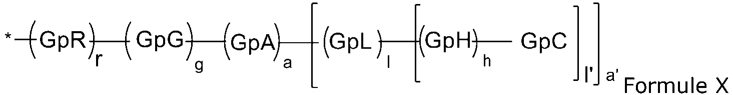 dans laquelle
   - GpR est choisi parmi les radicaux de formules VII, VII' ou VII" : 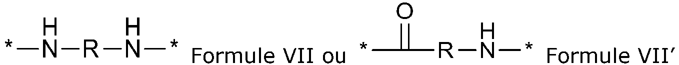 ou 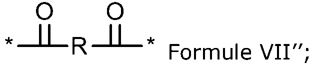
   - GpG et GpH identiques ou différents sont choisis parmi les radicaux de formules XI ou XI': 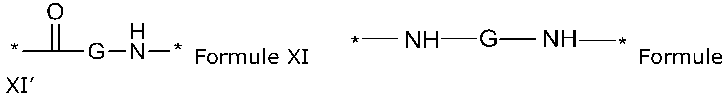
   - GpA est choisi parmi les radicaux de formule VIII 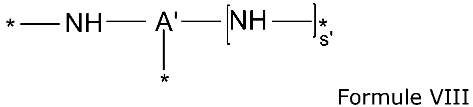 Dans laquelle A' est choisi parmi les radicaux de formule VIII',VIII" ou VIII‴ 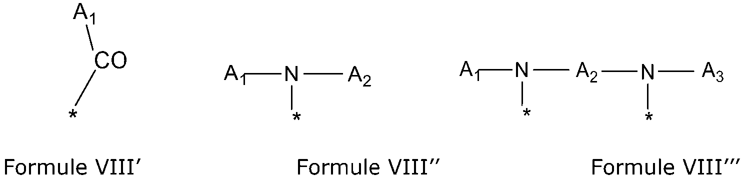
   - -GpL est choisi parmi les radicaux de formule XII 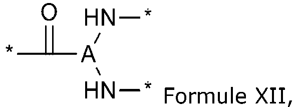
   - GpC est un radical de formule IX : 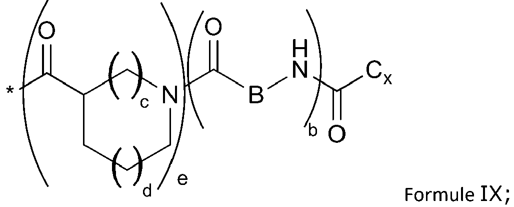
   - les * indiquent les sites de rattachement des différents groupes liés par des fonctions amides ;
   - a est un entier égal à 0 ou à 1 et a' = 1 si a = 0 et a' = 1, 2 ou 3 si a = 1 ;
   - a' est un entier égal à 1, à 2 ou à 3
   - b est un entier égal à 0 ou à 1 ;
   - c est un entier égal à 0 ou à 1, et si c est égal à 0 alors d est égal à 1 ou à 2;
   - d est un entier égal à 0, à 1 ou à 2 ;
   - e est un entier égal à 0 ou à 1 ;
   - g est un entier égal à 0, à 1, à 2, à 3 à 4 à 5 ou à 6;
   - h est un entier égal à 0, à 1, à 2, à 3 à 4 à 5 ou à 6,
   - l est un entier égal à 0 ou 1 et l' = 1 si l = 0 et l' = 2 si l = 1 ;
   - r est un entier égal à 0, à 1 ou à 2, et
   - s'est un entier égal à 0 ou 1, et
   - si e est différent de 0 alors au moins un des g, h ou l est différent de 0 ; et
   - si a = 0 alors l = 0 ;
   - A, A₁, A₂ et A₃ identiques ou différents sont des radicaux alkyles linéaires ou ramifiés, et éventuellement substitués par un radical issu d'un cycle saturé, insaturé ou aromatique,comprenant de 1 à 8 atomes de carbone;
   - B est un radical alkyle linéaire ou ramifié, éventuellement comprenant un noyau aromatique, comprenant de 1 à 9 atomes de carbone ou un radical éther ou polyéther non substitué comprenant de 4 à 14 atomes de carbone et de 1 à 5 atomes d'oxygène ;
   - Cₓ est un radical alkyl monovalent linéaire ou ramifié, éventuellement comprenant une partie cyclique, dans lequel x indique le nombre d'atomes de carbone et :
      ▪ Lorsque le radical hydrophobe -Hy porte 1 -GpC, alors 9 ≤ x ≤ 25,
      ▪ Lorsque le radical hydrophobe -Hy porte 2 -GpC, alors 9 ≤ x ≤ 15,
      ▪ Lorsque le radical hydrophobe -Hy porte 3 -GpC, alors 7 ≤ x ≤ 13,
      ▪ Lorsque le radical hydrophobe -Hy porte 4 -GpC, alors 7 ≤ x ≤ 11,
      ▪ Lorsque le radical hydrophobe -Hy porte au moins 5 -GpC alors, 6 ≤ x ≤ 11,
   - G est un radical alkyle ramifié de 1 à 8 atomes de carbone ledit radical alkyle portant une ou plusieurs fonction(s) acide carboxylique libre.
   - R est un radical choisi dans le groupe constitué par un radical alkyle divalent, linéaire ou ramifié comprenant de 1 à 12 atomes de carbone, un radical alkyle divalent, linéaire ou ramifié comprenant de 1 à 12 atomes de carbone portant une ou plusieurs fonctions -CONH₂ ou un radical éther ou polyéther non substitué comprenant de 4 à 14 atomes de carbone et de 1 à 5 atomes d'oxygène :
   - le ou les radicaux hydrophobes -Hy de formule X étant liés au PLG:
      ∘ via une liaison covalente entre un carbonyle du radical hydrophobe -Hy et un atome d'azote porté par le PLG formant ainsi une fonction amide issue de la réaction d'une fonction amine portée par le PLG et une fonction acide portée par le précurseur -Hy' du radical hydrophobe -Hy, et
      ∘ via une liaison covalente entre un atome d'azote du radical hydrophobe
         - Hy et un carbonyle porté par le PLG formant ainsi une fonction amide issue de la réaction d'une fonction amine du précurseur -Hy' du radical hydrophobe -Hy et une fonction acide portée par le PLG,
   - le ratio M entre le nombre de radicaux hydrophobes et le nombre d'unités glutamiques ou aspartiques étant compris entre 0 < M ≤ 0,5 ;
   - lorsque plusieurs radicaux hydrophobes sont portés par un co-polyaminoacide alors ils sont identiques ou différents,
   - le degré de polymérisation DP en unités glutamiques ou aspartiques pour les chaines PLG est compris entre 5 et 250 ;
   - les fonctions acides carboxyliques libres étant sous forme de sel de cation alcalin choisi dans le groupe constitué par Na⁺ et K⁺.

L'invention concerne également une méthode de préparation de compositions injectables stables.

Le pH des compositions selon l'invention est compris entre 6,0 et 8,0, de préférence compris entre 6,6 et 7,8 ou encore plus préférentiellement entre 6,8 et 7,6.

Ledit co-polyaminoacide porteur de charges carboxylates et de radicaux hydrophobes Hy est soluble en solution aqueuse à pH compris entre 6,0 et 8,0, à une température de 25 °C et à une concentration inférieure à 100 mg/ml.

Ledit co-polyaminoacide porteur de charges carboxylates et de radicaux hydrophobes Hy est soluble en solution aqueuse à pH compris entre 6,0 et 8,0, à une température de 25 °C et à une concentration inférieure à 60 mg/ml.

Le co-polyaminoacide est un co-polyaminoacide statistique dans l'enchaînement des unités glutamiques et/ou aspartiques.

On entend par « radical alkyle » une chaine carbonée, linéaire ou ramifiée, qui ne comprend pas d'hétéroatome.

Dans les formules les * indiquent les sites de rattachements des différents éléments représentés.

On entend par « composition stable physiquement » des compositions qui satisfont aux critères de l'inspection visuelle décrite dans la pharmacopée européenne, américaine et internationale, c'est-à-dire des compositions qui sont claires et qui ne contiennent pas de particules visibles, mais également incolores.

On entend par « solution aqueuse injectable » des solutions dont le solvant est l'eau et qui satisfont aux conditions des pharmacopées EP et US.

Les compositions sous forme d'une solution aqueuse injectable selon l'invention sont des solutions limpides. On entend par « solution limpide », des compositions qui satisfont aux critères décrits dans les pharmacopées américaine et européenne concernant les solutions injectables. Dans la pharmacopée US, les solutions sont définies dans la partie <1151> faisant référence à l'injection <1> (faisant référence à <788> selon USP 35 et précisé dans <788> selon USP 35 et dans <787>, <788> et <790> USP 38 (à partir du 1^{er} aout 2014), selon USP 38). Dans la pharmacopée européenne, les solutions injectables doivent remplir les critères donnés dans les sections 2.9.19 et 2.9.20.

On entend par « co-polyaminoacide étant constitué d'unités glutamiques ou aspartiques » des enchainements linéaires non cycliques d'unités acide glutamique ou acide aspartique liées entre elles par des liaisons peptidiques, lesdits enchainements présentant une partie C-terminale, correspondant à l'acide carboxylique d'une extrémité, et une partie N-terminale, correspondant à l'amine de l'autre extrémité de l'enchainement.

On entend par « soluble », susceptible de permettre de préparer une solution limpide et dépourvue de particules à une concentration inférieure à 100 mg/ml dans de l'eau distillée à 25 °C.

Les radicaux Hy, GpR, GpG, GpH, GpA, GpL et GpC sont chacun indépendamment identiques ou différents d'un résidu à l'autre.

Dans un mode de réalisation, la composition selon l'invention est caractérisée en ce que Hy comprend entre 15 et 100 atomes de carbone.

Dans un mode de réalisation, la composition selon l'invention est caractérisée en ce que Hy comprend entre 30 et 70 atomes de carbone.

Dans un mode de réalisation, la composition selon l'invention est caractérisée en ce que Hy comprend entre 40 et 60 atomes de carbone.

Dans un mode de réalisation, la composition selon l'invention est caractérisée en ce que Hy comprend entre 20 et 30 atomes de carbone.

Dans un mode de réalisation, la composition selon l'invention est caractérisée en ce que Hy comprend plus de 30 atomes de carbone.

Dans les formules, les * indiquent les sites de rattachement des radicaux hydrophobes au PLG ou entre les différents groupes GpR, GpG, GpH, GpA, GpL et GpC pour former des fonctions amides.

Les radicaux Hy sont rattachés au PLG via des fonctions amides.

Dans un mode de réalisation, r=0 et le radical hydrophobe de formule X est lié au PLG via une liaison covalente entre un carbonyl du radical hydrophobe et un atome d'azote porté par le PLG formant ainsi une fonction amide issue de la réaction d'une fonction amine portée par le précurseur du PLG et une fonction acide portée par le précurseur Hy' du radical hydrophobe.

Dans un mode de réalisation, r=1 ou 2 et le radical hydrophobe de formule X est lié au PLG :
▪ via une liaison covalente entre un atome d'azote du radical hydrophobe et un carbonyle porté par le PLG, formant ainsi une fonction amide issue de la réaction d'une fonction amine du précurseur -Hy' du radical hydrophobe et une fonction acide portée par le PLG ou,
▪ via une liaison covalente entre un carbonyl du radical hydrophobe et un atome d'azote porté par le PLG, formant ainsi une fonction amide issue de la réaction d'une fonction acide du précurseur Hy' du radical hydrophobe -Hy et une fonction amine du PLG.

Dans un mode de réalisation, si GpA est un radical de formule VIIIc et r=1, alors :
- les GpC sont liés directement ou indirectement à N_{α1} et N_{α2} et le PLG est lié directement ou indirectement via GpR à N_{β1} , ou
- ₂ les GpC sont liés directement ou indirectement à N_{α1} et N_{β1}, et le PLG est lié directement ou indirectement via GpR à N_{α2}, ou
- les GpC sont liés directement ou indirectement à N_{α2} et N_{β1}, et le PLG est lié directement ou indirectement via GpR à N_{α1}.

Dans un mode de réalisation, si GpA est un radical de formule VIIIc et r=0, alors :
- les GpC sont liés directement ou indirectement à N_{α1} et N_{α2} et le PLG est lié directement ou indirectement à N_{β1} ; ou
- les GpC sont liés directement ou indirectement à N_{α1} et N_{β1}, et le PLG est lié directement ou indirectement à N_{α2} ; ou
- les GpC sont liés directement ou indirectement à N_{α2} et N_{β1}, et le PLG est lié directement ou indirectement à N_{α1}.

Dans un mode de réalisation, si GpA est un radical de formule VIIId et r=1, alors
- les GpC sont liés directement ou indirectement à N_{α1}, N_{α2} et N_{β1} et le PLG est lié directement ou indirectement via GpR à N_{β2} ; ou
- les GpC sont liés directement ou indirectement à N_{α1}, N_{α2} et N_{β2} et le PLG est lié directement ou indirectement via GpR à N_{β1} ; ou
- les GpC sont liés directement ou indirectement à N_{α1}, N_{β1} et N_{β2} et le PLG est lié directement ou indirectement via GpR à N_{α2} ; ou
- les GpC sont liés directement ou indirectement à N_{α2}, N_{β1} et N_{β2} et le PLG est lié directement ou indirectement via GpR à N_{α1}.

Dans un mode de réalisation, si GpA est un radical de formule VIIId et r=0, alors
- les GpC sont liés directement ou indirectement à N_{α1}, N_{α2} et N_{β1} et le PLG est lié directement ou indirectement à N_{β2} ; ou
- les GpC sont liés directement ou indirectement à N_{α1}, N_{α2} et N_{β2} et le PLG est lié directement ou indirectement à N_{β1} ; ou
- les GpC sont liés directement ou indirectement à N_{α1}, N_{β1} et N_{β2} et le PLG est lié directement ou indirectement à N_{α2} ; ou
- les GpC sont liés directement ou indirectement à N_{α2}, N_{β1} et N_{β2} et le PLG est lié directement ou indirectement à N_{α1.*}

Dans un mode de réalisation, lorsque r=2 alors le groupe GpR lié au PLG est choisi parmi les GpR de formule VII.

Dans un mode de réalisation, lorsque r=2 alors le groupe GpR lié au PLG est choisi parmi les GpR de formule VII et le deuxième GpR est choisi parmi les GpR de formule VII".

Dans un mode de réalisation, un mode de réalisation, lorsque r=2 alors le groupe GpR lié au PLG est choisi parmi les GpR de formule VII".

Dans un mode de réalisation, un mode de réalisation, lorsque r=2 alors le groupe GpR lié au PLG est choisi parmi les GpR de formule VII" et le deuxième GpR est choisi parmi les GpR de formule VII.

Dans un mode de réalisation, a =0,

Dans un mode de réalisation h= 1 et g=0,

Dans un mode de réalisation h= 0 et g=1,

Dans un mode de réalisation, r = 0, g=1 et h=0.

Dans un mode de réalisation, au moins un des g, h ou l est différent de 0.

Dans un mode de réalisation au moins un de g et de h est égal à 1.

Dans un mode de réalisation au moins un de g et h est égal à 1.

Dans un mode de réalisation a = 1 et l = 1.

Dans un mode de réalisation, si l = 0, au moins un des g ou h est égal à 0.

Dans un mode de réalisation, si l = 1, au moins un des g ou h est égal à 0.

Dans un mode de réalisation g+h≥2.

Dans un mode de réalisation g est supérieur ou égal à 2 (g≥2).

Dans un mode de réalisation h est supérieur ou égal à 2 (h≥2).

Dans un mode de réalisation, g+h≥2 et a et l sont égaux à 0 (a=l=0).

Dans un mode de réalisation, g+h≥2 et b est égal à 0 (b=0).

Dans un mode de réalisation g ou h est supérieur ou égal à 2 (g≥2) et b est égal à 0.

Dans un mode de réalisation, g+h≥2, b est égal à 0 (b=0) et e est égal à 1 (e=1).

Dans un mode de réalisation g ou h est supérieur ou égal à 2 (g≥2) b est égal à 0 (b=0) et e est égal à 1 (e=1).

Dans un mode de réalisation, ledit au moins un radical hydrophobe -Hy est choisi parmi les radicaux de formule X dans laquelle r = 2 de formule Xc', telle que définie ci-dessous : 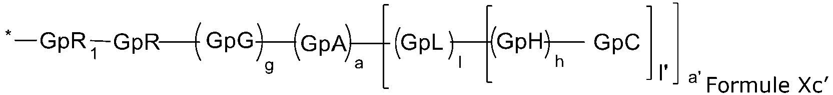 dans laquelle GpR₁ est un radical de formule VII. 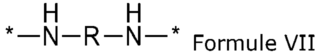 dans laquelle GpR, GpG, GpA, GpL, GpH, GpC, R, a, a', g, h, l et l' ont les définitions données précédemment.

Dans un mode de réalisation, ledit au moins un radical hydrophobe -Hy est choisi parmi les radicaux de formule X dans laquelle r = 2 de formule Xc', telle que définie ci-dessous : 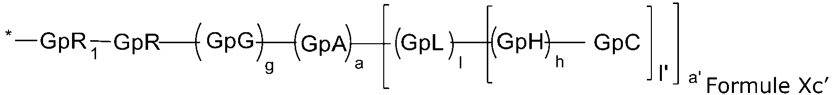 dans laquelle GpR₁ est un radical de formule VII". 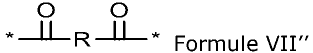 dans laquelle GpR, GpG, GpA, GpL, GpH, GpC, R, a, a', g, h, l et l' ont les définitions données précédemment.

Dans un mode de réalisation g = h = 0, a = 1, GpA est un radical de Formule VIII avec s' = 1 et A' de Formule VIII' ou VIII", et l = 1.

Dans un mode de réalisation, ledit au moins un radical hydrophobe -Hy est choisi parmi les radicaux de formule X dans laquelle r = 2 de formule Xc', telle que définie ci-dessous : 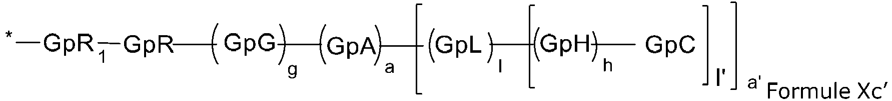 dans laquelle GpR₁ est un radical de formule VII. 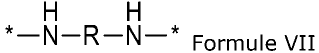 dans laquelle GpR, GpG, GpA, GpL, GpH, GpC, R, a, a', g, h, l et l' ont les définitions données précédemment.

Dans un mode de réalisation, ledit au moins un radical hydrophobe -Hy est choisi parmi les radicaux de formule X dans laquelle r = 2 de formule Xc', telle que définie ci-dessous : 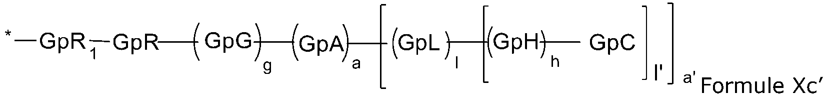 dans laquelle GpR₁ est un radical de formule VII". 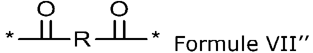 dans laquelle GpR, GpG, GpA, GpL, GpH, GpC, R, a, a', g, h, l et l' ont les définitions données précédemment.

Dans un mode de réalisation, ledit au moins un radical hydrophobe -Hy est choisi parmi les radicaux de formule X dans laquelle
- l = 0,
- de formule Xb' telle que définie ci-dessous 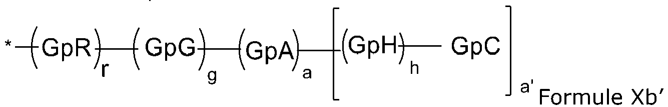 dans laquelle
- GpR est choisi parmi les radicaux de formules VII, VII' ou VII" : 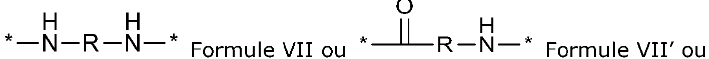 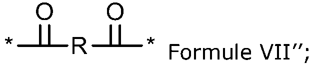
- GpG est choisi parmi les radicaux de formule XI ou XI' : 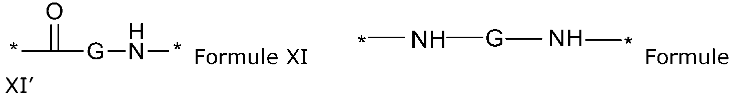
- GpA est choisi parmi les radicaux de formule VIII dans laquelle s' = 1 représentée par la formule VIIIa ou de formule VIII dans laquelle et s' = 0 représentée par la formule VIIIb : 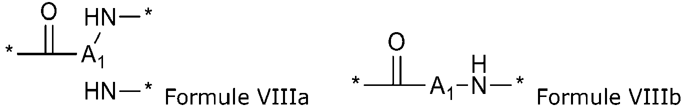
- GpC est un radical de formule IX : 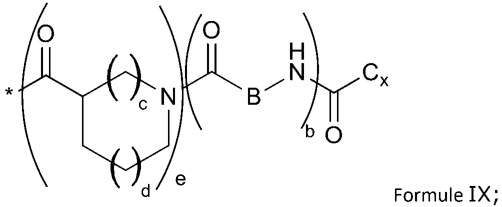
- les * indiquent les sites de rattachement des différents groupes liés par des fonctions amides ;
- a est un entier égal à 0 ou à 1 et a' = 1 si a = 0 et a' = 1 ou a' = 2 si a = 1 ;
- a' est un entier égal à 1 ou 2 et
   ∘ si a' est égal à 1 alors a est égal à 0 ou à 1 et GpA est un radical de formule VIIIb et,
   ∘ si a' est égal à 2 alors a est égal à 1, et GpA est un radical de formule VIIIa;
- b est un entier égal à 0 ou à 1 ;
- c est un entier égal à 0 ou à 1, et si c est égal à 0 alors d est égal à 1 ou à 2;
- d est un entier égal à 0, à 1 ou à 2 ;
- e est un entier égal à 0 ou à 1 ;
- g est un entier égal à 0, à 1, à 2, à 3 à 4 à 5 ou à 6 ;
- h est un entier égal à 0, à 1, à 2, à 3 à 4 à 5 ou à 6, et au moins un des g ou h est différent de 0 ;
- r est un entier égal à 0, 1 ou à 2, et
- s'est un entier égal à 0 ou 1 ;
- A₁ est un radical alkyle linéaire ou ramifié, et éventuellement substitués par un radical issu d'un cycle saturé, insaturé ou aromatique, comprenant de 1 à 6 atomes de carbone;
- B est un radical alkyle linéaire ou ramifié, éventuellement comprenant un noyau aromatique, comprenant de 1 à 9 atomes de carbone ou un radical éther ou polyéther non substitué comprenant de 4 à 14 atomes de carbone et de 1 à 5 atomes d'oxygène ;
- Cₓ est un radical alkyl monovalent linéaire ou ramifié, éventuellement comprenant une partie cyclique, dans lequel x indique le nombre d'atomes de carbone et :
   ▪ Lorsque le radical hydrophobe -Hy porte 1 -GpC, alors 9 ≤ x ≤ 25,
   ▪ Lorsque le radical hydrophobe -Hy porte 2 -GpC, alors 9 ≤ x ≤ 15,
   ▪ Lorsque le radical hydrophobe -Hy porte 3 -GpC, alors 7 ≤ x ≤ 13,
   ▪ Lorsque le radical hydrophobe -Hy porte 4 -GpC, alors 7 ≤ x ≤ 11,
   ▪ Lorsque le radical hydrophobe -Hy porte au moins 5 -GpC alors, 6 ≤ x ≤ 11,
- G est un radical alkyle ramifié de 1 à 8 atomes de carbone ledit radical alkyle portant une ou plusieurs fonction(s) acide carboxylique libre,
- R est un radical choisi dans le groupe constitué par un radical alkyle divalent, linéaire ou ramifié comprenant de 1 à 12 atomes de carbone, un radical alkyle divalent, linéaire ou ramifié comprenant de 1 à 12 atomes de carbone portant une ou plusieurs fonctions -CONH₂ ou un radical éther ou polyéther non substitué comprenant de 4 à 14 atomes de carbone et de 1 à 5 atomes d'oxygène :
- Le ou les radicaux hydrophobes Hy de formule X étant liés au PLG:
   ∘ via une liaison covalente entre un carbonyle du radical hydrophobe et un atome d'azote porté par le PLG formant ainsi une fonction amide issue de la réaction d'une fonction amine portée par le PLG et une fonction acide portée par le précurseur du radical hydrophobe , et
   ∘ via une liaison covalente entre un atome d'azote du radical hydrophobe et un carbonyle porté par le PLG, formant ainsi une fonction amide issue de la réaction d'une fonction amine du précurseur -Hy' du radical hydrophobe et une fonction acide portée par le PLG.
- le ratio M entre le nombre de radicaux hydrophobes et le nombre d'unités glutamiques ou aspartiques étant compris entre 0 < M ≤ 0,5 ;
- lorsque plusieurs radicaux hydrophobes sont portés par un co-polyaminoacide alors ils sont identiques ou différents,
- les fonctions acides carboxyliques libres étant sous forme de sel de cation alcalin choisi dans le groupe constitué par Na⁺ et K⁺.

Dans un mode de réalisation ledit au moins un radical hydrophobe -Hy est choisi parmi les radicaux de formule X telle que définie ci-dessous dans laquelle l = 0,
- GpA est choisi parmi les radicaux de formule VIII dans laquelle s' = 1 et A' choisi parmi les radicaux de formule VIII" ou VIII‴,
- de formule Xb' telle que définie ci-dessous : 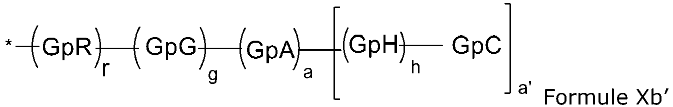 dans laquelle
- GpR est choisi parmi les radicaux de formules VII, VII' ou VII" : 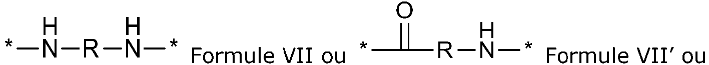 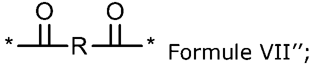
- GpG est choisi parmi les radicaux de formule XI ou XI': 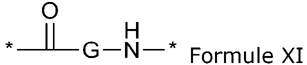 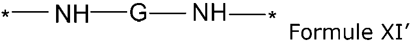
- GpA est choisi parmi les radicaux de formules, VIIIc ou VIIId : 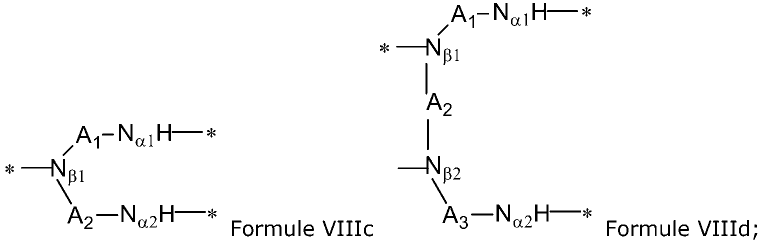
- GpC est un radical de formule IX : 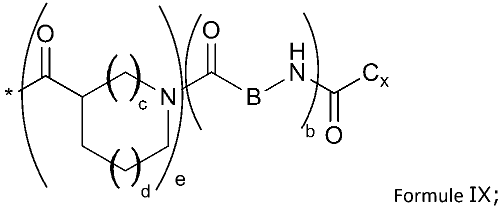
- les * indiquent les sites de rattachement des différents groupes liés par des fonctions amides ;
- a est un entier égal à 0 ou à 1 et a' = 1 si a = 0 et a'=2 ou 3 si a = 1 ;
- a' est un entier égal à 2 ou à 3 et
   ∘ si a' est égal à 1 alors a est égal à 0 et,
   ∘ si a' est égal à 2 ou 3 alors a est égal à 1, et GpA est un radical de formule VIIIc ou VIIId ;
- b est un entier égal à 0 ou à 1 ;
- c est un entier égal à 0 ou à 1, et si c est égal à 0 alors d est égal à 1 ou à 2;
- d est un entier égal à 0, à 1 ou à 2 ;
- e est un entier égal à 0 ou à 1 ;
- g est un entier égal à 0, à 1, à 2, à 3 à 4 à 5 ou à 6;
- h est un entier égal à 0, à 1, à 2, à 3 à 4 à 5 ou à 6, et au moins un des g ou h est différent de 0 ;
- r est un entier égal à 0, 1 ou à 2, et
- s'est un entier égal à 1 ;
- A₁, A₂, A₃ identiques ou différents sont des radicaux alkyles linéaires ou ramifiés, et éventuellement substitués par un radical issu d'un cycle saturé, insaturé ou aromatique, comprenant de 1 à 6 atomes de carbone;
- B est un radical alkyle linéaire ou ramifié, éventuellement comprenant un noyau aromatique, comprenant de 1 à 9 atomes de carbone ou un radical éther ou polyéther non substitué comprenant de 4 à 14 atomes de carbone et de 1 à 5 atomes d'oxygène ;
- Cₓ est un radical alkyl monovalent linéaire ou ramifié, éventuellement comprenant une partie cyclique, dans lequel x indique le nombre d'atomes de carbone et :
   ▪ Lorsque le radical hydrophobe -Hy porte 1 -GpC, alors 9 ≤ x ≤ 25,
   ▪ Lorsque le radical hydrophobe -Hy porte 2 -GpC, alors 9 ≤ x ≤ 15,
   ▪ Lorsque le radical hydrophobe -Hy porte 3 -GpC, alors 7 ≤ x ≤ 13,
   ▪ Lorsque le radical hydrophobe -Hy porte 4 -GpC, alors 7 ≤ x ≤ 11,
   ▪ Lorsque le radical hydrophobe -Hy porte au moins 5 -GpC alors, 6 ≤ x ≤ 11,
- Le ou les radicaux hydrophobes Hy de formule X étant liés au PLG:
   ∘ via une liaison covalente entre un carbonyle du radical hydrophobe et un atome d'azote porté par le PLG formant ainsi une fonction amide issue de la réaction d'une fonction amine portée par le PLG et une fonction acide portée par le précurseur -Hy' du radical hydrophobe, et
   ∘ via une liaison covalente entre un atome d'azote du radical hydrophobe et un carbonyle porté par le PLG, formant ainsi une fonction amide issue de la réaction d'une fonction amine du précurseur -Hy' du radical hydrophobe et une fonction acide portée par le PLG.
- G est un radical alkyle ramifié de 1 à 8 atomes de carbone ledit radical alkyle portant une ou plusieurs fonction(s) acide carboxylique libre,
- R est un radical choisi dans le groupe constitué par un radical alkyle divalent, linéaire ou ramifié comprenant de 1 à 12 atomes de carbone, un radical alkyle divalent, linéaire ou ramifié comprenant de 1 à 12 atomes de carbone portant une ou plusieurs fonctions -CONH₂ ou un radical éther ou polyéther non substitué comprenant de 4 à 14 atomes de carbone et de 1 à 5 atomes d'oxygène :
- le ratio M entre le nombre de radicaux hydrophobes et le nombre d'unités glutamiques ou aspartiques étant compris entre 0 < M≤ 0,5 ;
- lorsque plusieurs radicaux hydrophobes sont portés par un co-polyaminoacide alors ils sont identiques ou différents,
- les fonctions acides carboxyliques libres étant sous forme de sel de cation alcalin choisi dans le groupe constitué par Na⁺ et K⁺.

Dans un mode de réalisation, ledit au moins un radical hydrophobe -Hy est choisi parmi les radicaux de formule X dans laquelle a =1 et a' = 1 de formule Xa telle que définie ci-dessous : 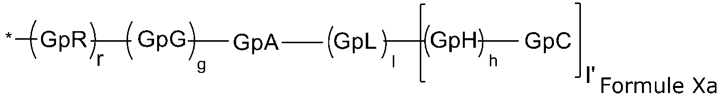 dans laquelle GpA est un radical de formule VIII et A' est choisi parmi les radicaux de formule VIII' avec s'=0 et GpA est un radical de Formule VIIIb 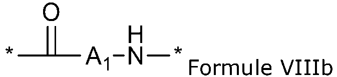 Et GpR, GpG, GpL, GpH, GpC, A₁, r, g, h, l et l' ont les définitions données précédemment.

Dans un mode de réalisation, ledit au moins un radical hydrophobe -Hy est choisi parmi les radicaux de formule X dans laquelle a =1 de formule Xb telle que définie ci-dessous : 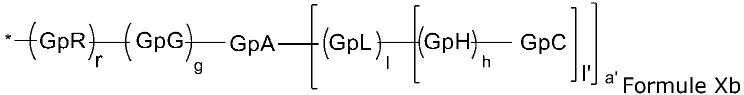 dans laquelle GpA est un radical de formule VIII et A' est choisi parmi les radicaux de formule VIII' avec s' = 1 et GpA est un radical de Formule VIIIa 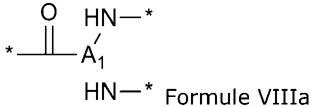 Et GpR, GpG, GpL, GpH, GpC, A₁, a', r, g, h, l et l' ont les définitions données précédemment.

Dans un mode de réalisation, ledit au moins un radical hydrophobe -Hy est choisi parmi les radicaux de formule X dans laquelle a =1 telle que définie ci-dessous : 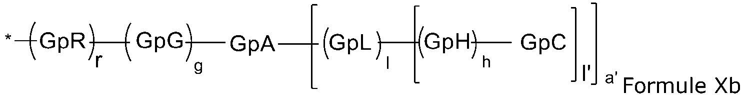 dans laquelle GpA est un radical de formule VIII et A est choisi parmi les radicaux de formule VIII" avec s'=1 et GpA est un radical de Formule VIIIc 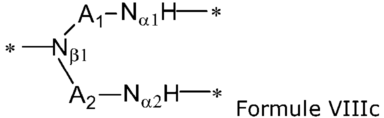 Et GpR, GpG, GpL, GpH, GpC, A₁, A₂, r, g, h, a', l et l' ont les définitions données précédemment.

Dans un mode de réalisation, ledit au moins un radical hydrophobe -Hy est choisi parmi les radicaux de formule X dans laquelle a =1 telle que définie ci-dessous : 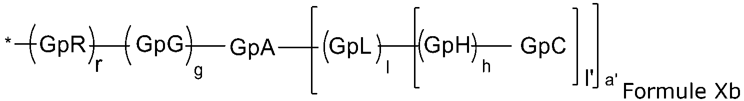 dans laquelle GpA est un radical de formule VIII et A est choisi parmi les radicaux de formule VIII‴ avec s'=1, et GpA est un radical de formule VIIId 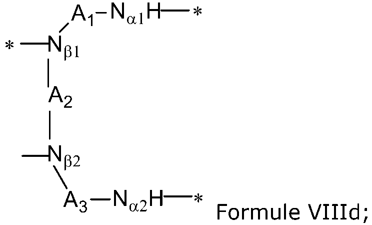 Et GpR, GpG, GpL, GpH, GpC, A₁, A₂, A₃, a', r, g, h, l et l' ont les définitions données précédemment.

Dans un mode de réalisation, la composition selon l'invention est caractérisée en ce que lesdits radicaux hydrophobes sont choisis parmi les radicaux hydrophobes de formule X dans laquelle GpA est un radical de formule VIIIb, a' = 1 et l = 0 représentée par la formule Xe suivante : 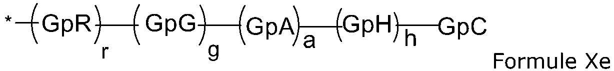 GpR, GpG, GpA, GpH, GpC, r, g, h, et a ont les définitions données précédemment.

Dans un mode de réalisation, r=0, et GpA est choisi parmi les radicaux de formule VIIIa et VIIIb.

Dans un mode de réalisation, r=0, g=0 et GpA est choisi parmi les radicaux de formule VIIIa et VIIIb.

Dans un mode de réalisation, r=0, GpA est choisi parmi les radicaux de formule VIIIa et VIIIb et h=0.

Dans un mode de réalisation, ledit au moins un radical hydrophobe -Hy est choisi parmi les radicaux de formule X dans laquelle r=1 de formule Xc, telle que définie ci-dessous : 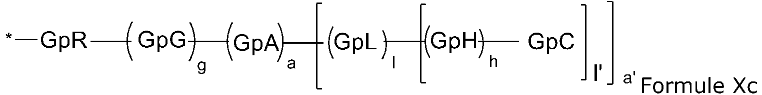 dans laquelle GpR est un radical de formule VII. 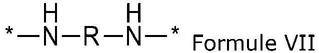 Et GpG, GpA, GpL, GpH, GpC, R, a, a', g, h, l, a' et l' ont les définitions données précédemment.

Dans un mode de réalisation, ledit au moins un radical hydrophobe -Hy est choisi parmi les radicaux de formule X dans laquelle r=1 de formule Xc, telle que définie ci-dessous : 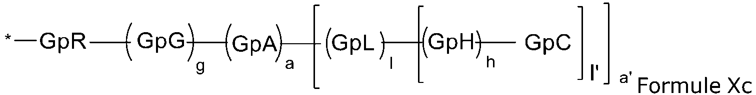 dans laquelle GpR est un radical de formule VII'. 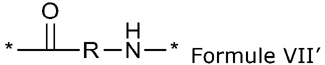 Et GpG, GpA, GpL, GpH, GpC, R, a, a', g, h, l, a' et l' ont les définitions données précédemment.

Dans un mode de réalisation, ledit au moins un radical hydrophobe -Hy est choisi parmi les radicaux de formule X dans laquelle r=1 de formule Xc telle que définie ci-dessous : 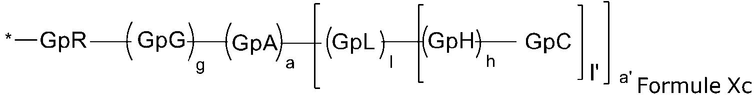 dans laquelle GpR est un radical de formule VII".

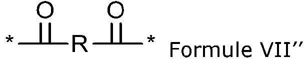

Dans un mode de réalisation, r=1 et GpR est choisi parmi les radicaux de formule VII' ou VII" et h = 0.

Dans un mode de réalisation, r=1, g=0 et GpR est un radical de formule VII' et h = 0.
- Dans un mode de réalisation, r=1, g=0 et GpR est un radical de formule VII' et h = 1.

Dans un mode de réalisation, r=1, g=0, GpR est un radical de formule VII', GpA est choisi parmi les radicaux de formule VIIIa ou VIIIb et h = 0.

Dans un mode de réalisation, r=1, g=0, GpR est un radical de formule VII', GpA est choisi parmi les radicaux de formule VIIIa ou VIIIb et h = 1.

Dans un mode de réalisation, r=1, g=0, GpR est un radical de formule VII', GpA est un radical de formule VIIIa et h = 0.

Dans un mode de réalisation, r=1, g=0, GpR est un radical de formule VII', GpA est un radical de formule VIIIa et h = 1.

Dans un mode de réalisation, r=1, g=0, GpR est un radical de formule VII', GpA est un radical de formule VIIIb et h = 0.

Dans un mode de réalisation, r=1, g=0, GpR est un radical de formule VII', GpA est un radical de formule VIIIb et h = 1.

Dans un mode de réalisation ledit au moins un radical hydrophobe -Hy est choisi parmi les radicaux de formule X telle que définie ci-dessous : 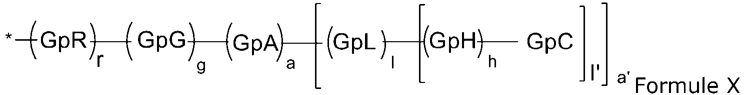 dans laquelle GpC est un radical de formule IX dans laquelle e=0 et GpC est un radical de formule IXa

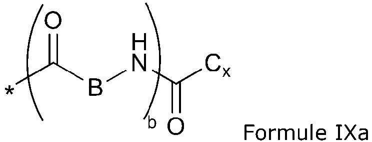

Dans un mode de réalisation ledit au moins un radical hydrophobe -Hy est choisi parmi les radicaux de formule X telle que définie ci-dessous : 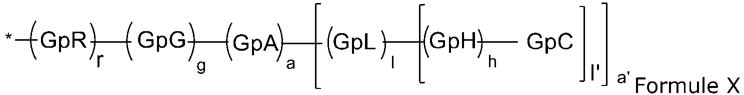 dans laquelle GpC est un radical de formule IX dans laquelle e=1, b = 0 et GpC est un radical de formule IXd

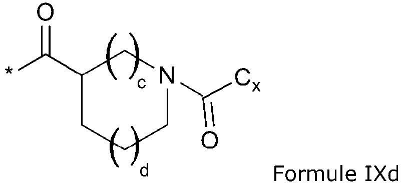

Dans un mode de réalisation ledit au moins un radical hydrophobe -Hy est choisi parmi les radicaux de formule X telle que définie ci-dessous : 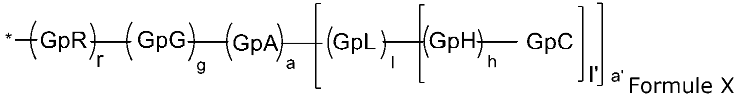 dans laquelle GpC est un radical de formule IX dans laquelle e=1 et GpC est un radical de formule IXb

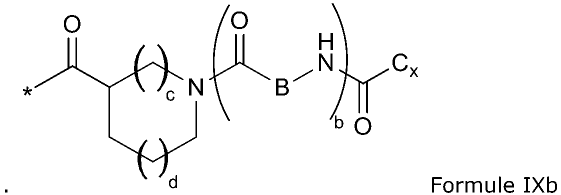

Dans un mode de réalisation leditau moins un radical hydrophobe -Hy est choisi parmi les radicaux de formule X dans laquelle r, g, a, l, h sont égaux à 0, de formule Xd telle que définie ci-dessous :

*-GpC Formule Xd.

dans laquelle GpC est un radical de formule IX dans laquelle e=0, b= 0 et GpC est un radical de formule IXc

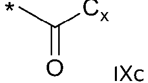

Dans un mode de réalisation, la composition selon l'invention est caractérisée en ce que lesdits radicaux hydrophobes sont choisis parmi les radicaux hydrophobes de formule X dans laquelle, a' = 2 et a = 1 et l = 0 représentée par la formule Xf suivante : 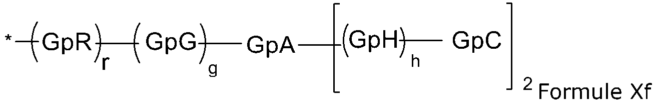 GpR, GpG, GpA, GpH, GpC, r, g et h ont les définitions données précédemment.

Dans un mode de réalisation, la composition selon l'invention est caractérisée en ce que lesdits radicaux hydrophobes sont choisis parmi les radicaux hydrophobes de formule X dans laquelle h = 0, l = 0 et l' = 1 représentée par la formule Xg suivante : 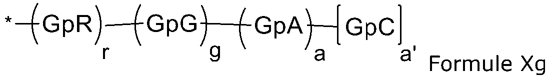 GpR, GpG, GpA, GpC, r, g, a et a' ont les définitions données précédemment.

Dans un mode de réalisation, la composition selon l'invention est caractérisée en ce que lesdits radicaux hydrophobes sont choisis parmi les radicaux hydrophobes de formule X dans laquelle h = 0, a'= 1 représentée par la formule Xh suivante : 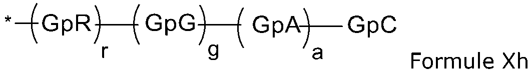 GpR, GpG, GpA, GpC, r, a et g ont les définitions données précédemment.

Dans un mode de réalisation, la composition selon l'invention est caractérisée en ce que lesdits radicaux hydrophobes sont choisis parmi les radicaux hydrophobes de formule X dans laquelle h = 0, a' = 2 et a = 1 représentée par la formule Xi suivante : 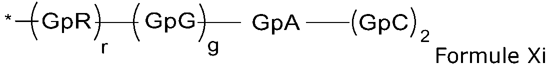 GpR, GpG, GpA, GpC, r et g ont les définitions données précédemment.

Dans un mode de réalisation, la composition est caractérisée en ce que le radical hydrophobe de formules X, Xa, Xb, Xb', Xc, Xe, Xf, Xg, Xh et Xi est un radical dans lequel R est un radical alkyle linéaire divalent comprenant de 2 à 12 atomes de carbone.

Dans un mode de réalisation, la composition est caractérisée en ce que le radical hydrophobe de formules X, Xa, Xb, Xb', Xc, Xe, Xf, Xg, Xh et Xi est un radical dans lequel R est un radical alkyle divalent comprenant de 2 à 6 atomes de carbone.

Dans un mode de réalisation, la composition est caractérisée en ce que le radical hydrophobe de formules X, Xa, Xb, Xb', Xc, Xe, Xf, Xg, Xh et Xi est un radical dans lequel R est un radical alkyle linéaire divalent comprenant de 2 à 6 atomes de carbone.

Dans un mode de réalisation, la composition est caractérisée en ce que le radical hydrophobe de formules X, Xa, Xb, Xb', Xc, Xe, Xf, Xg, Xh et Xi est un radical dans lequel R est un radical alkyle divalent comprenant de 2 à 4 atomes de carbone.

Dans un mode de réalisation, la composition est caractérisée en ce que le radical hydrophobe de formules X, Xa, Xb, Xb', Xc, Xe, Xf, Xg, Xh et Xi est un radical dans lequel R est un radical alkyle linéaire divalent comprenant de 2 à 4 atomes de carbone.

Dans un mode de réalisation, la composition est caractérisée en ce que le radical hydrophobe de formules X, Xa, Xb, Xb', Xc, Xe, Xf, Xg, Xh et Xi est un radical dans lequel R est un radical alkyle divalent comprenant 2 atomes de carbone.

Dans un mode de réalisation, la composition est caractérisée en ce que le radical hydrophobe de formules X, Xa, Xb, Xb', Xc, Xe, Xf, Xg, Xh et Xi est un radical dans lequel R est un radical alkyle linéaire divalent comprenant de 1 à 11 atomes de carbone.

Dans un mode de réalisation, la composition est caractérisée en ce que le radical hydrophobe de formules X, Xa, Xb, Xb', Xc, Xe, Xf, Xg, Xh et Xi est un radical dans lequel R est un radical alkyle divalent comprenant de 1 à 6 atomes de carbone.

Dans un mode de réalisation, la composition est caractérisée en ce que le radical hydrophobe de formules X, Xa, Xb, Xb', Xc, Xe, Xf, Xg, Xh et Xi est un radical dans lequel R est un radical alkyle divalent, comprenant de 2 à 5 atomes de carbone et portant une ou plusieurs fonctions amide (-CONH2).

Dans un mode de réalisation, la composition est caractérisée en ce que le radical hydrophobe de formules X, Xa, Xb, Xb', Xc, Xe, Xf, Xg, Xh et Xi est un radical dans lequel R est un radical alkyle linéaire divalent, comprenant de 2 à 5 atomes de carbone et portant une ou plusieurs fonctions amide (-CONH2).

Dans un mode de réalisation, la composition est caractérisée en ce que le radical hydrophobe de formules X, Xa, Xb, Xb', Xc, Xe, Xf, Xg, Xh et Xi est un radical dans lequel R est un radical choisi dans le groupe constitué par les radicaux représentés par les formules ci-dessous :

| | |
|---|---|
| 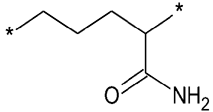 | Formule X1 |
| 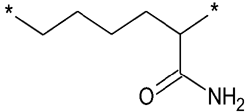 | Formule X2 |

Dans un mode de réalisation, la composition est caractérisée en ce que le radical hydrophobe de formules X, Xa, Xb, Xb', Xc, Xe, Xf, Xg, Xh et Xi est un radical dans lequel R est un radical de formule X1.

Dans un mode de réalisation, la composition est caractérisée en ce que le radical hydrophobe de formules X, Xa, Xb, Xb', Xc, Xe, Xf, Xg, Xh et Xi est un radical dans lequel R est un radical de formule X2.

Dans un mode de réalisation, la composition est caractérisée en ce que le radical hydrophobe de formules X, Xa, Xb, Xb', Xc, Xe, Xf, Xg, Xh et Xi est un radical dans lequel R est lié au co-polyaminoacide via une fonction amide portée par le carbone en position delta ou epsilon (ou en position 4 ou 5) par rapport à la fonction amide (-CONH₂).

Dans un mode de réalisation, la composition est caractérisée en ce que le radical hydrophobe de formules X, Xa, Xb, Xb', Xc, Xe, Xf, Xg, Xh et Xi est un radical dans lequel R est un radical linéaire éther ou polyéther non substitué comprenant de 4 à 14 atomes de carbone et de 1 à 5 atomes d'oxygène.

Dans un mode de réalisation, la composition est caractérisée en ce que le radical hydrophobe de formules X, Xa, Xb, Xb', Xc, Xe, Xf, Xg, Xh et Xi est un radical dans lequel R est un radical éther.

Dans un mode de réalisation, la composition est caractérisée en ce que le radical hydrophobe de formules X, Xa, Xb, Xb', Xc, Xe, Xf, Xg, Xh et Xi est un radical dans lequel R est un radical éther comprenant de 4 à 6 atomes de carbone.

Dans un mode de réalisation, la composition est caractérisée en ce que le radical hydrophobe de formules X, Xa, Xb, Xb', Xc, Xe, Xf, Xg, Xh et Xi est un radical dans lequel R est un radical alkyle divalent comprenant 6 atomes de carbone.

Dans un mode de réalisation, la composition est caractérisée en ce que le radical hydrophobe de formules X, Xa, Xb, Xb', Xc, Xe, Xf, Xg, Xh et Xi est un radical dans lequel R est un radical éther représenté par la formule

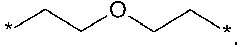

Dans un mode de réalisation, la composition est caractérisée en ce que le radical hydrophobe de formules X, Xa, Xb, Xb', Xc, Xe, Xf, Xg, Xh et Xi est un radical dans lequel R est un radical polyéther.

Dans un mode de réalisation, la composition est caractérisée en ce que le radical hydrophobe de formules X, Xa, Xb, Xb', Xc, Xe, Xf, Xg, Xh et Xi est un radical dans lequel R est un radical linéaire polyéther comprenant de 6 à 10 atomes de carbone et de 2 à 3 atomes d'oxygène.

Dans un mode de réalisation, la composition est caractérisée en ce que le radical hydrophobe de formules X, Xa, Xb, Xb', Xc, Xe, Xf, Xg, Xh et Xi est un radical dans lequel R est un radical polyéther choisi dans le groupe constitué par les radicaux représentés par les formules ci-dessous :

| | |
|---|---|
| 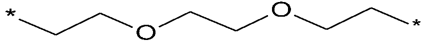 | Formule X3 |
| 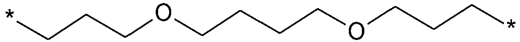 | Formule X4 |
| 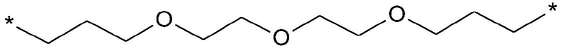 | Formule X5 |
| 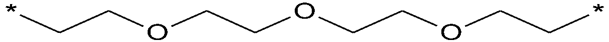 | Formule X6 |

Dans un mode de réalisation, la composition est caractérisée en ce que le radical hydrophobe de formules X, Xa, Xb, Xb', Xc, Xe, Xf, Xg, Xh et Xi est un radical dans lequel R est un radical de formule X3.

Dans un mode de réalisation, la composition est caractérisée en ce que le radical hydrophobe de formules X, Xa, Xb, Xb', Xc, Xe, Xf, Xg, Xh et Xi est un radical dans lequel R est un radical de formule X4.

Dans un mode de réalisation, la composition est caractérisée en ce que le radical hydrophobe de formules X, Xa, Xb, Xb', Xc, Xe, Xf, Xg, Xh et Xi est un radical dans lequel R est un radical polyéther choisi dans le groupe constitué par les radicaux représentés par les formules X5 et X6 ci-dessous :

Dans un mode de réalisation, la composition est caractérisée en ce que le radical hydrophobe de formules X, Xa, Xb, Xb', Xc, Xe, Xf, Xg, Xh et Xi est un radical dans lequel R est un radical polyéther de formule X5.

Dans un mode de réalisation, la composition est caractérisée en ce que le radical hydrophobe de formules X, Xa, Xb, Xb', Xc, Xe, Xf, Xg, Xh et Xi est un radical dans lequel R est un radical polyéther de formule X6.

| | |
|---|---|
| 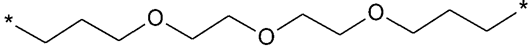 | Formule X5 |
| 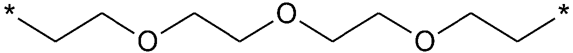 | Formule X6 |

Dans un mode de réalisation, la composition est caractérisée en ce que le radical hydrophobe de formules X, Xa, Xb, Xb', Xc, Xe, Xf, Xg, Xh et Xi est un radical dans lequel le radical GpG et/ou GpH est de formule XI' dans lequel G est un radical alkyle comprenant 6 atomes de carbone représenté par la formule Z ci-dessous :

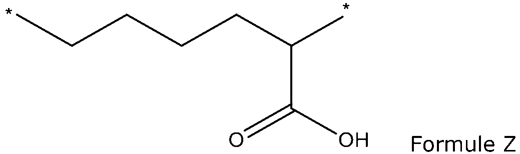

Dans un mode de réalisation, la composition est caractérisée en ce que le radical hydrophobe de formules X, Xa, Xb, Xb', Xc, Xe, Xf, Xg, Xh et Xi est un radical dans lequel le radical GpG et/ou GpH est de formule XI dans lequel G est un radical alkyle comprenant 4 atomes de carbone représenté par la formule Z ci-dessous :

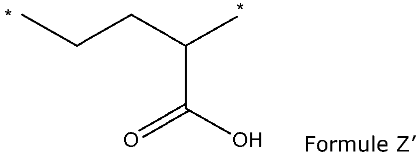

Dans un mode de réalisation, la composition est caractérisée en ce que le radical hydrophobe de formules X, Xa, Xb, Xb', Xc, Xe, Xf, Xg, Xh et Xi est un radical dans lequel le radical GpG et/ou GpH est de formule XI dans lequel G est un radical alkyle comprenant 4 atomes de carbone représenté par -(CH₂)₂-CH(COOH)-.

Dans un mode de réalisation, la composition est caractérisée en ce que le radical hydrophobe de formules X, Xa, Xb, Xb', Xc, Xe, Xf, Xg, Xh et Xi est un radical dans lequel le radical GpG et/ou GpH est de formule XI dans lequel G est un radical alkyle comprenant 4 atomes de carbone représenté par -CH((CH₂)₂COOH)-.

Dans un mode de réalisation, la composition est caractérisée en ce que le radical hydrophobe de formules X, Xa, Xb, Xb', Xc, Xe, Xf, Xg, Xh et Xi est un radical dans lequel le radical GpG et/ou GpH est de formule XI dans lequel G est un radical alkyle comprenant 3 atomes de carbone représenté par -CH₂-CH-(COOH).

Dans un mode de réalisation, la composition est caractérisée en ce que le radical hydrophobe de formules X, Xa, Xb, Xb', Xc, Xe, Xf, Xg, Xh et Xi est un radical dans lequel le radical GpG et/ou GpH est de formule XI dans lequel G est un radical alkyle comprenant 3 atomes de carbone représenté par -CH(CH₂)COOH)-.

Formules X, Xa, Xb... et GPADans un mode de réalisation, la composition est caractérisée en ce que le radical hydrophobe de formules X, Xa, Xb, Xb', Xc, Xe, Xf, Xg, Xh et Xi est un radical dans lequel le radical GpA est de formule VIII et dans laquelle A₁, A₂ ou A₃ est choisi dans le groupe constitué des radicaux représentés par les formules ci-dessous :

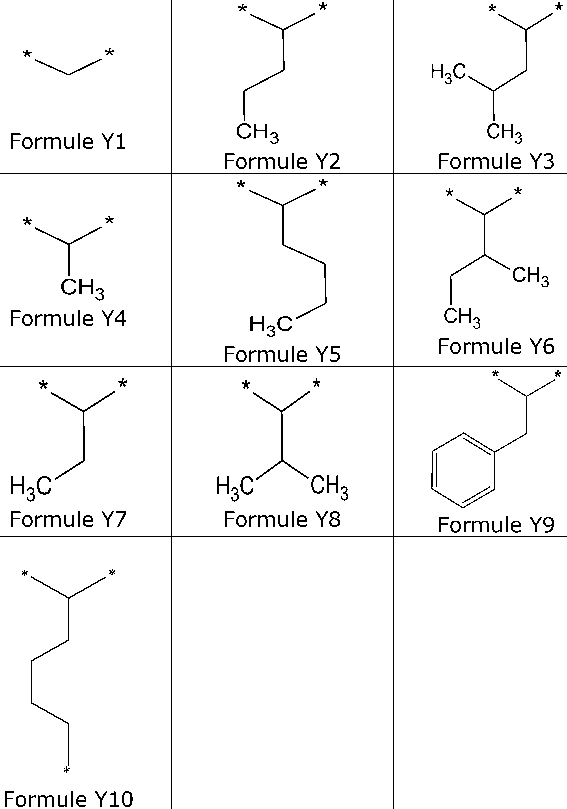

Dans un mode de réalisation, la composition est caractérisée en ce que le radical hydrophobe de formules X, Xa, Xb, Xb', Xc, Xd, Xe, Xf, Xg, Xh et Xi est un radical dans lequel le radical GpC de formule IX est choisi dans le groupe constitué des radicaux de formules IXe, IXf ou IXg ci-après représentées :

| | |
|---|---|
| 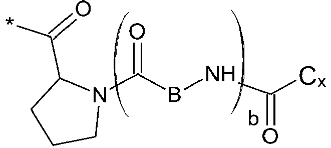 | Formule IXe |
| 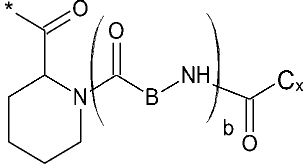 | Formule IXf |
| 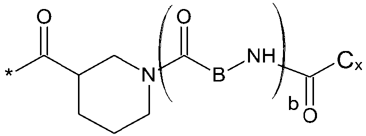 | Formule IXg |

Dans un mode de réalisation, la composition est caractérisée en ce que le radical hydrophobe de formules X, Xa, Xb, Xb', Xd, Xc, Xd, Xe, Xf, Xg, Xh et Xi est un radical dans lequel le radical GpC de formule IX est choisi dans le groupe constitué des radicaux de formules IXe, IXf ou IXg dans lesquels b est égal à 0, répondant respectivement aux formules IXh, IXi, et IXj ci-après représentées :

| | |
|---|---|
| 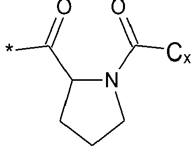 | Formule IXh |
| 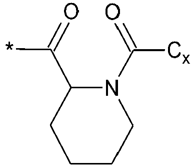 | Formule IXi |
| 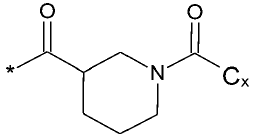 | Formule IXj |

Dans un mode de réalisation, la composition est caractérisée en ce que le radical hydrophobe de formule X, Xa, Xb, Xb', Xc, Xd, Xe, Xf, Xg, Xh et Xi est un radical dans lequel le radical GpC répond à la formule IX ou IXe dans lesquelles b = 0, et répond à la formule IXh.

Dans un mode de réalisation, la composition est caractérisée en ce que le radical hydrophobe de formule X, Xa, Xb, Xb', Xc, Xd, Xe, Xf, Xg, Xh et Xi est un radical dans lequel Cx est choisi dans le groupe constitué par les radicaux alkyles linéaires.

Dans un mode de réalisation, la composition est caractérisée en ce que le radical hydrophobe de formule X, Xa, Xb, Xb', Xc, Xd, Xe, Xf, Xg, Xh et Xi est un radical dans lequel Cx est choisi dans le groupe constitué par les radicaux alkyles ramifiés.

Dans un mode de réalisation, la composition est caractérisée en ce que le radical hydrophobe de formule X, Xa, Xb, Xb', Xc, Xd, Xe, Xf, Xg, Xh et Xi est un radical dans lequel Cx est choisi dans le groupe constitué par les radicaux alkyles comprenant entre 19 et 14 atomes de carbone.

Dans un mode de réalisation, la composition est caractérisée en ce que le radical hydrophobe de formule X, Xa, Xb, Xb', Xc, Xd, Xe, Xf, Xg, Xh et Xi est un radical dans lequel Cx est choisi dans le groupe constitué par les radicaux représentés par les formules ci-dessous :

| | |
|---|---|
| 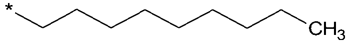 | x = 9 |
| 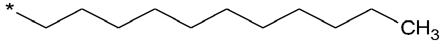 | x = 11 |
| 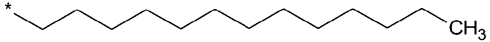 | x = 13 |

Dans un mode de réalisation, la composition est caractérisée en ce que le radical hydrophobe de formule X, Xa, Xb, Xb', Xc, Xd, Xe, Xf, Xg, Xh et Xi est un radical dans lequel Cx est choisi dans le groupe constitué par les radicaux alkyles comprenant entre 15 et 16 atomes de carbone.

Dans un mode de réalisation, la composition est caractérisée en ce que le radical hydrophobe de formule X, Xa, Xb, Xb', Xc, Xd, Xe, Xf, Xg, Xh et Xi est un radical dans lequel Cx est choisi dans le groupe constitué par les radicaux représentés par les formules ci-dessous :

| | |
|---|---|
| 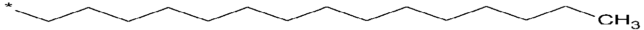 | x = 15 |

Dans un mode de réalisation, la composition est caractérisée en ce que le radical hydrophobe de formule X, Xa, Xb, Xb', Xc, Xd, Xe, Xf, Xg, Xh et Xi est un radical dans lequel Cx est choisi dans le groupe constitué par les radicaux représentés par les formules ci-dessous :

| | |
|---|---|
| 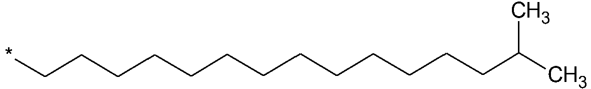 | x = 16 |

Dans un mode de réalisation, la composition est caractérisée en ce que le radical hydrophobe de formule X, Xa, Xb, Xb', Xc, Xd, Xe, Xf, Xg, Xh et Xi est un radical dans lequel Cx est choisi dans le groupe constitué par les radicaux alkyles comprenant entre 17 et 25 atomes de carbone.

Dans un mode de réalisation, la composition est caractérisée en ce que le radical hydrophobe de formule X, Xa, Xb, Xb', Xc, Xd, Xe, Xf, Xg, Xh et Xi est un radical dans lequel Cx est choisi dans le groupe constitué par les radicaux alkyles comprenant entre 17 et 18 atomes de carbone.

Dans un mode de réalisation, la composition est caractérisée en ce que le radical hydrophobe de formule X, Xa, Xb, Xb', Xc, Xd, Xe, Xf, Xg, Xh et Xi est un radical dans lequel Cx est choisi dans le groupe constitué par les radicaux alkyles représentés par les formules ci-dessous :

| | |
|---|---|
| 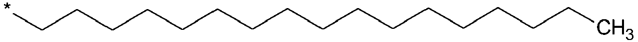 | x = 17 |

Dans un mode de réalisation, la composition est caractérisée en ce que le radical hydrophobe de formule X, Xa, Xb, Xb', Xc, Xd, Xe, Xf, Xg, Xh et Xi est un radical dans lequel Cx est choisi dans le groupe constitué par les radicaux alkyles comprenant entre 18 et 25 atomes de carbone.

Dans un mode de réalisation, la composition est caractérisée en ce que le radical hydrophobe de formule X, Xa, Xb, Xb', Xc, Xd, Xe, Xf, Xg, Xh et Xi est un radical dans lequel Cx est choisi dans le groupe constitué par les radicaux alkyles représentés par les formules ci-dessous :

| | |
|---|---|
| 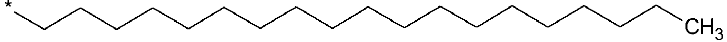 | x = 19 |
| 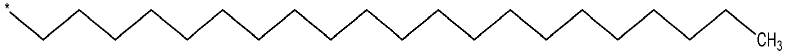 | x = 21 |

Dans un mode de réalisation, la composition est caractérisée en ce que le radical hydrophobe est un radical de formule X, Xa, Xb, Xb', Xc, Xd, Xe, Xf, Xg, Xh et Xi dans laquelle le radical GpC de formule IX est choisi dans le groupe constitué des radicaux dans lesquels Cx est choisi dans le groupe constitué par les radicaux alkyles comprenant 14 ou 15 atomes de carbone.

Dans un mode de réalisation, la composition est caractérisée en ce que le radical hydrophobe est un radical de formule X, Xa, Xb, Xb', Xc, Xd, Xe, Xf, Xg, Xh et Xi dans laquelle le radical GpC de formule IX est choisi dans le groupe constitué des radicaux dans lesquels Cx est choisi dans le groupe constitué par les radicaux représentés par les formules ci-dessous :

| | |
|---|---|
| 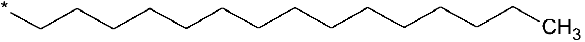 | x=15 |

Dans un mode de réalisation, lorsque a' = 1, x est compris entre 11 et 25 (11 ≤ x ≤ 25). En particulier, lorsque x est compris entre 15 et 16 (x = 15 ou 16) alors r = 1 et R est un radical éther ou polyéther et lorsque x est supérieur à 17 (x ≥ 17) alors r = 1 et R est un radical éther ou polyéther.

Dans un mode de réalisation, lorsque a' = 2, x est compris entre 9 et 15 (9 ≤ x ≤ 15).

Dans un mode de réalisation, le copolyaminoacide est choisi parmi les copolyaminoacides de formule XXXb dans laquelle le radical hydrophobe -Hy est choisi dans le groupe des radicaux hydrophobes de formule X, Xc', Xa, Xb', Xc, Xe, Xg et Xh dans laquelle a' = 1 et l'= 1 et GpC est un radical de formule IXe.

Dans un mode de réalisation, le copolyaminoacide est choisi parmi les copolyaminoacides de formule XXXb dans laquelle le radical hydrophobe -Hy est choisi dans le groupe des radicaux hydrophobes de formule X, Xc', Xa, Xb', Xc, Xe, Xg et Xh dans laquelle a' = 1 et l'= 1 et GpC est un radical de formule IX dans lequel e=0.

Dans un mode de réalisation, le copolyaminoacide est choisi parmi les copolyaminoacides de formule XXXb dans laquelle le radical hydrophobe -Hy est choisi dans le groupe des radicaux hydrophobes de formule X, Xc', Xa, Xb, Xc, Xf, Xg et Xi dans laquelle a' = 2 ou l'= 2 et GpC est un radical de formule IXe.

Dans un mode de réalisation, le copolyaminoacide est choisi parmi les copolyaminoacides de formule XXXb dans laquelle le radical hydrophobe -Hy est choisi dans le groupe des radicaux hydrophobes de formule X, Xc', Xa, Xb, Xc, Xf, Xg et Xi dans laquelle a' = 2 et l'= 2 et GpC est un radical de formule IX dans lequel e=0.

Dans un mode de réalisation, le copolyaminoacide est choisi parmi les copolyaminoacides de formule XXXa dans laquelle le radical hydrophobe -Hy est choisi dans le groupe des radicaux hydrophobes de formule X, Xc', Xa, Xb', Xc, Xe, Xg et Xh dans laquelle a' = 1 et l'= 1 et GpC est un radical de formule IXe.

Dans un mode de réalisation, le copolyaminoacide est choisi parmi les copolyaminoacides de formule XXXa dans laquelle le radical hydrophobe -Hy est choisi dans le groupe des radicaux hydrophobes de formule X, Xc', Xa, Xb, Xc, Xf, Xg et Xi dans laquelle a' = 2 ou l'= 2 et GpC est un radical de formule IXe.

Dans un mode de réalisation, le radical hydrophobe Hy est choisi dans le groupe des radicaux hydrophobes de formule X, dans laquelle h est supérieur ou égal à 2 et GpC est de formule Ixe.

Dans un mode de réalisation, le radical hydrophobe Hy est choisi dans le groupe des radicaux hydrophobes de formule X, dans laquelle g est supérieur ou égal à 2 et a, l et h sont égaux à 0 et GpC est de formule Ixe.

Dans un mode de réalisation, la composition est caractérisée en ce que le radical hydrophobe est choisi parmi les radicaux hydrophobes de formule X, Xc', Xa, Xb, Xb', Xc, Xe, Xg et Xh dans lequel a'=1 et l'=1 et dans lequel Cx est choisi dans le groupe constitué par les radicaux alkyles linéaires.

Dans un mode de réalisation, la composition est caractérisée en ce que le radical hydrophobe est choisi parmi les radicaux hydrophobes de formule X, Xc', Xa, Xb, Xb', Xc, Xf, Xg et Xi dans lequel a'=2 ou l'=2 et dans lequel Cx est choisi dans le groupe constitué par les radicaux alkyles linéaires.

Dans un mode de réalisation, le radical hydrophobe -Hy est choisi dans le groupe des radicaux hydrophobes de formule X, dans laquelle GpR est un radical de formule VII, GpH est un radical de formule XI et GpC est un radical de formule IX dans lequel e=1, b=0 et x=13.

Dans un mode de réalisation, le copolyaminoacide est un poly-L-glutamate de sodium modifié à une de ses extrémités de formule ci-après représentée décrit à l'exemple B1.

| | |
|---|---|
| B1 | 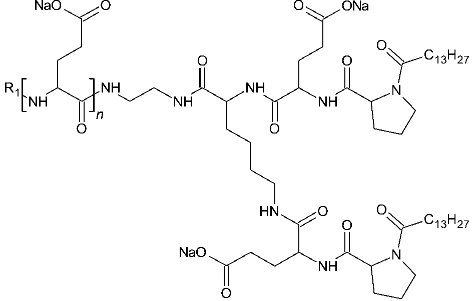 |
| | i = 0,038, DP = 26 |
| | R₁ = H ou pyroglutamate |

Dans un mode de réalisation, le copolyaminoacide est un poly-L-glutamate de sodium modifié à une de ses extrémités de formule ci-après représentée décrit à l'exemple B18.

| | |
|---|---|
| B18 | 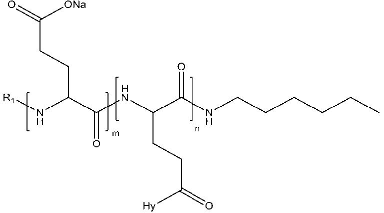 |
| | i= 0,15, DP (m + n) = 40 |
| | Hy = |
| | 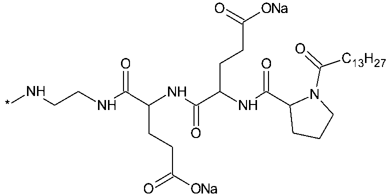 |
| | R₁ = H ou pyroglutamate |

Dans un mode de réalisation, la composition selon l'invention est caractérisée en ce que le ratio M entre le nombre de radicaux hydrophobes et le nombre d'unités glutamiques ou aspartiques est compris entre 0,007 et 0,3.

Dans un mode de réalisation, la composition selon l'invention est caractérisée en ce que le ratio M entre le nombre de radicaux hydrophobes et le nombre d'unités glutamiques ou aspartiques est compris entre 0,01 et 0,3.

Dans un mode de réalisation, la composition selon l'invention est caractérisée en ce que le ratio M entre le nombre de radicaux hydrophobes et le nombre d'unités glutamiques ou aspartiques est compris entre 0,02 et 0,2.

Dans un mode de réalisation, la composition selon l'invention est caractérisée en ce que le radical hydrophobe répond à la formule X et le ratio M entre le nombre de radicaux hydrophobes et le nombre d'unités glutamiques ou aspartiques est compris entre 0,007 et 0,15.

Dans un mode de réalisation, la composition selon l'invention est caractérisée en ce que le radical hydrophobe répond à la formule X et le ratio M entre le nombre de radicaux hydrophobes et le nombre d'unités glutamiques ou aspartiques est compris entre 0,01 et 0,1.

Dans un mode de réalisation, la composition selon l'invention est caractérisée en ce que le radical hydrophobe répond à la formule X et le ratio M entre le nombre de radicaux hydrophobes et le nombre d'unités glutamiques ou aspartiques est compris entre 0,02 et 0,08.

Dans un mode de réalisation, la composition selon l'invention est caractérisée en ce que le radical hydrophobe répond à la formule X dans laquelle le radical Cx comprend entre 9 et 10 atomes de carbone et le ratio M entre le nombre de radicaux hydrophobes et le nombre d'unités glutamiques ou aspartiques est compris entre 0,03 et 0,15.

Dans un mode de réalisation, la composition selon l'invention est caractérisée en ce que le radical hydrophobe répond à la formule X dans laquelle le radical Cx comprend entre 11 et 12 atomes de carbone et le ratio M entre le nombre de radicaux hydrophobes et le nombre d'unités glutamiques ou aspartiques est compris entre 0,015 et 0,1.

Dans un mode de réalisation, la composition selon l'invention est caractérisée en ce que le radical hydrophobe répond à la formule X dans laquelle le radical Cx comprend entre 11 et 12 atomes de carbone et le ratio M entre le nombre de radicaux hydrophobes et le nombre d'unités glutamiques ou aspartiques est compris entre 0,02 et 0,08.

Dans un mode de réalisation, la composition selon l'invention est caractérisée en ce que le radical hydrophobe répond à la formule X dans laquelle le radical Cx comprend entre 13 et 15 atomes de carbone et le ratio M entre le nombre de radicaux hydrophobes et le nombre d'unités glutamiques ou aspartiques est compris entre 0,01 et 0,1.

Dans un mode de réalisation, la composition selon l'invention est caractérisée en ce que le radical hydrophobe répond à la formule X dans laquelle le radical Cx comprend entre 13 et 15 atomes de carbone et le ratio M entre le nombre de radicaux hydrophobes et le nombre d'unités glutamiques ou aspartiques est compris entre 0,01 et 0,06.

Dans un mode de réalisation, la composition selon l'invention est caractérisée en ce que le radical hydrophobe répond à la formule X et le ratio M entre le nombre de radicaux hydrophobes et le nombre d'unités glutamiques ou aspartiques est compris entre 0,007 et 0,3.

Dans un mode de réalisation, la composition selon l'invention est caractérisée en ce que le radical hydrophobe répond à la formule X et le ratio M entre le nombre de radicaux hydrophobes et le nombre d'unités glutamiques ou aspartiques est compris entre 0,01 et 0,3.

Dans un mode de réalisation, la composition selon l'invention est caractérisée en ce que le radical hydrophobe répond à la formule X et le ratio M entre le nombre de radicaux hydrophobes et le nombre d'unités glutamiques ou aspartiques est compris entre 0,015 et 0,2.

Dans un mode de réalisation, la composition selon l'invention est caractérisée en ce que le radical hydrophobe répond à la formule X dans laquelle le radical Cx comprend entre 11 et 14 atomes de carbone et le ratio M entre le nombre de radicaux hydrophobes et le nombre d'unités glutamiques ou aspartiques est compris entre 0,1 et 0,2.

Dans un mode de réalisation, la composition selon l'invention est caractérisée en ce que le radical hydrophobe répond à la formule X dans laquelle le radical Cx comprend entre 15 et 16 atomes de carbone et le ratio M entre le nombre de radicaux hydrophobes et le nombre d'unités glutamiques ou aspartiques est compris entre 0,04 et 0,15.

Dans un mode de réalisation, la composition selon l'invention est caractérisée en ce que le radical hydrophobe répond à la formule X dans laquelle le radical Cx comprend entre 17 et 18 atomes de carbone et le ratio M entre le nombre de radicaux hydrophobes et le nombre d'unités glutamiques ou aspartiques est compris entre 0,02 et 0,06.

Dans un mode de réalisation, la composition selon l'invention est caractérisée en ce que le radical hydrophobe répond à la formule X dans laquelle le radical Cx comprend entre 19 et 25 atomes de carbone et le ratio M entre le nombre de radicaux hydrophobes et le nombre d'unités glutamiques ou aspartiques est compris entre 0,01 et 0,06.

Dans un mode de réalisation, la composition selon l'invention est caractérisée en ce que le radical hydrophobe répond à la formule X dans laquelle le radical Cx comprend entre 19 et 25 atomes de carbone et le ratio M entre le nombre de radicaux hydrophobes et le nombre d'unités glutamiques ou aspartiques est compris entre 0,01 et 0,05.

Dans un mode de réalisation, la composition selon l'invention est caractérisée en ce que le co-polyaminoacide porteur de charges carboxylates et de radicaux hydrophobes est choisi parmi les co-polyaminoacides de formule XXXa' suivante : 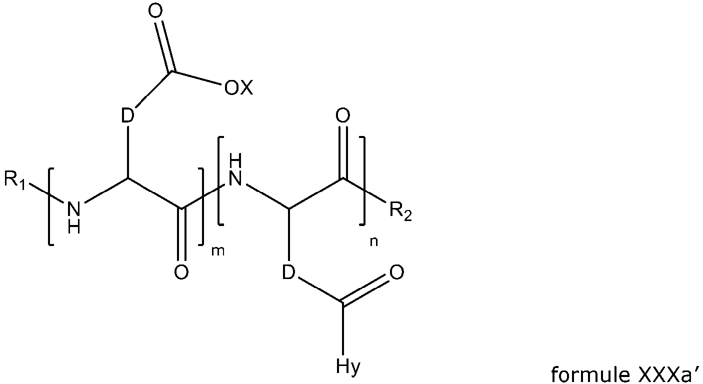 dans laquelle,
- D représente, indépendamment, soit un groupe -CH₂- (unité aspartique) soit un groupe -CH₂-CH₂- (unité glutamique),
- Hy est un radical hydrophobe choisi parmi les radicaux hydrophobes de formules X, dans lesquelles r = 1 et GpR est un radical de Formule VII,
- R₁ est un radical hydrophobe choisi parmi les radicaux hydrophobes de formules X dans lesquelles r = 0 ou r = 1 et GpR est un radical de Formule VII', ou un radical choisi dans le groupe constitué par un H, un groupe acyle linéaire en C2 à C10, un groupe acyle ramifié en C4 à C10, un benzyle, une unité « acide aminé » terminale et un pyroglutamate,
- R₂ est un radical hydrophobe choisi parmi les radicaux hydrophobes de formules X dans lesquelles r = 1 et GpR est un radical de Formule VII, ou un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et S ;
- X représente une entité cationique choisie dans le groupe comprenant les cations alcalins ;
- n + m représente le degré de polymérisation DP du co-polyaminoacide, c'est-à-dire le nombre moyen d'unités monomériques par chaîne de co-polyaminoacide et 5 ≤ n + m ≤ 250.

Lorsque le co-polyaminoacide comprend une ou plusieurs d'unité(s) aspartique(s), celle(s)-ci peu(ven)t subir des réarrangements structuraux.

Dans un mode de réalisation, la composition selon l'invention est caractérisée en ce que lorsque le co-polyaminoacide comprend des unités aspartate, alors le co-polyaminoacide peut en outre comprendre des unités monomériques de formule XXXI et/ou XXXI' :

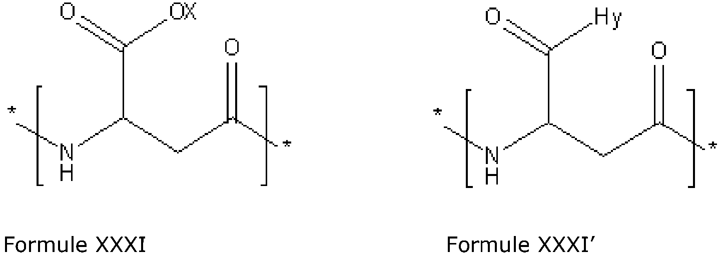

On appelle « co-polyaminoacide à greffage statistique » un co-polyaminoacide porteur de charges carboxylates et d'au moins un radical hydrophobe, un co-polyaminoacide de formule XXXa.

Dans un mode de réalisation, la composition selon l'invention est caractérisée en ce que le co-polyaminoacide porteur de charges carboxylates et de radicaux hydrophobes est choisi parmi les co-polyaminoacides de formules XXXa', dans laquelle R₁ = R'₁ et R₂ = R'₂, de formule XXXa suivante : 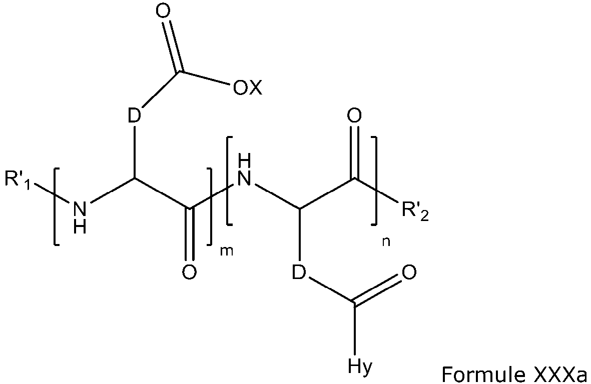 dans laquelle,
- m, n, X, D et Hy ont les définitions données précédemment,
- R'₁ est un radical choisi dans le groupe constitué par un H, un groupe acyle linéaire en C2 à C10, un groupe acyle ramifié en C4 à C10, un benzyle, une unité « acide aminé » terminale et un pyroglutamate,
   - R'₂ est un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et S.

Dans un mode de réalisation, la composition selon l'invention est caractérisée en ce que le co-polyaminoacide porteur de charges carboxylates et de radicaux hydrophobes est choisi parmi les co-polyaminoacides de formules XXXa, dans laquelle Hy est un radical de formule X.

Dans un mode de réalisation, la composition selon l'invention est caractérisée en ce que le co-polyaminoacide porteur de charges carboxylates et de radicaux hydrophobes est choisi parmi les co-polyaminoacides de formule XXXa, dans laquelle Hy est un radical de formule X, dans laquelle r=1.

Dans un mode de réalisation, la composition selon l'invention est caractérisée en ce que le co-polyaminoacide porteur de charges carboxylates et de radicaux hydrophobes est choisi parmi les co-polyaminoacides de formules XXXa, dans laquelle Hy est un radical de formule X, dans laquelle r=1, et pour GpC, b=0.

Dans un mode de réalisation, la composition selon l'invention est caractérisée en ce que le co-polyaminoacide porteur de charges carboxylates et de radicaux hydrophobes est choisi parmi les co-polyaminoacides de formule XXXa, dans laquelle Hy est un radical de formule X et dans laquelle GpC est un radical de formule IX.

Dans un mode de réalisation, la composition selon l'invention est caractérisée en ce que le co-polyaminoacide porteur de charges carboxylates et de radicaux hydrophobes est choisi parmi les co-polyaminoacides de formule XXXa, dans laquelle Hy est un radical de formule X et dans laquelle GpC est un radical de formule IX et r=1.

On appelle « co-polyaminoacide à greffage défini » un co-polyaminoacide porteur de charges carboxylates et d'au moins un radical hydrophobe, un co-polyaminoacide de formule XXXb.

Dans un mode de réalisation, la composition selon l'invention est caractérisée en ce que le co-polyaminoacide porteur de charges carboxylates et de radicaux hydrophobes est choisi parmi les co-polyaminoacides de formule XXXa' dans laquelle n = 0 de formule XXXb suivante : 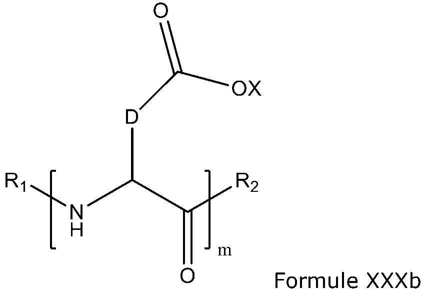 dans laquelle m, X, D, R₁ et R₂ ont les définitions données précédemment et au moins R₁ ou R₂ est un radical hydrophobe de formule X.

Dans un mode de réalisation, la composition selon l'invention est caractérisée en ce que le co-polyaminoacide porteur de charges carboxylates et de radicaux hydrophobes est choisi parmi les co-polyaminoacides de formule XXXa' dans laquelle n = 0 de formule XXXb et R₁ ou R₂ est un radical hydrophobe de formule X.

Dans un mode de réalisation, la composition selon l'invention est caractérisée en ce que le co-polyaminoacide porteur de charges carboxylates et de radicaux hydrophobes est choisi parmi les co-polyaminoacides de formules XXXb, dans laquelle R1 = R'1, de formule XXXb' : 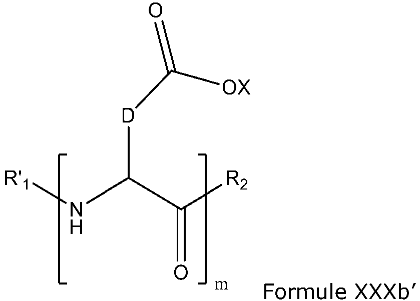 dans laquelle m, X, D, R'₁ et R₂ ont les définitions données précédemment et R₂ est un radical hydrophobe de formule X.

Dans un mode de réalisation, la composition selon l'invention est caractérisée en ce que le co-polyaminoacide porteur de charges carboxylates et de radicaux hydrophobes est choisi parmi les co-polyaminoacides de formules XXXb, dans laquelle R2 = R'2, de formule XXXb" : 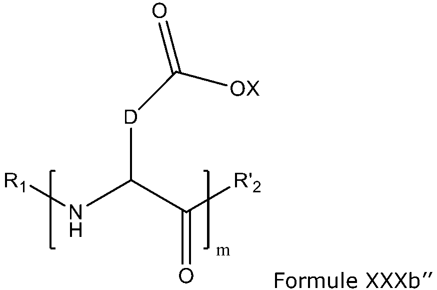 dans laquelle m, X, D, R₁ et R'₂ ont les définitions données précédemment et R₁ est un radical hydrophobe de formule X.

Dans un mode de réalisation, la composition selon l'invention est caractérisée en ce que le co-polyaminoacide porteur de charges carboxylates et de radicaux hydrophobes est choisi parmi les co-polyaminoacides de formule XXXb ou XXXb" dans laquelle R₁ est un radical hydrophobe de formule X et GpR est de formule VII'.

Dans un mode de réalisation, la composition selon l'invention est caractérisée en ce que le co-polyaminoacide porteur de charges carboxylates et de radicaux hydrophobes est choisi parmi les co-polyaminoacides de formule XXXb ou XXXb" dans laquelle R₁ est un radical hydrophobe de formule X et GpR est de formule VII".

Dans un mode de réalisation, la composition selon l'invention est caractérisée en ce que le co-polyaminoacide porteur de charges carboxylates et de radicaux hydrophobes est choisi parmi les co-polyaminoacides de formule XXXb ou XXXb" dans laquelle R₁ est un radical hydrophobe de formule X et GpR est de formule VII' et GpC est de formule IX.

Dans un mode de réalisation, la composition selon l'invention est caractérisée en ce que le co-polyaminoacide porteur de charges carboxylates et de radicaux hydrophobes est choisi parmi les co-polyaminoacides de formule XXXb ou XXXb" dans laquelle R₁ est un radical hydrophobe de formule X et GpR est de formule VII' et GpC est de formule IX.

Dans un mode de réalisation, la composition selon l'invention est caractérisée en ce que le co-polyaminoacide porteur de charges carboxylates et de radicaux hydrophobes est choisi parmi les co-polyaminoacides de formules XXXb ou XXXb' dans laquelle R₂ est un radical hydrophobe de formule X dans lesquelles r = 1 et GpR est de Formule VII.

Dans un mode de réalisation, la composition est caractérisée en ce que le co-polyaminoacide porteur de charges carboxylates et de radicaux hydrophobes est choisi parmi les co-polyaminoacides de formule XXXa', dans laquelle au moins un des R₁ ou R₂ est un radical hydrophobe tel que ci-dessus défini, de formule XXX suivante : 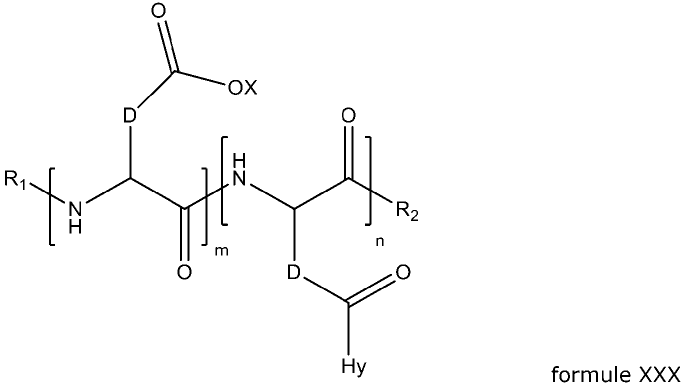 dans laquelle,
- D représente, indépendamment, soit un groupe -CH₂- (unité aspartique) soit un groupe -CH₂-CH₂- (unité glutamique),
- Hy est un radical hydrophobe choisi parmi les radicaux hydrophobes de formules X, dans lesquelles r = 1 et GpR est un radical de Formule VII,
- R₁ est un radical hydrophobe choisi parmi les radicaux hydrophobes de formules X dans lesquelles r=0 ou r=1 et GpR est un radical de Formule VII', ou un radical choisi dans le groupe constitué par un H, un groupe acyle linéaire en C2 à C10, un groupe acyle ramifié en C4 à C10, un benzyle, une unité « acide aminé » terminale et un pyroglutamate,
- R₂ est un radical hydrophobe choisi parmi les radicaux hydrophobes de formules X dans lesquelles r = 1 et GpR est un radical de Formule VII, ou un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et S,
- au moins un des R₁ ou R₂ est un radical hydrophobe tel que ci-dessus défini,
- X représente un H ou une entité cationique choisie dans le groupe comprenant les cations métalliques ;
- n + m représente le degré de polymérisation DP du co-polyaminoacide, c'est-à-dire le nombre moyen d'unités monomériques par chaîne de co-polyaminoacide et 5 ≤ n + m ≤ 250.

Dans un mode de réalisation, la composition selon l'invention est caractérisée en ce que le co-polyaminoacide porteur de charges carboxylates et de radicaux hydrophobes est choisi parmi les co-polyaminoacides de formule XXX, XXXa, XXXa', XXXb, XXXb' ou XXXb" dans lesquels le groupe D est un groupe -CH₂- (unité aspartique).

Dans un mode de réalisation, la composition selon l'invention est caractérisée en ce que le co-polyaminoacide porteur de charges carboxylates et de radicaux hydrophobes est choisi parmi les co-polyaminoacides de formule XXX, XXXa, XXXa', XXXb, XXXb' ou XXXb" dans lesquels le groupe D est un groupe -CH₂-CH₂-(unité glutamique).

Dans un mode de réalisation, la composition selon l'invention est caractérisée en ce que R₁ est un radical choisi dans le groupe constitué par un groupe acyle linéaire en C₂ à C₁₀, un groupe acyle ramifié en C₄ à C₁₀, un benzyle, une unité « acide aminé » terminale et un pyroglutamate.

Dans un mode de réalisation, la composition selon l'invention est caractérisée en ce que R₁ est un radical choisi dans le groupe constitué par un groupe acyle linéaire en C₂ à C₁₀ ou un groupe acyle ramifié en C₄ à C₁₀.

Dans un mode de réalisation, la composition selon l'invention est caractérisée en ce que le co-polyaminoacide porteur de charges carboxylates et de radicaux hydrophobes est choisi parmi les co-polyaminoacides de formules XXXa, XXXb, XXXb' ou XXXb" dans lesquels le co-polyaminoacide est choisi parmi les co-polyaminoacides dans lesquels le groupe D est un groupe -CH₂- (unité aspartique).

Dans un mode de réalisation, la composition selon l'invention est caractérisée en ce que le co-polyaminoacide porteur de charges carboxylates et de radicaux hydrophobes est choisi parmi les co-polyaminoacides de formules XXXa XXXb, XXXb' ou XXXb"dans lesquels le co-polyaminoacide est choisi parmi les co-polyaminoacides dans lesquels le groupe D est un groupe -CH₂-CH₂- (unité glutamique).

Dans un mode de réalisation, la composition selon l'invention est caractérisée en ce que n + m est compris entre 10 et 200.

Dans un mode de réalisation, la composition selon l'invention est caractérisée en ce que n + m est compris entre 15 et 150.

Dans un mode de réalisation, la composition selon l'invention est caractérisée en ce que n + m est compris entre 15 et 100.

Dans un mode de réalisation, la composition selon l'invention est caractérisée en ce que n + m est compris entre 15 et 80.

Dans un mode de réalisation, la composition selon l'invention est caractérisée en ce que n + m est compris entre 15 et 65.

Dans un mode de réalisation, la composition selon l'invention est caractérisée en ce que n + m est compris entre 20 et 60.

Dans un mode de réalisation, la composition selon l'invention est caractérisée en ce que n + m est compris entre 20 et 50.

Dans un mode de réalisation, la composition selon l'invention est caractérisée en ce que n + m est compris entre 20 et 40.

L'invention concerne également le co-polyaminoacide porteur de charges carboxylates et de radicaux hydrophobes Hy, ledit co-polyaminoacide étant constitué d'unités glutamiques ou aspartiques et lesdits radicaux hydrophobes Hy choisi parmi les radicaux de formule X telle que définie ci-dessous : 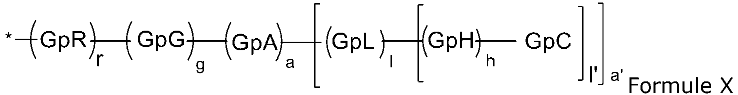 dans laquelle
GpR est choisi parmi les radicaux de formules VII, VII' ou VII" : 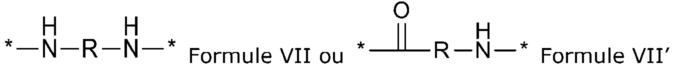 ou 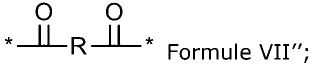
- GpG et GpH identiques ou différents sont choisis parmi les radicaux de formules XI ou XI': 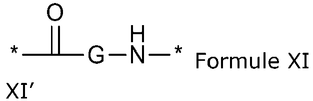 *

   -NH-G-NH-* Formule
- GpA est choisi parmi les radicaux de formule VIII 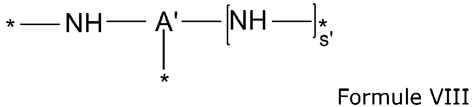 Dans laquelle A' est choisi parmi les radicaux de formule VIII',VIII" ou VIII‴ 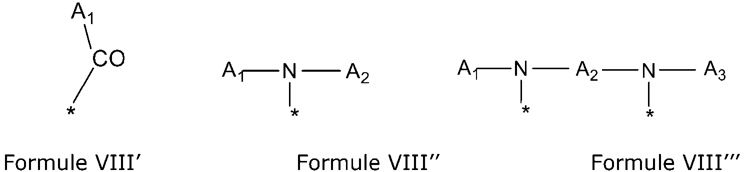
- -GpL est choisi parmi les radicaux de formule XII 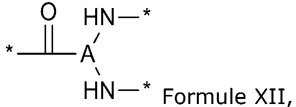
- GpC est un radical de formule IX : 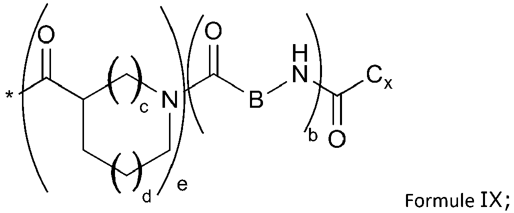
- les * indiquent les sites de rattachement des différents groupes liés par des fonctions amides ;
- a est un entier égal à 0 ou à 1 et a' = 1 si a = 0 et a' = 1, 2 ou 3 si a = 1 ;
- a' est un entier égal à 1, à 2 ou à 3 ;
- b est un entier égal à 0 ou à 1 ;
- c est un entier égal à 0 ou à 1, et si c est égal à 0 alors d est égal à 1 ou à 2;
- d est un entier égal à 0, à 1 ou à 2 ;
- e est un entier égal à 0 ou à 1 ;
- g est un entier égal à 0, à 1, à 2, à 3 à 4 à 5 ou à 6;
- h est un entier égal à 0, à 1, à 2, à 3 à 4 à 5 ou à 6, et au moins un des g, h ou l est différent de 0;
- l est un entier égal à 0 ou 1 et l' = 1 si l = 0 et l' = 2 si l = 1 ;
- r est un entier égal à 0, 1 ou à 2, et
- s'est un entier égal à 0 ou 1 ;
- et si e est différent de 0 alors au moins un des g, h ou l est différent de 0 ;
- et si a = 0 alors l = 0 ;
- A, A₁, A₂ et A₃ identiques ou différents sont des radicaux alkyles linéaires ou ramifiés comprenant de 1 à 8 atomes de carbone, et éventuellement substitués par un radical issu d'un cycle saturé, insaturé ou aromatique;
- B est un radical éther ou polyéther non substitué comprenant de 4 à 14 atomes de carbone et de 1 à 5 atomes d'oxygène ou un radical alkyle linéaire ou ramifié, éventuellement comprenant un noyau aromatique, comprenant de 1 à 9 atomes de carbone
- Cₓ est un radical alkyl monovalent linéaire ou ramifié éventuellement comprenant une partie cyclique, dans lequel x indique le nombre d'atomes de carbone et :
   ▪ Lorsque le radical hydrophobe -Hy porte 1 -GpC, alors 9 ≤ x ≤ 25,
   ▪ Lorsque le radical hydrophobe -Hy porte 2 -GpC, alors 9 ≤ x ≤ 15,
   ▪ Lorsque le radical hydrophobe -Hy porte 3 -GpC, alors 7 ≤ x ≤ 13,
   ▪ Lorsque le radical hydrophobe -Hy porte 4 -GpC, alors 7 ≤ x ≤ 11,
   ▪ Lorsque le radical hydrophobe -Hy porte au moins 5 -GpC alors, 6 ≤ x ≤ 11,
- G est un radical alkyle linéaire ou ramifié divalent de 1 à 8 atomes de carbone ledit radical alkyle portant une ou plusieurs fonction(s) acide carboxylique libre,
- R est un radical choisi dans le groupe constitué par un radical alkyle divalent, linéaire ou ramifié comprenant de 1 à 12 atomes de carbone, un radical alkyle divalent, linéaire ou ramifié comprenant de 1 à 12 atomes de carbone portant une ou plusieurs fonctions -CONH₂ ou un radical éther ou polyéther non substitué comprenant de 4 à 14 atomes de carbone et de 1 à 5 atomes d'oxygène,
- Le ou les radicaux hydrophobes -Hy de formule X étant liés au PLG:
   ∘ via une liaison covalente entre un carbonyle du radical hydrophobe -Hy et un atome d'azote porté par le PLG formant ainsi une fonction amide issue de la réaction d'une fonction amine portée par le PLG et une fonction acide portée par le précurseur -Hy' du radical hydrophobe -Hy, et
   ∘ via une liaison covalente entre un atome d'azote du radical hydrophobe -Hy et un carbonyle porté par le PLG formant ainsi une fonction amide issue de la réaction d'une fonction amine du précurseur -Hy' du radical hydrophobe -Hy et une fonction acide portée par le PLG,
- le ratio M entre le nombre de radicaux hydrophobes et le nombre d'unités glutamiques ou aspartiques étant compris entre 0 < M ≤ 0,5 ;
- lorsque plusieurs radicaux hydrophobes sont portés par un co-polyaminoacide alors ils sont identiques ou différents,
- le degré de polymérisation DP en unités glutamiques ou aspartiques pour les chaines PLG est compris entre 5 et 250 ;
- les fonctions acides carboxyliques libres étant sous forme de sel de cation alcalin choisi dans le groupe constitué par Na⁺ et K⁺.

L'invention concerne également le précurseur Hy' du radical hydrophobe -Hy de formule X' telle que définie ci-dessous : 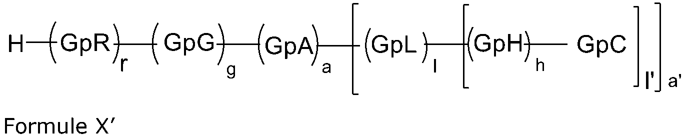 dans laquelle
GpR est choisi parmi les radicaux de formules VII, VII' ou VII" : 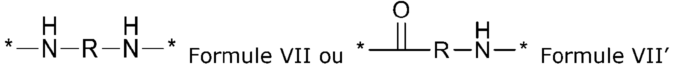 ou 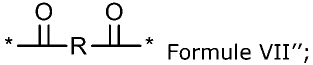
- GpG et GpH identiques ou différents sont choisis parmi les radicaux de formules XI ou XI': 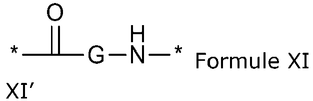 *

   -NH-G-NH-* Formule
- GpA est choisi parmi les radicaux de formule VIII 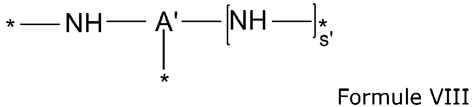 Dans laquelle A' est choisi parmi les radicaux de formule VIII',VIII" ou VIII"' 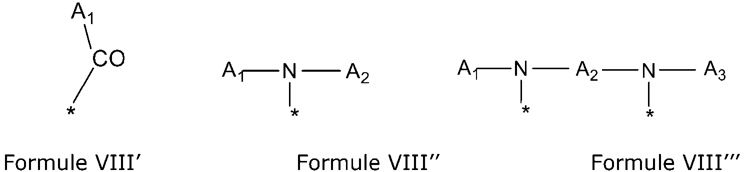
- -GpL est choisi parmi les radicaux de formule XII 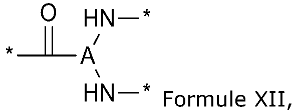
- GpC est un radical de formule IX : 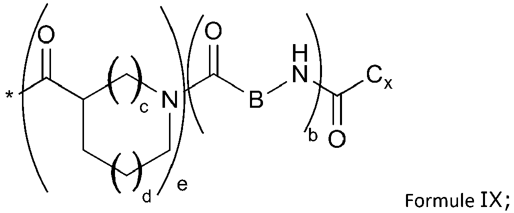
- les * indiquent les sites de rattachement des différents groupes liés par des fonctions amides ;
- a est un entier égal à 0 ou à 1 et a' = 1 si a = 0 et a' = 1, 2 ou 3 si a = 1 ;
- a' est un entier égal à 1, à 2 ou à 3 ;
- b est un entier égal à 0 ou à 1 ;
- c est un entier égal à 0 ou à 1, et si c est égal à 0 alors d est égal à 1 ou à 2;
- d est un entier égal à 0, à 1 ou à 2 ;
- e est un entier égal à 0 ou à 1 ;
- g est un entier égal à 0, à 1, à 2, à 3 à 4 à 5 ou à 6;
- h est un entier égal à 0, à 1, à 2, à 3 à 4 à 5 ou à 6, et au moins un des g, h ou l est différent de 0;
- l est un entier égal à 0 ou 1 et l' = 1 si l = 0 et l' = 2 si l = 1 ;
- r est un entier égal à 0, 1 ou à 2, et
- s'est un entier égal à 0 ou 1 ;
- et si e est différent de 0 alors au moins un des g, h ou l est différent de 0 ;
- et si a = 0 alors l = 0 ;
- A, A₁, A₂ et A₃ identiques ou différents sont des radicaux alkyles linéaires ou ramifiés comprenant de 1 à 8 atomes de carbone, et éventuellement substitués par un radical issu d'un cycle saturé, insaturé ou aromatique;
- B est un radical éther ou polyéther non substitué comprenant de 4 à 14 atomes de carbone et de 1 à 5 atomes d'oxygène ou un radical alkyle linéaire ou ramifié, éventuellement comprenant un noyau aromatique, comprenant de 1 à 9 atomes de carbone
- Cₓ est un radical alkyl monovalent linéaire ou ramifié éventuellement comprenant une partie cyclique, dans lequel x indique le nombre d'atomes de carbone et :
   ▪ Lorsque le radical hydrophobe -Hy porte 1 -GpC, alors 9 ≤ x ≤ 25,
   ▪ Lorsque le radical hydrophobe -Hy porte 2 -GpC, alors 9 ≤ x ≤ 15,
   ▪ Lorsque le radical hydrophobe -Hy porte 3 -GpC, alors 7 ≤ x ≤ 13,
   ▪ Lorsque le radical hydrophobe -Hy porte 4 -GpC, alors 7 ≤ x ≤ 11,
   ▪ Lorsque le radical hydrophobe -Hy porte au moins 5 -GpC alors, 6 ≤ x ≤ 11,
- G est un radical alkyle linéaire ou ramifié divalent de 1 à 8 atomes de carbone ledit radical alkyle portant une ou plusieurs fonction(s) acide carboxylique libre,
- R est un radical choisi dans le groupe constitué par un radical alkyle divalent, linéaire ou ramifié comprenant de 1 à 12 atomes de carbone, un radical alkyle divalent, linéaire ou ramifié comprenant de 1 à 12 atomes de carbone portant une ou plusieurs fonctions -CONH₂ ou un radical éther ou polyéther non substitué comprenant de 4 à 14 atomes de carbone et de 1 à 5 atomes d'oxygène,
- Le ou les radicaux hydrophobes -Hy de formule X étant liés au PLG:
   ∘ via une liaison covalente entre un carbonyle du radical hydrophobe -Hy et un atome d'azote porté par le PLG formant ainsi une fonction amide issue de la réaction d'une fonction amine portée par le PLG et une fonction acide portée par le précurseur -Hy' du radical hydrophobe -Hy, et
   ∘ via une liaison covalente entre un atome d'azote du radical hydrophobe -Hy et un carbonyle porté par le PLG formant ainsi une fonction amide issue de la réaction d'une fonction amine du précurseur -Hy' du radical hydrophobe -Hy et une fonction acide portée par le PLG,
- le ratio M entre le nombre de radicaux hydrophobes et le nombre d'unités glutamiques ou aspartiques étant compris entre 0 < M ≤ 0,5 ;
- lorsque plusieurs radicaux hydrophobes sont portés par un co-polyaminoacide alors ils sont identiques ou différents,
- les fonctions acides carboxyliques libres étant sous forme de sel de cation alcalin choisi dans le groupe constitué par Na⁺ et K⁺.

Dans un mode de réalisation, la composition selon l'invention est caractérisée en ce que le co-polyaminoacide est issu d'un polyaminoacide obtenu par polymérisation.

Dans un mode de réalisation, la composition selon l'invention est caractérisée en ce que le co-polyaminoacide est issu d'un polyaminoacide obtenu par polymérisation par ouverture de cycle d'un dérivé de N-carboxyanhydride d'acide glutamique ou d'un dérivé de N-carboxyanhydride d'acide aspartique.

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique comme décrit dans l'article de revue Adv. Polym. Sci. 2006, 202, 1-18 (Deming, T.J.).

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique.

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique choisi dans le groupe constitué par le N-carboxyanhydride poly-glutamate de méthyle (GluOMe-NCA), le N-carboxyanhydride poly-glutamate de benzyle (GluOBzl-NCA) et le N-carboxyanhydride poly glutamate de t-butyle (GluOtBu-NCA).

Dans un mode de réalisation, le dérivé de N-carboxyanhydride d'acide glutamique est le N-carboxyanhydride poly-L-glutamate de méthyle (L-GluOMe-NCA).

Dans un mode de réalisation, le dérivé de N-carboxyanhydride d'acide glutamique est le N-carboxyanhydride poly-L-glutamate de benzyle (L-GluOBzl-NCA).

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique en utilisant comme initiateur un complexe organométallique d'un métal de transition comme décrit dans la publication Nature 1997, 390, 386-389 (Deming, T.J.).

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique en utilisant comme initiateur l'ammoniaque ou une amine primaire comme décrit dans le brevet FR 2,801,226 (Touraud, F. ; et al.) et les références citées par ce brevet.

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique en utilisant comme initiateur l'hexaméthyldisilazane comme décrit dans la publication J. Am. Chem. Soc. 2007, 129, 14114-14115 (Lu H. ; et al.) ou une amine silylée comme décrit dans la publication J. Am. Chem. Soc. 2008, 130, 12562-12563 (Lu H. ; et al.).

Dans un mode de réalisation, la composition selon l'invention est caractérisée en ce que le procédé de synthèse du polyaminoacide obtenu par polymérisation d'un dérivé de N-carboxyanhydride d'acide glutamique ou d'un dérivé de N-carboxyanhydride d'acide aspartique dont est issu le co-polyaminoacide comprend une étape d'hydrolyse de fonctions ester.

Dans un mode de réalisation, cette étape d'hydrolyse de fonctions ester peut consister en une hydrolyse en milieu acide ou une hydrolyse en milieu basique ou être effectuée par hydrogénation.

Dans un mode de réalisation, cette étape d'hydrolyse de groupements ester est une hydrolyse en milieu acide.

Dans un mode de réalisation, cette étape d'hydrolyse de groupements ester est effectuée par hydrogénation.

Dans un mode de réalisation, la composition selon l'invention est caractérisée en ce que le co-polyaminoacide est issu d'un polyaminoacide obtenu par dépolymérisation d'un polyaminoacide de plus haut poids moléculaire.

Dans un mode de réalisation, la composition selon l'invention est caractérisée en ce que le co-polyaminoacide est issu d'un polyaminoacide obtenu par dépolymérisation enzymatique d'un polyaminoacide de plus haut poids moléculaire.

Dans un mode de réalisation, la composition selon l'invention est caractérisée en ce que le co-polyaminoacide est issu d'un polyaminoacide obtenu par dépolymérisation chimique d'un polyaminoacide de plus haut poids moléculaire.

Dans un mode de réalisation, la composition selon l'invention est caractérisée en ce que le co-polyaminoacide est issu d'un polyaminoacide obtenu par dépolymérisation enzymatique et chimique d'un polyaminoacide de plus haut poids moléculaire.

Dans un mode de réalisation, la composition selon l'invention est caractérisée en ce que le co-polyaminoacide est issu d'un polyaminoacide obtenu par dépolymérisation d'un polyaminoacide de plus haut poids moléculaire choisi dans le groupe constitué par le polyglutamate de sodium et le polyaspartate de sodium.

Dans un mode de réalisation, la composition selon l'invention est caractérisée en ce que le co-polyaminoacide est issu d'un polyaminoacide obtenu par dépolymérisation d'un polyglutamate de sodium de plus haut poids moléculaire.

Dans un mode de réalisation, la composition selon l'invention est caractérisée en ce que le co-polyaminoacide est issu d'un polyaminoacide obtenu par dépolymérisation d'un polyaspartate de sodium de plus haut poids moléculaire.

Dans un mode de réalisation, la composition selon l'invention est caractérisée en ce que le co-polyaminoacide est obtenu par greffage d'un groupe hydrophobe sur un poly-L-glutamique acide ou poly-L-aspartique acide en utilisant les procédés de formation de liaison amide bien connus de l'homme de l'art.

Dans un mode de réalisation, la composition selon l'invention est caractérisée en ce que le co-polyaminoacide est obtenu par greffage d'un groupe hydrophobe sur un poly-L-glutamique acide ou poly-L-aspartique acide en utilisant les procédés de formation de liaison amide utilisés pour la synthèse peptidique.

Dans un mode de réalisation, la composition selon l'invention est caractérisée en ce que le co-polyaminoacide est obtenu par greffage d'un groupe hydrophobe sur un poly-L-glutamique acide ou poly-L-aspartique acide comme décrit dans le brevet FR 2,840,614 (Chan, Y.P. ; et al.).

Dans la suite, les unités utilisées sont pour les insulines celles recommandées par les pharmacopées dont les correspondances en mg/ml sont données dans le tableau ci-après :

| **Insuline** | **Pharmacopée EP 8.0 (2014)** | **Pharmacopée US - USP38 (2015)** |
|---|---|---|
| Aspart | 1U = 0,0350 mg d'insuline aspart | 1 USP = 0,0350 mg d'insuline aspart |
| Lispro | 1U = 0,0347 mg d'insuline lispro | 1 USP = 0,0347 mg d'insuline lispro |
| Humaine | 1UI = 0,0347 mg d'insuline humaine | 1 USP = 0,0347 mg d'insuline humaine |
| Glargine | 1U = 0,0364 mg d'insuline glargine | 1 USP = 0,0364 mg d'insuline glargine |
| Porcine | 1UI = 0,0345 mg d'insuline porcine | 1 USP = 0,0345 mg d'insuline porcine |
| Bovine | 1UI = 0,0342 mg d'insuline bovine | 1 USP = 0,0342 mg d'insuline bovine |

On entend par insuline basale dont le point isoélectrique est compris entre 5,8 et 8,5 une insuline insoluble à pH 7 et dont la durée d'action est comprise entre 8 et 24 heures ou supérieure dans les modèles standards de diabète.

Ces insulines basales dont le point isoélectrique est compris entre 5,8 et 8,5 sont des insulines recombinantes dont la structure primaire a été modifiée principalement par introduction d'aminoacides basiques comme l'Arginine ou la Lysine. Elles sont décrites par exemple dans les brevets, demandes de brevets ou publications suivants WO 2003/053339, WO 2004/096854, US 5,656,722 et US 6,100,376 dont le contenu est incorporé par référence.

Dans un mode de réalisation, l'insuline basale dont le point isoélectrique est compris entre 5,8 et 8,5 est l'insuline glargine. L'insuline glargine est commercialisée sous la marque Lantus^{®} (100 U/ml) ou Toujeo^{®} (300 U/ml) par SANOFI.

Dans un mode de réalisation, l'insuline basale dont le point isoélectrique est compris entre 5,8 et 8,5 est une insuline glargine biosimilaire.

Une insuline glargine biosimilaire est en voie de commercialisation sous la marque Abasaglar^{®} ou Basaglar^{®} par ELI LILLY.

Dans un mode de réalisation, les compositions selon l'invention comprennent entre 40 et 500 U/mL d'insuline basale dont le point isoélectrique est compris entre 5,8 et 8,5.

Dans un mode de réalisation, les compositions selon l'invention comprennent 40 U/mL d'insuline basale dont le point isoélectrique est compris entre 5,8 et 8,5.

Dans un mode de réalisation, les compositions selon l'invention comprennent 75 U/mL d'insuline basale dont le point isoélectrique est compris entre 5,8 et 8,5.

Dans un mode de réalisation, les compositions selon l'invention comprennent 100 U/mL (soit environ 3,6 mg/mL) d'insuline basale dont le point isoélectrique est compris entre 5,8 et 8,5.

Dans un mode de réalisation, les compositions selon l'invention comprennent 150 U/mL d'insuline basale dont le point isoélectrique est compris entre 5,8 et 8,5.

Dans un mode de réalisation, les compositions selon l'invention comprennent 200 U/mL d'insuline basale dont le point isoélectrique est compris entre 5,8 et 8,5.

Dans un mode de réalisation, les compositions selon l'invention comprennent 225 U/mL d'insuline basale dont le point isoélectrique est compris entre 5,8 et 8,5.

Dans un mode de réalisation, les compositions selon l'invention comprennent 250 U/mL d'insuline basale dont le point isoélectrique est compris entre 5,8 et 8,5.

Dans un mode de réalisation, les compositions selon l'invention comprennent 300 U/mL d'insuline basale dont le point isoélectrique est compris entre 5,8 et 8,5.

Dans un mode de réalisation, les compositions selon l'invention comprennent 400 U/mL d'insuline basale dont le point isoélectrique est compris entre 5,8 et 8,5.

Dans un mode de réalisation, les compositions selon l'invention comprennent 500 U/mL d'insuline basale dont le point isoélectrique est compris entre 5,8 et 8,5.

Dans un mode de réalisation, le ratio massique entre l'insuline basale, dont le point isoélectrique est compris entre 5,8 et 8,5, et le co-polyaminoacide, soit co-polyaminoacide /insuline basale, est compris entre 0,2 et 8.

Dans un mode de réalisation, le ratio massique est compris entre 0,2 et 6.

Dans un mode de réalisation, le ratio massique est compris entre 0,2 et 5.

Dans un mode de réalisation, le ratio massique est compris entre 0,2 et 4.

Dans un mode de réalisation, le ratio massique est compris entre 0,2 et 3.

Dans un mode de réalisation, le ratio massique est compris entre 0,2 et 2.

Dans un mode de réalisation, le ratio massique est compris entre 0,2 et 1.

Dans un mode de réalisation, la concentration en co-polyaminoacide porteur de charges carboxylates et de radicaux hydrophobes est au plus de 60 mg/mL.

Dans un mode de réalisation, la concentration en co-polyaminoacide porteur de charges carboxylates et de radicaux hydrophobes est au plus de 40 mg/mL.

Dans un mode de réalisation, la concentration en co-polyaminoacide porteur de charges carboxylates et de radicaux hydrophobes est au plus de 20 mg/mL.

Dans un mode de réalisation, la concentration en co-polyaminoacide porteur de charges carboxylates et de radicaux hydrophobes est au plus de 10 mg/mL.

Dans un mode de réalisation, la concentration en co-polyaminoacide porteur de charges carboxylates et de radicaux hydrophobes est au plus 5 mg/ml.

Dans un mode de réalisation, la concentration en co-polyaminoacide porteur de charges carboxylates et de radicaux hydrophobes est au plus 2,5 mg/ml.

Dans un mode de réalisation, les compositions selon l'invention comprennent en outre une insuline prandiale. Les insulines prandiales sont solubles à pH 7.

On entend par insuline prandiale une insuline dite rapide ou « regular ».

Les insulines prandiales dites rapides sont des insulines qui doivent répondre aux besoins provoqués par l'ingestion des protéines et des glucides durant un repas, elles doivent agir en moins de 30 minutes.

Dans un mode de réalisation, l'insuline prandiale dite « regular » est de l'insuline humaine.

Dans un mode de réalisation, l'insuline prandiale est une insuline humaine recombinante telle que décrite dans la Pharmacopée Européenne et la Pharmacopée américaine.

L'insuline humaine est par exemple commercialisée sous les marques Humulin^{®} (ELI LILLY) et Novolin^{®} (NOVO NORDISK).

Les insulines prandiales dites très rapides (fast acting) sont des insulines qui sont obtenues par recombinaison et dont la structure primaire a été modifiée pour diminuer leur temps d'action.

Dans un mode de réalisation, les insulines prandiales dites très rapides (fast acting) sont choisies dans le groupe comprenant l'insuline lispro (Humalog^{®}), l'insuline glulisine (Apidra^{®}) et l'insuline aspart (NovoLog^{®}).

Dans un mode de réalisation, l'insuline prandiale est l'insuline lispro.

Dans un mode de réalisation, l'insuline prandiale est l'insuline glulisine.

Dans un mode de réalisation, l'insuline prandiale est l'insuline aspart.

Dans un mode de réalisation, les compositions selon l'invention comprennent au total entre 60 et 800 U/mL d'insuline avec une combinaison d'insuline prandiale et d'insuline basale dont le point isoélectrique est compris entre 5,8 et 8,5.

Dans un mode de réalisation, les compositions selon l'invention comprennent au total entre 100 et 500 U/mL d'insuline avec une combinaison d'insuline prandiale et d'insuline basale dont le point isoélectrique est compris entre 5,8 et 8,5.

Dans un mode de réalisation, les compositions selon l'invention comprennent au total 800 U/mL d'insuline avec une combinaison d'insuline prandiale et d'insuline basale dont le point isoélectrique est compris entre 5,8 et 8,5.

Dans un mode de réalisation, les compositions selon l'invention comprennent au total 700 U/mL d'insuline avec une combinaison d'insuline prandiale et d'insuline basale dont le point isoélectrique est compris entre 5,8 et 8,5.

Dans un mode de réalisation, les compositions selon l'invention comprennent au total 600 U/mL d'insuline avec une combinaison d'insuline prandiale et d'insuline basale dont le point isoélectrique est compris entre 5,8 et 8,5.

Dans un mode de réalisation, les compositions selon l'invention comprennent au total 500 U/mL d'insuline avec une combinaison d'insuline prandiale et d'insuline basale dont le point isoélectrique est compris entre 5,8 et 8,5.

Dans un mode de réalisation, les compositions selon l'invention comprennent au total 400 U/mL d'insuline avec une combinaison d'insuline prandiale et d'insuline basale dont le point isoélectrique est compris entre 5,8 et 8,5.

Dans un mode de réalisation, les compositions selon l'invention comprennent au total 300 U/mL d'insuline avec une combinaison d'insuline prandiale et d'insuline basale dont le point isoélectrique est compris entre 5,8 et 8,5.

Dans un mode de réalisation, les compositions selon l'invention comprennent au total 266 U/mL d'insuline avec une combinaison d'insuline prandiale et d'insuline basale dont le point isoélectrique est compris entre 5,8 et 8,5.

Dans un mode de réalisation, les compositions selon l'invention comprennent au total 200 U/mL d'insuline avec une combinaison d'insuline prandiale et d'insuline basale dont le point isoélectrique est compris entre 5,8 et 8,5.

Dans un mode de réalisation, les compositions selon l'invention comprennent au total 100 U/mL d'insuline avec une combinaison d'insuline prandiale et d'insuline basale dont le point isoélectrique est compris entre 5,8 et 8,5.

Les proportions entre l'insuline basale dont le point isoélectrique est compris entre 5,8 et 8,5 et l'insuline prandiale sont par exemple en pourcentage de 25/75, 30/70, 40/60, 50/50, 60/40, 63/37, 70/30, 75/25, 80/20, 83/17, 90/10pour des formulations telles que décrites ci-dessus comprenant de 60 à 800 U/mL. Cependant toute autre proportion peut être réalisée.

Dans un mode de réalisation, l'insuline basale dont le point isoélectrique est compris entre 5,8 et 8,5 et l'insuline prandiale sont présentes respectivement dans les concentrations suivantes (en U/ml) 75/25, 150/50, 200/66 ou 300/100.

Dans un mode de réalisation, l'insuline basale dont le point isoélectrique est compris entre 5,8 et 8,5 et l'insuline prandiale sont présentes respectivement dans les concentrations suivantes (en U/ml) 75/25.

Dans un mode de réalisation, l'insuline basale dont le point isoélectrique est compris entre 5,8 et 8,5 et l'insuline prandiale sont présentes respectivement dans les concentrations suivantes (en U/ml) 150/50.

On définit par le ratio radical hydrophobe par insuline basale comme étant le ratio de leurs concentrations molaires respectives : [Hy]/[insuline basale] (mol/mol) pour obtenir les performances attendues, à savoir la solubilisation de l'insuline basale à pH compris entre 6,0 et 8,0, la précipitation de l'insuline basale et la stabilité des compositions selon l'invention.

La valeur minimale du ratio radical hydrophobe par insuline basale [Hy]/[insuline basale], mesurée est la valeur à laquelle l'insuline basale est solubilisée, car la solubilisation est l'effet minimum à obtenir ; cette solubilisation conditionne tous les autres effets techniques qui ne peuvent être observés que si l'insuline basale est solubilisée à pH compris entre 6,0 et 8,0.

Dans les compositions selon l'invention, le ratio radical hydrophobe par insuline basale [Hy]/[insuline basale] peut être supérieur à la valeur minimale déterminée par la limite de solubilisation.

Dans un mode de réalisation, le ratio radical hydrophobe par insuline basale [Hy]/[insuline basale] ≤3.

Dans un mode de réalisation, le ratio radical hydrophobe par insuline basale [Hy]/[insuline basale] ≤2.

Dans un mode de réalisation, le ratio radical hydrophobe par insuline basale [Hy]/[insuline basale] ≤ 1,75.

Dans un mode de réalisation, le ratio radical hydrophobe par insuline basale [Hy]/[insuline basale] ≤ 1,5.

Dans un mode de réalisation, le ratio radical hydrophobe par insuline basale [Hy]/[insuline basale] ≤ 1,25.

Dans un mode de réalisation, le ratio radical hydrophobe par insuline basale [Hy]/[insuline basale] ≤ 1,00.

Dans un mode de réalisation, le ratio radical hydrophobe par insuline basale [Hy]/[insuline basale] ≤ 0,75.

Dans un mode de réalisation, le ratio radical hydrophobe par insuline basale [Hy]/[insuline basale] ≤ 0,5.

Dans un mode de réalisation, le ratio radical hydrophobe par insuline basale [Hy]/[insuline basale] ≤ 0,25.

Dans un mode de réalisation, les compositions selon l'invention comprennent en outre une hormone gastrointestinale.

On entend par « hormones gastrointestinales », les hormones choisies dans le groupe constitué par le GLP-1 RA (Glucagon like peptide-1 receptor agonist) et le GIP (Glucose-dependent insulinotropic peptide), l'oxyntomoduline (un dérivé du proglucagon), le peptide YY, l'amyline, la cholecystokinine, le polypeptide pancréatique (PP), la ghreline et l'entérostatine, leurs analogues ou dérivés et/ou leurs sels pharmaceutiquement acceptables.

Dans un mode de réalisation, les hormones gastro-intestinales sont des analogues ou dérivés de GLP-1 RA choisis dans le groupe constitué par l'exenatide ou Byetta^{®}(ASTRA-ZENECA), le liraglutide ou Victoza^{®} (NOVO NORDISK), le lixisenatide ou Lyxumia^{®} (SANOFI), l'albiglutide ou Tanzeum^{®} (GSK) ou le dulaglutide ou Trulicity^{®} (ELI LILLY & CO), leurs analogues ou dérivés et leurs sels pharmaceutiquement acceptables.

Dans un mode de réalisation, l'hormone gastro-intestinale est le pramlintide ou Symlin^{® ®}(ASTRA-ZENECA).

Dans un mode de réalisation, l'hormone gastrointestinale est l'exenatide ou Byetta^{®}, ses analogues ou dérivés et leurs sels pharmaceutiquement acceptables.

Dans un mode de réalisation, l'hormone gastrointestinale est le liraglutide ou Victoza^{®}, ses analogues ou dérivés et leurs sels pharmaceutiquement acceptables.

Dans un mode de réalisation, l'hormone gastrointestinale est le lixisenatide ou Lyxumia^{®}, ses analogues ou dérivés et leurs sels pharmaceutiquement acceptables.

Dans un mode de réalisation, l'hormone gastrointestinale est l'albiglutide ou Tanzeum^{®}, ses analogues ou dérivés et leurs sels pharmaceutiquement acceptables.

Dans un mode de réalisation, l'hormone gastrointestinale est le dulaglutide ou Trulicity^{®}, ses analogues ou dérivés et leurs sels pharmaceutiquement acceptables.

Dans un mode de réalisation, l'hormone gastrointestinale est le pramlintide ou Symlin^{®}, ses analogues ou dérivés et leurs sels pharmaceutiquement acceptables.

On entend par « analogue », lorsqu'il est utilisé par référence à un peptide ou une protéine, un peptide ou une protéine, dans lequel un ou plusieurs résidus d'acides aminés constitutifs ont été substitués par d'autres résidus d'acides aminés et/ou dans lequel un ou plusieurs résidus d'acides aminés constitutifs ont été supprimés et/ou dans lequel un ou plusieurs résidus d'acides aminés constitutifs ont été ajoutés. Le pourcentage d'homologie admis pour la présente définition d'un analogue est de 50 %.

On entend par « dérivé », lorsqu'il est utilisé par référence à un peptide ou une protéine, un peptide ou une protéine ou un analogue chimiquement modifié par un substituant qui n'est pas présent dans le peptide ou la protéine ou l'analogue de référence, c'est-à-dire un peptide ou une protéine qui a été modifié par création de liaisons covalentes, pour introduire des substituants.

Dans un mode de réalisation, le substituant est choisi dans le groupe constitué des chaines grasses.

Dans un mode de réalisation, la concentration en hormone gastrointestinale est comprise dans un intervalle de 0,01 à 100 mg/mL.

Dans un mode de réalisation, la concentration en exenatide, ses analogues ou dérivés et leurs sels pharmaceutiquement acceptables est comprise dans un intervalle de 0,04 à 0,5 mg/mL.

Dans un mode de réalisation, la concentration en liraglutide, ses analogues ou dérivés et leurs sels pharmaceutiquement acceptables est comprise dans un intervalle de 1 à 10 mg/mL.

Dans un mode de réalisation, la concentration en lixisenatide, ses analogues ou dérivés et leurs sels pharmaceutiquement acceptables est comprise dans un intervalle de 0,01 à 1 mg/mL.

Dans un mode de réalisation, la concentration en albiglutide, ses analogues ou dérivés et leurs sels pharmaceutiquement acceptables est comprise entre 5 à 100 mg/mL.

Dans un mode de réalisation, la concentration en dulaglutide, ses analogues ou dérivés et leurs sels pharmaceutiquement acceptables est comprise entre 0,1 à 10 mg/mL.

Dans un mode de réalisation, la concentration en pramlintide, ses analogues ou dérivés et leurs sels pharmaceutiquement acceptables est comprise entre 0,1 à 5 mg/mL.

Dans un mode de réalisation, les compositions selon l'invention sont réalisées par mélange de solutions commerciales d'insuline basale dont le point isoélectrique est compris entre 5,8 et 8,5 et de solutions commerciales de GLP-1 RA, d'analogue ou de dérivé de GLP-1 RA en ratios volumiques compris dans un intervalle de 10/90 à 90/10.

Dans un mode de réalisation, la composition selon l'invention comprend une dose journalière d'insuline basale et une dose journalière d'hormone gastro-intestinale.

Dans un mode de réalisation, les compositions selon l'invention comprennent entre 40 U/mL et 500 U/mL d'insuline basale dont le point isoélectrique est compris entre 5,8 et 8,5 et, entre 0,05 et 0,5 mg/mL d'exenatide.

Dans un mode de réalisation, les compositions selon l'invention comprennent entre 40 U/mL et 500 U/mL d'insuline basale dont le point isoélectrique est compris entre 5,8 et 8,5 et, de 1 à 10 mg/mL de liraglutide.

Dans un mode de réalisation, les compositions selon l'invention comprennent entre 40 U/mL et 500 U/mL d'insuline basale dont le point isoélectrique est compris entre 5,8 et 8,5 et, de 0,01 à 1 mg/mL de lixisenatide.

Dans un mode de réalisation, les compositions selon l'invention comprennent entre 40 U/mL et 500 U/mL d'insuline basale dont le point isoélectrique est compris entre 5,8 et 8,5 et, de 5 à 100 mg/mL d'albiglutide.

Dans un mode de réalisation, les compositions selon l'invention comprennent entre 40 U/mL et 500 U/mL d'insuline basale dont le point isoélectrique est compris entre 5,8 et 8,5 et, de 0,1 à 10 mg/mL de dulaglutide.

Dans un mode de réalisation, les compositions selon l'invention comprennent 500 U/mL d'insuline basale dont le point isoélectrique est compris entre 5,8 et 8,5 et, de 0,04 à 0,5 mg/mL d'exenatide.

Dans un mode de réalisation, les compositions selon l'invention comprennent 500 U/mL d'insuline basale dont le point isoélectrique est compris entre 5,8 et 8,5 et, de 1 à 10 mg/mL de liraglutide.

Dans un mode de réalisation, les compositions selon l'invention comprennent 500 U/mL d'insuline basale dont le point isoélectrique est compris entre 5,8 et 8,5 et, de 0,01 à 1 mg/mL de lixisenatide.

Dans un mode de réalisation, les compositions selon l'invention comprennent 500 U/mL d'insuline basale dont le point isoélectrique est compris entre 5,8 et 8,5 et, de 5 à 100 mg/mL d'albiglutide.

Dans un mode de réalisation, les compositions selon l'invention comprennent 500 U/mL d'insuline basale dont le point isoélectrique est compris entre 5,8 et 8,5 et, de 0,1 à 10 mg/mL de dulaglutide.

Dans un mode de réalisation, les compositions selon l'invention comprennent 400 U/mL d'insuline basale dont le point isoélectrique est compris entre 5,8 et 8,5 et, de 0,04 à 0,5 mg/mL d'exenatide.

Dans un mode de réalisation, les compositions selon l'invention comprennent 400 U/mL d'insuline basale dont le point isoélectrique est compris entre 5,8 et 8,5 et, de 1 à 10 mg/mL de liraglutide.

Dans un mode de réalisation, les compositions selon l'invention comprennent 400 U/mL d'insuline basale dont le point isoélectrique est compris entre 5,8 et 8,5 et, de 0,01 à 1 mg/mL de lixisenatide.

Dans un mode de réalisation, les compositions selon l'invention comprennent 400 U/mL d'insuline basale dont le point isoélectrique est compris entre 5,8 et 8,5 et, de 5 à 100 mg/mL d'albiglutide.

Dans un mode de réalisation, les compositions selon l'invention comprennent 400 U/mL d'insuline basale dont le point isoélectrique est compris entre 5,8 et 8,5 et, de 0,1 à 10 mg/mL de dulaglutide.

Dans un mode de réalisation, les compositions selon l'invention comprennent 300 U/mL d'insuline basale dont le point isoélectrique est compris entre 5,8 et 8,5 et, de 0,04 à 0,5 mg/mL d'exenatide.

Dans un mode de réalisation, les compositions selon l'invention comprennent 300 U/mL d'insuline basale dont le point isoélectrique est compris entre 5,8 et 8,5 et, de 1 à 10 mg/mL de liraglutide.

Dans un mode de réalisation, les compositions selon l'invention comprennent 300 U/mL d'insuline basale dont le point isoélectrique est compris entre 5,8 et 8,5 et, de 0,01 à 1 mg/mL de lixisenatide.

Dans un mode de réalisation, les compositions selon l'invention comprennent 300 U/mL d'insuline basale dont le point isoélectrique est compris entre 5,8 et 8,5 et, de 5 à 100 mg/mL d'albiglutide.

Dans un mode de réalisation, les compositions selon l'invention comprennent 300 U/mL d'insuline basale dont le point isoélectrique est compris entre 5,8 et 8,5 et, de 0,1 à 10 mg/mL de dulaglutide.

Dans un mode de réalisation, les compositions selon l'invention comprennent 225 U/mL d'insuline basale dont le point isoélectrique est compris entre 5,8 et 8,5 et, de 0,04 à 0,5 mg/mL d'exenatide.

Dans un mode de réalisation, les compositions selon l'invention comprennent 225 U/mL d'insuline basale dont le point isoélectrique est compris entre 5,8 et 8,5 et, de 1 à 10 mg/mL de liraglutide.

Dans un mode de réalisation, les compositions selon l'invention comprennent 225 U/mL d'insuline basale dont le point isoélectrique est compris entre 5,8 et 8,5 et, de 0,01 à 1 mg/mL de lixisenatide.

Dans un mode de réalisation, les compositions selon l'invention comprennent 225 U/mL d'insuline basale dont le point isoélectrique est compris entre 5,8 et 8,5 et, de 5 à 100 mg/mL d'albiglutide.

Dans un mode de réalisation, les compositions selon l'invention comprennent 225 U/mL d'insuline basale dont le point isoélectrique est compris entre 5,8 et 8,5 et, de 0,1 à 10mg/mL de dulaglutide.

Dans un mode de réalisation, les compositions selon l'invention comprennent 200 U/mL d'insuline basale dont le point isoélectrique est compris entre 5,8 et 8,5 et, de 0,04 à 0,5 mg/mL d'exenatide.

Dans un mode de réalisation, les compositions selon l'invention comprennent 200 U/mL d'insuline basale dont le point isoélectrique est compris entre 5,8 et 8,5 et, de 1 à 10 mg/mL de liraglutide.

Dans un mode de réalisation, les compositions selon l'invention comprennent 200 U/mL d'insuline basale dont le point isoélectrique est compris entre 5,8 et 8,5 et, de 0,01 à 1 mg/mL de lixisenatide.

Dans un mode de réalisation, les compositions selon l'invention comprennent 200 U/mL d'insuline basale dont le point isoélectrique est compris entre 5,8 et 8,5 et, de 5 à 100 mg/mL d'albiglutide.

Dans un mode de réalisation, les compositions selon l'invention comprennent 200 U/mL d'insuline basale dont le point isoélectrique est compris entre 5,8 et 8,5 et, de 0,1 à 10 mg/mL de dulaglutide.

Dans un mode de réalisation, les compositions selon l'invention comprennent 100 U/mL (soit environ 3,6 mg/mL) d'insuline basale dont le point isoélectrique est compris entre 5,8 et 8,5 et, de 0,04 à 0,5 mg/mL d'exenatide.

Dans un mode de réalisation, les compositions selon l'invention comprennent 100 U/mL (soit environ 3,6 mg/mL) d'insuline basale dont le point isoélectrique est compris entre 5,8 et 8,5 et, de 1 à 10 mg/mL de liraglutide.

Dans un mode de réalisation, les compositions selon l'invention comprennent 100 U/mL (soit environ 3,6 mg/mL) d'insuline basale dont le point isoélectrique est compris entre 5,8 et 8,5 et, de 0,01 à 1 mg/mL de lixisenatide.

Dans un mode de réalisation, les compositions selon l'invention comprennent 100 U/mL d'insuline basale dont le point isoélectrique est compris entre 5,8 et 8,5 et, de 5 à 100 mg/mL d'albiglutide.

Dans un mode de réalisation, les compositions selon l'invention comprennent 100 U/mL d'insuline basale dont le point isoélectrique est compris entre 5,8 et 8,5 et, de 0,1 à 10 mg/mL de dulaglutide.

Dans un mode de réalisation, les compositions selon l'invention comprennent 40 U/mL d'insuline basale dont le point isoélectrique est compris entre 5,8 et 8,5 et, de 0,04 à 0,5 mg/mL d'exenatide.

Dans un mode de réalisation, les compositions selon l'invention comprennent 40 U/mL d'insuline basale dont le point isoélectrique est compris entre 5,8 et 8,5 et, de 1 à 10 mg/mL de liraglutide.

Dans un mode de réalisation, les compositions selon l'invention comprennent 40 U/mL d'insuline basale dont le point isoélectrique est compris entre 5,8 et 8,5 et, de 0,01 à 1 mg/mL de lixisenatide.

Dans un mode de réalisation, les compositions selon l'invention comprennent 40 U/mL d'insuline basale dont le point isoélectrique est compris entre 5,8 et 8,5 et, de 5 à 100 mg/mL d'albiglutide.

Dans un mode de réalisation, les compositions selon l'invention comprennent 40 U/mL d'insuline basale dont le point isoélectrique est compris entre 5,8 et 8,5 et, de 0,1 à 10 mg/mL de dulaglutide.

L'invention concerne également des compositions qui comprennent en outre des espèces ioniques, lesdites espèces ioniques permettant d'améliorer la stabilité des compositions.

L'invention concerne également l'utilisation d'espèces ioniques choisies dans le groupe des anions, des cations et/ou zwitterions pour améliorer la stabilité physico-chimique des compositions.

Dans un mode de réalisation les espèces ioniques comprennent moins de 10 atomes de carbone.

Lesdites espèces ioniques sont choisies dans le groupe des anions, des cations et/ou zwitterions. Par zwitterion, on entend une espèce portant au moins une charge positive et au moins une charge négative sur deux atomes non adjacents.

Lesdites espèces ioniques sont utilisées seules ou en mélange et de préférence en mélange.

Dans un mode de réalisation, les anions sont choisis parmi les anions organiques.

Dans un mode de réalisation les anions organiques comprennent moins de 10 atomes de carbone.

Dans un mode de réalisation, les anions organiques sont choisis dans le groupe constitué par l'acétate, le citrate et le succinate

Dans un mode de réalisation, les anions sont choisis parmi les anions d'origine minérale.

Dans un mode de réalisation, les anions d'origine minérale sont choisis dans le groupe constitué par les sulfates, les phosphates et les halogénures, notamment les chlorures.

Dans un mode de réalisation, les cations sont choisis parmi les cations organiques.

Dans un mode de réalisation les cations organiques comprennent moins de 10 atomes de carbone.

Dans un mode de réalisation, les cations organiques sont choisis dans le groupe constitué par les ammoniums, par exemple le 2-Amino-2-(hydroxyméthyl)propane-1,3-diol où l'amine est sous forme d'ammonium.

Dans un mode de réalisation, les cations sont choisis parmi les cations d'origine minérale.

Dans un mode de réalisation, les cations d'origine minérale sont choisis dans le groupe constitué par le zinc, en particulier Zn2+ et les métaux alcalins, en particulier Na+ et K+,

Dans un mode de réalisation, les zwitterions sont choisis parmi les zwitterions d'origine organique.

Dans un mode de réalisation, les zwitterions d'origine organique sont choisis parmi les aminoacides.

Dans un mode de réalisation les aminoacides sont choisis parmi les aminoacides aliphatiques dans le groupe constitué par la glycine, l'alanine, la valine, l'isoleucine et la leucine.

Dans un mode de réalisation les aminoacides sont choisis parmi les aminoacides cycliques dans le groupe constitué par la proline.

Dans un mode de réalisation les aminoacides sont choisis parmi les aminoacides hydroxylés ou soufrés dans le groupe constitué par la cystéine, la sérine, la thréonine, et la méthionine.

Dans un mode de réalisation les aminoacides sont choisis parmi les aminoacides aromatiques dans le groupe constitué par la phénylalanine, la tyrosine et le tryptophane.

Dans un mode de réalisation les aminoacides sont choisis parmi les aminoacides dont la fonction carboxyle de la chaine latérale est amidifiée dans le groupe constitué par l'asparagine et la glutamine.

Dans un mode de réalisation, les zwitterions d'origine organique sont choisis dans le groupe constitué par les aminoacides ayant une chaine latérale non chargée.

Dans un mode de réalisation, les zwitterions d'origine organique sont choisis dans le groupe constitué par les aminodiacides ou acides aminés acides.

Dans un mode de réalisation, les aminodiacides sont choisis dans le groupe constitué par l'acide glutamique et l'acide aspartique, éventuellement sous forme de sels.

Dans un mode de réalisation, les zwitterions d'origine organique sont choisis dans le groupe constitué par les acides aminés basiques ou dits « cationiques ».

Dans un mode de réalisation les aminoacides dits « cationiques » sont choisis parmi l'arginine, l'histidine et la lysine, en particulier arginine et lysine.

Tout particulièrement les zwitterions comprennent autant de charges négatives que de charges positives et donc une charge globale nulle au point isoélectrique et/ou à un pH compris entre 6,0 et 8,0.

Lesdites espèces ioniques sont introduites dans les compositions sous forme de sels. L'introduction de ceux-ci peut se faire sous forme solide avant mise en solution dans les compositions, ou sous forme de solution, en particulier de solution concentrée.

Par exemple, les cations d'origine minérale sont apportés sous forme de sels choisis parmi le chlorure de sodium, le chlorure de zinc, le phosphate de sodium, le sulfate de sodium, etc.

Par exemples, les anions d'origine organique sont apportés sous forme des sels choisis parmi le citrate de sodium ou de potassium, l'acétate de sodium.

Par exemple, les acides aminés sont ajoutés sous forme de sels choisis parmi le chlorhydrate d'arginine, le chlorhydrate d'histidine ou sous forme non salifiée comme par exemple l'histidine, l'arginine.

Dans un mode de réalisation, la concentration molaire totale en espèces ioniques dans la composition est supérieure ou égale à 10 mM.

Dans un mode de réalisation, la concentration molaire totale en espèces ioniques dans la composition est supérieure ou égale à 20 mM.

Dans un mode de réalisation, la concentration molaire totale en espèces ioniques dans la composition est supérieure ou égale à 30 mM.

Dans un mode de réalisation, la concentration molaire totale en espèces ioniques dans la composition est supérieure ou égale à 50 mM.

Dans un mode de réalisation, la concentration molaire totale en espèces ioniques dans la composition est supérieure ou égale à 75 mM.

Dans un mode de réalisation, la concentration molaire totale en espèces ioniques dans la composition est supérieure ou égale à 100 mM.

Dans un mode de réalisation, la concentration molaire totale en espèces ioniques dans la composition est supérieure ou égale à 200 mM.

Dans un mode de réalisation, la concentration molaire totale en espèces ioniques dans la composition est supérieure ou égale à 300 mM.

Dans un mode de réalisation, la concentration molaire totale en espèces ioniques dans la composition est supérieure ou égale à 500 mM.

Dans un mode de réalisation, la concentration molaire totale en espèces ioniques dans la composition est supérieure ou égale à 600 mM.

Dans un mode de réalisation, la concentration molaire totale en espèces ioniques dans la composition est supérieure ou égale à 700 mM.

Dans un mode de réalisation, la concentration molaire totale en espèces ioniques dans la composition est supérieure ou égale à 800 mM.

Dans un mode de réalisation, la concentration molaire totale en espèces ioniques dans la composition est supérieure ou égale à 900 mM.

Dans un mode de réalisation, la concentration molaire totale en espèces ioniques dans la composition est inférieure ou égale à 1000 mM.

Dans un mode de réalisation, la concentration molaire totale en espèces ioniques dans la composition est inférieure ou égale à 1500 mM.

Dans un mode de réalisation, la concentration molaire totale en espèces ioniques dans la composition est inférieure ou égale à 1200 mM.

Dans un mode de réalisation, la concentration molaire totale en espèces ioniques dans la composition est inférieure ou égale à 1000 mM.

Dans un mode de réalisation, la concentration molaire totale en espèces ioniques dans la composition est inférieure ou égale à 900 mM.

Dans un mode de réalisation, la concentration molaire totale en espèces ioniques dans la composition est inférieure ou égale à 800 mM.

Dans un mode de réalisation, la concentration molaire totale en espèces ioniques dans la composition est inférieure ou égale à 700 mM.

Dans un mode de réalisation, la concentration molaire totale en espèces ioniques dans la composition est inférieure ou égale à 600 mM.

Dans un mode de réalisation, la concentration molaire totale en espèces ioniques dans la composition est inférieure ou égale à 500 mM.

Dans un mode de réalisation, la concentration molaire totale en espèces ioniques dans la composition est inférieure ou égale à 400 mM.

Dans un mode de réalisation, la concentration molaire totale en espèces ioniques dans la composition est inférieure ou égale à 300 mM.

Dans un mode de réalisation, la concentration molaire totale en espèces ioniques dans la composition est inférieure ou égale à 200 mM.

Dans un mode de réalisation, la concentration molaire totale en espèces ioniques dans la composition est inférieure ou égale à 100 mM.

Dans un mode de réalisation, la concentration molaire totale en espèces ioniques dans la composition est comprise entre 10 et 1000 mM.

Dans un mode de réalisation, la concentration molaire totale en espèces ioniques dans la composition est comprise entre 20 et 1000 mM.

Dans un mode de réalisation, la concentration molaire totale en espèces ioniques dans la composition est comprise entre 30 et 1000 mM.

Dans un mode de réalisation, la concentration molaire totale en espèces ioniques dans la composition est comprise entre 50 et 1000 mM.

Dans un mode de réalisation, la concentration molaire totale en espèces ioniques dans la composition est comprise entre 75 et 1000 mM.

Dans un mode de réalisation, la concentration molaire totale en espèces ioniques dans la composition est comprise entre 100 et 1000 mM.

Dans un mode de réalisation, la concentration molaire totale en espèces ioniques dans la composition est comprise entre 200 et 1000 mM.

Dans un mode de réalisation, la concentration molaire totale en espèces ioniques dans la composition est comprise entre 300 et 1000 mM.

Dans un mode de réalisation, la concentration molaire totale en espèces ioniques dans la composition est comprise entre 400 et 1000 mM.

Dans un mode de réalisation, la concentration molaire totale en espèces ioniques dans la composition est comprise entre 500 et 1000 mM.

Dans un mode de réalisation, la concentration molaire totale en espèces ioniques dans la composition est comprise entre 600 et 1000 mM.

Dans un mode de réalisation, la concentration molaire totale en espèces ioniques dans la composition est comprise entre 10 et 900 mM.

Dans un mode de réalisation, la concentration molaire totale en espèces ioniques dans la composition est comprise entre 20 et 900 mM.

Dans un mode de réalisation, la concentration molaire totale en espèces ioniques dans la composition est comprise entre 30 et 900 mM.

Dans un mode de réalisation, la concentration molaire totale en espèces ioniques dans la composition est comprise entre 50 et 900 mM.

Dans un mode de réalisation, la concentration molaire totale en espèces ioniques dans la composition est comprise entre 75 et 900 mM.

Dans un mode de réalisation, la concentration molaire totale en espèces ioniques dans la composition est comprise entre 100 et 900 mM.

Dans un mode de réalisation, la concentration molaire totale en espèces ioniques dans la composition est comprise entre 200 et 900 mM.

Dans un mode de réalisation, la concentration molaire totale en espèces ioniques dans la composition est comprise entre 300 et 900 mM.

Dans un mode de réalisation, la concentration molaire totale en espèces ioniques dans la composition est comprise entre 400 et 900 mM.

Dans un mode de réalisation, la concentration molaire totale en espèces ioniques dans la composition est comprise entre 500 et 900 mM.

Dans un mode de réalisation, la concentration molaire totale en espèces ioniques dans la composition est comprise entre 600 et 900 mM.

Dans un mode de réalisation, la concentration molaire totale en espèces ioniques dans la composition est comprise entre 10 et 900 mM.

Dans un mode de réalisation, la concentration molaire totale en espèces ioniques dans la composition est comprise entre 20 et 800 mM.

Dans un mode de réalisation, la concentration molaire totale en espèces ioniques dans la composition est comprise entre 30 et 800 mM.

Dans un mode de réalisation, la concentration molaire totale en espèces ioniques dans la composition est comprise entre 50 et 800 mM.

Dans un mode de réalisation, la concentration molaire totale en espèces ioniques dans la composition est comprise entre 75 et 800 mM.

Dans un mode de réalisation, la concentration molaire totale en espèces ioniques dans la composition est comprise entre 100 et 800 mM.

Dans un mode de réalisation, la concentration molaire totale en espèces ioniques dans la composition est comprise entre 200 et 800 mM.

Dans un mode de réalisation, la concentration molaire totale en espèces ioniques dans la composition est comprise entre 300 et 800 mM.

Dans un mode de réalisation, la concentration molaire totale en espèces ioniques dans la composition est comprise entre 400 et 800 mM.

Dans un mode de réalisation, la concentration molaire totale en espèces ioniques dans la composition est comprise entre 500 et 800 mM.

Dans un mode de réalisation, la concentration molaire totale en espèces ioniques dans la composition est comprise entre 600 et 800 mM.

Dans un mode de réalisation, la concentration molaire totale en espèces ioniques dans la composition est comprise entre 10 et 700 mM.

Dans un mode de réalisation, la concentration molaire totale en espèces ioniques dans la composition est comprise entre 20 et 700 mM.

Dans un mode de réalisation, la concentration molaire totale en espèces ioniques dans la composition est comprise entre 30 et 700 mM.

Dans un mode de réalisation, la concentration molaire totale en espèces ioniques dans la composition est comprise entre 50 et 700 mM.

Dans un mode de réalisation, la concentration molaire totale en espèces ioniques dans la composition est comprise entre 75 et 700 mM.

Dans un mode de réalisation, la concentration molaire totale en espèces ioniques dans la composition est comprise entre 100 et 700 mM.

Dans un mode de réalisation, la concentration molaire totale en espèces ioniques dans la composition est comprise entre 200 et 700 mM.

Dans un mode de réalisation, la concentration molaire totale en espèces ioniques dans la composition est comprise entre 300 et 700 mM.

Dans un mode de réalisation, la concentration molaire totale en espèces ioniques dans la composition est comprise entre 400 et 700 mM.

Dans un mode de réalisation, la concentration molaire totale en espèces ioniques dans la composition est comprise entre 500 et 700 mM.

Dans un mode de réalisation, la concentration molaire totale en espèces ioniques dans la composition est comprise entre 600 et 700 mM.

Dans un mode de réalisation, la concentration molaire totale en espèces ioniques dans la composition est comprise entre 10 et 600 mM.

Dans un mode de réalisation, la concentration molaire totale en espèces ioniques dans la composition est comprise entre 20 et 600 mM.

Dans un mode de réalisation, la concentration molaire totale en espèces ioniques dans la composition est comprise entre 30 et 600 mM.

Dans un mode de réalisation, la concentration molaire totale en espèces ioniques dans la composition est comprise entre 50 et 600 mM.

Dans un mode de réalisation, la concentration molaire totale en espèces ioniques dans la composition est comprise entre 75 et 600 mM.

Dans un mode de réalisation, la concentration molaire totale en espèces ioniques dans la composition est comprise entre 100 et 600 mM.

Dans un mode de réalisation, la concentration molaire totale en espèces ioniques dans la composition est comprise entre 200 et 600 mM.

Dans un mode de réalisation, la concentration molaire totale en espèces ioniques dans la composition est comprise entre 300 et 600 mM.

Dans un mode de réalisation, la concentration molaire totale en espèces ioniques dans la composition est comprise entre 400 et 600 mM.

Dans un mode de réalisation, la concentration molaire totale en espèces ioniques dans la composition est comprise entre 500 et 600 mM.

Dans un mode de réalisation, la concentration molaire totale en espèces ioniques dans la composition est comprise entre 10 et 500 mM.

Dans un mode de réalisation, la concentration molaire totale en espèces ioniques dans la composition est comprise entre 20 et 500 mM.

Dans un mode de réalisation, la concentration molaire totale en espèces ioniques dans la composition est comprise entre 30 et 500 mM.

Dans un mode de réalisation, la concentration molaire totale en espèces ioniques dans la composition est comprise entre 50 et 500 mM.

Dans un mode de réalisation, la concentration molaire totale en espèces ioniques dans la composition est comprise entre 75 et 500 mM.

Dans un mode de réalisation, la concentration molaire totale en espèces ioniques dans la composition est comprise entre 100 et 500 mM.

Dans un mode de réalisation, la concentration molaire totale en espèces ioniques dans la composition est comprise entre 200 et 500 mM.

Dans un mode de réalisation, la concentration molaire totale en espèces ioniques dans la composition est comprise entre 300 et 500 mM.

Dans un mode de réalisation, la concentration molaire totale en espèces ioniques dans la composition est comprise entre 400 et 500 mM.

Dans un mode de réalisation, la concentration molaire totale en espèces ioniques dans la composition est comprise entre 10 et 400 mM.

Dans un mode de réalisation, la concentration molaire totale en espèces ioniques dans la composition est comprise entre 20 et 400 mM.

Dans un mode de réalisation, la concentration molaire totale en espèces ioniques dans la composition est comprise entre 30 et 400 mM.

Dans un mode de réalisation, la concentration molaire totale en espèces ioniques dans la composition est comprise entre 50 et 400 mM.

Dans un mode de réalisation, la concentration molaire totale en espèces ioniques dans la composition est comprise entre 75 et 400 mM.

Dans un mode de réalisation, la concentration molaire totale en espèces ioniques dans la composition est comprise entre 100 et 400 mM.

Dans un mode de réalisation, la concentration molaire totale en espèces ioniques dans la composition est comprise entre 200 et 400 mM.

Dans un mode de réalisation, la concentration molaire totale en espèces ioniques dans la composition est comprise entre 300 et 400 mM.

Dans un mode de réalisation, la concentration molaire totale en espèces ioniques dans la composition est comprise entre 10 et 300 mM.

Dans un mode de réalisation, la concentration molaire totale en espèces ioniques dans la composition est comprise entre 20 et 300 mM.

Dans un mode de réalisation, la concentration molaire totale en espèces ioniques dans la composition est comprise entre 30 et 300 mM.

Dans un mode de réalisation, la concentration molaire totale en espèces ioniques dans la composition est comprise entre 50 et 300 mM.

Dans un mode de réalisation, la concentration molaire totale en espèces ioniques dans la composition est comprise entre 75 et 300 mM.

Dans un mode de réalisation, la concentration molaire totale en espèces ioniques dans la composition est comprise entre 100 et 300 mM.

Dans un mode de réalisation, la concentration molaire totale en espèces ioniques dans la composition est comprise entre 200 et 300 mM.

Dans un mode de réalisation, la concentration molaire totale en espèces ioniques dans la composition est comprise entre 10 et 200 mM.

Dans un mode de réalisation, la concentration molaire totale en espèces ioniques dans la composition est comprise entre 20 et 200 mM.

Dans un mode de réalisation, la concentration molaire totale en espèces ioniques dans la composition est comprise entre 30 et 200 mM.

Dans un mode de réalisation, la concentration molaire totale en espèces ioniques dans la composition est comprise entre 50 et 200 mM.

Dans un mode de réalisation, la concentration molaire totale en espèces ioniques dans la composition est comprise entre 75 et 200 mM.

Dans un mode de réalisation, la concentration molaire totale en espèces ioniques dans la composition est comprise entre 100 et 200 mM.

Dans un mode de réalisation, la concentration molaire totale en espèces ioniques dans la composition est comprise entre 10 et 100 mM.

Dans un mode de réalisation, la concentration molaire totale en espèces ioniques dans la composition est comprise entre 20 et 100 mM.

Dans un mode de réalisation, la concentration molaire totale en espèces ioniques dans la composition est comprise entre 30 et 100 mM.

Dans un mode de réalisation, la concentration molaire totale en espèces ioniques dans la composition est comprise entre 50 et 100 mM.

Dans un mode de réalisation, la concentration molaire totale en espèces ioniques dans la composition est comprise entre 75 et 100 mM.

Dans un mode de réalisation, la concentration molaire totale en espèces ioniques dans la composition est comprise entre 10 et 75 mM.

Dans un mode de réalisation, la concentration molaire totale en espèces ioniques dans la composition est comprise entre 20 et 75 mM.

Dans un mode de réalisation, la concentration molaire totale en espèces ioniques dans la composition est comprise entre 30 et 75 mM.

Dans un mode de réalisation, la concentration molaire totale en espèces ioniques dans la composition est comprise entre 50 et 75 mM.

Dans un mode de réalisation, la concentration molaire totale en espèces ioniques dans la composition est comprise entre 10 et 50 mM.

Dans un mode de réalisation, la concentration molaire totale en espèces ioniques dans la composition est comprise entre 20 et 50 mM.

Dans un mode de réalisation, la concentration molaire totale en espèces ioniques dans la composition est comprise entre 30 et 50 mM.

Dans un mode de réalisation, lesdites espèces ioniques sont présentes en une concentration allant de 5 à 400 mM.

Dans un mode de réalisation, lesdites espèces ioniques sont présentes en une concentration allant de 5 à 300 mM.

Dans un mode de réalisation, lesdites espèces ioniques sont présentes en une concentration allant de 5 à 200 mM.

Dans un mode de réalisation, lesdites espèces ioniques sont présentes en une concentration allant de 5 à 100 mM.

Dans un mode de réalisation, lesdites espèces ioniques sont présentes en une concentration allant de 5 à 75 mM.

Dans un mode de réalisation, lesdites espèces ioniques sont présentes en une concentration allant de 5 à 50 mM.

Dans un mode de réalisation, lesdites espèces ioniques sont présentes en une concentration allant de 5 à 25 mM.

Dans un mode de réalisation, lesdites espèces ioniques sont présentes en une concentration allant de 5 à 20 mM.

Dans un mode de réalisation, lesdites espèces ioniques sont présentes en une concentration allant de 5 à 10 mM.

Dans un mode de réalisation, lesdites espèces ioniques sont présentes en une concentration allant de 10 à 400 mM.

Dans un mode de réalisation, lesdites espèces ioniques sont présentes en une concentration allant de 10 à 300 mM.

Dans un mode de réalisation, lesdites espèces ioniques sont présentes en une concentration allant de 10 à 200 mM.

Dans un mode de réalisation, lesdites espèces ioniques sont présentes en une concentration allant de 10 à 100 mM.

Dans un mode de réalisation, lesdites espèces ioniques sont présentes en une concentration allant de 10 à 75 mM.

Dans un mode de réalisation, lesdites espèces ioniques sont présentes en une concentration allant de 10 à 50 mM.

Dans un mode de réalisation, lesdites espèces ioniques sont présentes en une concentration allant de 10 à 25 mM.

Dans un mode de réalisation, lesdites espèces ioniques sont présentes en une concentration allant de 10 à 20 mM.

Dans un mode de réalisation, lesdites espèces ioniques sont présentes en une concentration allant de 20 à 300 mM.

Dans un mode de réalisation, lesdites espèces ioniques sont présentes en une concentration allant de 20 à 200 mM.

Dans un mode de réalisation, lesdites espèces ioniques sont présentes en une concentration allant de 20 à 100 mM.

Dans un mode de réalisation, lesdites espèces ioniques sont présentes en une concentration allant de 20 à 75 mM.

Dans un mode de réalisation, lesdites espèces ioniques sont présentes en une concentration allant de 20 à 50 mM.

Dans un mode de réalisation, lesdites espèces ioniques sont présentes en une concentration allant de 20 à 25 mM.

Dans un mode de réalisation, lesdites espèces ioniques sont présentes en une concentration allant de 50 à 300 mM.

Dans un mode de réalisation, lesdites espèces ioniques sont présentes en une concentration allant de 50 à 200 mM.

Dans un mode de réalisation, lesdites espèces ioniques sont présentes en une concentration allant de 50 à 100 mM.

Dans un mode de réalisation, lesdites espèces ioniques sont présentes en une concentration allant de 50 à 75 mM.

S'agissant des cations d'origine minérale et en particulier de Zn²⁺, sa concentration molaire au sein de la composition peut être comprise entre 0,25 et 20 mM, en particulier entre 0,25 et 10 mM ou entre 0,25 et 5 mM.

Dans un mode de réalisation, la composition comprend du zinc.

Dans un mode de réalisation, la composition comprend de 0,2 à 2 mM de zinc.

Dans un mode de réalisation, la composition comprend du NaCl.

Dans un mode de réalisation le NaCl est présent en une concentration allant de 2 à 25 mM.

Dans un mode de réalisation le NaCl est présent en une concentration allant de 2,5 à 20 mM.

Dans un mode de réalisation le NaCl est présent en une concentration allant de 4 à 15 mM.

Dans un mode de réalisation le NaCl est présent en une concentration allant de 5 à 10 mM.

Dans un mode de réalisation, les compositions selon l'invention comprennent en outre des tampons.

Dans un mode de réalisation, les compositions selon l'invention comprennent des tampons à des concentrations comprises entre 0 et 100 mM.

Dans un mode de réalisation, les compositions selon l'invention comprennent des tampons à des concentrations comprises entre 15 et 50 mM.

Dans un mode de réalisation, les compositions selon l'invention comprennent un tampon choisi dans le groupe constitué par un tampon phosphate, le Tris (trishydroxyméthylaminométhane) et le citrate de sodium.

Dans un mode de réalisation, le tampon est le phosphate de sodium.

Dans un mode de réalisation, le tampon est le Tris (trishydroxyméthylaminométhane).

Dans un mode de réalisation, le tampon est le citrate de sodium.

Dans un mode de réalisation, les compositions selon l'invention comprennent en outre des sels de zinc à une concentration comprise entre 0 et 5000 µM.

Dans un mode de réalisation, les compositions selon l'invention comprennent en outre des sels de zinc à une concentration comprise entre 0 et 4000 µM.

Dans un mode de réalisation, les compositions selon l'invention comprennent en outre des sels de zinc à une concentration comprise entre 0 et 3000 µM.

Dans un mode de réalisation, les compositions selon l'invention comprennent en outre des sels de zinc à une concentration comprise entre 0 et 2000 µM.

Dans un mode de réalisation, les compositions selon l'invention comprennent en outre des sels de zinc à une concentration comprise entre 0 et 1000 µM.

Dans un mode de réalisation, les compositions selon l'invention comprennent en outre des sels de zinc à une concentration comprise entre 50 et 600 µM.

Dans un mode de réalisation, les compositions selon l'invention comprennent en outre des sels de zinc à une concentration comprise entre 100 et 500 µM.

Dans un mode de réalisation, les compositions selon l'invention comprennent en outre des sels de zinc à une concentration comprise entre 200 et 500 µM.

Dans un mode de réalisation, les compositions selon l'invention comprennent en outre des conservateurs.

Dans un mode de réalisation, les conservateurs sont choisis dans le groupe constitué par le m-crésol et le phénol, seuls ou en mélange.

Dans un mode de réalisation, la concentration des conservateurs est comprise entre 10 et 50 mM.

Dans un mode de réalisation, la concentration des conservateurs est comprise entre 10 et 40 mM.

Dans un mode de réalisation, les compositions selon l'invention comprennent en outre un tensioactif.

Dans un mode de réalisation, le tensioactif est choisi dans le groupe constitué par le propylène glycol et le polysorbate.

Les compositions selon l'invention peuvent en outre comprendre des additifs tels que des agents de tonicité.

Dans un mode de réalisation, les agents de tonicité sont choisis dans le groupe constitué par la glycérine, le chlorure de sodium, le mannitol et la glycine.

Les compositions selon l'invention peuvent comprendre en outre tous les excipients conformes aux pharmacopées et compatibles avec les insulines utilisées aux concentrations d'usage.

L'invention concerne également une formulation pharmaceutique selon l'invention, caractérisée en ce qu'elle est obtenue par séchage et/ou lyophilisation.

Dans le cas des libérations locale et systémique, les modes d'administration envisagés sont par voie intraveineuse, sous-cutanée, intradermique ou intramusculaire.

Les voies d'administration transdermique, orale, nasale, vaginale, oculaire, buccale, pulmonaire sont également envisagées.

Dans un mode de réalisation, la composition selon l'invention est caractérisée en ce qu'elle est administrée 1 fois par jour.

Dans un mode de réalisation, la composition selon l'invention est caractérisée en ce qu'elle est administrée au moins 2 fois par jour.

Dans un mode de réalisation, la composition selon l'invention est caractérisée en ce qu'elle est administrée 2 fois par jour.

Dans un mode de réalisation, la composition selon l'invention est caractérisée en ce qu'elle comprend en outre une insuline prandiale.

Dans un mode de réalisation, la composition selon l'invention comprenant en outre au moins une insuline prandiale est caractérisée en ce qu'elle est administrée 1 fois par jour.

Dans un mode de réalisation, la composition selon l'invention comprenant en outre au moins une insuline prandiale est caractérisée en ce qu'elle est administrée au moins 2 fois par jour.

Dans un mode de réalisation, la composition selon l'invention comprenant en outre au moins une insuline prandiale est caractérisée en ce qu'elle est administrée 2 fois par jour.

Dans un mode de réalisation, la composition selon l'invention est caractérisée en ce qu'elle comprend en outre une hormone gastro-intestinale.

Dans un mode de réalisation, la composition selon l'invention comprenant en outre au moins une hormone gastro-intestinale est caractérisée en ce qu'elle est administrée 1 fois par jour.

Dans un mode de réalisation, la composition selon l'invention comprenant en outre au moins une hormone gastro-intestinale est caractérisée en ce qu'elle est administrée au moins 2 fois par jour.

Dans un mode de réalisation, la composition selon l'invention comprenant en outre au moins une hormone gastro-intestinale est caractérisée en ce qu'elle est administrée 2 fois par jour.

Dans un mode de réalisation, la composition selon l'invention est caractérisée en ce que l'hormone gastro-intestinale est un GLP-1 RA.

Dans un mode de réalisation, la composition selon l'invention comprenant en outre au un GLP-1 RA est caractérisée en ce qu'elle est administrée 1 fois par jour.

Dans un mode de réalisation, la composition selon l'invention comprenant en outre au moins un GLP-1 RA est caractérisée en ce qu'elle est administrée au moins 2 fois par jour.

Dans un mode de réalisation, la composition selon l'invention comprenant en outre au moins un GLP-1 RA est caractérisée en ce qu'elle est administrée 2 fois par jour.

L'invention concerne également des formulations unidoses à pH compris entre 6,0 et 8,0 comprenant une insuline basale dont le point isoélectrique est compris entre 5,8 et 8,5.

L'invention concerne également des formulations unidoses à pH compris entre 6,0 et 8,0 comprenant une insuline basale dont le point isoélectrique est compris entre 5,8 et 8,5 et une insuline prandiale.

L'invention concerne également des formulations unidoses à pH compris entre 6,0 et 8,0 comprenant une insuline basale dont le point isoélectrique est compris entre 5,8 et 8,5 et une hormone gastrointestinale, telles que définies précédemment.

L'invention concerne également des formulations unidoses à pH compris entre 6,0 et 8,0 comprenant une insuline basale dont le point isoélectrique est compris entre 5,8 et 8,5, une insuline prandiale et une hormone gastrointestinale, telles que définies précédemment.

L'invention concerne également des formulations unidoses à pH compris entre 6,6 et 7,8 comprenant une insuline basale dont le point isoélectrique est compris entre 5,8 et 8,5.

L'invention concerne également des formulations unidoses à pH compris entre 6,6 et 7,8 comprenant une insuline basale dont le point isoélectrique est compris entre 5,8 et 8,5 et une insuline prandiale.

L'invention concerne également des formulations unidoses à pH compris entre 6,6 et 7,8 comprenant une insuline basale dont le point isoélectrique est compris entre 5,8 et 8,5 et une hormone gastrointestinale, telles que définies précédemment.

L'invention concerne également des formulations unidoses à pH compris entre 6,6 et 7,8 comprenant une insuline basale dont le point isoélectrique est compris entre 5,8 et 8,5, une insuline prandiale et une hormone gastrointestinale, telles que définies précédemment.

L'invention concerne également des formulations unidoses à pH compris entre 6,6 et 7,6 comprenant une insuline basale dont le point isoélectrique est compris entre 5,8 et 8,5.

L'invention concerne également des formulations unidoses à pH compris entre 6,6 et 7,6 comprenant une insuline basale dont le point isoélectrique est compris entre 5,8 et 8,5 et une insuline prandiale.

L'invention concerne également des formulations unidoses à pH compris entre 6,6 et 7,6 comprenant une insuline basale dont le point isoélectrique est compris entre 5,8 et 8,5 et une hormone gastrointestinale, telles que définies précédemment.

L'invention concerne également des formulations unidoses à pH compris entre 6,6 et 7,6 comprenant une insuline basale dont le point isoélectrique est compris entre 5,8 et 8,5 , une insuline prandiale et une hormone gastrointestinale, telles que définies précédemment.

Dans un mode de réalisation, les formulations unidoses comprennent en outre un co-polyaminoacide tel que défini précédemment.

Dans un mode de réalisation, les formulations sont sous forme d'une solution injectable.

Dans un mode de réalisation, l'insuline basale dont le point isoélectrique est compris entre 5,8 et 8,5 est l'insuline glargine.

Dans un mode de réalisation, le GLP-1 RA, analogue ou dérivé de GLP-1 RA est choisi dans le groupe comprenant exenatide (Byetta^{®}), liraglutide (Victoza^{®}), lixisenatide (Lyxumia^{®}), albiglutide (Tanzeum^{®}), dulaglutide (Trulicity^{®}) ou l'un de leurs dérivés.

Dans un mode de réalisation, l'hormone gastrointestinale est l'exenatide.

Dans un mode de réalisation, l'hormone gastrointestinale est le liraglutide.

Dans un mode de réalisation, l'hormone gastrointestinale est le lixisenatide.

Dans un mode de réalisation, l'hormone gastrointestinale est l'albiglutide.

Dans un mode de réalisation, l'hormone gastrointestinale est le dulaglutide.

La solubilisation à pH compris entre 6,0 et 8,0 des insulines basales dont le point isoélectrique est compris entre 5,8 et 8,5, par les co-polyaminoacides porteurs de charges carboxylates et d'au moins un radical hydrophobe selon l'invention, peut être constatée et contrôlée de manière simple, à l'œil nu, grâce à un changement d'aspect de la solution.

La solubilisation à pH compris entre 6,6 et 7,8 des insulines basales dont le point isoélectrique est compris entre 5,8 et 8,5, par les co-polyaminoacides porteurs de charges carboxylates et d'au moins un radical hydrophobe selon l'invention, peut être constatée et contrôlée de manière simple, à l'œil nu, grâce à un changement d'aspect de la solution.

Par ailleurs et de façon toute aussi importante, la demanderesse a pu vérifier qu'une insuline basale dont le point isoélectrique est compris entre 5,8 et 8,5, solubilisée à pH compris entre 6,0 et 8,0 en présence d'un co-polyaminoacide porteur de charges carboxylates et d'au moins un radical hydrophobe selon l'invention conserve son action d'insuline lente que ce soit seule ou en combinaison avec une insuline prandiale ou une hormone gastrointestinale.

La demanderesse a également pu vérifier qu'une insuline prandiale mélangée à pH compris entre 6,0 et 8,0 en présence d'un co-polyaminoacide porteur de charges carboxylates et d'au moins un radical hydrophobe selon l'invention et d'une insuline basale dont le point isoélectrique est compris entre 5,8 et 8,5, conserve son action d'insuline rapide.

La préparation d'une composition selon l'invention présente l'avantage de pouvoir être réalisée par simple mélange d'une solution aqueuse d'insuline basale dont le point isoélectrique est compris entre 5,8 et 8,5, et d'un co-polyaminoacide porteur de charges carboxylates et d'au moins un radical hydrophobe selon l'invention, en solution aqueuse ou sous forme lyophilisée. Si nécessaire, le pH de la préparation est ajusté à pH compris entre 6,0 et 8,0.

La préparation d'une composition selon l'invention présente l'avantage de pouvoir être réalisée par simple mélange d'une solution aqueuse d'insuline basale dont le point isoélectrique est compris entre 5,8 et 8,5, d'une solution d'insuline prandiale, et d'un co-polyaminoacide porteur de charges carboxylates et d'au moins un radical hydrophobe selon l'invention, en solution aqueuse ou sous forme lyophilisée. Si nécessaire, le pH de la préparation est ajusté à pH compris entre 6,0 et 8,0.

La préparation d'une composition selon l'invention présente l'avantage de pouvoir être réalisée par simple mélange d'une solution aqueuse d'insuline basale dont le point isoélectrique est compris entre 5,8 et 8,5, d'une solution de GLP-1 RA, un analogue ou un dérivé de GLP-1 RA, et d'un co-polyaminoacide porteur de charges carboxylates et d'au moins un radical hydrophobe selon l'invention, en solution aqueuse ou sous forme lyophilisée. Si nécessaire, le pH de la préparation est ajusté à pH compris entre 6,0 et 8,0.

La préparation d'une composition selon l'invention présente l'avantage de pouvoir être réalisée par simple mélange d'une solution aqueuse d'insuline basale dont le point isoélectrique est compris entre 5,8 et 8,5, d'une solution d'insuline prandiale, d'une solution de GLP-1 RA ou d'un analogue ou dérivé de GLP-1 RA et d'un co-polyaminoacide porteur de charges carboxylates et d'au moins un radical hydrophobe selon l'invention, en solution aqueuse ou sous forme lyophilisée. Si nécessaire, le pH de la préparation est ajusté à pH compris entre 6,0 et 8,0.

Dans un mode de réalisation, le mélange d'insuline basale et de co-polyaminoacide est concentré par ultrafiltration avant le mélange avec l'insuline prandiale en solution aqueuse ou sous forme lyophilisée.

Si nécessaire, la composition du mélange est ajustée en excipients tels que glycérine, m-crésol, chlorure de zinc, et polysorbate (Tween^{®}) par ajout de solutions concentrées de ces excipients au sein du mélange. Si nécessaire, le pH de la préparation est ajusté à pH compris entre 6,0 et 8,

### Partie A - Synthèse des composés intermédiaires hydrophobes Hv permettant d'obtenir les radicaux -Hy.

| **N°** | **COMPOSES INTERMEDIAIRES HYDROPHOBES** |
|---|---|
| A1 | 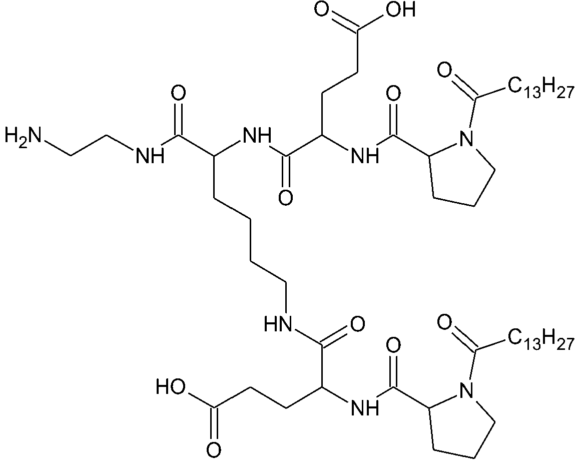 |
| A2 | 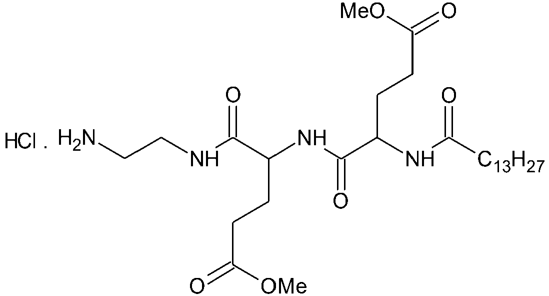 |
| A3 | 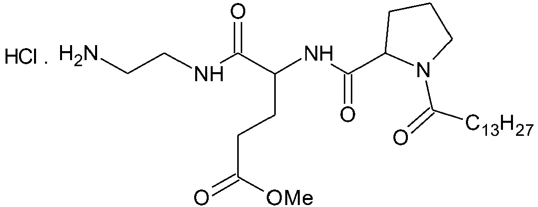 |
| A4 | 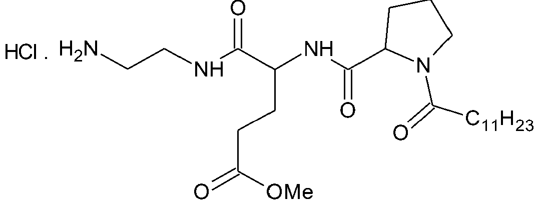 |
| A5 | 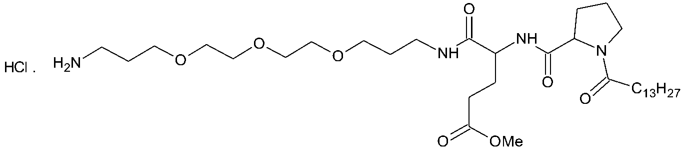 |
| A7 | 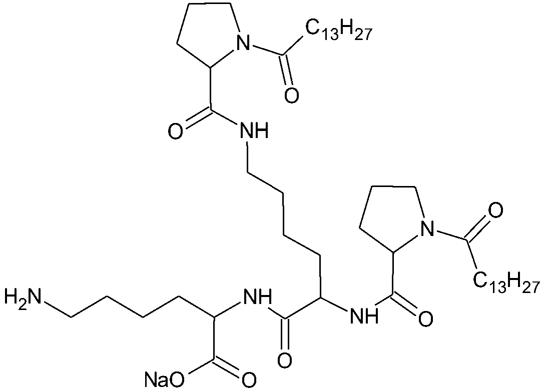 |
| A5a | 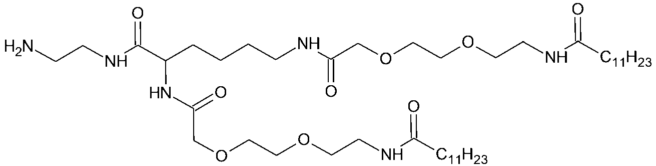 |
| A6a | 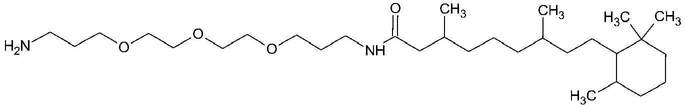 |
| A8 | 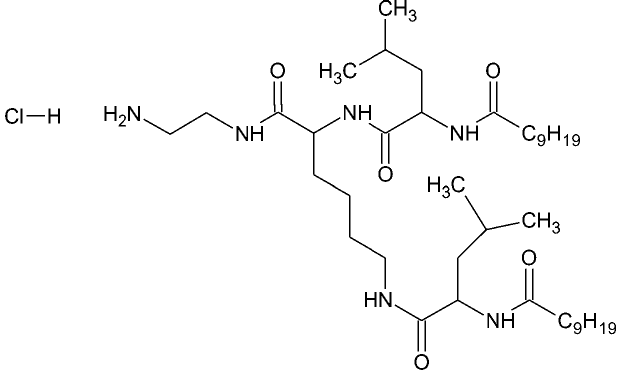 |
| A9 | 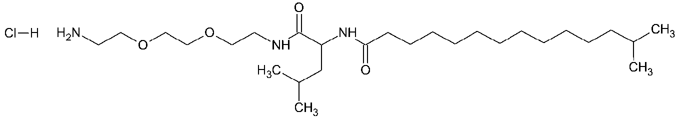 |
| A10 | 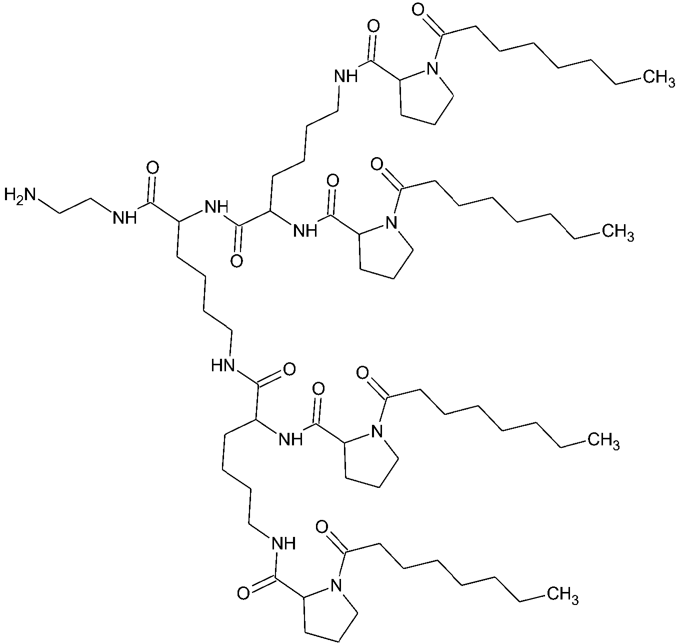 |
| A11 | 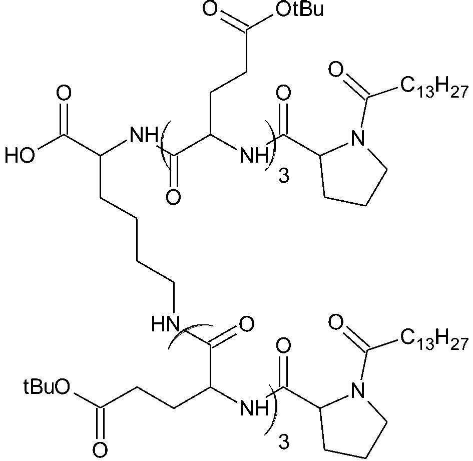 |
| A12 | 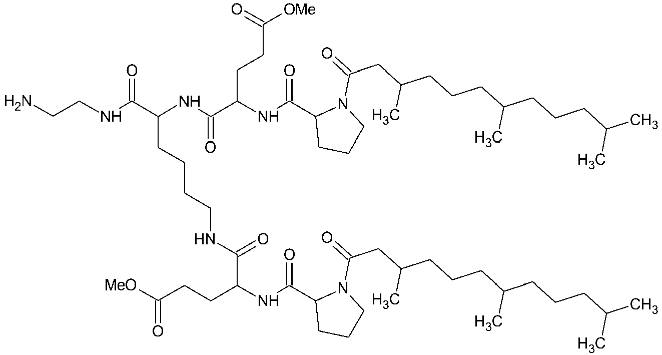 |
| A13 | 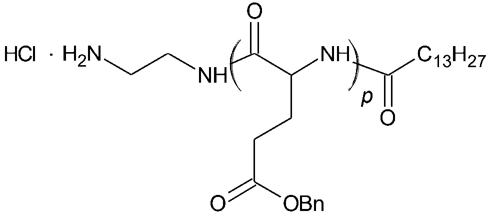 DP (p) = 5,2 |
| A14 | 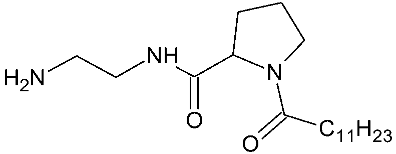 |
| A15 | 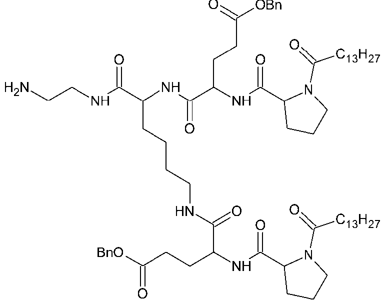 |
| A16 | 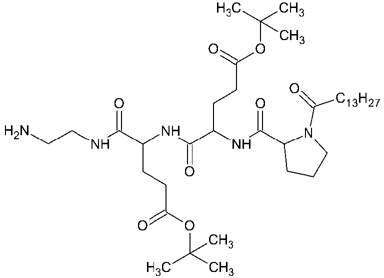 |
| A17 | 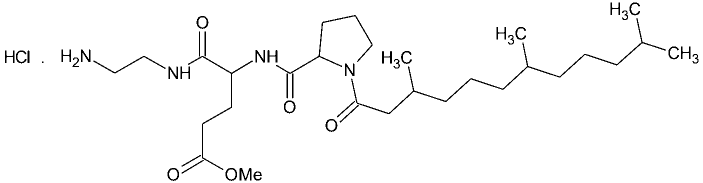 |
| A18 | 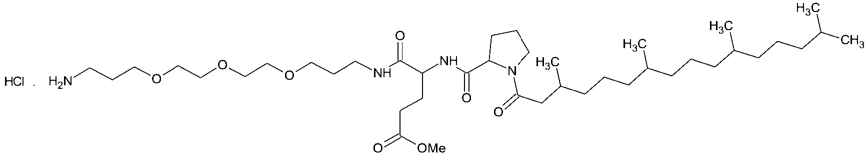 |
| A19 | 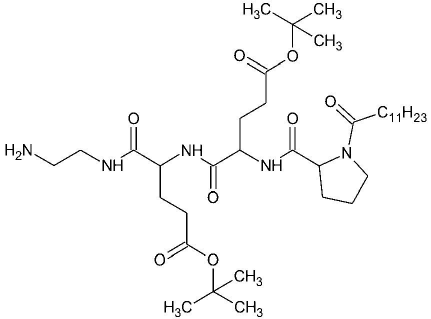 |
| A20 | 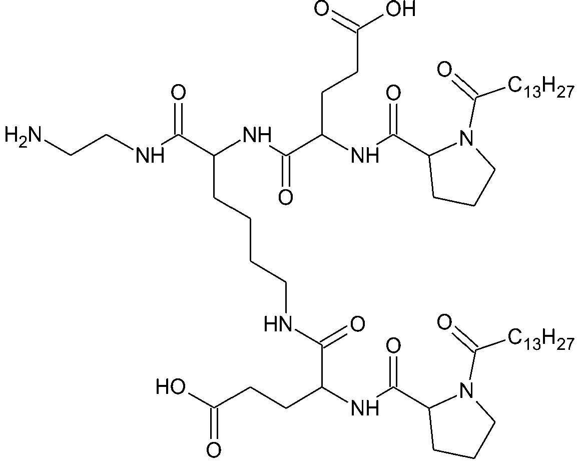 |

### Exemple A1 : molécule A1

### Molécule 1 : Produit obtenu par la réaction entre le Fmoc-Lys(Fmoc)-OH et la résine 2-Cl-trityl chloride.

À une suspension de Fmoc-Lys(Fmoc)-OH (7,32 g, 12,40 mmol) dans du dichlorométhane (60 mL) à température ambiante est ajoutée de la DIPEA (4,32 mL, 24,80 mmol). Après solubilisation complète (10 min), la solution obtenue est versée sur de la résine 2-Cl-trityl chloride préalablement lavée au dichlorométhane (100-200 mesh, 1 % DVB, 1,24 mmol/g) (4,00 g, 4,96 mmol), dans un réacteur adapté à la synthèse peptidique sur support solide. Après 2 h d'agitation à température ambiante, du méthanol grade HPLC (0,8 mL/g résine, 3,2 mL) est ajouté et le milieu est agité à température ambiante pendant 15 min. La résine est filtrée, lavée successivement avec du dichlorométhane (3 x 60 mL), du DMF (2 x 60 mL), du dichlorométhane (2 x 60 mL), de l'isopropanol (1 x 60 mL) et du dichlorométhane (3 x 60 mL).

### Molécule 2 : Produit obtenu par la réaction entre la molécule 1 et un mélange DMF/pipéridine 80:20.

La molécule 1, préalablement lavée au DMF, est traitée avec un mélange DMF/pipéridine 80:20 (60 mL). Après 30 min d'agitation à température ambiante, la résine est filtrée, lavée successivement avec du DMF (3 x 60 mL), de l'isopropanol (1 x 60 mL) et du dichlorométhane (3 x 60 mL).

### Molécule 3 : Produit obtenu par la réaction entre la molécule 2 et le Fmoc-Glu(OtBu)-OH.

À une suspension de Fmoc-Glu(OtBu)-OH (10,55 g, 24,80 mmol) et de 1-[bis(dimethylamino)methylèene]-1H-1,2,3-triazolo[4,5-b]pyridinium 3-oxide hexafluorophosphate (HATU, 9,43 g, 24,80 mmol) dans un mélange DMF/dichlorométhane 1:1 (60 mL) est ajoutée de la DIPEA (8,64 mL, 49,60 mmol). Après solubilisation complète, la solution obtenue est versée sur la molécule 2. Après 2 h d'agitation à température ambiante, la résine est filtrée, lavée successivement avec du DMF (3 x 60 mL), de l'isopropanol (1 x 60 mL) et du dichlorométhane (3 x 60 mL).

### Molécule 4 : Produit obtenu par la réaction entre la molécule 3 et un mélange DMF/morpholine 50:50.

La molécule 3, préalablement lavée au DMF, est traitée avec un mélange DMF/morpholine 50:50 (60 mL). Après 1 h 15 d'agitation à température ambiante, la résine est filtrée, lavée successivement avec du DMF (3 x 60 mL), de l'isopropanol (1 x 60 mL) et du dichlorométhane (3 x 60 mL).

### Molécule 5 : Produit obtenu par la réaction entre la molécule 4 et la molécule 11.

Par un procédé similaire à celui utilisé pour la molécule 3 appliqué à la molécule 4 et à la molécule 11 (8,07 g, 24,80 mmol) dans du DMF (60 mL), la molécule 5 est obtenue.

### Molécule 6 : Produit obtenu par la réaction entre la molécule 5 et un mélange dichlorométhane/1,1,1,3,3,3-hexafluoro-2-propanol (HFIP) 80:20.

La molécule 5 est traitée avec un mélange dichlorométhane/1,1,1,3,3,3-hexafluoro-2-propanol (HFIP) 80:20 (60 mL). Après 20 min d'agitation à température ambiante, la résine est filtrée et lavée avec du dichlorométhane (2 x 60 mL). Les solvants sont évaporés sous pression réduite. Deux co-évaporations sont ensuite effectuées sur le résidu avec du dichlorométhane (60 mL) puis du diisopropyléther (60 mL). Le produit est purifié par chromatographie sur gel de silice (dichlorométhane, méthanol). Un solide blanc de molécule 6 est obtenu.
Rendement : 2,92 g (52 % sur 6 étapes)
RMN ¹H (CD₃OD, ppm) : 0,90 (6H) ; 1,22-2,47 (88H) ; 3,13-3,25 (2H) ; 3,45-3,76 (4H) ; 4,24-4,55 (5H).
LC/MS (ESI+) : 1131,9 (calculé ([M+H]⁺) : 1131,8).

### Molécule 7 : Produit obtenu par la réaction entre la molécule 6 et la N-Boc éthylènediamine.

À une solution de la molécule 6 (2,82 g, 2,49 mmol) dans le Me-THF (20 mL) à température ambiante sont ajoutés successivement du N-hydroxybenzotriazole (HOBt, 496 mg, 3,24 mmol) et de la N-Boc éthylènediamine (BocEDA, 440 mg, 2,74 mmol). Le mélange est refroidi à 0 °C puis du chlorhydrate de (3-diméthylaminopropyl)-N'-éthylcarbodiimide (EDC, 621 mg, 3,24 mmol) est ajouté. Le milieu réactionnel est agité pendant 15 min à 0 °C puis 18 h à température ambiante. La phase organique est diluée avec du dichlorométhane (30 mL) et lavée par une solution aqueuse saturée en NH₄Cl (2 x 20 mL), une solution aqueuse saturée en NaHCO₃ (2 x 20 mL), et une solution aqueuse saturée en NaCl (2 x 20 mL). La phase organique est séchée sur Na₂SO₄, filtrée et concentrée sous pression réduite. Un solide blanc de la molécule 7 est obtenu après recristallisation dans l'acétonitrile.
Rendement : 2,47 g (78 %)
RMN ¹H (CDCl₃, ppm) : 0,87 (6H) ; 1,09-1,77 (77H) ; 1,84-2,49 (20H) ; 2,99-3,83 (10H) ; 4,16-4,25 (1H) ; 4,27-4,47 (4H) ; 5,68 (0,1H) ; 5,95-6,08 (0,9H) ; 6,91-7,14 (2H) ; 7,43-7,57 (1H) ; 7,68-7,78 (1H) ; 8,22-8,35 (1H).
LC/MS (ESI+) : 1273,9 (calculé ([M+H]⁺) : 1273,9).

### Molécule A1

À une solution de la molécule 7 (2,47 g, 1,94 mmol) dans le dichlorométhane (20 mL) à température ambiante est ajoutée une solution de HCl 4 N dans le dioxane (7,27 mL) puis le milieu est agité pendant 16 h à température ambiante. Après concentration sous pression réduite, co-évaporation et lavage au diisopropyléther, un solide blanc de la molécule A1 sous forme sel de HCl est obtenu. Ce solide est solubilisé dans de l'eau (100 mL) puis le pH est ajusté à 7 par ajout d'une solution aqueuse de NaOH 1 N. La solution est lyophilisée puis le lyophilisat est séché par co-évaporation au toluène. Un solide blanc de molécule A1 est obtenu.
Rendement : 1,64 g (80 %)
RMN ¹H (D₂O, ppm) : 0,90 (6H) ; 1,15-2,59 (70H) ; 3,06-3,86 (10H) ; 4,19-4,43 (5H).
LC/MS (ESI+) : 1061,8 (calculé ([M+H]⁺) : 1061,8).

### Exemple A2 : molécule A2

### Molécule 8 : Produit obtenu par le couplage entre l'acide myristique et le L-glutamate de méthyle.

À une solution d'acide myristique (35,0 g, 153,26 mmol) dans le tétrahydrofurane (THF, 315 mL) à 0 °C sont ajoutés successivement du N-hydroxysuccinimide (NHS, 17,81 g, 154,79 mmol) et du *N,N-*dicyclohexylcarboxydiimide (DCC, 31,94 g, 154,79 mmol). Le milieu est agité pendant 48 h tout en remontant la température à l'ambiante, filtré sur fritté puis ajouté à une solution de L-glutamate de méthyle (24,95 g, 154,79 mmol) et de *N,N-*diisopropyléthylamine (DIPEA, 99,0 g, 766,28 mmol) dans l'eau (30 mL). Le mélange réactionnel est agité à 20 °C pendant 48 h puis concentré sous pression réduite. De l'eau (200 mL) est ajoutée et le mélange obtenu est traitée par addition successive d'acétate d'éthyle (AcOEt, 100 mL) puis d'une solution aqueuse de Na₂CO₃ à 5 % (50 mL). La phase aqueuse est ensuite lavée une nouvelle fois à l'AcOEt (100 mL), acidifiée par ajout d'une solution aqueuse de HCl 10 % et le produit est extrait au dichlorométhane (DCM, 3 x 150 mL). La phase organique est séchée sur Na₂SO₄, filtrée et concentrée sous pression réduite. Un solide blanc de molécule 8 est obtenu.
Rendement : 47,11 g (84 %)
RMN ¹H (CDCl₃, ppm) : 0,87 (3H) ; 1,07-1,66 (22H) ; 2,02-2,11 (1H) ; 2,18-2,36 (3H) ; 2,39-2,47 (1H) ; 2,50-2,58 (1H) ; 3,69 (3H) ; 4,54-4,59 (1H) ; 6,62 (1H) ; 8,26 (1H).
LC/MS (ESI+) : 372,2 (calculé ([M+H]⁺) : 372,3).

### Molécule 9 : Produit obtenu par le couplage entre la molécule 8 et le L-glutamate de méthyle.

Par un procédé similaire à celui utilisé pour la préparation de la molécule 8 et appliqué à la molécule 8 (35,0 g, 94,21 mmol) et au L-glutamate de méthyle (15,33 g, 95,15 mmol), un solide blanc de la molécule 9 est obtenu après recristallisation dans l'acétonitrile.
Rendement : 24,0 g (49 %)
RMN ¹H (DMSO-d6, ppm) : 0,85 (3H) ; 1,06-1,51 (22H) ; 1,70-1,94 (3H) ; 1,96-2,15 (3H) ; 2,29-2,40 (4H) ; 3,58 (3H) ; 3,58 (3H) ; 4,16-4,22 (1H) ; 4,25-4,32 (1H) ; 7,93 (1H) ; 8,16 (1H) ; 12,66 (1H).
LC/MS (ESI+) : 515,3 (calculé ([M+H]⁺) : 515,3).

### Molécule 10 : Produit obtenu par le couplage entre la molécule 9 et la N-Boc éthylènediamine.

À une suspension de la molécule 9 (24,0 g, 46,63 mmol) dans le DCM (285 mL) à 0 °C sont ajoutés successivement du HOBt (714 mg, 46,66 mmol), de la BocEDA (8,97 g, 55,96 mmol) en solution dans le DCM (25 mL) puis du EDC (9,83 g, 51,30 mmol). Le milieu réactionnel est agité pendant 1 h à 0 °C puis 18 h à température ambiante. La phase organique est lavée par une solution aqueuse saturée en NaHCO₃ (2 x 300 mL), une solution aqueuse de HCl 1 N (2 x 300 mL), une solution aqueuse saturée en NaCl (500 mL). Du méthanol (40 mL) est ajouté, la phase organique est séchée sur Na₂SO₄, filtrée et concentrée sous pression réduite. Un solide blanc de la molécule 10 est obtenu après recristallisation dans l'acétonitrile.
Rendement : 27,15 g (89 %)
RMN ¹H (CDCl₃, ppm) : 0,87 (3H) ; 1,07-1,68 (22H) ; 1,42 (9H) ; 1,97-2,18 (4H) ; 2,22-2,31 (2H) ; 2,35-2,55 (4H) ; 3,19-3,29 (2H) ; 3,30-3,38 (2H) ; 3,66 (3H) ; 3,68 (3H) ; 4,34-4,41 (1H) ; 4,42-4,48 (1H) ; 5,54 (1H) ; 6,99-7,18 (2H) ; 7,56 (1H).
LC/MS (ESI+) : 657,4 (calculé ([M+H]⁺) : 657,4).

### Molécule A2

À une solution de la molécule 10 (27,15 g, 41,33 mmol) dans un mélange DCM/méthanol (410 mL) à 0 °C est ajoutée une solution de HCl 4 N dans le dioxane (51,7 mL) et le milieu est agité pendant 2 h à 0 °C puis 16 h à température ambiante. Après concentration sous pression réduite et co-évaporation au méthanol (2 x 150 mL), un solide blanc de la molécule A2 sous la forme d'un sel de chlorhydrate est obtenu après recristallisation dans l'acétonitrile.
Rendement : 23,2 g (95 %)
RMN ¹H (DMSO-d6, ppm) : 0,85 (3H) ; 1,05-1,52 (22H) ; 1,71-1,85 (2H) ; 1,87-2,03 (2H) ; 2,07-2,18 (2H) ; 2,24-2,37 (4H) ; 2,84 (2H) ; 3,24-3,38 (2H) ; 3,58 (3H) ; 3,58 (3H) ; 4,17-4,24 (2H) ; 7,95-8,08 (5H) ; 8,14 (1H).
LC/MS (ESI+) : 557,3 (calculé ([M+H]⁺) : 557,4).

### Exemple A3 : molécule A3

### Molécule 11 : Produit obtenu par la réaction entre le chlorure de myristoyle et la L-proline.

A une solution de L-proline (300,40 g, 2,61 mol) dans de la soude aqueuse 2 N (1,63 L) à 0 °C est ajouté lentement sur 1 h du chlorure de myristoyle (322 g, 1,30 mol) en solution dans du dichlorométhane (DCM, 1,63 L). A la fin de l'ajout, le milieu réactionnel est remonté à 20 °C en 3 h, puis agité 2 h supplémentaires. Le mélange est refroidi à 0 °C puis une solution aqueuse de HCl à 37 % (215 mL) est ajoutée en 15 min. Le milieu réactionnel est agité pendant 1 h entre 0 °C et 20 °C. La phase organique est séparée, lavée avec une solution aqueuse de HCl 10 % (3 x 430 mL), une solution aqueuse saturée en NaCl (430 mL), séchée sur Na₂SO₄, filtrée sur coton puis concentrée sous pression réduite. Le résidu est solubilisé dans de l'heptane (1,31 L) à 50 °C, puis la solution est ramenée progressivement à température ambiante. Après amorçage de la cristallisation à l'aide d'une tige en verre, le milieu est à nouveau chauffé à 40 °C pendant 30 min puis ramené à température ambiante pendant 4 h. Un solide blanc est obtenu après filtration sur fritté, lavage à l'heptane (2 x 350 mL) et séchage sous pression réduite.
Rendement : 410 g (97 %)
RMN ¹H (CDCl₃, ppm) : 0,88 (3H) ; 1,28 (20H) ; 1,70 (2H) ; 1,90-2,10 (3H) ; 2,36 (2H) ; 2,51 (1H) ; 3,47 (1H) ; 3,56 (1H) ; 4,61 (1H).
LC/MS (ESI) : 326,4 ; 651,7 ; (calculé ([M+H]⁺) : 326,3 ; ([2M+H]⁺) : 651,6).

### Molécule 12 : Produit obtenu par le couplage entre la molécule 11 et le L-glutamate de méthyle.

Par un procédé similaire à celui utilisé pour la préparation de la molécule 8 et appliqué à la molécule 11 (30,0 g, 92,17 mmol) et au L-glutamate de méthyle (15,60 g, 96,78 mmol), un solide blanc de la molécule 12 est obtenu après solubilisation dans l'acétone au reflux, refroidissement à température ambiante et filtration sur fritté. Le filtrat est évaporé et le résidu est précipité dans l'acétone comme précédemment, cette opération étant répétée 3 fois.
Rendement : 15,5 g (36 %)
RMN ¹H (DMSO-d6, ppm) : 0,85 (3H) ; 1,07-1,37 (20H) ; 1,40-1,50 (2H) ; 1,71-2,27 (8H) ; 2,30-2,40 (2H) ; 3,28-3,54 (2H) ; 3,58 (1,3H) ; 3,59 (1,7H); 4,14-4,28 (1H); 4,28-4,37 (1H) ; 8,06 (0,55H) ; 8,33 (0,45H) ; 12,64 (1H).
LC/MS (ESI+) : 469,2 (calculé ([M+H]⁺) : 469,3).

### Molécule 13 : Produit obtenu par le couplage entre la molécule 12 et la N-Boc éthylènediamine.

Par un procédé similaire à celui utilisé pour la préparation de la molécule 10 et appliqué à la molécule 12 (15,5 g, 33,05 mmol) et à la BocEDA (5,83 g, 36,36 mmol), un solide blanc de la molécule 13 est obtenu après recristallisation dans l'acétonitrile.
Rendement : 19,8 g (83 %)
RMN ¹H (DMSO-d6, ppm) : 0,85 (3H) ; 1,07-1,55 (22H) ; 1,37 (9H) ; 1,69-2,19 (7H) ; 2,22-2,36 (3H) ; 2,91-3,17 (4H) ; 3,28-3,60 (5H) ; 4,11-4,18 (0,7H) ; 4,20-4,28 (1H) ; 4,38-4,42 (0,3H) ; 6,74 (1H) ; 7,64 (0,7H) ; 7,87 (0,7H) ; 7,98 (0,3H) ; 8,22 (0,3H).
LC/MS (ESI+) : 611,4 (calculé ([M+H]⁺) : 611,4).

### Molécule A3

Par un procédé similaire à celui utilisé pour la préparation de la molécule A2 et appliqué à la molécule 13 (16,8 g, 27,50 mmol), un solide blanc de la molécule A3 sous la forme d'un sel de chlorhydrate est obtenu après recristallisation dans l'acétonitrile.
Rendement : 13,5 g (90 %)
RMN ¹H (DMSO-d6, ppm) : 0,85 (3H) ; 1,08-1,52 (22H) ; 1,70-2,37 (10H) ; 2,80-2,90 (2H) ; 3,22-3,62 (4H) ; 3,57 (3H) ; 4,15-4,28 (1,75H) ; 4,41-4,44 (0,25H) ; 7,81-8,13 (4,5H) ; 8,24-8,29 (0,25H) ; 8,33-8,39 (0,25H).
LC/MS (ESI+) : 511,3 (calculé ([M+H]⁺) : 511,4).

### Exemple A4 : Molécule A4

### Molécule 14 : Produit obtenu par la réaction entre le chlorure de lauroyle et la L-proline

Par un procédé similaire à celui utilisé pour la préparation de la molécule 11 et appliqué à au chlorure de lauroyle (27,42 g, 685,67 mmol) et à la L-proline (60,0 g, 247,27 mmol), un solide blanc de la molécule 14 est obtenu.
Rendement : 78,35 g (96 %)
RMN ¹H (CDCl₃, ppm) : 0,87 (3H) ; 1,26 (16H) ; 1,70 (2H) ; 1,90-2,10 (3H) ; 2,35 (2H) ; 2,49 (1H) ; 3,48 (1H) ; 3,56 (1H) ; 4,60 (1H).
LC/MS (ESI+) : 298,1 (calculé ([M+H]⁺) : 298,2).

### Molécule 15 : Produit obtenu par le couplage entre la molécule 14 et le L-glutamate de méthyle.

Par un procédé similaire à celui utilisé pour la préparation de la molécule 8 et appliqué à la molécule 14 (34,64 g, 116,46 mmol) et au L-glutamate de méthyle (19,14 g, 118,79 mmol), un solide blanc de la molécule 15 est obtenu après recristallisation dans l'acétonitrile.
Rendement : 37,28 g (73 %)
RMN ¹H (CDCl₃, ppm) : 0,85 (3H) ; 1,08-1,42 (16H) ; 1,54-1,06 (2H) ; 1,80-2,47 (10H) ; 3,42-3,80 (2H) ; 3,65 (2,55H) ; 3,67 (0,45H) ; 4,37-4,40 (0,15H) ; 4,51-4,58 (0,85H) ; 4,58-4,67 (1H) ; 7,26 (0,15H) ; 7,65 (0,85H) ; 8,06 (1H).
LC/MS (ESI+) : 441,1 (calculé ([M+H]⁺) : 441,3).

### Molécule 16 : Produit obtenu par le couplage entre la molécule 15 et la N-Boc éthylènediamine.

Par un procédé similaire à celui utilisé pour la préparation de la molécule 10 et appliqué à la molécule 15 (37,30 g, 84,66 mmol) et à la BocEDA (14,92 g, 93,13 mmol), un solide blanc de la molécule 16 est obtenu après recristallisation dans l'acétonitrile.
Rendement : 43,10 g (87 %)
RMN ¹H (DMSO-d6, ppm) : 0,85 (3H) ; 1,08-1,53 (18H) ; 1,37 (9H) ; 1,70-2,36 (10H) ; 2,91-3,60 (9H) ; 4,11-4,18 (0,7H) ; 4,21-4,28 (1H) ; 4,38-4,42 (0,3H) ; 6,38 (0,1H) ; 6,74 (0,9H) ; 7,65 (0,7H) ; 7,87 (0,7H) ; 7,99 (0,3H) ; 8,22 (0,3H).
LC/MS (ESI+) : 583,4 (calculé ([M+H]⁺) : 583,4).

### Molécule A4

Par un procédé similaire à celui utilisé pour la préparation de la molécule A2 et appliqué à la molécule 16 (43,10 g, 73,96 mmol), un solide blanc de la molécule A4 sous la forme d'un sel de chlorhydrate est obtenu après recristallisation dans l'acétonitrile.
Rendement : 31,90 g (83 %)
RMN ¹H (DMSO-d6, ppm) : 0,85 (3H) ; 1,05-1,37 (16H) ; 1,39-1,52 (2H) ; 1,70-2,37 (10H) ; 2,29-2,91 (2H) ; 3,20-3,62 (7H) ; 4,16-4,29 (1,7H) ; 4,42-4,46 (0,3H) ; 7,86-8,18 (4,6H) ; 8,32 (0,3H) ; 8,40 (0,3H).
LC/MS (ESI+) : 483,2 (calculé ([M+H]⁺) : 483,3).

### Exemple A5 : molécule A5

### Molécule 17 : Produit obtenu par la réaction entre la 1-amino-4,7,10-trioxa-13-tridécane amine et le tert-butyl phénylcarbonate.

À une solution de 1-amino-4,7,10-trioxa-13-tridécane amine (112,29 g, 509,71 mmol) dans l'éthanol (510 mL) à 80 °C est ajouté au goutte à goutte du *tert-*butyl phénylcarbonate (49,50 g, 254,86 mmol). Le milieu réactionnel est agité à 80 °C pendant 3 h 30 puis concentré sous pression réduite. Le résidu est solubilisé dans de l'eau (250 mL), le pH est ajusté à 2,3 avec une solution de HCl 37 % et le mélange est extrait au méthyl tert-butyléther (MTBE, 2 x 150 mL). La phase aqueuse est basifiée à pH 12,6 par addition d'une solution de NaOH 2 N et extraite au DCM (3 x 250 mL). La phase organique est lavée avec une solution aqueuse de NaOH 1 N (1 x 100 mL), une solution aqueuse saturée en NaCl (100 mL), séchée sur Na₂SO₄, filtrée et concentrée sous pression réduite. Une huile jaune de molécule 17 est obtenue.
Rendement : 54,4 g (67 %)
RMN ¹H (CDCl₃, ppm) : 1,40-1,58 (11H) ; 1,73-1,81 (4H) ; 2,80-2,84 (2H) ; 3,20-3,70 (14H) ; 5,11 (1H).
LC/MS (ESI+) : 321,2 (calculé ([M+H]⁺) : 321,2).

### Molécule 18 : Produit obtenu par le couplage entre la molécule 12 et la molécule 17.

Par un procédé similaire à celui utilisé pour la préparation de la molécule 10 et appliqué à la molécule 12 (20,46 g, 43,66 mmol) et à la molécule 17 (16,79 g, 52,39 mmol), une cire blanche de la molécule 18 est obtenue après purification par chromatographie flash (éluant : DCM, méthanol), solubilisation du résidu dans le DCM (300 mL), lavages de la phase organique avec une solution aqueuse de NaHCO₃ (2 x 150 mL), une solution aqueuse de HCl 10 % (2 x 150 mL), une solution aqueuse saturée en NaCl (2 x 150 mL), séchage sur Na₂SO₄ et concentration sous pression réduite.
Rendement : 30,15 g (90 %)
RMN ¹H (DMSO-d6, ppm) : 0,85 (3H) ; 1,09-1,52 (31H) ; 1,55-1,67 (4H) ; 1,69-2,36 (10H) ; 2,91-2,98 (2H) ; 3,02-3,17 (2H) ; 3,28-3,61 (17H) ; 4,12-4,17 (0,7H) ; 4,20-4,28 (1H) ; 4,39-4,42 (0,3H) ; 6,37 (0,1H) ; 6,71 (0,9H) ; 7,59 (0,7H) ; 7,85 (0,7H) ; 7,94 (0,3H) ; 8,21 (0,3H).
LC/MS (ESI+) : 771,4 (calculé ([M+H]⁺) : 771,5).

### Molécule A5

Par un procédé similaire à celui utilisé pour la préparation de la molécule A2 et appliqué à la molécule 18 (30,0 g, 38,91 mmol), un solide blanc de la molécule A5 sous la forme d'un sel de chlorhydrate est obtenu après solubilisation du résidu dans l'eau (500 mL) et lyophilisation.
Rendement : 25,2 g (91 %)
RMN ¹H (DMSO-d6, ppm) : 0,85 (3H) ; 1,06-1,37 (20H) ; 1,39-1,52 (2H) ; 1,58-1,66 (2H) ; 1,70-2,37 (12H) ; 2,78-2,85 (2H) ; 3,01-3,15 (2H) ; 3,31-3,62 (17H) ; 4,11-4,17 (0,7H) ; 4,19-4,27 (1H) ; 4,41-4,44 (0,3H) ; 7,63-7,71 (0,7H) ; 7,90-8,24 (4H) ; 8,28-8,35 (0,3H).
LC/MS (ESI+) : 671,4 (calculé ([M+H]⁺) : 671,5).

### Exemple A7 : molécule A7

### Molécule 21 : Produit obtenu par couplage entre la molécule 11 et la L-lysine.

Par un procédé similaire à celui utilisé pour la préparation de la molécule 8 appliqué à la molécule 11 (133,00 g, 408,61 mmol) et à la L-lysine (31,36 g, 214,52 mmol), un solide blanc de molécule 21 est obtenu après cristallisation 2 fois dans l'acétone.
Rendement : 106,50 g (68 %)
RMN ¹H (DMSO-d₆, ppm) : 0,85 (6H) ; 1,26 (40H) ; 1,35-1,50 (6H) ; 1,50-2,10 (10H) ; 2,10-2,25 (4H) ; 3,01 (2H) ; 3,31-3,55 (4H) ; 4,10-4,40 (3H) ; 7,68 (0,6H) ; 7,97 (1H) ; 8,27 (0,4H) ; 12,50 (1H).
LC/MS (ESI): 761,8 ; (calculé ([M+H]⁺): 762,1).

### Molécule 22 : Produit obtenu par couplage entre la molécule 21 et la N-Boc-L-lysinate de méthyle.

Par un procédé similaire à celui utilisé pour la préparation de la molécule 10 appliqué à la molécule 21 (43,00 g, 56,50 mmol) en solution dans le THF et au chlorhydrate de *N*-Boc-L-lysinate de méthyle (20,12 g, 67,79 mmol), un solide transparent de molécule 22 est obtenu et utilisé sans purification complémentaire.
Rendement : 55,80 g (98 %)
RMN ¹H (DMSO-d6, ppm) : 0,86 (6H) ; 1,08-2,03 (64H) ; 1,37 (9H) ; 2,07-2,30 (4H) ; 2,84-3,09 (4H) ; 3,29-3,57 (4H) ; 3,58-3,65 (3H) ; 4,14-4,43 (4H) ; 6,40 (0,1H) ; 6,74 (0,9H) ; 7,69 (0,6H) ; 7,82 (0,6H) ; 7,95-8,06 (1H) ; 8,11-8,20 (0,4H) ; 8,26 (0,4H).
LC/MS (ESI) : 1003,8 (calculé ([M+H]⁺) : 1003,8).

### Molécule 23 : Produit obtenu par saponification de la molécule 23.

Une solution de molécule 22 (55,80 g, 55,61 mmol) dans un mélange THF/eau 1:1 (370 mL) à 0 °C est traitée par addition lente d'une solution de LiOH (2,00 g, 83,41 mmol) dans l'eau (185 mL). Après 16 h d'agitation à 0 °C, le milieu est concentré sous pression réduite et le résidu est repris dans l'eau (500 mL). Du DCM (500 mL) est ajouté, le mélange hétérogène est refroidi à 10 °C et acidifié par ajout d'une solution aqueuse de HCl 10 % jusqu'à pH 1. La phase aqueuse est extraite au DCM (2 x 300 mL), les phases organiques combinées sont lavées par une solution aqueuse saturée en NaCl (2 x 300 mL), séchées sur Na₂SO₄, filtrées et concentrées sous pression réduite. Un solide blanc de molécule 23 est obtenu après cristallisation dans l'acétone.
Rendement : 46,10 g (84 %)
RMN ¹H (pyridine-d6, ppm) : 0,85 (6H) ; 1,05-2,03 (67H) ; 2,07-2,61 (10H) ; 3,12-3,93 (8H) ; 4,54-4,93 (2H) ; 4,98-5,16 (2H) ; 7,35-7,45 (1H) ; 8,34-8,63 (1H) ; 8,94-9,41 (2H).
LC/MS (ESI) : 989,8 (calculé ([M+H]⁺) : 989,8).

### Molécule A7

À une solution de la molécule 23 (12,00 g, 12,13 mmol) dans le dichlorométhane (40 mL) à 0 °C est ajoutée une solution de HCl 4 N dans le dioxane (15,20 mL) puis le milieu est agité pendant 15 h à 0 °C et 5 h température ambiante. Le mélange réactionnel est concentré sous pression réduite, le résidu est solubilisé dans un mélange de DCM (120 mL) et de NaOH 2 N (60 mL). Après séparation des phases, la phase organique est lavée par une solution de NaOH 2 N (60 mL), séchée sur Na₂SO₄ et concentrée sous pression réduite.
Rendement : 10,90 g (98 %)
RMN ¹H (DMSO-d6, ppm) : 0,86 (6H) ; 1,05-2,27 (70H) ; 2,45-2,52 (2H) ; 2,90-3,58 (6H) ; 3,67-3,76 (1H) ; 4,02-4,10 (0,6H) ; 4,11-4,17 (0,4H) ; 4,20-4,26 (0,6H) ; 4,30-4,39 (1H) ; 4,42-4,46 (0,4H) ; 7,29-7,42 (1H) ; 7,71-7,80 (0,6H) ;
7,97-8,05 (0,6H) ; 8,10-8,24 (0,4H) ; 8,33-8,45 (0,4H).

LC/MS (ESI) : 887,7 (calculé ([M-H]⁻) : 887,7).

### Exemple A5a : molécule A5a

### Molécule 3a : Produit obtenu par la réaction entre le Fmoc-Lys(Fmoc)-OH et la résine 2-Cl-trityl chloride.

À une suspension de Fmoc-Lys(Fmoc)-OH (7,32 g, 12,40 mmol) dans du DCM (60 mL) à température ambiante est ajoutée de la DIPEA (4,32 mL, 24,80 mmol). Après solubilisation complète (10 min), la solution obtenue est versée sur de la résine 2-Cl-trityl chloride (100-200 mesh, 1% DVB, 1,24 mmol/g) (4,00 g, 4,96 mmol) préalablement lavée au DCM, dans un réacteur adapté à la synthèse peptidique sur support solide. Après 2 h d'agitation à température ambiante, du méthanol grade HPLC (0,8 mL/g résine, 3,2 mL) est ajouté et le milieu est agité à température ambiante pendant 15 min. La résine est filtrée, lavée successivement avec du DCM (3 x 60 mL), du DMF (2 x 60 mL), du DCM (2 x 60 mL), de l'isopropanol (1 x 60 mL) et du DCM (3 x 60 mL).

### Molécule 4a : Produit obtenu par réaction entre la molécule 3a et un mélange DMF/pipéridine 80:20.

La molécule 3a, préalablement lavée au DMF, est traitée avec un mélange DMF/pipéridine 80:20 (60 mL). Après 30 min d'agitation à température ambiante, la résine est filtrée, lavée successivement avec du DMF (3 x 60 mL), de l'isopropanol (1 x 60 mL) et du DCM (3 x 60 mL).

### Molécule 5a : Produit obtenu par réaction entre la molécule 4a et l'acide 8-(9-Fluorénylméthyloxycarbonyl-amino)-3,6-dioxaoctanoique (Fmoc-O2Oc-OH).

À une suspension de Fmoc-O2Oc-OH (9,56 g, 24,80 mmol) et de 1-[bis(diméthylamino)methylene]-1H-1,2,3-triazolo[4,5-b]pyridinium 3-oxide hexafluorophosphate (HATU, 9,43 g, 24,80 mmol) dans un mélange DMF/DCM 1:1 (60 mL) est ajoutée de la DIPEA (8,64 mL, 49,60 mmol). Après solubilisation complète, la solution obtenue est versée sur la molécule 4a. Après 2 h d'agitation à température ambiante, la résine est filtrée, lavée successivement avec du DMF (3 x 60 mL), de l'isopropanol (1 x 60 mL) et du dichlorométhane (3 x 60 mL).

### Molécule 6a : Produit obtenu par réaction entre la molécule 5a et un mélange DMF/pipéridine 80:20.

Par un procédé similaire à celui utilisé pour la molécule 4a appliqué à la molécule 5a, la molécule 6a est obtenue.

### Molécule 7a : Produit obtenu par réaction entre la molécule 6a et l'acide laurique.

Par un procédé similaire à celui utilisé pour la molécule 5a appliqué à la molécule 6a et à l'acide laurique (4,97 g, 24,80 mmol) dans du DMF (60 mL), la molécule 7a est obtenue.

### Molécule 8a : Produit obtenu par réaction entre la molécule 7a et un mélange dichlorométhane/1,1,1,3,3,3-hexafluoro-2-propanol (HFIP) 80:20.

La molécule 7a est traitée avec un mélange dichlorométhane/1,1,1,3,3,3-hexafluoro-2-propanol (HFIP) 80:20 (60 mL). Après 20 min d'agitation à température ambiante, la résine est filtrée et lavée avec du dichlorométhane (2 x 60 mL). Les solvants sont évaporés sous pression réduite. Deux co-évaporations sont ensuite effectuées sur le résidu avec du dichlorométhane (60 mL) puis du diisopropyléther (60 mL). Un solide blanc de molécule 8a est obtenu après recristallisation dans l'acétonitrile.
Rendement : 2,63 g (66 % sur 6 étapes)
RMN ¹H (CDCl₃, ppm) : 0,87 (6H) ; 1,09-1,66 (40H) ; 1,77-1,98 (2H) ; 2,13-2,29 (4H) ; 3,24-3,75 (18H) ; 3,95-4,07 (4H) ; 4,65-4,70 (1H) ; 6,23-6,37 (1H) ; 6,39-6,62 (1H) ; 6,74-6,91 (1H) ; 7,38-7,54 (1H).
LC/MS (ESI) : 801,6 (calculé ([M+H]⁺) : 801,6).

### Molécule 9a : Produit obtenu par la réaction entre la molécule 8a et la N-Boc éthylènediamine.

À une solution de la molécule 8a (2,63 g, 3,29 mmol) dans le chloroforme (20 mL) à température ambiante sont ajoutés successivement du HOBt (654 mg, 4,27 mmol) et de la BocEDA (580 mg, 3,62 mmol). Le mélange est refroidi à 0 °C puis du EDC (819 mg, 4,27 mmol) est ajouté. Le milieu réactionnel est agité pendant 15 min à 0 °C puis 18 h à température ambiante. La phase organique est lavée par une solution aqueuse saturée en NH₄Cl (2 x 10 mL), une solution aqueuse saturée en NaHCO₃ (2 x 10 mL), et une solution aqueuse saturée en NaCl (2 x 10 mL). La phase organique est séchée sur Na₂SO₄, filtrée et concentrée sous pression réduite. Un solide blanc de la molécule 9a est obtenu après purification par chromatographie sur gel de silice (éluant : dichlorométhane, méthanol).
Rendement : 2,37 g (76 %)
RMN ¹H (CDCl₃, ppm) : 0,87 (6H) ; 1,08-1,47 (34H) ; 1,43 (9H) ; 1,48-1,70 (7H) ; 1,78-1,87 (1H) ; 2,14-2,25 (4H) ; 3,16-3,71 (22H) ; 3,92-4,04 (4H) ; 4,47-4,52 (1H) ; 5,33 (1H) ; 6,10 (1H) ; 6,65-7,01 (1H) ; 7,11-7,30 (2H) ; 7,47-7,63 (1H).

### Molécule A5a

À une solution de la molécule 9a (2,37 g, 2,51 mmol) dans le dichlorométhane (50 mL) à température ambiante est ajoutée une solution de HCl 4 M dans le dioxane (6,3 mL) puis le milieu est agité pendant 2 h à température ambiante. Après concentration sous pression réduite, le résidu est solubilisé dans du dichlorométhane (50 mL) puis lavé avec une solution aqueuse de NaOH 1 N (2 x 12,5 mL) et une solution aqueuse saturée en NaCl (25 mL). La phase organique est séchée sur Na₂SO₄, filtrée et concentrée sous pression réduite. Un solide blanc de la molécule A5a est obtenu après recristallisation dans l'acétonitrile.
Rendement : 1,57 g (74 %)
RMN ¹H (CDCl₃, ppm) : 0,87 (6H) ; 1,08-1,43 (34H) ; 1,48-1,71 (7H) ; 1,74-1,93 (3H) ; 2,14-2,25 (4H) ; 2,79-2,86 (2H) ; 3,17-3,71 (20H) ; 3,93-4,05 (4H) ; 4,47-4,54 (1H) ; 6,08-6,29 (1H) ; 6,84-7,01 (1H) ; 7,15-7,32 (2H) ; 7,50-7,64 (1H).
LC/MS (ESI) : 843,6 (calculé ([M+H]⁺) : 843,7).

### Exemple A6a : molécule A6a

### Molécule 10a : Produit obtenu par hydrogénation de l'acide rétinoïque.

Une solution d'acide rétinoïque (19,0 g, 63,24 mmol) dans du méthanol (450 mL) en présence de palladium sur charbon 10 % (1,9 g) est placée sous atmosphère d'hydrogène (1 atm) à température ambiante. Après une nuit, le milieu réactionnel est filtré sur fritté puis le filtrat est concentré sous pression réduite. Une huile incolore de molécule 10a est obtenue.
Rendement : 19,50 g (99 %)
RMN ¹H (CDCl₃, ppm) : 0,45-2,01 (35 H) ; 2,10-2,17 (1H) ; 2,33-2,38 (1H) ; 11,14 (1H).
LC/MS (ESI) : 309,3 ; (calculé ([M-H]⁻) : 309,3).

### Molécule 11a : Produit obtenu par couplage entre la Boc-1-amino-4,7,10-trioxa-13-tridécane amine (BocTOTA) et la molécule 10a.

Par un procédé similaire à celui utilisé pour la préparation de la molécule 9a appliqué à la molécule 10a (19,3 g, 62,15 mmol) et à la BocTOTA (23,9 g, 74,58 mmol), une huile orange de la molécule 11a est obtenue.
Rendement : 37,05 g (97 %)
RMN ¹H (CDCl₃, ppm) : 0,43-1,71 (49 H) ; 2,13-2,17 (1H) ; 3,17-3,24 (2H); 3,32-3,39 (2H) ; 3,51-3,66 (12H) ; 4,77 (0,1H) ; 4,94 (0,9H) ; 6,13 (0,9H) ; 6,29 (0,1H). LC/MS (ESI) : 613,5 ; (calculé ([M+H]⁺) : 613,5).

### Molécule A6a

Par un procédé similaire à celui utilisé pour la préparation de la molécule A5a appliqué à la molécule 11a (34,9 g, 56,94 mmol), une huile orange de la molécule A6a est obtenue.
Rendement : 28,5 g (97 %)
RMN ¹H (CDCl₃, ppm) : 0,41-1,96 (42 H) ; 2,13 (1H) ; 2,78 (2H) ; 3,31-3,36 (2H) ; 3,53 (4H) ; 3,55-3,58 (4H) ; 3,60-3,63 (4H) ; 6,43 (1H).
LC/MS (ESI) : 513,5 ; (calculé ([M+H]⁺) : 513,5).

### Exemple A8 : Molécule A8

### Molécule 15a : Produit obtenu par la réaction entre l'acide décanoïque et la L-leucine.

Par un procédé similaire à celui utilisé pour la préparation de la molécule 8 appliqué à l'acide décanoïque (8,77 g, 50,94 mmol) et à la L-leucine (7,00 g, 53,36 mmol), un solide blanc de la molécule 15a est obtenu.
Rendement : 9,17 g (66 %)
RMN ¹H (DMSO-d6, ppm) : 0,82-0,89 (9H) ; 1,18-1,65 (17H) ; 2,04-2,14 (2H) ; 4,19-4,23 (1H) ; 7,98 (1H) ; 12,40 (1H).
LC/MS (ESI) : 286,2 (calculé ([M+H]⁺) : 286,2).

### Molécule 16a : Produit obtenu par la réaction entre la molécule 15a et l'ester méthylique de la L-lysine.

À une solution de molécule 15a (9,16 g, 32,11 mmol) dans le THF (160 mL) sont ajoutés successivement de la triéthylamine (8,12 g, 80,27 mmol) et du 2-(1H-benzotriazol-1-yl)-1,1,3,3-tétraméthyluronium tétrafluoroborate (TBTU) et le milieu est agité pendant 30 min à température ambiante. Le dichlorhydrate d'ester méthylique de la L-lysine (3,93 g, 16,86 mmol) est ajouté et le milieu réactionnel est agité pendant 3 h puis concentré sous pression réduite. Le résidu est dilué à l'AcOEt (200 mL), la phase organique est filtrée et lavée par une solution aqueuse de HCl 1 N puis à l'eau, séchée sur Na₂SO₄, filtrée et concentrée sous pression réduite. Un solide blanc de la molécule 16a est obtenu après trituration du résidu dans l'acétonitrile.
Rendement : 7,33 g (66 %)
RMN ¹H (DMSO-d6, ppm) : 0,80-0,91 (18H) ; 1,06-1,72 (38H) ; 2,03-2,16 (4H) ; 2,91-3,07 (2H) ; 3,60 (1,15H) ; 3,61 (1,85H) ; 4,13-4,28 (2H) ; 4,33-4,44 (1H) ; 7,79-7,92 (3H) ; 8,13-8,26 (1H).
LC/MS (ESI) 695,7 (calculé ([M+H]⁺) : 695,6).

Molécule 17a : Produit obtenu par la saponification de la molécule 16a.

À une solution de molécule 16a (7,33 g, 10,55 mmol) dans un mélange THF/méthanol/eau (105 mL) est ajouté du LiOH (505,13 mg, 21,09 mmol) à 0 °C puis le milieu est agité pendant 20 h à température ambiante et concentré sous pression réduite. La phase aqueuse est acidifiée avec une solution de HCl 1 N jusqu'à pH 1 et le solide formé est filtré, lavé à l'eau et séché sous pression réduite pour conduire à un solide blanc de la molécule 17a.
Rendement : 7,09 g (99 %)
RMN ¹H (DMSO-d6, ppm) : 0,80-0,89 (18H) ; 1,18-1,73 (40H) ; 2,03-2,16 (4H) ; 2,91-3,05 (2H) ; 4,03-4,13 (1H) ; 4,21-4,27 (1H) ; 4,31-4,40 (1H) ; 7,79-8,02 (4H). LC/MS (ESI) : 681,7 (calculé ([M+H]⁺) : 681,6).

### Molécule 18a : Produit obtenu par la réaction entre la molécule 17a et la N-Boc éthylènediamine.

Par un procédé similaire à celui utilisé pour la préparation de la molécule 16a appliqué à la molécule 17a (7,09 g, 10,41 mmol) et à la N-Boc éthylènediamine (1,83 g, 11,45 mmol), un solide blanc de molécule 18a est obtenu après trituration dans l'acétonitrile.
Rendement : 6,64 g (77 %)
RMN ¹H (DMSO-d6, ppm) : 0,80-0,91 (18H) ; 1,15-1,73 (49H) ; 2,03-2,18 (4H) ; 2,92-3,13 (6H) ; 4,05-4,30 (3H) ; 6,71-6,83 (1H) ; 7,69-8,23 (5H).
LC/MS (ESI) : 824,0 (calculé ([M+H]⁺) : 823,7).

### Molécule A8

Par un procédé similaire à celui utilisé pour la molécule A5a appliqué à la molécule 18a (3,00 g, 3,64 mmol) sans lavage basique, un solide beige de molécule A8 sous la forme d'un sel de chlorhydrate est obtenu après co-évaporation 4 fois du résidu dans le méthanol.
Rendement : 2,66 g (96 %)
RMN ¹H (DMSO-d6, ppm) : 0,80-0,91 (18H) ; 1,15-1,76 (40H) ; 2,03-2,19 (4H) ; 1,78-2,89 (2H) ; 2,91-3,07 (2H) ; 3,22-3,37 (2H) ; 4,08-4,14 (1H) ; 4,17-4,28 (2H) ; 7,81-8,36 (8H).
LC/MS (ESI) : 723,7 (calculé ([M+H]⁺) : 723,6).

### Exemple A9 : Molécule A9

### Molécule 19a : Acide 13-méthyltétradécanoïque.

Dans un tricol sec sous argon est introduit du magnésium (5,50 g, 226,3 mmol) en copeaux. Le magnésium est recouvert de THF (25 mL) anhydre et quelques gouttes de 1-bromo-2-méthylpropane sont ajoutées à température ambiante pour initier la réaction. Après l'observation d'un exotherme et un léger trouble du milieu, le reste du 1-bromo-2-méthylpropane (28,42 g, 207 mmol) dilué dans du THF (60 mL) est ajouté au goutte-à-goutte en 1 h alors que la température du milieu reste stable entre 65 et 70 °C. Le milieu réactionnel est ensuite chauffé à reflux pendant 2 h.

Dans un tricol sous argon, à une solution de CuCl (280 mg, 2,83 mmol) dissout dans la *N*-méthylpyrrolidone (NMP) préalablement distillée à 0 °C est ajoutée au goutte-à-goutte une solution d'acide 11-bromoundécanoïque (25 g, 94,27 mmol) dissout dans le THF (60 mL). À cette solution est ensuite ajoutée au goutte-à-goutte la solution de l'organomagnésien légèrement chaude diluée dans le THF (50 mL) de façon à maintenir la température du milieu en dessous de 25 °C. Le mélange est ensuite agité à température ambiante pendant 16 h. Le milieu est refroidi à 0 °C et la réaction est stoppée par addition lente d'une solution aqueuse d'HCl 1 N jusqu'à pH 1 (300 mL) et le milieu est extrait à l'hexane (100 mL) et à l'acétate d'éthyle (2 x 75 mL). Après lavage de la phase organique avec une solution aqueuse d'HCl 1 N (100 mL), de l'eau (100 mL) et séchage sur Na₂SO₄, la solution est filtrée et concentrée sous vide pour donner un solide brun. Après purification par chromatographie flash (cyclohexane, acétate d'éthyle), un solide blanc est obtenu.
Rendement : 18,1 g (79 %)
RMN ¹H (CDCl₃, ppm) : 0,87 (6H) ; 1,11-1,18 (2H) ; 1,20-1,38 (16H) ; 1,51 (1H) ; 1,63 (2H) ; 2,35 (2H).

### Molécule 20 : Produit obtenu par la réaction entre la molécule 19a et la L-leucine.

À une solution de molécule 19a (18,05 g, 74,46 mmol) dans le THF (745 mL) à température ambiante sont ajoutés successivement du DCC (14,63 g, 70,92 mmol) et du NHS (8,16 g, 70,92 mmol). Après 40 h d'agitation à température ambiante, le milieu est refroidi à 0 °C pendant 20 min, filtré sur fritté. De la L-leucine (9,77 g, 74,46 mmol), de la DIPEA (86 mL) et de l'eau (150 mL) sont ajoutés au filtrat. Après 20 h d'agitation à température ambiante, le milieu est dilué avec une solution aqueuse saturée de NaHCO₃ (200 mL). La phase aqueuse est lavée à l'acétate d'éthyle (2 x 200 mL) et acidifiée avec une solution aqueuse d'HCl 2 N jusqu'à pH 1. Le précipité est filtré, rincé abondamment à l'eau et séché sous vide à 50 °C. Par 3 fois, le solide est trituré dans le pentane, soniqué puis filtré pour donner un solide blanc.
Rendement : 18,8 g (75 %)
RMN ¹H (CDCl₃, ppm) : 0,86 (6H) ; 0,96 (6H) ; 1,12-1,18 (2H) ; 1,20-1,78 (22H) ; 2,24 (2H) ; 4,58-4,63 (1H) ; 5,89 (1H).
LC/MS (ESI): 356,2 ; (calculé ([M+H]⁺): 356,6).

### Molécule 21a : Produit obtenu par la réaction entre la molécule 20 et la Boctri(éthylèneglycol)diamine.

À une solution de molécule 20 (16,7 g, 46,97 mmol) dans le THF (235 mL) sont ajoutés de la DIPEA (20,3 mL) et du TBTU à température ambiante. Après 20 min d'agitation, de la Boc-tri(éthylèneglycol)diamine (14 g, 56,36 mmol) est ajoutée. Après agitation à température ambiante pendant 5 h, le mélange est concentré sous vide. Le résidu est repris dans l'acétate d'éthyle (500 mL), lavé avec une solution aqueuse saturée de NaHCO₃ (3 x 200 mL), une solution aqueuse de HCl 1 N (3 x 200 mL) et une solution aqueuse saturée en NaCl (3 x 200 mL). Après séchage sur Na₂SO₄, filtration et concentration sous vide, le résidu est purifié par chromatographie flash (cyclohexane, acétate d'éthyle, méthanol) pour donner une huile incolore.
Rendement : 23,5 g (85 %)
RMN ¹H (CDCl₃, ppm) : 0,86 (6H) ; 0,93 (6H) ; 1,10-1,17 (2H) ; 1,19-1,08 (31H) ; 2,18 (2H) ; 3,23-3,65 (12H) ; 4,41-4,56 (1H) ; 5,12-5,47 (1H) ; 5,99-6,11 (0,75H) ; 6,48-6,65 (1H) ; 7,30-7,40 (0,25H).

### Molécule A9

Par un procédé similaire à celui utilisé pour la préparation de la molécule A5a appliqué à la molécule 21a (23,46 g, 40,04 mmol) sans lavage basique, le résidu obtenu après concentration sous vide est trituré dans un mélange acétonitrile/acétone. Le surnageant est retiré et le résidu pâteux est séché sous vide. Le résidu est ensuite trituré dans de l'acétone (150 mL) et le solide blanc de molécule A9 sous forme de sel de chlorhydrate est filtré, rincé à l'acétone puis séché sous vide.
Rendement : 13,0 g (64 %)
RMN ¹H (DMSO-d6, ppm) : 0,79-0,90 (12H) ; 1,09-1,61 (24H) ; 2,03-2,17 (2H) ; 2,92-2,98 (2H) ; 3,15-3,23 (2H) ; 3,40 (2H) ; 3,50-3,58 (4H) ; 3,61 (2H) ; 4,30-4,23 (1H) ; 7,88-8,14 (5H).
LC/MS (ESI): 486,4 ; (calculé ([M-Cl]⁺): 486,8).

### Exemple A10 : Molécule A10

### Molécule 22a : Produit obtenu par la réaction entre le chlorure d'octanoyle et la L-proline.

Par un procédé similaire à celui utilisé pour la préparation de la molécule 11 et appliqué au chlorure d'octanoyle (150,0 g, 0,922 mol) et à la L-proline (212,3 g, 1,844 mol), une huile incolore de molécule 22a est obtenue après lavages de la phase organique avec une solution aqueuse de HCl 10 % (3 x 300 mL), une solution aqueuse saturée en NaCl (300 mL), séchage sur Na₂SO₄, filtration sur coton, concentration sous pression réduite, puis le résidu est purifié par chromatographie flash (éluant : DCM, MeOH)
Rendement : 134 g (60 %)
RMN ¹H (CDCl₃, ppm) : 0,87 (3H) ; 1,10-1,52 (8H) ; 1,57-1,74 (2H) ; 1,79-2,52 (6H) ; 3,37-3,67 (2H) ; 4,37-4,42 (0,07H) ; 4,53-5,63 (0,93H) ; 9,83 (1H).
LC/MS (ESI) : 242,1 ; (calculé ([M+H]⁺) : 242,2).

### Molécule 23a : Produit obtenu par couplage entre la molécule 22a et la L-lysine.

À une solution de la molécule 22a (132 g, 0,547 mol) dans le THF (924 mL) refroidie à une température inférieure à 5 °C sont ajoutés successivement du NHS (66,1 g, 0,574 mol) et du DCC (118,5 g, 0,574 mol). Après 21 h d'agitation, le précipité est éliminé par précipitation et le filtrat est additionné en 30 min sur une solution de L-lysine (41,98 g, 0,287 mol) dans un mélange d'eau déionisé (82 mL) et de DIPEA (476 mL, 2,735 mol) à 15 °C. Après 23 h d'agitation à température ambiante, le milieu réactionnel est concentré sous pression réduite pour donner un résidu huileux qui est dilué dans de l'eau (1,3 L). La phase aqueuse est lavée deux fois avec de l'AcOEt (2 x 0,5 L), refroidie à une température inférieure à 10 °C, acidifiée par ajout d'une solution de HCl 6 N (120 mL) pour atteindre un pH de 1 puis extraite trois fois avec du DCM (3 x 0,6 L). Les phases organiques sont réunies, lavées avec une solution saturée de NaCl (0,6 L), séchées sur Na₂SO₄ puis concentrées sous pression réduite. La mousse obtenue est reprise dans de l'acétone (240 mL) au reflux pendant 2 h. Après une nuit à 10 °C, du pentane (240 mL) est ajouté goutte-à-goutte. Après 1 h d'agitation, le précipité est récupéré par filtration sous vide, lavé avec un mélange 1:1 de pentane et d'acétone (150mL) puis séché sous vide.
Rendement : 83,9 g (52 %)
RMN ¹H (CDCl₃, ppm) : 0,87 (6H) ; 1,06-1,78 (25H) ; 1,80-2,41 (13H) ; 2,80-3,72 (6H) ; 4,30-4,39 (0,15H) ; 4,46-4,70 (2,85H) ; 7,84 (1H) ; 7,93 (1H).
LC/MS (ESI) : 593,5 ; (calculé ([M+H]⁺) : 593,4).

### Molécule 24 : Produit obtenu par couplage entre la molécule 23a et l'ester méthylique de la L-lysine.

Sur la molécule 23a (76,26 g, 0,129 mol) sont successivement ajoutés du HOPO (3,57 g, 32,1 mmol), de la LysOMe dihydrochloride (15,0 g, 64,3 mmol) et du EDC (34,53 g, 0,18 mol) puis du DMF (600 mL) préalablement refroidie à 5 °C est ajouté. Après dissolution, de la triéthylamine (43,9 mL, 0,315 mol) est ajouté goutte-à-goutte en maintenant la température inférieure à 5 °C pendant encore 2 h après la fin de l'addition. Après une nuit à température ambiante, le milieu réactionnel est versé sur un mélange eau/glace (2 kg) et DCM (0,5 L). Après 15 min d'agitation, les phases sont séparées. La phase aqueuse est extraite deux fois avec du DCM (2 x 0,4 L). Les phases organiques sont réunies, lavées avec une solution de HCl 1 N (0,5 L) puis avec une solution saturée de NaCl (0,5 L), séchées sur Na₂SO₄, concentrées sous pression réduite, puis le résidu est purifié par chromatographie flash (éluant : DCM, MeOH).
Rendement : 56,7 g (67 %)
RMN ¹H (CDCl₃, ppm) : 0,87 (12H) ; 1,10-2,40 (82H) ; 2,86-3,72 (17H) ; 4,16-4,60 (7H) ; 6,83-8,01 (6H).

### Molécule A10

Une solution de molécule 24 (4,0 g, 3,05 mmol) dans de l'éthylènediamine (30 mL) est chauffée à 50 °C pendant une nuit. Le milieu réactionnel est alors dilué avec du méthyl-tétrahydrofuranne puis la phase organique est lavée 4 fois avec une solution saturée de NaCl (4 x 30 mL) puis 2 fois avec de l'eau (2 x 50 mL) avant d'être séchée sur Na₂SO₄ puis concentrée sous pression réduite. Le résidu est solubilisé dans de l'acétonitrile au reflux pendant 30 min puis la solution est refroidie à température ambiante sous agitation pendant une nuit. Le précipité blanc est alors récupéré par filtration sous vide, lavé avec de l'acétonitrile froid (2 x 20 mL) puis séché sous vide.
Rendement : 3,0 g (74 %)
RMN ¹H (CDCl₃, ppm) : 0,87 (12H) ; 1,09-2,37 (84H) ; 2,74-4,56 (25H) ; 6,85-8,00 (7H).
LC/MS (ESI) : 1338,0 (calculé ([M+H]⁺) : 1338,0).

### Exemple A11 : Molécule A11

La molécule A11 est obtenue par la méthode conventionnelle de synthèse peptidique en phase solide (SPPS) sur résine 2-chlorotrityle chloride (CTC) (40,0 g, 1,16 mmol/g).
Le greffage du premier acide aminé Fmoc-Lys(Fmoc)-OH (1,5 équivalents) est effectué dans le DCM (10V), en présence de DIPEA (3,0 équivalents). Les sites n'ayant pas réagi sont cappés au méthanol (0,8 mL/g résine) en fin de réaction.
Les couplages des acides aminés protégés Fmoc-Glu(OtBu)-OH (2,5 équivalents), Fmoc-Pro-OH (2,5 équivalents) et de l'acide myristique (2,5 équivalents) sont effectués dans le DMF (10V), en présence de HATU (2,5 équivalents) et de DIPEA (3,7 équivalents).

Les groupements protecteurs Fmoc sont retirés à l'aide d'une solution de DMF/pipéridine 80:20 (10 V).
Le produit est clivé de la résine à l'aide d'une solution de DCM/HFIP 80:20 (10 V).
Après concentration sous pression réduite, le résidu est purifié par chromatographie sur gel de silice (dichlorométhane, méthanol).
Rendement : 56,5 g (65 %)
RMN ¹H (CD₃OD, ppm) : 0,90 (6H) ; 1,22-2,53 (140H) ; 3,12-3,25 (2H) ; 3,43-3,80 (4H) ; 4,17-4,54 (9H).
LC/MS (ESI+) : 1894,5 (calculé ([M+Na]⁺) : 1894,2).

### Exemple A12 : Molécule A12

### Molécule 25 : Produit obtenu par hydrogénation du farnésol.

À une solution de farnésol (60,00 g, 269,82 mmol) dans le THF (1200 mL) sous argon est ajouté de l'oxyde de platine (PtO₂, 613 mg, 2,70 mmol) et le milieu est placé sous 1 atm de dihydrogène puis agité pendant 6 jours à température ambiante. Après filtration sur célite en rinçant au THF, une huile noire de molécule 25 est obtenue après concentration sous pression réduite. Ce composé est utilisé sans purification supplémentaire.
Rendement : 61,60 g (100%)
RMN ¹H (CDCl₃, ppm) : 0,85 (3H) ; 0,87 (6H) ; 0,90 (3H) ; 1,01-1,43 (15H) ; 1,47-1,66 (3H) ; 3,62-3,76 (2H).

### Molécule 26 : Produit obtenu par oxydation de la molécule 25

À une solution de molécule 25 (61,60 g, 269,68 mmol) dans un mélange dichloroéthane/eau (1350 mL/1080 mL) sont ajoutés successivement du bromure de tétrabutylammonium (46,95 g, 145,63 mmol), de l'acide acétique (416 mL, 7,28 mol) puis du KMnO₄ (127,85 g, 809,04 mmol) par petites fractions en maintenant la température entre 11 et 13 °C. Le milieu réactionnel est ensuite agité pendant 4 h 30 au reflux, refroidi à 0 °C puis acidifié jusqu'à pH 1 avec une solution de HCl 37 % (50 mL). Du Na₂SO₃ (186,94 g) est ajouté progressivement en maintenant la température entre 0 et 10 °C et le milieu est agité jusqu'à décoloration complète. Le milieu est acidifié jusqu'à pH 1 avec une solution de HCl 37 % puis de l'eau (500 mL) et du DCM (500 mL) sont ajoutés. Les phases sont séparées et la phase aqueuse est extraite au DCM (2 x 500 mL). Les phases organiques combinées sont lavées par une solution aqueuse de HCl à 10 % (400 mL), de l'eau (2 x 400 mL), une solution aqueuse saturée en NaCl (400 mL), séchées sur Na₂SO₄, filtrées et concentrées sous pression réduite. Une huile jaune de molécule 26 est obtenue après purification par chromatographie flash (éluant : cyclohexane, AcOEt).
Rendement : 54,79 g (84 %)
RMN ¹H (CDCl₃, ppm) : 0,85 (3H) ; 0,87 (6H) ; 0,97 (3H) ; 1,03-1,43 (13H) ; 1,52 (1H) ; 1,91-2,01 (1H) ; 2,11-2,18 (1H) ; 2,32-2,39 (1H).
LC/MS (ESI-) : 241,3 (calculé ([M-H]⁻) : 241,2).

### Molécule 27 : Produit obtenu par couplage entre la molécule 26 et la L-prolinate de méthyle.

À une solution de molécule 26 (54,70 g, 225,66 mmol) dans du DCM (1500 mL) à 0 °C sont ajoutés successivement du HOBt (3,46 g, 22,57 mmol), de la DIPEA (117,92 mL, 676,97 mmol), le chlorhydrate de L-prolinate de méthyle (56,06 g, 338,49 mmol) puis du EDC (64,89 g, 338,49 mmol). Le mélange réactionnel est agité à 0 °C pendant 1 h puis à température ambiante pendant 18 h. Le milieu est ensuite dilué avec du DCM (1000 mL) puis lavé par une solution aqueuse saturée en NaHCO₃ (2 x 1 L), une solution aqueuse de HCl 1 N (2 x 1000 mL) et une solution aqueuse saturée en NaCl (2 x 1000 mL). La phase organique est séchée sur Na₂SO₄, filtrée et concentrée sous pression réduite pour donner une huile jaune de molécule 27 qui est utilisée sans purification supplémentaire.
Rendement : 77,15 g (97 %)
RMN ¹H (DMSO-d₆, ppm) : 0,79-0,89 (12H) ; 0,98-1,43 (13H) ; 1,51 (1H) ; 1,70-2,32 (7H) ; 3,33-3,42 (0,4H) ; 3,46-3,57 (1,6H) ; 3,59 (2,4H) ; 3,67 (0,6H) ; 4,23-4,32 (0,8H) ; 4,53-4,62 (0,2H).
LC/MS (ESI+) : 354,2 (calculé ([M+H]⁺) : 354,3).

### Molécule 28 : Produit obtenu par la saponification de la molécule 27.

À une solution de molécule 27 (77,15 g, 218,22 mmol) dans un mélange THF/MeOH 1:1 (1454 mL) à 0 °C est ajoutée goutte-à-goutte une solution de LiOH (7,84 g, 327,33 mmol) dans de l'eau (727 mL). Le mélange réactionnel est agité à 0 °C pendant 18 h puis à température ambiante pendant 5 h. Les solvants organiques sont évaporés sous pression réduite. De l'eau (500 mL), une solution aqueuse de HCl à 10 % (200 mL) et du DCM (800 mL) sont ajoutés et les phases sont séparées. La phase aqueuse est extraite par du DCM (2 x 1 L). Les phases organiques réunies sont lavées par de l'eau (500 mL), une solution aqueuse saturée en NaCl (500 mL), séchées sur Na₂SO₄, filtrées et concentrées sous pression réduite pour donner une huile jaune de molécule 28 qui est utilisée sans purification supplémentaire.
Rendement : 71,72 g (97 %)
RMN ¹H (DMSO-d₆, ppm) : 0,73-0,95 (12H) ; 0,95-1,42 (13H) ; 1,51 (1H) ; 1,65-2,32 (7H) ; 3,24-3,64 (2H) ; 4,13-4,28 (0,8H) ; 4,37-4,50 (0,2H) ; 12,44 (1H).
LC/MS (ESI+) : 340,2 (calculé ([M+H]⁺) : 340,3).

### Molécule A12

La molécule A12 est obtenue par la méthode conventionnelle de synthèse peptidique en phase solide (SPPS) sur résine 2-chlorotrityle chloride (CTC) (34,5 g, 1,16 mmol/g).

Le greffage de l'éthylène diamine (10,0 équivalents) est effectué dans le DCM (10V), en présence de DIPEA (10,0 équivalents). Les sites n'ayant pas réagi sont cappés au méthanol (0,8 mL/g résine) en fin de réaction.

Les couplages des acides aminés protégés Fmoc-Lys(Fmoc)-OH (1,5 équivalents), Fmoc-Glu(OMe)-OH (3,0 équivalents) et de la molécule 28 (3,0 équivalents) sont effectués dans un mélange DCM/DMF 1:1 (10V), en présence de HATU (1,0 équivalent par rapport à l'acide) et de DIPEA (2,0 équivalents par rapport à l'acide).

Les groupements protecteurs Fmoc sont retirés à l'aide d'une solution de DMF/pipéridine 80:20 (10 V) (après couplage de la lysine) ou une solution de morpholine à 50 % dans le DMF (après couplage des acides glutamiques).

Le produit est clivé de la résine à l'aide d'une solution de DCM/TFA 50:50 (10 V). Après évaporation, le résidu est solubilisé dans du MeTHF (450 mL) et la phase organique est lavée par une solution aqueuse saturée en NaHCO3 (3 x 450 mL) puis une solution aqueuse saturée en NaCl (200 mL). Après séchage sur Na2SO4, la phase organique est filtrée, concentrée sous pression réduite et le résidu est purifié par chromatographie sur gel de silice (dichlorométhane, méthanol, NH4OH).
Rendement : 13,95 g (31 % global sur 7 étapes).
RMN ¹H (DMSO-d₆, ppm) : 0,73-0,91 (24H) ; 0,96-2,41 (56H) ; 2,72 (2H) ; 2,89-3,10 (2H) ; 3,15-3,26 (2H) ; 3,26-3,51 (4H) ; 3,57 (3H) ; 3,58 (3H) ; 3,99-4,50 (5H) ; 6,07 (2H) ; 7,59-8,39 (5H).
LC/MS (ESI+) : 1118,2 (calculé ([M+H]⁺) : 1117,8).

### Exemple A13 : Molécule A13

### Molécule 29 : Produit obtenu par polymérisation du γ-benzyl-L-glutamate N-carboxyanhydride initiée par la N-Boc-ethylènediamine.

Dans un réacteur, du γ-benzyl-L-glutamate N-carboxyanhydride (39,44 g, 149,82 mmol) est solubilisé dans du DMF (81 mL) à 25 °C. Le mélange est alors agité jusqu'à complète dissolution, refroidi à -10 °C, puis une solution de BocEDA (6,00 g, 37,45 mmol) dans le DMF (7 mL) est introduite rapidement. Le milieu réactionnel est agité à 0 °C pendant 3 h puis une solution de HCl dans le 1,4-dioxane (3,33 M, 11,8 mL, 39,29 mmol) est ajoutée. Le milieu réactionnel est agité à température ambiante puis coulé sur une solution MeOH/IPE (125 mL/495 mL) refroidie par un bain de glace. Après 65 h d'agitation à température ambiante, le précipité est filtré sur fritté, lavé par de l'IPE (2 x 90 mL) et séché à 30 °C sous pression réduite.
Rendement : 21,71 g (54 %)
DP (estimé d'après la RMN ¹H) : 4,9
La masse molaire moyenne calculée de la molécule 29 sous forme de sel de chlorhydrate est de 1270,9 g/mol.
RMN ¹H (DMSO-d6, ppm) : 1,35 (9H) ; 1,72-2,09 (9,8H) ; 2,23-2,60 (9,8H) ; 2,86-3,19 (4H) ; 3,85 (1H) ; 4,14-4,52 (3,9H) ; 4,86-5,23 (9,8H) ; 6,33-6,85 (1H) ; 7,09-7,55 (24,5H) ; 7,88-8,42 (6,9H) ; 8,67 (1H).

### Molécule 30 : Produit obtenu entre le couplage du chlorure de myristoyle et la molécule 29.

Après solubilisation de la molécule 29 sous forme de sel de chlorhydrate (12,46 g, 9,80 mmol) dans du DCM (115 mL), la solution est refroidie à 0 °C. Puis sont ajoutés successivement de la triéthylamine (2,35 g, 23,24 mmol) et une solution de chlorure de myristoyle (3,16 g, 12,79 mmol) dans le DCM (16 mL). Le milieu réactionnel est agité à 0 °C pendant 4 h puis à température pendant 2 h avant d'être coulé sur de l'IPE (920 mL). Après 14 h d'agitation à température ambiante, le précipité est filtré, lavé par de l'EtOH (2 x 145 mL puis 100 mL) et séché à 30 °C sous pression réduite.
Rendement : 9,77 g (69 %)
DP (estimé d'après la RMN ¹H) : 5,1
La masse molaire moyenne calculée de la molécule 30 est de 1488,7 g/mol.
RMN ¹H (CDCl₃, ppm) : 0,87 (3H) ; 1,07-1,51 (29H) ; 1,51-1,64 (2H) ; 1,80-2,75 (22,4H) ; 2,98-3,73 (4H) ; 3,84-4,50 (5,1H) ; 4,86-5,32 (10,2H) ; 5,71-6,47 (1H) ; 6,72-8,38 (31,6H).

### Molécule A13

À une solution de la molécule 30 (4,70 g, 3,16 mmol) dans du DCM (31 mL) à 0 °C est ajouté du TFA (31 mL). Le milieu réactionnel est agité à 0 °C pendant 2 h puis concentré sous pression réduite et à température ambiante. Le résidu est repris dans du DCM (100 mL) puis concentré à sec sous pression réduite et à température ambiante. Le résidu est solubilisé dans du DCM (100 mL) et lavé par une solution aqueuse de tampon carbonate à pH = 10,4 (326 mL puis 2 x 200 mL) puis par une solution aqueuse de HCl (0,1 N, 2 x 200 mL). La solution organique est séchée sur Na₂SO₄, filtrée puis concentrée à sec à 40 °C sous pression réduite.
Rendement : 3,96 g (88 %)
DP (estimé d'après la RMN ¹H) : 5,2
La masse molaire moyenne calculée de la molécule A13 sous forme de sel de chlorhydrate est de 1446,9 g/mol.
RMN ¹H (TFA-d, ppm) : 0,91 (3H) ; 1,17-1,47 (20H) ; 1,60-1,74 (2H) ; 1,99-2,78 (22,8H) ; 3,41-4,05 (4H) ; 4,62-4,83 (5,2H) ; 5,05-5,35 (10,4H) ; 6,99-8,02 (26H).

### Exemple A14 : Molécule A14

### Molécule 31 : Produit obtenu par la réaction entre la molécule 14 et la Boc-éthylènediamine.

Par un procédé similaire à celui utilisé pour la préparation de la molécule 10 appliqué à la molécule 14 (12,00 g, 40,35 mmol) et à la BocEDA (7,76 g, 48,42 mmol), une huile incolore de la molécule 31 est obtenue et utilisée sans autre purification.
Rendement : 17,40 g (94 %)
RMN ¹H (CDCl₃, ppm) : 0,86 (3H) ; 1,11-1,68 (18H) ; 1,41 (9H) ; 1,80-2,38 (6H) ; 3,06-3,35 (4H) ; 3,37-3,49 (1H) ; 3,51-3,73 (1H) ; 4,26-4,31 (0,1H) ; 4,45-4,52 (0,9H) ; 4,91-5,19 (1H) ; 6,97 (0,1H) ; 7,23 (0,9H).
LC/MS (ESI+) : 440,4 (calculé ([M+H]⁺) : 440,3).

### Molécule A14

Après un procédé similaire à celui utilisé pour la préparation de la molécule A2 appliqué à la molécule 31 (8,85 g, 20,13 mmol) en solution dans le DCM, un solide blanc de molécule A14 est obtenu après lavage basique, concentration sous pression réduite puis recristallisation dans l'acétonitrile.
Rendement : 6,53 g (96 %)
RMN ¹H (DMSO, ppm) : 0,85 (3H) ; 1,07-1,56 (20H) ; 1,68-2,03 (4H) ; 2,09-2,29 (2H) ; 2,50-2,58 (2H) ; 2,96-3,11 (2H) ; 3,21-3,59 (2H) ; 4,17-4,21 (0,65H) ; 4,25-4,29 (0,35H) ; 7,68 (0,65H) ; 8,00 (0,35H)
LC/MS (ESI) : 340,3 ; (calculé ([M+H]⁺) : 340,3).
,3).

### Molécule A15

La molécule A15 est obtenue par la méthode conventionnelle de synthèse peptidique en phase solide (SPPS) sur résine 2-chlorotrityle chloride (CTC) (16,0 g, 1,16 mmol/g).

Le greffage de l'éthylène diamine (20,0 équivalents) est effectué dans le DCM (10V). Les sites n'ayant pas réagi sont cappés au méthanol (0,8 mL/g résine) en fin de réaction.

Les couplages des acides aminés protégés Fmoc-Lys(Fmoc)-OH (3,0 équivalents), Fmoc-Glu(OBn)-OH (4,0 équivalents) et de la molécule 11 (3,0 équivalents) sont effectués dans le DMF (10V) (couplages Lys et molécule 11) ou un mélange DCM/DMF 1:1 (10V) (couplage Glu), en présence de HATU (1,0 équivalent par rapport à l'acide) et de DIPEA (1,5 équivalents par rapport à l'acide).

Les groupements protecteurs Fmoc sont retirés à l'aide d'une solution de DMF/pipéridine 80:20 (10 V) (après couplage de la lysine) ou une solution de DBU à 1 % dans le DMF (après couplage des acides glutamiques)

Le produit est clivé de la résine à l'aide d'une solution de DCM/TFA 50:50 (10 V). Après évaporation, le résidu est solubilisé dans de l'acétate d'éthyle (400 mL) et la phase organique est lavée par une solution aqueuse de tampon carbonate à pH 10 (1 M) (2 x 400 mL) puis une solution aqueuse saturée en NaCl (400 mL). Après séchage sur Na₂SO₄, la phase organique est filtrée, concentrée sous pression réduite et le résidu est purifié par chromatographie sur gel de silice (dichlorométhane, méthanol, NH₄OH), puis par une recristallisation dans l'acétonitrile.
Rendement : 16,20 g (70 % global sur 7 étapes).
RMN ¹H (DMSO-d₆, ppm) : 0,85 (6H) ; 1,11-2,57 (72H) ; 2,50-5,57 (2H) ; 2,90-3,08 (4H) ; 3,36-3,61 (4H) ; 4,06-4,43 (5H) ; 5,08 (4H) ; 7,27-7,40 (10H) ; 7,51-8,31 (5H).
LC/MS (ESI+) : 1242,0 (calculé ([M+H]⁺) : 1241,9).

### Exemple A16 : Molécule A16

### Molécule 32 : Produit obtenu par SPPS

La molécule 32 est obtenue par la méthode conventionnelle de synthèse peptidique en phase solide (SPPS) sur résine 2-chlorotrityle chloride (CTC) (50,0 g, 1,14 mmol/g).

Le greffage du premier acide aminé Fmoc-Glu(OtBu)-OH (1,3 équivalents) est effectué dans le DCM (10V), en présence de DIPEA (2,6 équivalents). Les sites n'ayant pas réagi sont cappés au méthanol (0,8 mL/g résine) en fin de réaction.

Les couplages de l'acide aminé protégé Fmoc-Glu(OtBu)-OH (1,3 équivalents) et de la molécule 11 (3,0 équivalents) sont effectués dans le DMF (10V), en présence de HATU (1,0 équivalent par rapport à l'acide) et de DIPEA (1,5 équivalents par rapport à l'acide).

Les groupements protecteurs Fmoc sont retirés à l'aide d'une solution de DMF/pipéridine 80:20 (10 V).

Le produit est clivé de la résine à l'aide d'une solution de DCM/HFIP 80:20 (10 V).
Après concentration sous pression réduite, le résidu est purifié par trituration dans le diisopropyléther.
Rendement : 35,78 g (90 %)
RMN ¹H (CDCl₃, ppm) : 0,88 (3H) ; 1,19-1,35 (20H) ; 1,43 (9H) ; 1,44 (9H) ; 1,55-1,67 (2H) ; 1,90-2,46 (14H) ; 3,46-3,54 (1H) ; 3,63-3,71 (1H) ; 4,33-4,40 (1H) ; 4,43-4,52 (2H) ; 7,35 (0,05H) ; 7,40 (0,05H) ; 7,63 (0,95H) ; 7,94 (0,95H).
LC/MS (ESI+) : 696,4 (calculé ([M+H]⁺) : 696,5).

### Molécule 33 : Produit obtenu par la réaction entre la molécule 32 et la N-CBz éthylènediamine.

Par un procédé similaire à celui utilisé pour la préparation de la molécule 7 et appliqué à la molécule 32 (30,0 g, 43,11 mmol) et au chlorhydrate de N-CBz éthylènediamine (CBzEDA•HCl, 11,93 g, 51,73 mmol), et en présence de DIPEA (15,0 mL, 86,22 mmol), un solide beige de la molécule 33 est obtenu. Il est utilisé sans purification supplémentaire.
Rendement : 37,6 g (100 %)
RMN ¹H (CDCl₃, ppm) : 0,88 (3H) ; 1,19-1,34 (20H) ; 1,42 (9H) ; 1,44 (9H) ; 1,52-2,54 (16H) ; 3,16-3,70 (6H) ; 4,08-4,15 (1H) ; 4,19-4,25 (1H) ; 4,43-4,53 (1H) ; 5,00 (1H) ; 5,08 (1H) ; 6,56 (1H) ; 7,00 (1H) ; 7,24-7,37 (5H) ; 7,59 (1H) ; 8,41 (1H).
LC/MS (ESI+) : 872,5 (calculé ([M+H]⁺) : 872,6).

### Molécule A16

À une solution de molécule 33 (37,6 g, 43,11 mmol) dans du méthanol (376 mL) est ajouté du Pd/Al₂O₃ (3,76 g) sous atmosphère d'argon. Le mélange est placé sous atmosphère d'hydrogène (7 bar) et agité à température ambiante pendant 72 h. Après filtration du catalyseur sur fritté P4 puis sur une membrane Omnipore 0,2 µm PTFE hydrophile, le filtrat est évaporé sous pression réduite pour donner la molécule A16 sous forme d'une huile collante.
Rendement : 31,06 g (98 %)
RMN ¹H (CDCl₃, ppm) : 0,88 (3H) ; 1,19-1,35 (20H) ; 1,43 (9H) ; 1,46 (9H) ; 1,56-1,67 (2H) ; 1,92-2,12 (6H) ; 2,24-2,54 (8H) ; 2,71 (2H) ; 2,90 (2H) ; 3,22-3,32 (1H) ; 3,42-3,51 (1H) ; 3,55-3,64 (1H) ; 3,73-3,81 (1H) ; 4,13-4,21 (1H) ; 4,26-4,33 (1H) ; 4,39-4,48 (1H) ; 7,10 (1H) ; 7,71 (1H) ; 8,45 (1H).
LC/MS (ESI+) : 738,5 (calculé ([M+H]⁺) : 738,5).

### Molécule A17

La molécule A17 est obtenue par la méthode conventionnelle de synthèse peptidique en phase solide (SPPS) sur résine 2-chlorotrityle chloride (CTC) (64,66 g, 1,16 mmol/g).

Le greffage de l'éthylène diamine (10,0 équivalents) est effectué dans le DCM (10V), en présence de DIPEA (10,0 équivalents). Les sites n'ayant pas réagi sont cappés au méthanol (0,8 mL/g résine) en fin de réaction.

Les couplages de l'acide aminé protégé Fmoc-Glu(OMe)-OH (1,5 équivalents) et de la molécule 28 (1,5 équivalents) sont effectués dans un mélange DCM/DMF 1:1 (10V) pour le couplage de l'acide glutamique ou dans du DMF (10V) pour le couplage de la molécule 28, en présence de HATU (1,0 équivalent par rapport à l'acide) et de DIPEA (2,0 équivalents par rapport à l'acide).

Les groupements protecteurs Fmoc sont retirés à l'aide d'une solution de DMF/morpholine 50:50 (10 V).

Le produit est clivé de la résine à l'aide d'une solution de DCM/TFA 50:50 (10 V). Après évaporation, le résidu est solubilisé dans du MeTHF (500 mL) et la phase organique est lavée par une solution aqueuse de Na₂CO₃ à 5 % (3 x 250 mL) puis les phases aqueuses sont extraites au MeTHF (1 x 150 mL). Les phases organiques réunies sont séchées sur Na₂SO₄ et filtrées. Une solution de HCl dans MeOH (1,25 M) est ajoutée puis le milieu est concentré sous pression réduite. Le résidu est purifié par chromatographie sur gel de silice (dichlorométhane, méthanol) pour donner le sel de chlorhydrate de la molécule A17 sous la forme d'un solide brun clair.
Rendement : 12,48 g (30 % global sur 5 étapes).
RMN ¹H (DMSO-d₆, ppm) : 0,76-0,90 (12H) ; 0,97-1,41 (13H) ; 1,45-1,55 (1H) ; 1,68-2,40 (11H) ; 2,77-2,92 (2H) ; 3,20-3,64 (4H) ; 3,57 (3H) ; 4,15-4,49 (2H) ; 7,90-8,48 (5H).
LC/MS (ESI+) : 525,5 (calculé ([M+H]⁺) : 525,4).

### Exemple A18 : Molécule A18

### Molécule 34 : Produit obtenu par hydrogénation du phytol.

À une solution de phytol (260,00 g, 878,78 mmol) dans l'éthanol (1,25 L) sous argon est ajouté du Nickel de Raney à 50 % dans l'eau (30,75 g, 175,36 mmol). Le milieu est placé sous 1 bar de dihydrogène puis agité pendant 8 jours à température ambiante. Après filtration sur un pad de célite/silice/célite en rinçant à l'éthanol, une huile incolore de molécule 34 est obtenue après concentration sous pression réduite.
Rendement : 261,40 g (quant.)
RMN ¹H (CDCl₃, ppm) : 0,84 (6H) ; 0,86 (6H) ; 0,89 (3H) ; 1,00-1,46 (22H) ; 1,46-1,68 (3H) ; 3,61-3,73 (2H).

### Molécule 35 : Produit obtenu par oxydation de la molécule 34.

Par un procédé similaire à celui utilisé pour la préparation de la molécule 26 appliqué à la molécule 34 (29,00 g, 97,13 mmol), une huile jaune de la molécule 35 est obtenue.
Rendement : 28,70 g (94 %)
RMN ¹H (CDCl₃, ppm) : 0,84 (6H) ; 0,86 (6H) ; 0,97 (3H) ; 1,00-1,41 (20H) ; 1,52 (1H) ; 1,96 (1H) ; 2,14 (1H) ; 2,35 (1H) ; 11,31 (1H).
LC/MS (ESI) : 311,1 (calculé ([M-H]⁻) : 311,3).

### Molécule 36 : Produit obtenu par couplage entre la molécule 35 et la L-prolinate de méthyle.

Par un procédé similaire à celui utilisé pour la préparation de la molécule 27 appliqué à la molécule 35 (18,00 g, 57,59 mmol) et au chlorhydrate de L-prolinate de méthyle (14,31 g, 86,39 mmol), une huile jaune de la molécule 36 est obtenue.
Rendement : 23,20 g (95 %)
RMN ¹H (DMSO-d₆, ppm) : 0,78-0,89 (15H) ; 0,97-1,43 (20H) ; 1,43-1,56 (1H) ; 1,70-1,96 (4H) ; 1,96-2,32 (3H) ; 3,33-3,56 (2H) ; 3,59 (0,6H) ; 3,67 (2,4H) ; 4,27 (0,8H) ; 4,57 (0,2H).
LC/MS (ESI) : 424,4 (calculé ([M+H]⁺) : 424,4).

### Molécule 37 : Produit obtenu par la saponification de la molécule 36.

Par un procédé similaire à celui utilisé pour la préparation de la molécule 28 appliqué à la molécule 36 (21,05 g, 49,68 mmol), une huile jaune de la molécule 37 est obtenue.
Rendement : 20,40 g (99 %)
RMN ¹H (DMSO-d₆, ppm) : 0,77-0,91 (15H) ; 0,97-1,43 (20H) ; 1,43-1,56 (1H) ; 1,67-1,96 (4H) ; 1,96-2,29 (3H) ; 3,26-3,56 (2H) ; 4,20 (0,8H) ; 4,41 (0,2H).
LC/MS (ESI) : 410,3 (calculé ([M+H]⁺): 410,4).

### Molécule A18

La molécule A18 est obtenue par la méthode conventionnelle de synthèse peptidique en phase solide (SPPS) sur résine 2-chlorotrityle chloride (CTC) (26,72 g, 1,16 mmol/g).

Par un procédé similaire à celui utilisé pour la préparation de la molécule A17 appliqué au 4,7,10-trioxa-1,13-tridecanediamine (TOTA, 68,30 g, 310,0 mmol), au Fmoc-Glu(OMe)-OH (23,77 mmol, 62,00 mmol) et à la molécule 37 (19,04 g, 46,50 mmol), une huile jaune de molécule A18 sous forme de chlorhydrate est obtenue.
Rendement : 5,53 g (23 % global sur 5 étapes).
RMN ¹H (DMSO-d₆, ppm) : 0,76-0,89 (15H) ; 0,97-2,38 (36H) ; 2,77-2,87 (2H) ; 3,00-3,17 (3H) ; 3,32-3,54 (13H) ; 3,57 (3H) ; 4,09-4,18 (0,75H) ; 4,20-4,29 (1H) ; 4,39-4,47 (0,25H) ; 7,63-8,36 (5H).
LC/MS (ESI+) : 755,7 (calculé ([M+H]⁺) : 755,6).

### Exemple A19 : Molécule A19

La molécule A19 est synthétisée de la même manière que la molécule A16 en utilisant la molécule 14 à la place de la molécule 11 lors de l'étape de SPPS. Rendement global (3 étapes) : 32,6 g (81 %)
RMN ¹H (CDCl₃, ppm) : 0,88 (3H) ; 1,20-1,35 (16H) ; 1,43 (9H) ; 1,46 (9H) ; 1,56-1,68 (2H) ; 1,93-2,11 (6H) ; 2,24-2,55 (10H) ; 2,85 (2H) ; 3,19-3,29 (1H) ; 3,38-3,48 (1H) ; 3,55-3,64 (1H) ; 3,74-3,82 (1H) ; 4,14-4,21 (1H) ; 4,25-4,32 (1H) ; 4,41-4,50 (1H) ; 7,03 (1H) ; 7,69 (1H) ; 8,42 (1H).
LC/MS (ESI) : 710,4 (calculé ([M+H]⁺): 710,5).

### Exemple A20 : Molécule A20

La molécule A20 est obtenue par la méthode conventionnelle de synthèse peptidique en phase solide (SPPS) sur résine 2-chlorotrityle chloride (CTC) (40,00 g, 1,16 mmol/g).

Le greffage de l'éthylène diamine (20,0 équivalents) est effectué dans le DCM (10V). Les sites n'ayant pas réagi sont cappés au méthanol (0,8 mL/g résine) en fin de réaction.

Les couplages des acides aminés protégés Fmoc-Lys(Fmoc)-OH (1,5 équivalents), Fmoc-Glu(OtBu)-OH (2,5 équivalents) et de la molécule 11 (2,5 équivalents) sont effectués dans le DMF (10 V), en présence de HATU (1,0 équivalent par rapport à l'acide) et de DIPEA (1,5 équivalents par rapport à l'acide).

Les groupements protecteurs Fmoc sont retirés à l'aide d'une solution de DMF/piperidine 80:20 (10 V).

Le produit est clivé de la résine à l'aide d'une solution de DCM/TFA 50:50 (10 V). Après évaporation, le résidu est solubilisé dans de l'eau (600 mL), le pH de la solution est ajusté à 7 par ajout d'une solution de NaOH 5 N, puis le produit est lyophilisé. Le lyophilisat est purifié par colonne de chromatographie sur gel de silice (dichlorométhane, méthanol, NH₄OH) pour donner la molécule A20 sous la forme d'un solide blanc.
Rendement : 24,6 g (50 % global sur 7 étapes).
RMN ¹H (MeOD-d4, ppm) : 0,90 (6H) ; 1,18-2,45 (68H) ; 2,45-2,60 (2H) ; 3,05-3,11 (2H) ; 3,11-3,19 (1H) ; 3,23-3,33 (1H) ; 3,43-3,66 (4H) ; 3,82-3,94 (2H) ; 4,10-4,51 (5H).
LC/MS (ESI+) : 1061,9 (calculé ([M+H]⁺) : 1061,8).

### Partie B - Synthèse des co-polvaminoacides hydrophobes

### i) Co-polyaminoacides de formule XXXa, XXXb et XXXb', XXXb"

| **N°** | **CO-POLYAMINOACIDES PORTEUR DE CHARGES CARBOXYLATES ET DE RADICAUX HYDROPHOBE** |
|---|---|
| B1 | 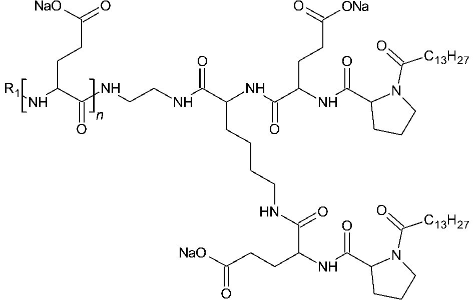 |
| | i = 0,038, DP = 26 |
| | R₁ = H ou pyroglutamate |
| B2 | 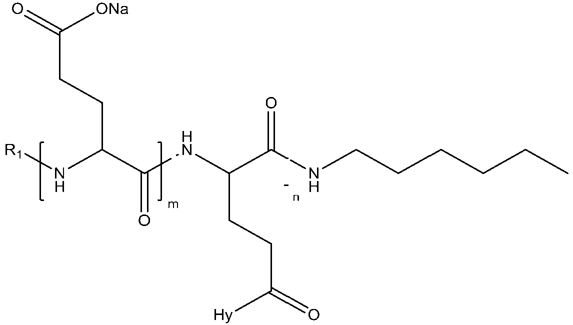 |
| | i= 0,15, DP (m + n) = 40 |
| | Hy = |
| | 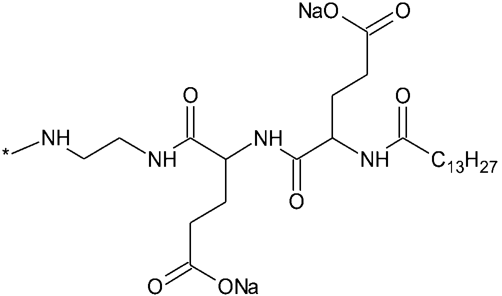 |
| | R₁ = H ou pyroglutamate |
| B3 | 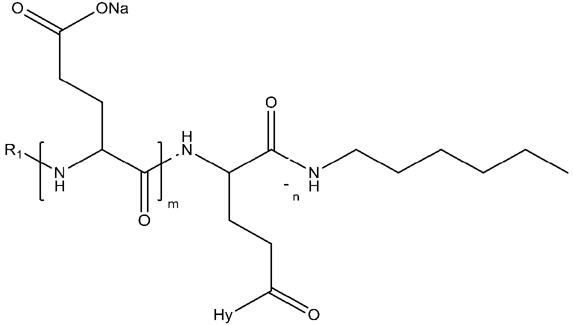 |
| | i= 0,15, DP (m + n) = 40 |
| | Hy = |
| | 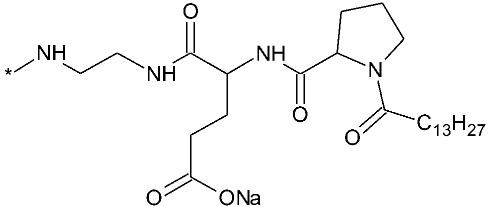 |
| | R₁ = H ou pyroglutamate |
| B4 | 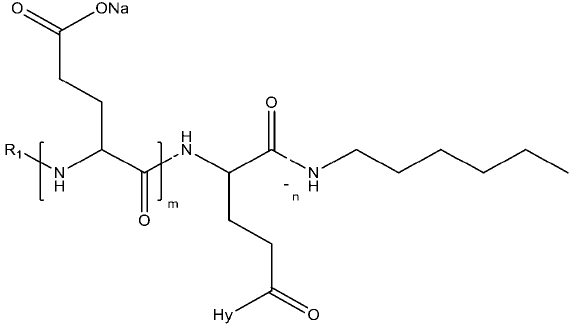 |
| | i= 0,15, DP (m + n) = 40 |
| | Hy = |
| | 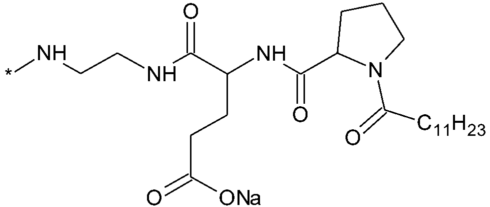 |
| | R₁ = H ou pyroglutamate |
| B5 | 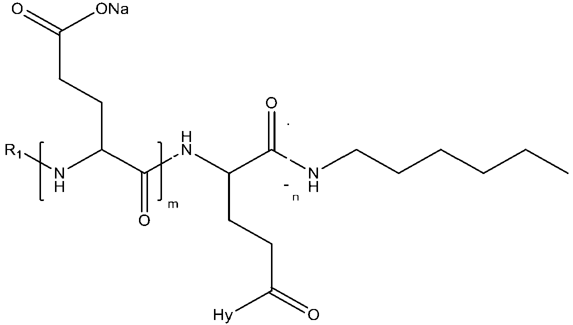 |
| | i= 0,15, DP (m + n) = 40 |
| | Hy = |
| | 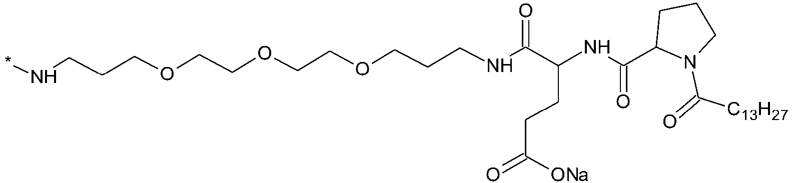 |
| | R₁ = H ou pyroglutamate |
| B7 | 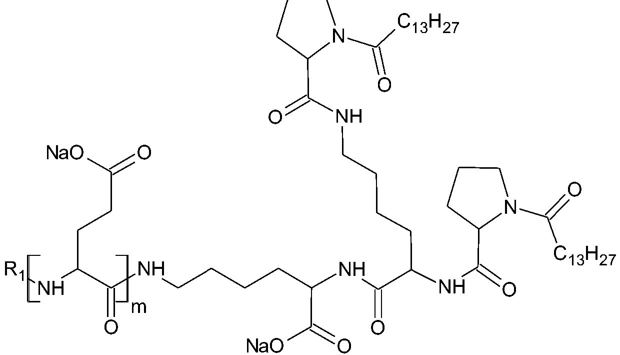 |
| | i = 0,038, DP = 26 |
| | R₁ = H ou pyroglutamate |
| B13 | 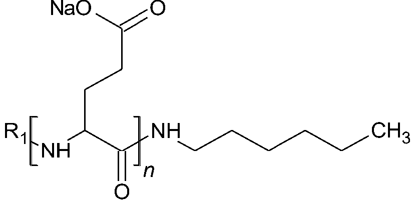 |
| | i = 0,042, DP = 24 |
| | R₁ = |
| | 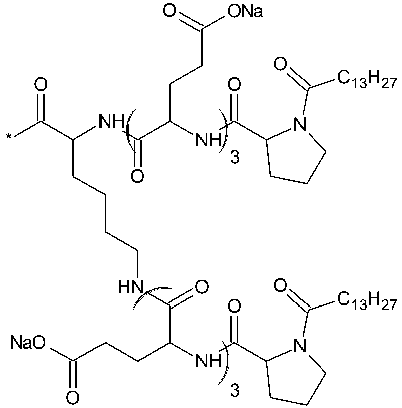 |
| B14 | 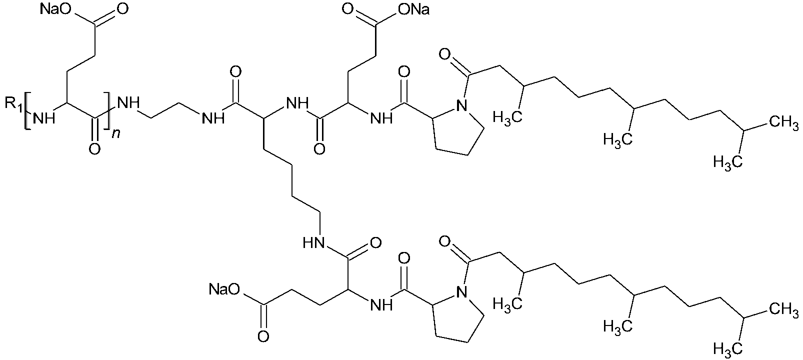 |
| | i = 0,042, DP = 24 |
| | R₁ = H ou pyroglutamate |
| B15 | 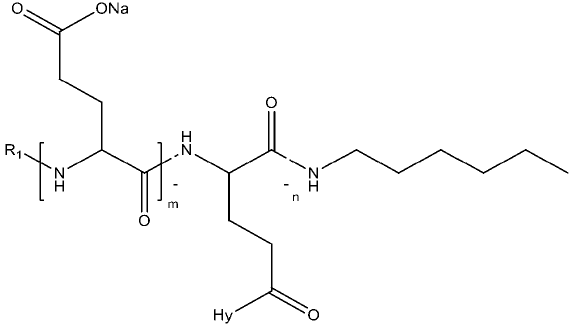 |
| | i= 0,15, DP (m + n) = 40 |
| | Hy = |
| | 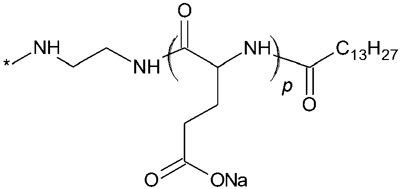 |
| | DP (p) = 5,2 |
| | R₁ = H ou pyroglutamate |
| B17 | 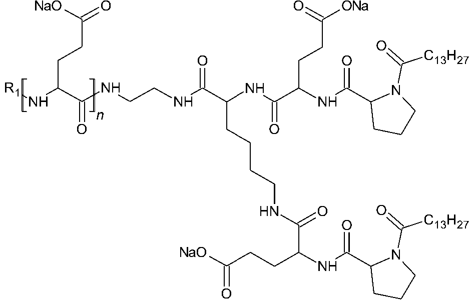 |
| | i = 0,1, DP = 10 |
| | R₁ = H ou pyroglutamate |
| B18 | 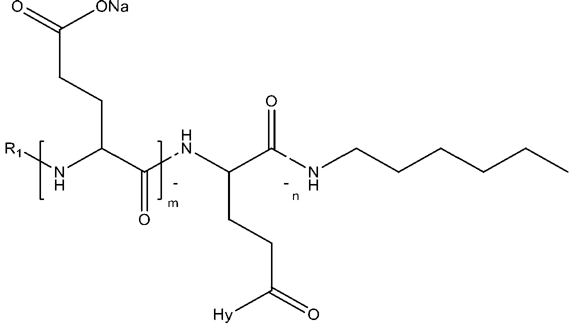 |
| | i= 0,15, DP (m + n) = 40 |
| | Hy = |
| | 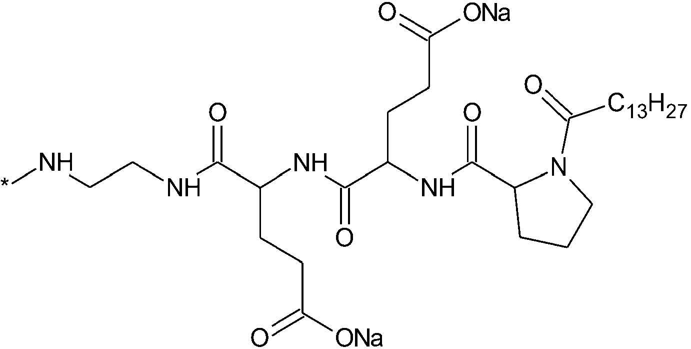 |
| | R₁ = H ou pyroglutamate |
| B19 | 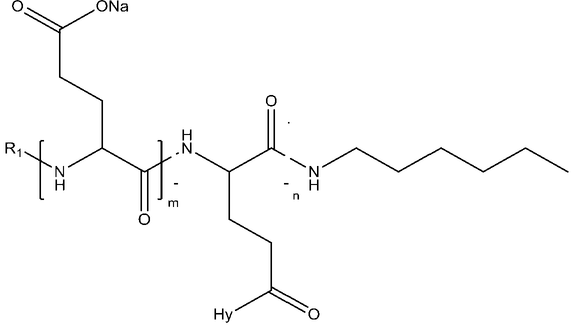 |
| | i= 0,15, DP (m + n) = 40 |
| | Hy = |
| | 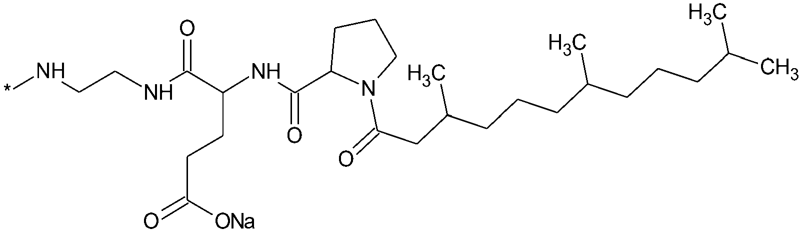 |
| | R₁ = H ou pyroglutamate |
| B20 | 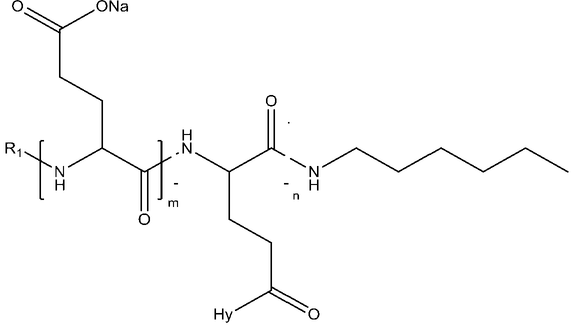 |
| | i= 0,15, DP (m + n) = 40 |
| | Hy = |
| | 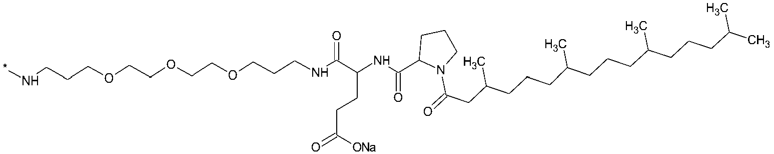 |
| | R₁ = H ou pyroglutamate |
| B21 | 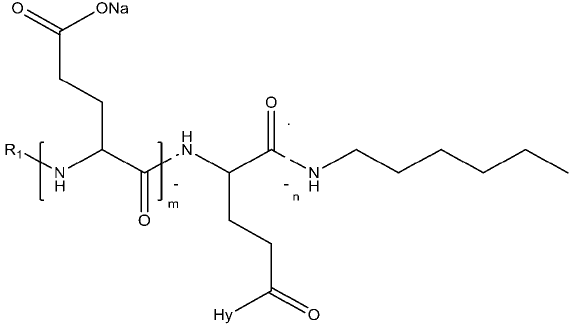 |
| | i= 0,15, DP (m + n) = 40 |
| | Hy = |
| | 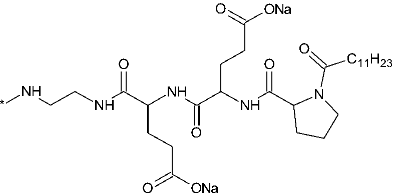 |
| | R₁ = H ou pyroglutamate |
| B22 | 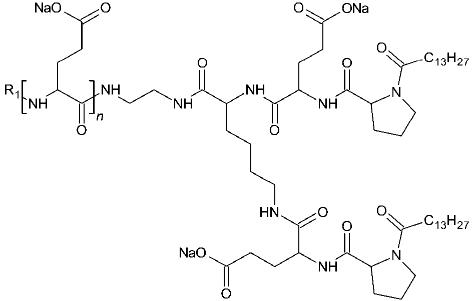 |
| | i = 0,05, DP = 20 |
| | R₁ = H ou pyroglutamate |

### Co-polyaminoacide B1 : poly-L-glutamate de sodium modifié à une de ses extrémités par la molécule A1 et ayant une masse molaire moyenne en nombre (Mn) de 2800 g/mol

Dans un ballon préalablement séché à l'étuve, du γ-benzyl-L-glutamate N-carboxyanhydride (8,95 g, 34 mmol) est solubilisé dans du DMF anhydre (34 mL). Le mélange est refroidi à 4 °C, puis une solution de molécule A1 (1,64 g, 1,55 mmol) dans le chloroforme (6,6 mL) est introduite rapidement. Le mélange est agité entre 4 °C et température ambiante pendant 68 h, puis chauffé à 65 °C pendant 2 h. La moitié du solvant est distillé sous pression réduite puis le milieu réactionnel est refroidi à température ambiante et versé goutte à goutte dans du diisopropyléther (300 mL) sous agitation. Le précipité blanc est récupéré par filtration, lavé avec du diisopropyléther (5 x 50 mL) puis séché sous pression réduite à 30 °C pour obtenir un solide blanc. Le solide (7,9 g) est dilué dans du TFA (30 mL), et une solution d'acide bromhydrique (HBr) à 33 % dans de l'acide acétique (21 mL, 120 mmol) est alors ajoutée au goutte à goutte à 0 °C. La solution est agitée pendant 2 h à température ambiante puis est coulée goutte à goutte sur un mélange 1:1 (v/v) de diisopropyléther/eau sous agitation (360 mL). Après 2 h d'agitation, le mélange hétérogène est laissé au repos pendant une nuit. Le précipité blanc est récupéré par filtration, lavé successivement avec de l'IPE (2 x 30 mL) puis avec de l'eau (2 x 30 mL). Le solide obtenu est solubilisé dans de l'eau (200 mL) en ajustant le pH à 7 par ajout d'une solution aqueuse de soude 1 N. De l'eau (65 mL) est ajoutée. Le mélange est filtré sur filtre 0,45 µm puis purifié par ultrafiltration contre une solution de NaCl 0,9 % puis de l'eau jusqu'à ce que la conductimétrie du perméat soit inférieure à 50 µS/cm. La solution de co-polyaminoacide est ensuite concentrée à environ 25 g/L théorique, le pH est ajusté à 7 et la solution aqueuse est filtrée sur 0,2 µm. Cette solution est diluée avec de l'eau et de l'acétone afin d'obtenir une solution à 12 g/L contenant 30 % massique d'acétone, puis elle est filtrée sur filtre de charbon actif (3M R53SLP). L'acétone est distillée (40 °C, 100 mbar) et la solution est purifiée par ultrafiltration contre une solution de NaCl 0,9 % puis de l'eau jusqu'à ce que la conductimétrie du perméat soit inférieure à 50 µS/cm. La solution de co-polyaminoacide est ensuite concentrée et le pH est ajusté à 7. La solution aqueuse est filtrée sur 0,2 µm et conservée à 4 °C.
Extrait sec : 17,8 mg/g
DP (estimé d'après la RMN ¹H) : 26
D'après la RMN ¹H : i = 0,038
La masse molaire moyenne calculée du co-polyaminoacide B1 est de 4994 g/mol.
HPLC-SEC organique (calibrant PEG) : Mn = 2800 g/mol

### Co-polyaminoacide B2 : poly-L-glutamate de sodium modifié par la molécule A2 dont les esters sont saponifiés et ayant une masse molaire moyenne en nombre (Mn) de 5200 g/mol

### Co-polyaminoacide B2-1 : acide poly-L-glutamique issu de la polymérisation du γ-benzyl-L-glutamate N-carboxyanhydride initiée par l'hexylamine

Dans un réacteur à double enveloppe, du γ-benzyl-L-glutamate N-carboxyanhydride (500 g, 1,90 mol) est solubilisé dans du DMF anhydre (1100 mL). Le mélange est alors agité jusqu'à complète dissolution, refroidi à 0 °C, puis de l'hexylamine (6,27 mL, 47,5 mmol) est introduite rapidement. Le mélange est agité à 0 °C pendant 5 h, entre 0 °C et 20 °C pendant 7 h, puis à 20 °C pendant 7 h. Le milieu réactionnel est ensuite chauffé à 65 °C pendant 2 h, refroidi à 55 °C et du méthanol (3300 mL) est introduit en 1 h 30. Le mélange réactionnel est alors refroidi à 0 °C et laissé sous agitation pendant 18 h. Le précipité blanc est récupéré par filtration, lavé au diisopropyléther (2 x 800 mL) puis séché sous pression réduite à 30 °C pour donner un acide poly(gamma-benzyl-L-glutamique) (PBLG).

A une solution de PBLG (180 g) dans du N,N-diméthylacétamide (DMAc, 450 mL) est ajouté du Pd/Al₂O₃ (36 g) sous atmosphère d'argon. Le mélange est placé sous atmosphère d'hydrogène (10 bar) et agité à 60 °C pendant 24 h. Après refroidissement à température ambiante et filtration du catalyseur sur fritté P4 puis sur membrane Omnipore 0,2 µm PTFE hydrophile, une solution d'eau à pH 2 (2700 mL) est coulée goutte à goutte sur la solution de DMAc, sur une période de 45 min et sous agitation. Après 18 h sous agitation, le précipité blanc est récupéré par filtration, lavé avec de l'eau (4 x 225 mL) puis séché sous pression réduite à 30 °C

### Co-polyaminoacide B2

Le co-polyaminoacide B2-1 (15,0 g) est solubilisé dans du DMF (230 mL) à 40 °C puis de la N-méthylmorpholine (NMM, 11,57 g, 114,4 mmol) est ajoutée. En parallèle, la molécule A2 sous forme de sel de chlorhydrate (10,17 g, 17,2 mmol) est mise en suspension dans du DMF (250 mL) et de la triéthylamine (2,39 mL, 17,2 mmol) est ajoutée, puis le mélange est légèrement chauffé sous agitation jusqu'à complète dissolution. À la solution de co-polyaminoacide, refroidie à 25 °C, sont successivement ajoutés la solution de molécule A2, de la N-oxyde de 2-hydroxypyridine (HOPO, 3,81 g, 34,3 mmol) puis du EDC (6,58 g, 34,3 mmol). Le milieu réactionnel est agité à 25 °C pendant 2 h, filtré sur filtre tissé 0,2 mm et coulé au goutte-à-goutte sur 2,6 L d'eau contenant du NaCl à 15 % massique et du HCl (pH 2) sous agitation. A la fin de l'ajout, le pH est réajusté à 2 avec une solution de HCl 1 N, et la suspension est laissée reposer une nuit. Le précipité est collecté par filtration, puis rincé par 2 x 100 mL d'eau. Le solide blanc obtenu est solubilisé dans 1,2 L d'eau par ajout lent d'une solution aqueuse de NaOH 1 N jusqu'à pH 7 sous agitation, puis la solution est filtrée sur filtre 0,45 µm. De l'éthanol (30 % massique) est ajouté puis la solution est filtrée sur filtre de charbon actif (3M R53SLP). Une solution de NaOH 10 N est lentement ajoutée sous agitation jusqu'à pH 13 puis le mélange est laissé sous agitation pendant 2 h. Après neutralisation à pH 7 par ajout d'une solution de HCl 37 %, la solution limpide obtenue est purifiée par ultrafiltration contre une solution de NaCl 0,9 % puis de l'eau, jusqu'à ce que la conductimétrie du perméat soit inférieure à 50 µS/cm. La solution de co-polyaminoacide est ensuite concentrée et le pH est ajusté à 7. La solution aqueuse est filtrée sur 0,2 µm et conservée à 4 °C.
Extrait sec : 22,6 mg/g
DP (estimé d'après la RMN ¹H) : 40
D'après la RMN ¹H : i = 0,15
La masse molaire moyenne calculée du co-polyaminoacide B2 est de 9301 g/mol.
HPLC-SEC organique (calibrant PEG) : Mn = 5200 g/mol.

### Co-polyaminoacide B3 : poly-L-glutamate de sodium modifié par la molécule A3 dont l'ester est saponifié et ayant une masse molaire moyenne en nombre (Mn) de 4900 g/mol

Le co-polyaminoacide B2-1 (12,0 g) est solubilisé dans du DMF (92 mL) à 40 °C puis de la N-méthylmorpholine (NMM, 9,25 g, 91,5 mmol) est ajoutée. En parallèle, une solution de la molécule A3 sous forme de sel de chlorhydrate (7,51 g, 13,7 mmol) et de N,N-diisopropylethylamine (DIPEA, 2,39 mL, 13,7 mmol) dans du DMF (27 mL) est préparée. À la solution de co-polyaminoacide refroidie à 25 °C, sont successivement ajoutés la solution de molécule A3 et de la N-oxyde de 2-hydroxypyridine (HOPO, 3,05 g, 27,4 mmol). Le mélange est refroidi à 0 °C puis du EDC (5,26 g, 27,4 mmol) est ajouté. Après 5 min à 0 °C, le milieu réactionnel est agité à 25 °C pendant 2 h, filtré sur filtre tissé 0,2 mm et coulé au goutte-à-goutte sur 950 mL d'eau contenant du NaCl à 15 % massique et du HCl (pH 2) sous agitation. A la fin de l'ajout, le pH est réajusté à 2 avec une solution de HCl 1 N, et la suspension est laissée reposer une nuit. Le précipité est collecté par filtration, puis rincé par 3 x 100 mL d'eau. Le solide obtenu est solubilisé dans 1 L d'eau par ajout lent d'une solution aqueuse de NaOH 1 N jusqu'à pH 7 sous agitation. Une fois la solubilisation complète, le pH est ajusté à pH 12 pendant 2 h puis à pH 13 pendant 1 h par ajout d'une solution de NaOH 10 N. Après neutralisation à pH 7 par ajout d'une solution de HCl 37 %, cette solution est diluée avec de l'eau et de l'éthanol afin d'obtenir une solution à 12 g/L contenant 30 % massique d'éthanol, puis elle est filtrée sur filtre de charbon actif (3M R53SLP). La solution obtenue est filtrée sur 0,45 µm et purifiée par ultrafiltration contre une solution de NaCl 0,9 % puis de l'eau jusqu'à ce que la conductimétrie du perméat soit inférieure à 50 µS/cm. La solution de co-polyaminoacide est ensuite concentrée et le pH est ajusté à 7. La solution aqueuse est filtrée sur 0,2 µm et conservée à 4 °C.
Extrait sec : 20,6 mg/g
DP (estimé d'après la RMN ¹H) : 40
D'après la RMN ¹H : i = 0,15
La masse molaire moyenne calculée du co-polyaminoacide B3 est de 8977 g/mol.
HPLC-SEC organique (calibrant PEG) : Mn = 4900 g/mol.

### Co-polyaminoacide B4 : poly-L-glutamate de sodium modifié par la molécule A4 dont l'ester est saponifié et ayant une masse molaire moyenne en nombre (Mn) de 4700 g/mol

Par un procédé similaire à celui utilisé pour la préparation du co-polyaminoacide B3 appliqué au sel de chlorhydrate de la molécule A4 (7,12 g, 13,7 mmol) et au co-polyaminoacide B2-1 (12,0 g), un poly-L-glutamate de sodium modifié par la molécule A4 dont l'ester est saponifié est obtenu.
Extrait sec : 19,4 mg/g
DP (estimé d'après la RMN ¹H) : 40
D'après la RMN ¹H : i = 0,15
La masse molaire moyenne calculée du co-polyaminoacide B4 est de 8809 g/mol.
HPLC-SEC organique (calibrant PEG) : Mn = 4700 g/mol.

### Co-polyaminoacide B5 : poly-L-glutamate de sodium modifié par la molécule A5 dont l'ester est saponifié et ayant une masse molaire moyenne en nombre (Mn) de 5400 g/mol

Par un procédé similaire à celui utilisé pour la préparation du co-polyaminoacide B3 appliqué au sel de chlorhydrate de la molécule A5 (9,71 g, 13,7 mmol) et au co-polyaminoacide B2-1 (12,0 g), un poly-L-glutamate de sodium modifié par la molécule A5 dont l'ester est saponifié est obtenu.
Extrait sec : 20,8 mg/g
DP (estimé d'après la RMN ¹H) : 40
D'après la RMN ¹H : i = 0,15
La masse molaire moyenne calculée du co-polyaminoacide B5 est de 9939 g/mol.
HPLC-SEC organique (calibrant PEG) : Mn = 5400 g/mol.

### Co-polyaminoacide B7 : poly-L-glutamate de sodium modifié à l'une de ses extrémités par la molécule A7 et ayant une masse molaire moyenne en nombre (Mn) de 2500 g/mol

Par un procédé similaire à celui utilisé pour la préparation du co-polyaminoacide B1 appliqué à la molécule A7 (2,50 g, 2,74 mmol) et à du γ-benzyl-L-glutamate N-carboxyanhydride (15,89 g, 60,4 mmol), un poly-L-glutamate de sodium modifié à l'une de ses extrémités par la molécule A7 est obtenu.
Extrait sec : 20,3 mg/g
DP (estimé d'après la RMN ¹H) : 26
D'après la RMN ¹H : i = 0,038
La masse molaire moyenne calculée du co-polyaminoacide B7 est de 3893 g/mol.
HPLC-SEC organique (calibrant PEG) : Mn = 2500 g/mol

### Co-polyaminoacide B13 : poly-L-glutamate de sodium modifié à l'une de ses extrémités par la molécule A11 dont les esters sont déprotégés et ayant une masse molaire en nombre (Mn) de 3000 g/mol

Dans un réacteur à double enveloppe, du γ-benzyl-L-glutamate N-carboxyanhydride (24,50 g, 93,05 mmol) est solubilisé dans du DMF anhydre (55 mL). Le mélange est alors agité jusqu'à complète dissolution, refroidi à 0 °C, puis de l'hexylamine (0,56 mL, 4,23 mmol) est introduite rapidement. Le mélange est agité à 0 °C pendant 48 h puis sont successivement ajoutés une solution de molécule A11 (9,51 g, 5,08 mmol) dans le DMF (50 mL), HOPO (564 mg, 5,08 mmol) et EDC (973 mg, 5,08 mmol). Le milieu réactionnel est agité à 0 °C pendant 1 h, entre 0 °C et 20 °C pendant 2 h, puis à 20 °C pendant 16 h. Cette solution est ensuite coulée dans un mélange H₂O/MeOH 1:1 (10 V) à température ambiante et sous agitation. Après 4 h sous agitation, le précipité blanc est récupéré par filtration, lavé avec du diisopropyl éther (2 x 100 mL), de l'eau (2 x 100 mL) et un mélange H₂O/MeOH 1:1 (2 x 100 mL) puis séché sous pression réduite.

Le solide obtenu est solubilisé dans du TFA (220 mL) et agité à température ambiante pendant 2 h 30. Cette solution est ensuite coulée dans de l'eau (10 V) à température ambiante et sous agitation. Après 2 h 30 sous agitation, le précipité blanc est récupéré par filtration, lavé avec de l'eau (2 x 200 mL) puis séché sous pression réduite.

Le solide obtenu est solubilisé dans du *N,N*-diméthylacétamide (DMAc, 210 mL) puis du Pd/Al₂O₃ (2,1 g) est ajouté sous atmosphère d'argon. Le mélange est placé sous atmosphère d'hydrogène (6 bar) et agité à 60 °C pendant 24 h. Après refroidissement à température ambiante et filtration du catalyseur sur fritté P4 puis sur membrane Omnipore 0,2 µm PTFE hydrophile, une solution d'eau à pH 2 contenant 15 % de NaCl (6 V) est coulée goutte-à-goutte sur la solution de DMAc, sur une période de 45 min et sous agitation. Après 18 h sous agitation, le précipité blanc est récupéré par filtration, lavé avec de l'eau puis séché sous pression réduite. Le solide obtenu est solubilisé dans de l'eau (600 mL) en ajustant le pH à 7 par ajout d'une solution aqueuse de soude 1 N. Le pH est ensuite ajusté à pH 12 et la solution est maintenue sous agitation pendant 1 h. Après neutralisation à pH 7, la solution est filtrée sur 0,2 µm, diluée avec de l'éthanol afin d'obtenir une solution contenant 30 % massique d'éthanol, puis filtrée sur filtre de charbon actif (3M R53SLP). La solution obtenue est filtrée sur 0,45 µm et purifiée par ultrafiltration contre une solution de NaCl 0,9 % puis de l'eau jusqu'à ce que la conductimétrie du perméat soit inférieure à 50 µS/cm. La solution de co-polyaminoacide est ensuite concentrée et le pH est ajusté à 7. La solution aqueuse est filtrée sur 0,2 µm et conservée à 4 °C.
Extrait sec : 23,5 mg/g
DP (estimé par RMN ¹H) = 24 donc i = 0,042
La masse molaire moyenne calculée du co-polyaminoacide B13 est de 5377 g/mol.
HPLC-SEC organique (calibrant PEG) : Mn = 3000 g/mol.
Co-polyaminoacide B14 : poly-L-glutamate de sodium modifié à l'une de ses extrémités par la molécule A12 dont les esters sont déprotégés et ayant une masse molaire en nombre (Mn) de 3300 g/mol

### Co-polyaminoacide B14-1 : poly-L-benzylglutamate modifié à l'une de ses extrémités par la molécule A12.

Dans un ballon préalablement séché à l'étuve, du γ-benzyl-L-glutamate N-carboxyanhydride (50,00 g, 189,39 mmol) est solubilisé dans du DMF anhydre (65 mL). Le mélange est alors agité jusqu'à complète dissolution, refroidi à 0 °C, puis une solution de la molécule A12 (9,65 g, 8,63 mmol) dans le DMF (50 mL) est introduite rapidement. Le mélange est agité entre 0 °C et température ambiante pendant 2 jours, puis chauffé à 65 °C pendant 2 h. Le mélange réactionnel est alors refroidi à température ambiante puis versé goutte-à-goutte dans du diisopropyléther (1,8 L) sous agitation. Le précipité blanc est récupéré par filtration, lavé deux fois avec du diisopropyléther puis séché sous vide à 30 °C pour obtenir un solide blanc.

### Co-polyaminoacide B14

Le co-polyaminoacide B14-1 est solubilisé dans du DMAc (250 mL) puis du Pd/Al₂O₃ (5,0 g) est ajouté sous atmosphère d'argon. Le mélange est placé sous atmosphère d'hydrogène (10 bar) et agité à 60 °C pendant 24 h. Après refroidissement à température ambiante et filtration du catalyseur sur fritté P4 puis sur membrane Omnipore 0,2 µm PTFE hydrophile, une solution d'eau à pH 2 (6 V) est coulée goutte-à-goutte sur la solution de DMAc, sur une période de 45 min et sous agitation. Après 18 h sous agitation, le précipité blanc est récupéré par filtration, lavé avec de l'eau puis séché sous pression réduite. Le solide obtenu est solubilisé dans de l'eau (1,25 L) en ajustant le pH à 7 par ajout d'une solution aqueuse de soude 1 N. Le pH est ensuite ajusté à pH 13 et la solution est maintenue sous agitation pendant 3 h. Après neutralisation à pH 7, la solution est filtrée sur 0,2 µm, diluée avec de l'éthanol afin d'obtenir une solution contenant 30 % massique d'éthanol, puis filtrée sur filtre de charbon actif (3M R53SLP). La solution obtenue est filtrée sur 0,45 µm et purifiée par ultrafiltration contre une solution de NaCl 0,9 % puis de l'eau jusqu'à ce que la conductimétrie du perméat soit inférieure à 50 µS/cm. La solution de co-polyaminoacide est ensuite concentrée et le pH est ajusté à 7. La solution aqueuse est filtrée sur 0,2 µm et conservée à 4 °C.
Extrait sec : 25,7 mg/g
DP (estimé par RMN ¹H) = 24 donc i = 0,042
La masse molaire moyenne calculée du co-polyaminoacide B14 est de 4720 g/mol.
HPLC-SEC organique (calibrant PEG) : Mn = 3300 g/mol.
Co-polyaminoacide B15 : poly-L-glutamate de sodium modifié par la molécule A13 dont les esters sont déprotégés et ayant une masse molaire en nombre (Mn) de 4400 g/mol

Par un procédé similaire à celui utilisé pour la préparation du co-polyaminoacide B3 appliqué au sel de chlorhydrate de la molécule A13 (3,39 g, 2,34 mmol) et au co-polyaminoacide B2-1 (2,04 g), avec une étape de saponification à pH 13 pendant 5 h dans un mélange d'eau contenant 30 % massique d'éthanol, un poly-L-glutamate de sodium modifié par la molécule A13 dont les esters sont déprotégés est obtenu.
Extrait sec : 15,7 mg/g
DP (estimé d'après la RMN ¹H) : 40
D'après la RMN ¹H : i = 0,15
La masse molaire moyenne calculée du co-polyaminoacide B15 est de 12207 g/mol.
HPLC-SEC organique (calibrant PEG) : Mn = 4400 g/mol.
Co-polyaminoacide B17 : poly-L-glutamate de sodium modifié à l'une de ses extrémités par la molécule A15 dont les esters sont déprotégés et ayant une masse molaire moyenne en nombre (Mn) de 1000 g/mol

Par un procédé similaire à celui utilisé pour la préparation du co-polyaminoacide B14 appliqué à la molécule A15 (10,85 g, 8,74 mmol) et au γ-benzyl-L-glutamate *N*-carboxyanhydride (23,00 g, 87,37 mmol), avec une étape de saponification à pH 12 pendant 2 h, un poly-L-glutamate de sodium modifié à l'une de ses extrémités par la molécule A15 dont les esters sont déprotégés est obtenu.
Extrait sec : 23,9 mg/g
DP (estimé d'après la RMN ¹H) : 10
D'après la RMN ¹H : i = 0,1
La masse molaire moyenne calculée du co-polyaminoacide B17 est de 2576 g/mol.
HPLC-SEC aqueuse (calibrant PEG) : Mn = 1000 g/mol.
Co-polyaminoacide B18 : poly-L-glutamate de sodium modifié par la molécule A16 dont les esters sont déprotégés et ayant une masse molaire en nombre (Mn) de 5000 g/mol

Par un procédé de couplage similaire à celui utilisé pour la préparation du co-polyaminoacide B3 appliqué à la molécule A16 (31,06 g, 42,08 mmol) et au co-polyaminoacide B2-1 (36,80 g), un solide beige est obtenu après l'étape de précipitation acide. Ce solide est dilué dans du TFA (100 g/L) et le mélange est agité à température ambiante pendant 3 h. La solution est ensuite coulée goutte à goutte sur de l'eau (3 V) sous agitation. Après 16 h d'agitation, le précipité est récupéré par filtration puis lavé avec de l'eau. Le solide obtenu est solubilisé dans de l'eau en ajustant le pH à 7 par ajout d'une solution aqueuse de soude 10 N. Une fois la solubilisation complète, le pH est ajusté à pH 12 pendant 1 h par ajout d'une solution de NaOH 1 N. Après neutralisation à pH 7 par ajout d'une solution de HCl 1 N, le produit est purifié par un procédé similaire à celui utilisé pour la préparation du co-polyaminoacide B3 (carbofiltration et ultrafiltration). Un poly-L-glutamate de sodium modifié par la molécule A16 dont les esters sont déprotégés est obtenu.
Extrait sec : 28,2 mg/g
DP (estimé d'après la RMN ¹H) : 40
D'après la RMN ¹H : i = 0,15
La masse molaire moyenne calculée du co-polyaminoacide B18 est de 9884 g/mol.
HPLC-SEC organique (calibrant PEG) : Mn = 5000 g/mol.
Co-polyaminoacide B19 : poly-L-glutamate de sodium modifié par la molécule A17 dont les esters sont déprotégés et ayant une masse molaire en nombre (Mn) de 4900 g/mol

Par un procédé similaire à celui utilisé pour la préparation du co-polyaminoacide B3 appliqué au sel de chlorhydrate de la molécule A17 (7,35 g, 13,09 mmol) et au co-polyaminoacide B2-1 (11,45 g), avec une étape de saponification à pH 13 pendant 3 h dans un mélange d'eau contenant 30 % massique d'éthanol, un poly-L-glutamate de sodium modifié par la molécule A17 dont les esters sont déprotégés est obtenu.
Extrait sec : 25,7 mg/g
DP (estimé d'après la RMN ¹H) : 40
D'après la RMN ¹H : i = 0,15
La masse molaire moyenne calculée du co-polyaminoacide B19 est de 9062 g/mol.
HPLC-SEC organique (calibrant PEG) : Mn = 4900 g/mol.
Co-polyaminoacide B20 : poly-L-glutamate de sodium modifié par la molécule A18 dont les esters sont déprotégés et ayant une masse molaire en nombre (Mn) de 5800 g/mol

Par un procédé similaire à celui utilisé pour la préparation du co-polyaminoacide B3 appliqué au sel de chlorhydrate de la molécule A18 (5,43 g, 6,86 mmol) et au co-polyaminoacide B2-1 (6,00 g), avec une étape de saponification à pH 13 pendant 3 h dans un mélange d'eau contenant 30 % massique d'éthanol, un poly-L-glutamate de sodium modifié par la molécule A18 dont les esters sont déprotégés est obtenu.
Extrait sec : 22,0 mg/g
DP (estimé d'après la RMN ¹H) : 40
D'après la RMN ¹H : i = 0,15
La masse molaire moyenne calculée du co-polyaminoacide B20 est de 10444 g/mol.
HPLC-SEC organique (calibrant PEG) : Mn = 5800 g/mol.
Co-polyaminoacide B21 : poly-L-glutamate de sodium modifié par la molécule A19 dont les esters sont déprotégés et ayant une masse molaire en nombre (Mn) de 5000 g/mol

Par un procédé similaire à celui utilisé pour la préparation du co-polyaminoacide B18 appliqué à la molécule A19 (32,64 g, 45,97 mmol) et au co-polyaminoacide B2-1 (40,20 g), un poly-L-glutamate de sodium modifié par la molécule A19 dont les esters sont déprotégés est obtenu.
Extrait sec : 26,2 mg/g
DP (estimé d'après la RMN ¹H) : 40
D'après la RMN ¹H : i = 0,15
La masse molaire moyenne calculée du co-polyaminoacide B21 est de 9716 g/mol.
HPLC-SEC organique (calibrant PEG) : Mn = 5000 g/mol.
Co-polyaminoacide B22 : poly-L-glutamate de sodium modifié à l'une de ses extrémités par la molécule A20 et ayant une masse molaire moyenne en nombre (Mn) de 1900 g/mol

Par un procédé similaire à celui utilisé pour la préparation du co-polyaminoacide B14 appliqué à la molécule A20 (13,28 g, 12,51 mmol) dans du CHCl₃ (53 mL) et au γ-benzyl-L-glutamate N-carboxyanhydride (72,46 g, 275,2 mmol) dans du DMF (270 mL), avec une étape de saponification à pH 12 pendant 1 h 30, un poly-L-glutamate de sodium modifié à l'une de ses extrémités par la molécule A20 est obtenu.
Extrait sec : 27,3 mg/g
DP (estimé d'après la RMN ¹H) : 20
D'après la RMN ¹H : i = 0,05
La masse molaire moyenne calculée du co-polyaminoacide B22 est de 4087 g/mol.
HPLC-SEC aqueuse (calibrant PEG) : Mn = 1900 g/mol.

### ii) Co-polyaminoacides de formules XXXa, XXXb, XXXb' et XXXb"

| **N°** | **CO-POLYAMINOACIDES PORTEUR DE CHARGES CARBOXYLATES ET DE RADICAUX HYDROPHOBES** |
|---|---|
| B7' | 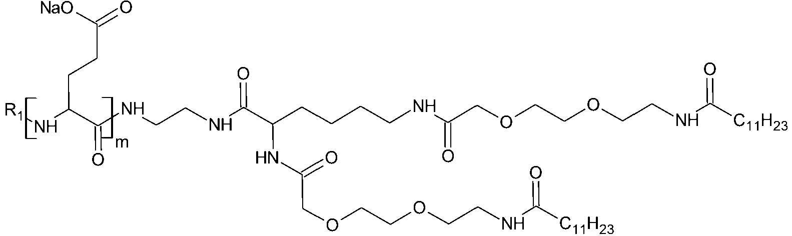 |
| | i = 0,042, DP (m) = 24 |
| | R₁ = H ou pyroglutamate |
| B8 | 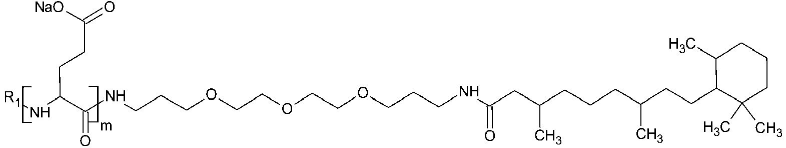 |
| | i = 0,043, DP (m) = 23 |
| | R₁ = H ou pyroglutamate |
| B10 | 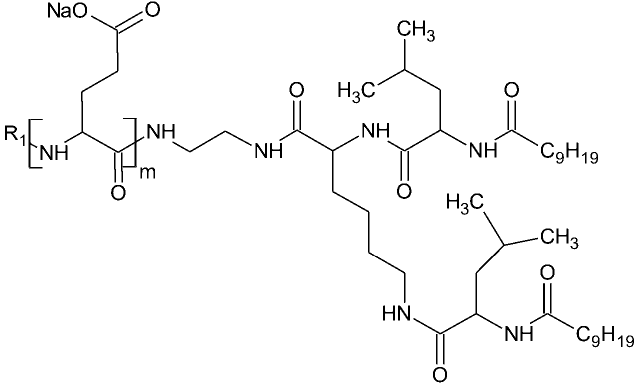 |
| | i = 0,032, DP (m) = 31 |
| | R₁ = H ou pyroglutamate |
| B11 | 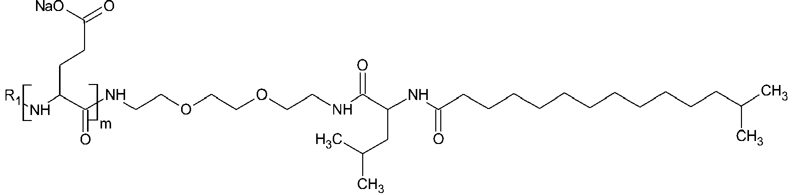 |
| | i = 0,034, DP (m) = 29 |
| | R₁ = H ou pyroglutamate |
| B12 | 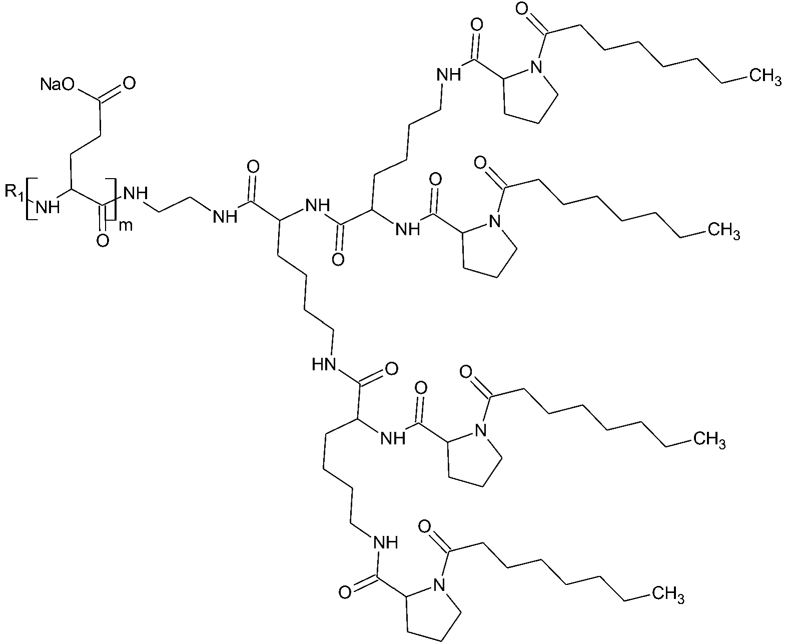 |
| | i = 0,042, DP (m) = 24 |
| | R₁ = H ou pyroglutamate |

### Co-polyaminoacide B7' : poly-L-glutamate de sodium modifié à l'une de ses extrémités par la molécule A5a et ayant une masse molaire moyenne en nombre (Mn) de 2600 g/mol

### Co-polyaminoacide B7'-1 : poly-L-benzylglutamate modifié à l'une de ses extrémités par la molécule A5a.

Dans un ballon préalablement séché à l'étuve, du γ-benzyl-L-glutamate N-carboxyanhydride (10,1 g, 38,4 mmol) est solubilisé dans du DMF anhydre (19 mL). Le mélange est alors agité jusqu'à complète dissolution, refroidi à 0 °C, puis une solution de la molécule A5a (1,47 g, 1,74 mmol) dans le chloroforme (3,7 mL) est introduite rapidement. Le mélange est agité entre 0 °C et température ambiante pendant 2 jours, puis chauffé à 65 °C pendant 2 h. Le mélange réactionnel est alors refroidi à température ambiante puis versé goutte-à-goutte dans du diisopropyléther (0,29 L) sous agitation. Le précipité blanc est récupéré par filtration, lavé deux fois avec du diisopropyléther (5 × 50 mL) puis séché sous vide à 30 °C pour obtenir un solide blanc.

### Co-polyaminoacide B7'

Le co-polyaminoacide B7'-1 (8,33 g, 33,0 mmol) est dilué dans de l'acide trifuloroacétique (TFA, 132 mL), puis la solution est refroidie à 4 °C. Une solution de HBr à 33 % dans l'acide acétique (92,5 mL, 0,528 mol) est alors ajoutée goutte-à-goutte. Le mélange est agité à température ambiante pendant 2 h, puis coulé goutte-à-goutte sur un mélange 1:1 (v/v) de diisopropyléther et d'eau sous agitation (0,8 L). Après 2 h d'agitation, le mélange hétérogène est laissé au repos pendant une nuit. Le précipité blanc est récupéré par filtration, lavé avec de l'IPE (2 x 66 mL) puis avec de l'eau (2 x 66 mL). Le solide obtenu est alors solubilisé dans de l'eau (690 mL) en ajustant le pH à 7 par ajout d'une solution aqueuse de soude 1 N. Après solubilisation, la concentration théorique est ajustée à 20 g/L théorique par addition d'eau (310 mL), la solution est filtrée sur filtre 0,45 µm puis purifiée par ultrafiltration contre une solution de NaCl 0,9 %, puis de l'eau jusqu'à ce que la conductimétrie du perméat soit inférieure à 50 µS/cm. La solution obtenue est filtrée sur filtre 0,2 µm et stockée à 2-8 °C.
Extrait sec : 17,3 mg/g
DP (estimé d'après la RMN ¹H) : 24
D'après la RMN ¹H : i = 0,042
La masse molaire moyenne calculée du co-polyaminoacide B7' est de 4430 g/mol.
HPLC-SEC organique (calibrant PEG) : Mn = 2600 g/mol.

### Exemple B8 : co-polyaminoacide B8 - poly-L-glutamate de sodium modifié à l'une de ses extrémités par la molécule A6a et ayant une masse molaire moyenne en nombre (Mn) de 2400 g/mol

### Co-polyaminoacide B8-1 : poly-L-benzylglutamate modifié à l'une de ses extrémités par la molécule A6.

Dans un ballon préalablement séché à l'étuve, du γ-benzyl-L-glutamate N-carboxyanhydride (19,0 g, 72,2 mmol) est solubilisé dans du DMF anhydre (19 mL). Le mélange est alors agité jusqu'à complète dissolution, refroidi à 0 °C, puis une solution de la molécule A6a (1,68 g, 3,28 mmol) dans le chloroforme (3,7 mL) est introduite rapidement. Le mélange est agité entre 0 °C et température ambiante pendant 2 jours, puis chauffé à 65 °C pendant 2 h. Le mélange réactionnel est alors refroidi à température ambiante puis versé goutte-à-goutte dans du diisopropyléther (0,29 L) sous agitation. Le précipité blanc est récupéré par filtration, lavé deux fois avec du diisopropyléther (5 × 50 mL) puis séché sous vide à 30 °C pour obtenir un solide blanc.

### Co-polyaminoacide B8

Par un procédé similaire à celui utilisé pour la préparation du co-polyaminoacide B7' appliqué au co-polyaminoacide B8-1 (14,6 g, 61,5 mmol), un poly-L-glutamate de sodium modifié à l'une de ses extrémités par la molécule A6a est obtenu.
Extrait sec : 21,3 mg/g
DP (estimé d'après la RMN ¹H) : 23
D'après la RMN ¹H : i = 0,043
La masse molaire moyenne calculée du co-polyaminoacide B8 est de 3948 g/mol.
HPLC-SEC organique (calibrant PEG) : Mn = 2400 g/mol.

### Co-polyaminoacide B10 : poly-L-glutamate de sodium modifié à l'une de ses extrémités par la molécule A8 et ayant une masse molaire moyenne en nombre (Mn) de 3100 g/mol

### Co-polyaminoacide B10-1 : poly-L-benzylglutamate modifié à l'une de ses extrémités par la molécule A8.

Dans un contenant adapté sont introduits successivement le sel de chlorhydrate de la molécule A8 (2,308 g, 3,04 mmol), du chloroforme (120 mL), du tamis moléculaire 4 Å (1,5 g), ainsi que de la résine échangeuse d'ion Amberlite IRN 150 (1,5 g). Après 1 h d'agitation sur rouleaux, le milieu est filtré et la résine est rincée avec du chloroforme. Le mélange est évaporé puis co-évaporé avec du toluène. Le résidu est solubilisé dans du DMF anhydre (40 mL) pour être utilisé directement dans la réaction de polymérisation.

Dans un ballon préalablement séché à l'étuve, du γ-benzyl-L-glutamate N-carboxyanhydride (20,0 g, 76,0 mmol) est solubilisé dans du DMF anhydre (19 mL). Le mélange est alors agité jusqu'à complète dissolution, refroidi à 0 °C, puis une solution de la molécule A8, préalablement préparée, dans le chloroforme (3,7 mL) est introduite rapidement. Le mélange est agité entre 0 °C et température ambiante pendant 2 jours, puis chauffé à 65 °C pendant 2 h. Le mélange réactionnel est alors refroidi à température ambiante puis versé goutte-à-goutte dans du diisopropyléther (0,29 L) sous agitation. Le précipité blanc est récupéré par filtration, lavé deux fois avec du diisopropyléther (5 × 50 mL) puis séché sous vide à 30 °C pour obtenir un solide blanc.

### Co-polyaminoacide B10

Par un procédé similaire à celui utilisé pour la préparation du co-polyaminoacide B7' appliqué au co-polyaminoacide B10-1 (15,2 g, 60,8 mmol), un poly-L-glutamate de sodium modifié à l'une de ses extrémités par la molécule A8 est obtenu.
Extrait sec : 34,1 mg/g
DP (estimé d'après la RMN ¹H) : 31
D'après la RMN ¹H : i = 0,032
La masse molaire moyenne calculée du co-polyaminoacide B10 est de 5367 g/mol.
HPLC-SEC organique (calibrant PEG) : Mn = 3100 g/mol.

### Exemple B11 : co-polyaminoacide B11 - poly-L-glutamate de sodium modifié à l'une de ses extrémités par la molécule A9 et ayant une masse molaire moyenne en nombre (Mn) de 3000 g/mol

### Co-polyaminoacide B11-1 : poly-L-benzylglutamate modifié à l'une de ses extrémités par la molécule A9.

Dans un contenant adapté sont introduits successivement le sel de chlorhydrate de la molécule A9 (2,023 g, 3,87 mmol), du chloroforme (120 mL), du tamis moléculaire 4 Å (1,5 g), ainsi que de la résine échangeuse d'ion Amberlite IRN 150 (1,5 g). Après 1 h d'agitation sur rouleaux, le milieu est filtré et la résine est rincée avec du chloroforme. Le mélange est évaporé puis co-évaporé avec du toluène. Le résidu est solubilisé dans du DMF anhydre (40 mL) pour être utilisé directement dans la réaction de polymérisation.

Par un procédé similaire à celui utilisé pour la préparation du co-polyaminoacide B8-1 appliqué à la solution de la molécule A9 préalablement préparée et au γ-benzyl-L-glutamate *N*-carboxyanhydride (25,5 g, 96,8 mmol), le co-polyaminoacide B11-1 est obtenu.

### Co-polyaminoacide B11

Par un procédé similaire à celui utilisé pour la préparation du co-polyaminoacide B7' appliqué au co-polyaminoacide B11-1 (18,4 g, 77,3 mmol), un poly-L-glutamate de sodium modifié à l'une de ses extrémités par la molécule A9 est obtenu.
Extrait sec : 28,0 mg/g
DP (estimé d'après la RMN ¹H) : 29
D'après la RMN ¹H : i = 0,034
La masse molaire moyenne calculée du co-polyaminoacide B11 est de 4828 g/mol.
HPLC-SEC organique (calibrant PEG) : Mn = 3000 g/mol.

### Co-polyaminoacide B12 : poly-L-glutamate de sodium modifié à l'une de ses extrémités par la molécule A10 et ayant une masse molaire moyenne en nombre (Mn) de 2700 g/mol

### Co-polyaminoacide B12-1 : poly-L-benzylglutamate modifié à l'une de ses extrémités par la molécule A10.

Par un procédé similaire à celui utilisé pour la préparation du co-polyaminoacide B10-1 appliqué à la molécule A10 (3,0 g, 2,24 mmol) et au γ-benzyl-L-glutamate N-carboxyanhydride (12,99 g, 49,3 mmol), le co-polyaminoacide B12-1 est obtenu.

### Co-polyaminoacide B12

Par un procédé similaire à celui utilisé pour la préparation du co-polyaminoacide B7' appliqué au co-polyaminoacide B12-1 (13,2 g, 48,0 mmol), un poly-L-glutamate de sodium modifié à l'une de ses extrémités par la molécule A10 est obtenu.
Extrait sec : 13,2 mg/g
DP (estimé d'après la RMN ¹H) : 24
D'après la RMN ¹H : i = 0,042
La masse molaire moyenne calculée du co-polyaminoacide B12 est de 4924 g/mol.
HPLC-SEC organique (calibrant PEG) : Mn = 2700 g/mol.

### Partie CE - Co-polyaminoacides contre-exemples

| **N°** | |
|---|---|
| CE1 | 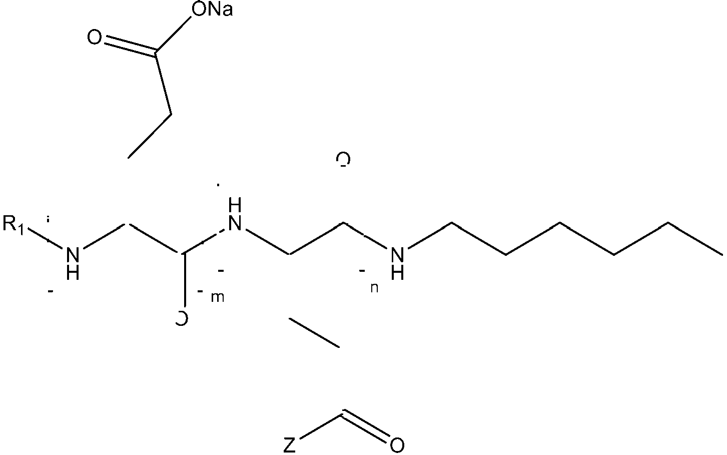 |
| | i = 0,05, DP (m + n) = 22 |
| | 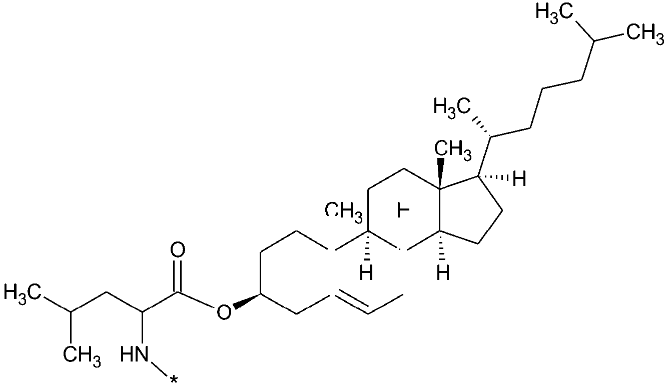 Z = |
| | R₁=CH₃-CO-, H ou pyroglutamate |
| CE2 | 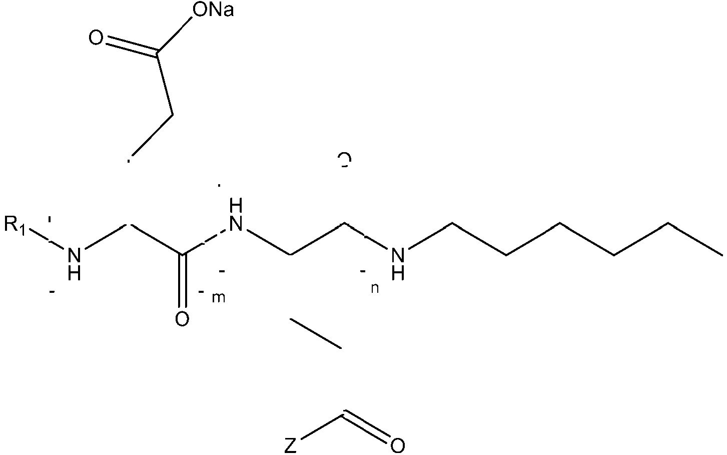 |
| | i = 0,05, DP (m + n) = 43 |
| | 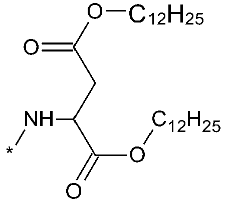 Z = |
| | R₁=CH₃-CO-, H ou pyroglutamate |

Les co-polyaminoacides CE1 et CE2 sont synthétisés selon le procédé décrit dans la demande WO2017211916.

### Partie C - Compositions

### Exemple C1 : Solution d'insuline analogue rapide (Humalog^{®}) à 100 U/mL

Cette solution est une solution commerciale d'insuline lispro commercialisée par la société ELI LILLY sous le nom de Humalog^{®}. Ce produit est une insuline analogue rapide. Les excipients dans Humalog^{®} sont le méta-crésol (3,15 mg/mL), le glycérol (16 mg/mL), le phosphate de disodium (1,88 mg/mL), l'oxyde de zinc (pour avoir 0,0197 mg d'ion zinc/mL), l'hydroxyde de sodium et l'acide chlorhydrique pour l'ajustement du pH (pH 7-7,8) et de l'eau.

### Exemple C2 : Solution d'insuline analogue rapide lispro à 100-600 U/mL

Cette solution est une solution d'insuline préparée à partir de poudre d'insuline lispro produite par la société Gan&Lee. Ce produit est une insuline analogue rapide. Les excipients utilisés sont le m-crésol, le glycérol, l'oxyde de zinc, l'hydroxyde de sodium et l'acide chlorhydrique pour l'ajustement du pH (pH 7-7,8) et de l'eau. La concentration de zinc est de 300 µM pour 100 UI/mL d'insuline. La concentration des autres excipients varie en fonction de celle de lispro pour obtenir les concentrations souhaitées dans les formulations finales.

### Exemple C3 : Solution d'insuline analogue lente (Lantus^{®}) à 100 U/mL

Cette solution est une solution commerciale d'insuline glargine commercialisée par la société SANOFI sous le nom de Lantus^{®}. Ce produit est une insuline analogue lente. Les excipients dans Lantus^{®} sont le chlorure de zinc (30 µg/mL), le m-crésol (2,7 mg/mL), le glycérol (20 mg/mL), le polysorbate 20 (16 µM), l'hydroxyde de sodium et l'acide chlorhydrique pour l'ajustement du pH (pH 4) et de l'eau.

### Exemple C4 : Solution d'insuline glargine à 100-400 U /mL

Cette solution est une solution d'insuline glargine préparée à partir de poudre d'insuline glargine produite par la société Gan&Lee. Ce produit est une insuline analogue lente. Les excipients utilisés sont le chlorure de zinc, le m-crésol, le glycérol, l'hydroxyde de sodium et l'acide chlorhydrique pour l'ajustement du pH (pH 4) et de l'eau. La concentration de zinc est de 460 µM pour 100 UI/mL d'insuline. La concentration des autres excipients varie en fonction de celle de glargine pour obtenir les concentrations souhaitées dans les formulations finales.

### Partie CA - Compositions comprenant de l'insuline glargine

Procédé de préparation CA1 : Préparation d'une composition diluée co-polyaminoacide/insuline glargine 50 U/mL à pH 7,1, suivant un procédé utilisant l'insuline glargine sous forme liquide (en solution) et un co-polyaminoacide sous forme liquide (en solution).

À une solution mère de co-polyaminoacide à pH 7,1 sont ajoutées des solutions concentrées de méta-crésol et de glycérine de manière à obtenir une solution de co-polyaminoacide de concentration C_{mère co-polyaminoacide/excipients} (mg/mL). La quantité d'excipients ajoutée est ajustée de manière à obtenir une concentration de méta-crésol de 35 mM et glycérine de 184 mM dans la composition co-polyaminoacide/insuline glargine 50 U/mL à pH 7,1.

Dans un pot stérile, un volume V_{insuline glargine} d'une solution d'insuline glargine à une concentration de 100 U/mL décrite en C3 ou C4 est ajouté à un volume V_{mère co-polyaminoacide /excipients} d'une solution de co-polyaminoacide à la concentration C_{mère co-polyaminoacide /excipients} (mg/mL) de manière à obtenir une composition diluée co-polyaminoacide C_{co-polyaminoacide dilué} (mg/mL)/insuline glargine 50 U/mL à pH 7,1. Un trouble apparaît. Le pH est ajusté à pH 7,1 par ajout de NaOH concentrée et la solution est placée en statique à 40 °C pendant 2 h jusqu'à solubilisation complète. Cette solution visuellement limpide est placée à 4 °C.

### Procédé de préparation CA2 : Préparation d'une composition co-polyaminoacide/insuline glargine concentrée à pH 7,1 à l'aide d'un co-polyaminoacide, suivant un procédé de concentration d'une composition diluée.

Une composition co-polyaminoacide/insuline glargine 50 U/mL à pH 7,1 décrite dans l'exemple CA1 est concentrée par ultrafiltration sur une membrane 3 kDa en cellulose régénérée (Amicon^{®} Ultra-15 commercialisée par la société Millipore). A l'issue de cette étape d'ultrafiltration, le rétentat est limpide et la concentration en insuline glargine dans la composition est déterminée par chromatographie en phase inverse (RP-HPLC). La concentration en insuline glargine dans la composition est ensuite ajustée à la valeur souhaitée par dilution dans une solution d'excipients m-crésol/glycérine/Tween 20 de manière à obtenir une concentration finale en m-crésol de 35 mM, en Tween 20 de 52 µM et une osmolarité de 300 mOsm/kg. Le pH est mesuré et ajusté à pH 7,1 par ajout de NaOH et HCl concentré. Cette solution à pH 7,1, visuellement limpide, présente une concentration en insuline glargine C_{insuline glargine} (U/mL) et une concentration en co-polyaminoacide C_{co-polyaminoacide} (mg/mL) = C_{co-polyaminoacide dilué} (mg/mL) x C_{insuline glargine} (U/mL)/50 (U/mL).

### Procédé de préparation CA3 : Préparation d'une composition concentrée co-polyaminoacide / insuline glargine à pH 7,1, suivant un procédé utilisant l'insuline glargine sous forme liquide (en solution) et un co-polyaminoacide sous forme liquide (en solution).

À une solution mère de co-polyaminoacide à pH 7,1 est ajoutée une solution de glargine à 220-400 UI/mL contenant les excipients décrits dans l'exemple C4. La concentration des excipients dans la solution de glargine est ajustée de manière à obtenir une concentration de m-crésol de 35 mM, et en glycérine de 184 mM dans la composition co-polyaminoacide/insuline glargine à pH 7,1. Un trouble apparaît. Le pH est ajusté à pH 7,1 par ajout de NaOH concentré et la solution est placée en statique dans une étuve à 40 °C pendant 2 h jusqu'à solubilisation complète. Cette solution visuellement limpide est placée à 4 °C après ajout d'un volume de solution concentrée de polysorbate 20 pour obtenir une concentration finale de 52 µM.

### Procédé de préparation CA3a : Préparation d'une composition co-polyaminoacide / insuline glargine à pH 7,1, suivant un procédé utilisant l'insuline glargine sous forme liquide (en solution) et un co-polyaminoacide sous forme liquide (en solution).

À une solution mère de co-polyaminoacide à pH 7,0-7,5 est ajoutée une solution de glargine à 100-220 U/mL contenant les excipients décrits dans l'exemple C4. Les concentrations des excipients dans la solution de glargine sont ajustées de manière à obtenir une concentration en m-crésol de 35 mM, et en glycérine de 230 mM dans la composition co-polyaminoacide/insuline glargine. Un trouble apparaît. Le pH est ajusté à pH 7,5 par ajout de NaOH concentré et la solution est placée en statique dans une étuve à 40 °C pendant 2 h jusqu'à solubilisation complète. La solution obtenue est visuellement limpide.

### Procédé de préparation CA3b : Préparation d'une composition co-polyaminoacide / insuline glargine à pH 7,1, suivant un procédé utilisant l'insuline glargine sous forme liquide (en solution) et un co-polyaminoacide sous forme liquide (en solution).

À une solution mère de co-polyaminoacide à pH 7,0-7,5 sont ajoutées, dans l'ordre, une solution de chlorure de sodium et une solution de glargine à 100-220 U/mL décrite dans l'exemple C4. Les concentrations des excipients dans la solution de glargine sont ajustées de manière à obtenir une concentration en m-crésol de 35 mM et en glycérine de 230 mM dans la composition co-polyaminoacide/insuline glargine à pH 7,1. Un trouble apparaît. Le pH est ajusté à pH 7,5 par ajout de NaOH concentré et la solution est placée en statique dans une étuve à 40 °C pendant 2 h jusqu'à solubilisation complète. La solution obtenue est visuellement limpide.

Selon les procédés de préparation CA2, CA3 ou CA3a et CA3b, des compositions co-polyaminoacide/insuline glargine ont été préparées par exemple avec des concentrations en insuline glargine entre 100 U/mL et 300 U/mL.

### Exemple CA4 : Préparation de compositions co-polyaminoacide/insuline glargine 200 U/mL à pH 7,1.

Des compositions co-polyaminoacide/insuline glargine 200 U/mL sont préparées selon les procédés décrits dans CA2 et CA3 de manière à obtenir une concentration en insuline glargine C_{insuline glargine} = 200 U/mL et une concentration en co-polyaminoacide Cco-polyaminoacide (mg/mL).
Ces compositions sont présentées dans le tableau 1 et 1a.

**Tableau 1 : Compositions d'insuline glargine (200 U/mL) en présence de co-polyaminoacide.**

| **Composition** | **Co-polyaminoacide** | **Concentration en co-polyaminoacide (en mg/ml)** | **Insuline glargine (U/mL)** | **Aspect visuel de la solution** |
|---|---|---|---|---|
| CA3-1 | B7 | 5 | 200 | limpide |
| CA2-1 | B1 | 6 | 200 | limpide |
| CA3-2 | B9 | 5 | 200 | limpide |

**Tableau 1a : Compositions d'insuline glargine (200 U/mL) en présence du co-polyaminoacide B8.**

| **Composition** | **Co-polyaminoacide** | | **Concentration en co-polyaminoacide (en mg/ml)** | | **Insuline glargine (U/mL)** | | **Aspect visuel de la solution** |
|---|---|---|---|---|---|---|---|
| CA3-3 | B8 | | 9 | | 200 | | limpide |
| CA3-4 | B7' | 17 | | 200 | | limpide | |

Les co-polyaminoacides permettent de solubiliser l'insuline glargine à pH neutre et de conduire à une solution limpide.

### Procédé de préparation CA5 : Protocole pour la détermination de la concentration minimale pour solubiliser insuline glargine à 50 U/mL à pH 7,1.

A une solution mère de co-polyaminoacide à pH 7-7,5 sont ajoutées des solutions concentrées de m-crésol et de glycérine. Les quantités d'excipients ajoutées sont ajustées de manière à obtenir une concentration en m-crésol de 35 mM et en glycérine de 184 mM dans une composition co-polyaminoacide / insuline glargine 50 U/mL.

Dans un vial de 3 mL, 0,5 mL d'une solution d'insuline glargine à une concentration de 100 U/mL, décrite dans les exemples C3 ou C4, est ajouté à un volume de 0,5 mL de la solution de co-polyaminoacide/m-crésol/glycérine, de manière à obtenir une composition co-polyaminoacide (mg/mL)/ insuline glargine 50 U/mL. Un trouble apparaît. Le pH est ajusté à pH 7,1 par ajout de NaOH concentré et la solution est placée en statique dans une étuve à 40 °C pendant 1 nuit. Cette opération est réalisée pour différentes concentrations de co-polyaminoacide. Après la nuit à 40 °C les échantillons sont inspectés visuellement et soumis à une mesure de diffusion statique de la lumière à un angle de 173° à l'aide d'un Zetasizer (Malvern). La concentration minimale de co-polyaminoacide permettant de solubiliser l'insuline glargine est définie comme la concentration la plus faible pour laquelle le mélange co-polyaminoacide / insuline glargine à pH 7,1 est visuellement limpide et présente une intensité diffusée inférieure ou égale à 1000 kcps (milliers de photons par seconde). Les concentrations minimales de co-polyaminoacide sont présentées dans le tableau 1b suivant.

**Tableau 1b : Concentration minimum en co-polyaminoacide pour solubiliser l'insuline glargine**

| **Co-polyaminoacide** | **Concentration minimale de co-polyaminoacide pour la solubilisation de glargine 50 U/mL à pH 7,1** | |
|---|---|---|
| | **(mg/mL)** | **Ratio hydrophobe/insuline glargine (mol/mol)** |
| B22 | 0,75 | 0,61 |
| B7 | 0,75 | 0,64 |
| B13 | 0,88 | 0,54 |
| CE1 | 1,2 | 1,08 |
| CE2 | 1,0 | 0,83 |

Les co-polyaminoacides B22, B7 et B13 permettent de solubiliser l'insuline glargine avec une concentration massique inférieure ou égale à 0,88 mg/mL et avec un ratio molaire hydrophobe/insuline glargine inférieur ou égal à 0,64.

**Tableau 1c : Ratio minimum pour solubiliser l'insuline glargine.**

| **Co-polyaminoacide** | **Concentration minimale de co-polyaminoacide pour la solubilisation de glargine 50 U/mL à pH 7,1** | |
|---|---|---|
| | **(mg/mL)** | **Ratio co-polyaminoacide /insuline glargine (mol/mol)** |
| B2 | 0,75 | 0,27 |
| B3 | 0,87 | 0,32 |
| B9 | 0,87 | 0,33 |
| B15 | 1,0 | 0,27 |
| B18 | 0,79 | 0,26 |
| B19 | 1,0 | 0,37 |
| B21 | 0,7 | 0,24 |
| CE1 | 1,2 | 1,01 |
| CE2 | 1,0 | 0,45 |

Les co-polyaminoacides B2, B3, B9, B15, B18, B19 et B21 permettent de solubiliser l'insuline glargine avec une concentration massique inférieure ou égale à 1,25 mg/mL et avec un ratio molaire co-polyaminoacide /insuline glargine inférieur ou égal à 0,42.

### Procédé de préparation CA6 : Détermination de la concentration minimale pour solubiliser insuline glargine à 50 U/mL en présence de chlorure de sodium à pH 7,1.

Le procédé de préparation suit le procédé de préparation CA5 avec une seule différence : à la solution mère de co-polyaminoacide à pH 7-7,5, une solution concentrée de chlorure de sodium est ajoutée pour atteindre les cibles dans la composition finale, en plus des solutions de m-crésol et de glycérine. Les résultats sont décrits dans le tableau 1d suivant.

**Tableau 1d : ratios minimum pour solubiliser l'insuline glargine**

| **co-polyaminoacid e** | **NaCl (mM)** | **Concentration minimale en co-polyaminoacide pour la solubilisation de glargine 50 U/mL à pH 7,1** | |
|---|---|---|---|
| | | **(mg/mL)** | **Ratio hydrophobe/insulin e glargine (mol/mol)** |
| B22 | 0 | 0,77 | 0,62 |
| | 5 | 0,63 | 0,51 |
| | 10 | 0,59 | 0,48 |

L'ajout de sel permet de diminuer La concentration en co-polyaminoacide B22 au seuil de solubilisation de glargine.

### Partie CB - Compositions comprenant de l'insuline glargine et insuline lispro

### Procédé de préparation CB1 : Préparation d'une composition diluée co-polyaminoacide/insuline glargine 43 (U/mL)/insuline lispro 13,5 (U/mL)

À un volume V_{co-polyaminoacide / insuline glargine dilué} de la composition diluée co-polyaminoacide / insuline glargine 50 U/mL à pH 7,1 décrite dans l'exemple CA1 est ajoutée un volume V_{insuline lispro} d'une solution lispro à 100 U/mL et de l'eau de manière à obtenir une composition co-polyaminoacide/insuline glargine 43 (U/mL)/insuline lispro 13,5 (U/mL).

### Procédé de préparation CB2 : Préparation d'une composition co-polyaminoacide / insuline glargine / insuline lispro concentrée à pH 7,1

Une composition co-polyaminoacide/insuline glargine 43 (U/mL)/insuline lispro 13,5 (U/mL) décrite dans l'exemple CB1 est concentrée par ultrafiltration sur une membrane 3 kDa en cellulose régénérée (Amicon^{®} Ultra-15 commercialisée par la société MILLIPORE). À l'issue de cette étape d'ultrafiltration, le rétentat est limpide et la concentration en insuline glargine dans la composition est déterminée par chromatographie en phase inverse (RP-HPLC). Les concentrations en insuline glargine et insuline lispro dans la composition sont ensuite ajustées à la valeur souhaitée par dilution dans une solution d'excipients m-crésol/glycérine/Tween 20 de manière à obtenir une concentration finale en m-crésol de 35 mM, en Tween de 52 µM et une osmolarité de 300 mOsmole/kg. Le pH est mesuré et ajusté si nécessaire à pH 7,1 par ajout de NaOH et HCl concentré. Cette solution à pH 7,1, visuellement limpide, présente une concentration en insuline glargine C_{insuline glargine} (U/mL), une concentration en insuline lispro C_{insuline lispro} = C_{insuline glargine} x 0,33 et une concentration en co-polyaminoacide C_{co-polyaminoacide} (mg/mL) = C_{co-polyaminoacide dilué} (mg/mL) x C_{insuline glargine} (U/mL) / 50 (U/mL).

### Procédé de préparation CB3 : Préparation d'une composition co-polyaminoacide/insuline glargine/insuline lispro concentrée à pH 7,1

À un volume V_{co-polyaminoacide / insuline glargine concentré} de la composition concentrée co-polyaminoacide/insuline glargine à pH 7,1 décrite dans l'exemple CA3 est ajoutée un volume V_{insuline lispro} d'une solution lispro décrite dans l'exemple C2. Un volume de solution de polysorbate 20 est aussi ajouté pour obtenir une concentratione finale de 52 µM. La solution résultante à pH 7,1, visuellement limpide, présente une concentration en insuline glargine C_{insuline glargine} (U/mL), une concentration en insuline lispro C_{insuline lispro} = C_{insuline glargine} X 0,33 et une concentration en co-polyaminoacide C_{co-polyaminoacide} (mg/mL) = C_{co-polyaminoacide dilué} (mg/mL) x C_{insuline glargine} (U/mL)/50 (U/mL). La concentration de m-crésol est de 35 mM et celle de glycérine 230 mM.

### Procédé de préparation CB4 : Préparation d'une composition co-polyaminoacide/insuline glargine 75 U/mL/insuline lispro 25 U/mL à pH 7,2

À un volume de la composition concentrée co-polyaminoacide/insuline glargine à pH 7,2 décrite dans l'exemple CA3a ou CA3b est ajoutée un volume d'une solution lispro décrite dans l'exemple C2. La concentration de zinc est ajustée à 0,5 mM par ajout d'un volume d'une solution concentrée de ZnCl₂. Le pH est ajusté à 7,2 par ajout d'une solution concentrée d'acide chloridrique. La solution résultante, visuellement limpide, présente une concentration en insuline glargine de 75 U/mL et en insuline lispro de 25 U/mL. La concentration de m-crésol est de 35 mM et celle de glycérine 230 mM.

### Procédé de préparation CB5 : Préparation d'une composition co-polyaminoacide/insuline glargine 150 U/mL /insuline lispro 50 U/mL à pH 7,2

À un volume de la composition concentrée co-polyaminoacide/insuline glargine à pH 7,2 décrite dans l'exemple CA4 ou CA5 est ajoutée un volume d'une solution lispro décrite dans l'exemple C2. La concentration de zinc est ajustée à 1 mM par ajout d'un volume d'une solution concentrée de ZnCl₂. Le pH est ajusté à 7,2 par ajout d'une solution concentrée d'acide chloridrique. La solution résultante, visuellement limpide, présente une concentration en insuline glargine de 150 U/mL et en insuline lispro 50 U/mL. La concentration de m-crésol est de 35 mM et celle de glycérine 230 mM.

### Exemple CB2 et CB3 : Préparation de compositions co-polyaminoacide/insuline glargine 200 U/mL/insuline lispro 66 U/mL à pH 7,1

Des compositions co-polyaminoacide/insuline glargine 200 U/mL/insuline lispro 66 U/mL sont préparées selon un des procédés décrits dans les exemple CB2 ou CB3 de manière à obtenir une concentration en insuline glargine C_{insuline glargine} = 200 U/mL, une concentration en insuline lispro C_{insuline lispro} = 66 U/mL et une concentration en co-polyaminoacide C_{co-polyaminoacide} (mg/mL).
Ces compositions sont présentées dans le Tableau 2 et 2a.

**Tableau 2 : Compositions d'insuline glargine (200 U/mL) et d'insuline lispro (66 U/mL) en présence de co-polyaminoacide.**

| **Compositio n** | **Co-polyaminoacid e** | **Concentration en co-polyaminoacid e (en mg/ml)** | **Insulin e glargin e (U/mL)** | **Insulin e Lispro (U /mL )** | **Aspect visuel de la solutio n** |
|---|---|---|---|---|---|
| CB3-1 | B7 | 5 | 200 | 66 | limpide |
| CB2-2 | B1 | 6 | 200 | 66 | limpide |
| CB3-2 | B4 | 5 | 200 | 66 | limpide |

**Tableau 2a : Compositions d'insuline glargine (200 U/mL) et d'insuline lispro (66 U/mL) en présence de co-polyaminoacide.**

| **Compositio n** | **Co-polyaminoacid e** | **Concentration en co-polyaminoacid e (en mg/ml)** | **Insulin e glargin e (U/mL)** | **Insulin e Lispro (U /mL )** | **Aspect visuel de la solutio n** |
|---|---|---|---|---|---|
| CB3-2' | B8 | 9 | 200 | 66 | limpide |
| CB3-3 | B7' | 17 | 200 | 66 | limpide |

### Exemples CB4 et CB5: Préparation de compositions co-polyaminoacide/insuline glargine /insuline lispro à pH 7,2

Des compositions de co-polyaminoacide/insuline glargine 75 U/mL/insuline lispro 25 U/mL et des compositions de co-polyaminoacide/insuline glargine 150 U/mL/insuline lispro 50 U/mL sont préparées selon les procédés décrits dans les exemple CB4 et CB5.

**Tableau 2b : Compositions d'insuline glargine (75 et 150 U/mL) et d'insuline lispro (25 et 50 U/mL) en présence de co-polyaminoacide.**

| **Comp ositio n** | **Co-polyaminoacide** | **Concen tration en co-polyam inoacid e (en mg/ml )** | **Insuline glargine (U/mL)** | **Insuline Lispro (U /mL)** | **NaCl (mM )** | **Aspect visuel** |
|---|---|---|---|---|---|---|
| CB4-1 | B15 | 2,0 | 75 | 25 | 0 | Limpide |
| CB5-1 | | 4,0 | 150 | 50 | 0 | Limpide |
| CB4-2 | B2 | 1,5 | 75 | 25 | 0 | Limpide |
| CB5-2 | | 3,0 | 150 | 50 | 0 | Limpide |
| CB4-3 | B18 | 1,5 | 75 | 25 | 0 | Limpide |
| CB4-4 | | 1,2 | 75 | 25 | 5 | Limpide |
| CB5-3 | | 3,0 | 150 | 50 | 0 | Limpide |
| CB4-5 | B3 | 1,8 | 75 | 25 | 0 | Limpide |
| CB5-4 | | 3,6 | 150 | 50 | 0 | Limpide |
| CB4-6 | B9 | 1,8 | 75 | 25 | 0 | Limpide |
| CB5-5 | | 3,6 | 150 | 50 | 0 | Limpide |
| CB4-7 | B22 | 1,5 | 75 | 25 | 0 | Limpide |
| CB4-8 | | 1,3 | 75 | 25 | 5 | Limpide |
| CB5-6 | | 3,0 | 150 | 50 | 0 | Limpide |
| CB4-9 | B13 | 1,8 | 75 | 25 | 0 | Limpide |
| CB5-7 | | 3,6 | 150 | 50 | 0 | Limpide |
| CB4-10 | B7 | 1,4 | 75 | 25 | 0 | Limpide |
| CB4-11 | B14 | 2,0 | 75 | 25 | 0 | Limpide |
| CB4-12 | B19 | 2,0 | 75 | 25 | 0 | Limpide |
| CB4-13 | B20 | 3,6 | 75 | 25 | 0 | Limpide |
| CB4-14 | B21 | 1,4 | 75 | 25 | 0 | Limpide |
| CB5-9 | | 2,8 | 150 | 50 | 0 | Limpide |
| CB4-15 | CE1 | 2,0 | 75 | 25 | 0 | Limpide |
| CB4-16 | CE2 | 2,0 | 75 | 25 | 0 | Limpide |

Les co-polyaminoacides permettent de solubiliser l'insuline glargine en présence d'insuline lispro à pH neutre et de conduire à une solution limpide.

### Partie CD - Résultats

### Mise en évidence de la stabilité physique des compositions selon l'invention par l'étude des compositions précédemment préparées

### Exemple CD1 : Stabilité accélérée à 25 °C en dynamique

3 vials de 3 mL remplis avec 1 mL de composition co-polyaminoacide/insuline glargine ou co-polyaminoacide/insuline glargine/insuline prandiale sont placés verticalement sur un agitateur orbital. L'agitateur est placé dans une étuve à 25 °C et les vials sont soumis à une agitation de 250 rpm. Les vials sont inspectés visuellement de manière quotidienne/hebdomadaire afin de détecter l'apparition de particules visibles ou d'une turbidité. Cette inspection est réalisée selon les recommandations de la Pharmacopée Européenne (EP 2.9.20) : les vials sont soumis à un éclairage d'au moins 2000 Lux et sont observés face à un fond blanc et un fond noir. Le nombre de jours de stabilité correspond à la durée à partir de laquelle au moins 2 vials présentent des particules visibles ou sont turbides.

Le résultat de stabilité accélérée avec le co-polyaminoacide B4 est présenté dans le Tableau 3.

**Tableau 3 : résultats de la stabilités de la composition co-polyaminoacide B4/insuline glargine (200 U/mL)/insuline lispro (66 U/mL) à 25 °C en dynamique.**

| **Composition** | **Co-polyaminoacide** | **Concentration en co-polyaminoacide (en mg/ml)** | **Stabilité en jours** |
|---|---|---|---|
| CA3-1 | B4 | 5 | > 12 |

Le co-polyaminoacide B4 permet de solubiliser l'insuline glargine en présence d'insuline lispro à pH neutre et de conduire à une composition présentant une bonne stabilité physique.

### Exemple CD2 : Stabilité accélérée à 30 °C en statique

Au moins 5 cartouches de 3 mL remplies avec 1 mL de composition co-polyaminoacide/insuline glargine/insuline prandiale sont placées dans une étuve à 30 °C en conditions statiques. Les cartouches sont inspectées visuellement de manière bimensuelle afin de détecter l'apparition de particules visibles ou d'une turbidité. Cette inspection est réalisée selon les recommandations de la Pharmacopée Européenne (EP 2.9.20) : les cartouches sont soumises à un éclairage d'au moins 2000 Lux et sont observés face à un fond blanc et un fond noir. Le nombre de semaines de stabilité correspond à la durée à partir de laquelle la majorité des cartouches présente des particules visibles ou est turbide comparé à un standard.

Le résultat de stabilité accélérée en conditions statiques sont présentés dans le tableau 4 suivant.

**Tableau 4 : Résultats des stabilités des compositions co-polyaminoacide/insuline glargine/insuline lispro à 30 °C en conditions statiques.**

| **Composition** | **Co-polyaminoacide** | **Concentration en co-polyaminoacide (mg/ml)** | **Stabilité 30°C (semaine)** |
|---|---|---|---|
| CB4-2 | B2 | 1,5 | > 8 |
| CB4-7 | B1 | 1,5 | >18 |
| CB4-9 | B13 | 1,8 | > 23 |
| CB4-12 | B19 | 2,0 | > 8 |
| CB4-13 | B20 | 3,6 | > 8 |
| CB5-6 | B22 | 3,0 | >18 |

### Exemple CD3 Précipitation de l'insuline glargine après mélange des compositions co-polyaminoacide/insuline glargine 75 U/mL/insuline lispro 25 U/mL avec albumine.

Ce test met en évidence la précipitation de l'insuline glargine lors de l'injection dans un milieu physiologique simulé à pH et force ionique physiologiques et contenant de l'albumine. Ces conditions permettent de mimer le comportement de la composition lors de l'injection sous-cutanée. A 100 µL de composition co-polyaminoacide/insuline glargine 75 U/mL/insuline lispro 25 U/mL sont ajoutés 100 µL d'une solution d'albumine bovine à 20 mg/mL dans un tampon phosphate à pH 7.4. Le tampon phosphate (PBS ou phosphate buffer saline) est concentré de manière à ce que les teneurs en NaCl et en phosphate soient respectivement de 140 mM et 10 mM à l'issue du mélange avec la composition. La précipitation de glargine dans ce milieu est suivie à température ambiante (20-25 °C) par des mesures d'absorbance à 450 nm des mélanges pendant 30 minutes. Les mesures d'absorbance sont réalisées à l'aide d'un lecteur UV-visible de plaques multipuits.

L'absorbance augmente jusqu'à atteindre un plateau. On défini le temps de précipitation de glargine comme le temps nécessaire pour que l'absorbance mesurée soit supérieure ou égale à 80 % de la valeur du plateau. Les temps de précipitations obtenues avec les compositions précdemment décrites sont présenté dans le tableau 5.

**Tableau 5 : Temps de précipitation de l'insuline glargine après mélange des compositions co-polyaminoacide/insuline glargine/insuline lispro à un milieu qui simule le milieu sous-cutanée.**

| **Composition** | **Co-polyaminoacide** | **Concentration en co-polyaminoacide (mg/ml)** | **Temps de précipitation (minute)** |
|---|---|---|---|
| CB4-2 | B2 | 1,5 | 3 |
| CB4-7 | B1 | 1,5 | 0,5 |
| CB4-9 | B13 | 1,7 | 0,5 |
| CB4-12 | B19 | 2,0 | 2 |
| CB4-13 | B20 | 3,6 | 3 |
| CB4-15 | CE1 | 2,0 | 4 |
| CB4-16 | CE2 | 2,0 | 4 |

Les compositions co-polyaminoacide/insuline glargine/insuline lispro de l'invention mènent à une précipitation rapide de glargine après mélange avec un milieu qui simule le milieu sous-cutanée.

### Partie D Pharmacocinétique

**D1:** Protocole de mesure de la pharmacocinétique de formulations d'insuline glargine et d'insuline lispro.

Des études chez le chien ont été conduites dans l'objectif d'évaluer la pharmacocinétique des insulines après administration d'une composition co-polyaminoacide B22/insuline glargine (150 U/mL)/insuline lispro (50 U/mL).

Les profils pharmacocinétiques de l'insuline glargine (somme de la concentration circulante en insuline glargine et en son principal métabolite M1) et de l'insuline lispro ont été obtenus pour cette composition.

Dix animaux qui ont été mis à jeûn depuis 17,5 heures environ ont été injectés par voie sous-cutanée à la dose de 0,68 U/kg d'insuline. Des prélèvements sanguins sont réalisés pendant les 16h suivant l'administration pour décrire la pharmacocinétique des insulines. Les niveaux de glargine, de glargine-M1 et de lispro sont déterminés par une méthode de bioanalyse spécifique.

Les paramètres pharmacocinétiques déterminés sont les suivants :
- AUC₀₋₁ₕ, AUC₀₋₂ₕ, AUC₁₀₋₁₆ₕ correspondant à la surface sous la courbe des concentrations d'insuline glargine (et son métabolite M1) en fonction du temps entre respectivement 0 et 1 h, 0 et 2 h et 10 et 16 h post-administration ;
- AUC₀₋₃₀ₘᵢₙ, AUC₀₋₁ₕ, AUC₈₋₁₆ₕ correspondant à la surface sous la courbe des concentrations d'insuline lispro en fonction du temps entre respectivement 0 et 0,5 h, 0 et 1 h et 8 et 16 h post-administration ;
- AUCₗₐₛₜ correspondant à la surface sous la courbe entre le temps 0 et le dernier temps de mesure effectuée sur le sujet..

Le tableau 6 suivant reporte différents paramètres pharmacocinétiques d'insuline glargine et d'insuline lispro.

**Tableau 6 : Paramètres pharmacocinétiques moyens (ratio des moyennes) de la composition CB5-6 comprenant le co-polyaminoacide B22/insuline glargine 150 U/mL/insuline lispro 50 U/mL.**

| | **Insuline glargine (150 U/mL)** | | | **Insulin Lispro (50 U/mL)** | | |
|---|---|---|---|---|---|---|
| | **AUC₀₋₁ₕ/AUCₗₐₛₜ (%)** | **AUC₀₋₂ₕ/AUCₗₐₛₜ (%)** | **AUC₁₀₋₁₆ₕ/AUCₗₐₛₜ (%)** | **AUC₀₋₃₀ₘᵢₙ/AUCₗₐₛₜ (%)** | **AUC₀₋₁ₕ/AUCₗₐₛₜ (%)** | **AUC₈₋₁₆ₕ/AUCₗₐₛₜ (%)** |
| CB5-6 | 24.7 | 35.4 | 14.9 | 33.5 | 60.1 | 0.6 |

Les résultats obtenus montrent que, d'une part, la composante glargine de la formulation est absorbée rapidement (AUC₀₋₁ₕ et AUC₀₋₂ₕ) tout en conservant son caractère basal avec une couverture importante sur la partie terminale du temps d'observation (AUC₁₀₋₁₆ₕ).

D'autre part la composante lispro est rapidement absorbée (AUC₀₋₃₀ₘᵢₙ et AUC₀₋₁ₕ) et conserve son caractère prandial. En effet il n'y a plus de lispro observée au-delà de 8h (AUC₈₋₁₆ₕ).

## Revendications

1. Composition sous forme d'une solution aqueuse injectable, dont le pH est compris entre 6, 0 et 8,0, comprenant au moins :
- une insuline basale dont le point isoélectrique pI est compris entre 5,8 et 8,5 ;
- un co-polyaminoacide porteur de charges carboxylates et de radicaux hydrophobes Hy, ledit co-polyaminoacide étant constitué d'unités glutamiques ou aspartiques et lesdits radicaux hydrophobes Hy étant de formule X suivante : 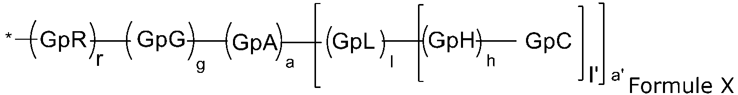 dans laquelle
- GpR est choisi parmi les radicaux de formules VII, VII' ou VII" : 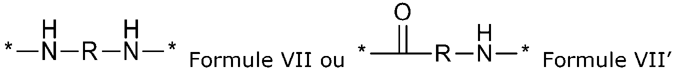 ou 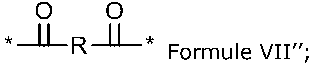
- GpG et GpH identiques ou différents sont choisis parmi les radicaux de formules XI ou XI': 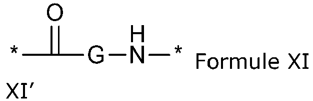 *
-NH-G-NH-* Formule
- - GpA est choisi parmi les radicaux de formule VIII 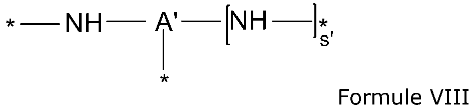 Dans laquelle A' est choisi parmi les radicaux de formule VIII',VIII" ou VIII‴ 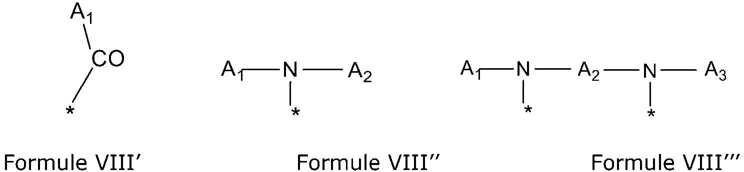
- -GpL est choisi parmi les radicaux de formule XII 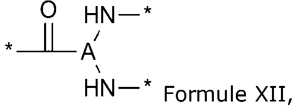
- GpC est un radical de formule IX : 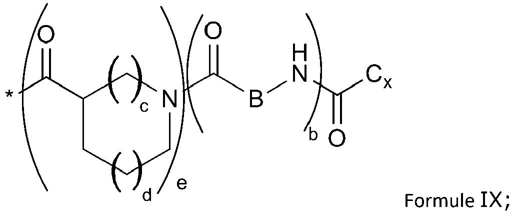
- les * indiquent les sites de rattachement des différents groupes liés par des fonctions amides ;
- a est un entier égal à 0 ou à 1 et a' = 1 si a = 0 et a' = 1, 2 ou 3 si a = 1 ;
- a' est un entier égal à 1, à 2 ou à 3
- b est un entier égal à 0 ou à 1 ;
- c est un entier égal à 0 ou à 1, et si c est égal à 0 alors d est égal à 1 ou à 2;
- d est un entier égal à 0, à 1 ou à 2 ;
- e est un entier égal à 0 ou à 1 ;
- g est un entier égal à 0, à 1, à 2, à 3 à 4 à 5 ou à 6;
- h est un entier égal à 0, à 1, à 2, à 3 à 4 à 5 ou à 6,
- l est un entier égal à 0 ou 1 et l' = 1 si l = 0 et l' = 2 si l = 1 ;
- r est un entier égal à 0, à 1 ou à 2, et
- s'est un entier égal à 0 ou 1, et
- si e est différent de 0 alors au moins un des g, h ou l est différent de 0 ; et
- si a = 0 alors l = 0 ;
- A, A₁, A₂ et A₃ identiques ou différents sont des radicaux alkyles linéaires ou ramifiés, et éventuellement substitués par un radical issu d'un cycle saturé, insaturé ou aromatique,comprenant de 1 à 8 atomes de carbone;
- **B est un** radical alkyle linéaire ou ramifié, éventuellement comprenant un noyau aromatique, comprenant de 1 à 9 atomes de carbone ou un radical éther ou polyéther non substitué comprenant de 4 à 14 atomes de carbone et de 1 à 5 atomes d'oxygène ;
- Cₓ est un radical alkyl monovalent linéaire ou ramifié, éventuellement comprenant une partie cyclique, dans lequel x indique le nombre d'atomes de carbone et :
▪ Lorsque le radical hydrophobe -Hy porte 1 -GpC, alors 9 ≤ x ≤ 25,
▪ Lorsque le radical hydrophobe -Hy porte 2 -GpC, alors 9 ≤ x ≤ 15,
▪ Lorsque le radical hydrophobe -Hy porte 3 -GpC, alors 7 ≤ x ≤ 13,
▪ Lorsque le radical hydrophobe -Hy porte 4 -GpC, alors 7 ≤ x ≤ 11,
▪ Lorsque le radical hydrophobe -Hy porte au moins 5 -GpC alors, 6 ≤ x ≤ 11,
- G est un radical alkyle ramifié de 1 à 8 atomes de carbone ledit radical alkyle portant une ou plusieurs fonction(s) acide carboxylique libre.
- R est un radical choisi dans le groupe constitué par un radical alkyle divalent, linéaire ou ramifié comprenant de 1 à 12 atomes de carbone, un radical alkyle divalent, linéaire ou ramifié comprenant de 1 à 12 atomes de carbone portant une ou plusieurs fonctions -CONH₂ ou un radical éther ou polyéther non substitué comprenant de 4 à 14 atomes de carbone et de 1 à 5 atomes d'oxygène :
- le ou les radicaux hydrophobes -Hy de formule X étant liés au PLG:
o via une liaison covalente entre un carbonyle du radical hydrophobe -Hy et un atome d'azote porté par le PLG formant ainsi une fonction amide issue de la réaction d'une fonction amine portée par le PLG et une fonction acide portée par le précurseur -Hy' du radical hydrophobe -Hy, et
∘ via une liaison covalente entre un atome d'azote du radical hydrophobe -Hy et un carbonyle porté par le PLG formant ainsi une fonction amide issue de la réaction d'une fonction amine du précurseur -Hy' du radical hydrophobe -Hy et une fonction acide portée par le PLG,
- le ratio M entre le nombre de radicaux hydrophobes et le nombre d'unités glutamiques ou aspartiques étant compris entre 0 < M ≤ 0,5 ;
- lorsque plusieurs radicaux hydrophobes sont portés par un co-polyaminoacide alors ils sont identiques ou différents,
- le degré de polymérisation DP en unités glutamiques ou aspartiques pour les chaines PLG est compris entre 5 et 250 ;
- les fonctions acides carboxyliques libres étant sous forme de sel de cation alcalin choisi dans le groupe constitué par Na⁺ et K⁺.

2. Composition selon la revendication 1, **caractérisée en ce que** le co-polyaminoacide porteur de charges carboxylates et de radicaux hydrophobes est choisi parmi les co-polyaminoacides de formule XXXa' suivante : 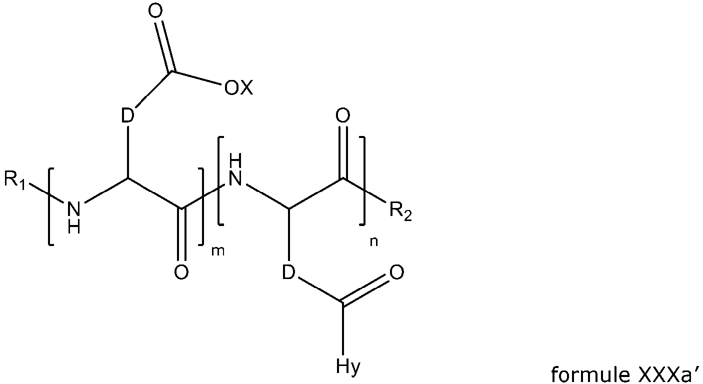 dans laquelle,
- D représente, indépendamment, soit un groupe -CH₂- (unité aspartique) soit un groupe -CH₂-CH₂- (unité glutamique),
- Hy est un radical hydrophobe choisi parmi les radicaux hydrophobes de formules X, dans lesquelles r = 1 et GpR est un radical de Formule VII,
- R₁ est un radical hydrophobe choisi parmi les radicaux hydrophobes de formules X dans lesquelles r = 0 ou r = 1 et GpR est un radical de Formule VII', ou un radical choisi dans le groupe constitué par un H, un groupe acyle linéaire en C2 à C10, un groupe acyle ramifié en C4 à C10, un benzyle, une unité « acide aminé » terminale et un pyroglutamate,
- R₂ est un radical hydrophobe choisi parmi les radicaux hydrophobes de formules X dans lesquelles r = 1 et GpR est un radical de Formule VII, ou un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et S ;
- X représente une entité cationique choisie dans le groupe comprenant les cations alcalins ;
- n + m représente le degré de polymérisation DP du co-polyaminoacide, c'est-à-dire le nombre moyen d'unités monomériques par chaîne de co-polyaminoacide et 5 ≤ n + m ≤ 250.

3. Composition selon la revendication précédente, **caractérisée en ce que** le co-polyaminoacide porteur de charges carboxylates et de radicaux hydrophobes est choisi parmi les co-polyaminoacides de formules XXXa', dans laquelle R₁ = R'₁ et R₂ = R'₂, de formule XXXa suivante : 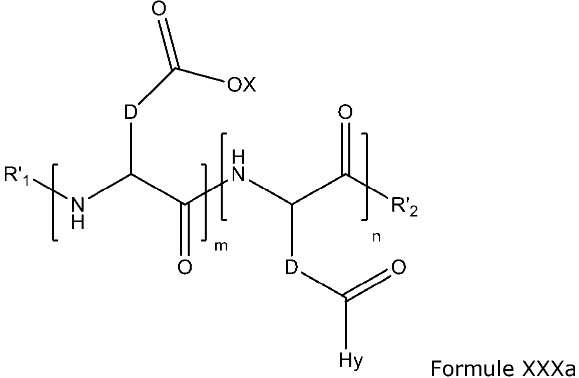 dans laquelle,
- m, n, X, D et Hy ont les définitions données précédemment,
- R'₁ est un radical choisi dans le groupe constitué par un H, un groupe acyle linéaire en C2 à C10, un groupe acyle ramifié en C4 à C10, un benzyle, une unité « acide aminé » terminale et un pyroglutamate,
- R'₂ est un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et S.

4. Composition selon la revendication 2, **caractérisée en ce que** le co-polyaminoacide porteur de charges carboxylates et de radicaux hydrophobes est choisi parmi les co-polyaminoacides de formule XXXa' dans laquelle n = 0 de formule XXXb suivante : 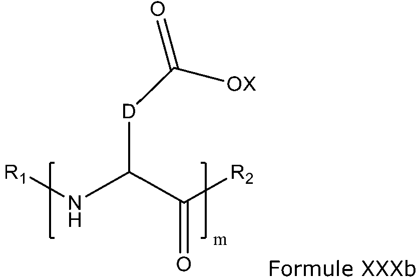 dans laquelle m, X, D, R₁ et R₂ ont les définitions données précédemment et au moins R₁ ou R₂ est un radical hydrophobe de formule X.

5. Composition selon la revendication 4 **caractérisée en ce que** le co-polyaminoacide porteur de charges carboxylates et de radicaux hydrophobes est choisi parmi les co-polyaminoacides de formule XXXa' dans laquelle n = 0 de formule XXXb et R₁ ou R₂ est un radical hydrophobe de formule X.

6. Composition selon la revendication l'une quelconque des revendications 4 et 5, **caractérisée en ce que** le co-polyaminoacide porteur de charges carboxylates et de radicaux hydrophobes est choisi parmi les co-polyaminoacides de formules XXXb dans laquelle R₂ est un radical hydrophobe de formule X dans laquelle r = 1 et GpR est de Formule VII.

7. Composition selon l'une quelconque des revendications 2 à 6, **caractérisé en ce que** R₁ est un radical choisi dans le groupe constitué par un groupe acyle linéaire en C₂ à C₁₀, un groupe acyle ramifié en C₄ à C₁₀, un benzyle, une unité « acide aminé » terminale et un pyroglutamate.

8. Composition selon la revendication précédente, **caractérisé en ce que** R₁ est un radical choisi dans le groupe constitué par un groupe acyle linéaire en C₂ à C₁₀ ou un groupe acyle ramifié en C₄ à C₁₀.

9. Composition selon l'une quelconque des revendications 2 à 8, **caractérisée en ce que** le co-polyaminoacide porteur de charges carboxylates et de radicaux hydrophobes est choisi parmi les co-polyaminoacides de formules XXXa', XXXa ou XXXb dans lesquels le co-polyaminoacide est choisi parmi les co-polyaminoacides dans lesquels le groupe D est un groupe -CH₂- (unité aspartique).

10. Composition selon l'une quelconque des revendications 2 à 8, **caractérisée en ce que** le co-polyaminoacide porteur de charges carboxylates et de radicaux hydrophobes est choisi parmi les co-polyaminoacides de formules XXXa', XXXa ou XXXb dans lesquels le co-polyaminoacide est choisi parmi les co-polyaminoacides dans lesquels le groupe D est un groupe -CH₂-CH₂- (unité glutamique).

11. Composition selon l'une quelconque des revendications précédentes, **caractérisée en ce que** l'insuline basale dont le point isoélectrique est compris entre 5,8 et 8,5 est l'insuline glargine

12. Composition selon l'une quelconque des revendications précédentes, **caractérisée en ce qu'**elle comprend entre 40 et 500 U/mL d'insuline basale dont le point isoélectrique est compris entre 5,8 et 8,5.

13. Composition selon l'une quelconque des revendications précédentes, **caractérisée en ce que** la concentration en co-polyaminoacide porteur de charges carboxylates et de radicaux hydrophobes est au plus de 60 mg/mL.

14. Composition selon l'une quelconque des revendications précédentes, **caractérisée en ce que** la concentration en co-polyaminoacide porteur de charges carboxylates et de radicaux hydrophobes est au plus de 40 mg/mL.

15. Composition selon l'une quelconque des revendications précédentes, **caractérisée en ce que** la concentration en co-polyaminoacide porteur de charges carboxylates et de radicaux hydrophobes est au plus de 20 mg/mL.

16. Composition selon l'une quelconque des revendications précédentes, **caractérisée en ce que** la concentration en co-polyaminoacide porteur de charges carboxylates et de radicaux hydrophobes est au plus de 10 mg/mL.

17. Co-polyaminoacide porteur de charges carboxylates et de radicaux hydrophobes Hy, ledit co-polyaminoacide étant constitué d'unités glutamiques ou aspartiques et lesdits radicaux hydrophobes Hy choisi parmi les radicaux de formule X telle que définie ci-dessous : 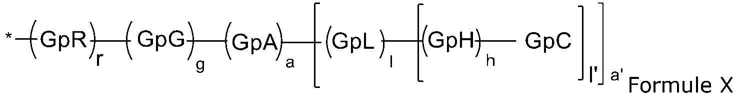 dans laquelle
GpR est choisi parmi les radicaux de formules VII, VII' ou VII" : 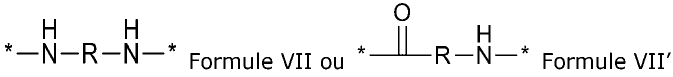 ou 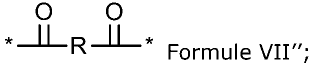
- GpG et GpH identiques ou différents sont choisis parmi les radicaux de formules XI ou XI': 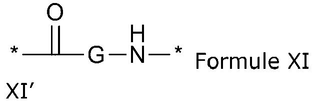 *
-NH-G-NH-* Formule
- GpA est choisi parmi les radicaux de formule VIII 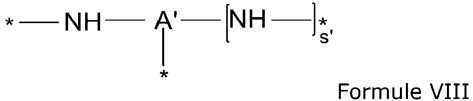 Dans laquelle A' est choisi parmi les radicaux de formule VIII',VIII" ou VIII‴ 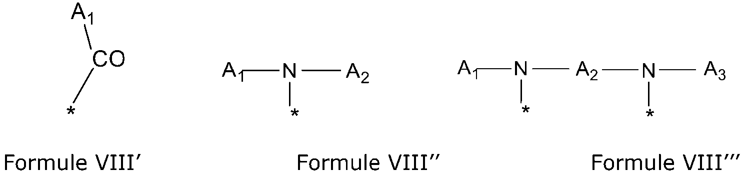
- -GpL est choisi parmi les radicaux de formule XII 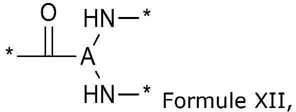
- GpC est un radical de formule IX : 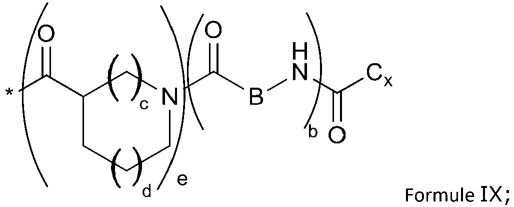
- les * indiquent les sites de rattachement des différents groupes liés par des fonctions amides ;
- a est un entier égal à 0 ou à 1 et a' = 1 si a = 0 et a' = 1, 2 ou 3 si a = 1 ;
- a' est un entier égal à 1, à 2 ou à 3 ;
- b est un entier égal à 0 ou à 1 ;
- c est un entier égal à 0 ou à 1, et si c est égal à 0 alors d est égal à 1 ou à 2;
- d est un entier égal à 0, à 1 ou à 2 ;
- e est un entier égal à 0 ou à 1 ;
- g est un entier égal à 0, à 1, à 2, à 3 à 4 à 5 ou à 6;
- h est un entier égal à 0, à 1, à 2, à 3 à 4 à 5 ou à 6, et au moins un des g, h ou l est différent de 0;
- l est un entier égal à 0 ou 1 et l' = 1 si l = 0 et l' = 2 si l = 1 ;
- r est un entier égal à 0, 1 ou à 2, et
- s'est un entier égal à 0 ou 1 ;
- et si e est différent de 0 alors au moins un des g, h ou l est différent de 0 ;
- et si a = 0 alors l = 0 ;
- A, A₁, A₂ et A₃ identiques ou différents sont des radicaux alkyles linéaires ou ramifiés comprenant de 1 à 8 atomes de carbone, et éventuellement substitués par un radical issu d'un cycle saturé, insaturé ou aromatique;
- B est un radical éther ou polyéther non substitué comprenant de 4 à 14 atomes de carbone et de 1 à 5 atomes d'oxygène ou un radical alkyle linéaire ou ramifié, éventuellement comprenant un noyau aromatique, comprenant de 1 à 9 atomes de carbone
- Cₓ est un radical alkyl monovalent linéaire ou ramifié éventuellement comprenant une partie cyclique, dans lequel x indique le nombre d'atomes de carbone et :
▪ Lorsque le radical hydrophobe -Hy porte 1 -GpC, alors 9 ≤ x ≤ 25,
▪ Lorsque le radical hydrophobe -Hy porte 2 -GpC, alors 9 ≤ x ≤ 15,
▪ Lorsque le radical hydrophobe -Hy porte 3 -GpC, alors 7 ≤ x ≤ 13,
▪ Lorsque le radical hydrophobe -Hy porte 4 -GpC, alors 7 ≤ x ≤ 11,
▪ Lorsque le radical hydrophobe -Hy porte au moins 5 -GpC alors, 6 ≤ x ≤ 11,
- G est un radical alkyle linéaire ou ramifié divalent de 1 à 8 atomes de carbone ledit radical alkyle portant une ou plusieurs fonction(s) acide carboxylique libre,
- R est un radical choisi dans le groupe constitué par un radical alkyle divalent, linéaire ou ramifié comprenant de 1 à 12 atomes de carbone, un radical alkyle divalent, linéaire ou ramifié comprenant de 1 à 12 atomes de carbone portant une ou plusieurs fonctions -CONH₂ ou un radical éther ou polyéther non substitué comprenant de 4 à 14 atomes de carbone et de 1 à 5 atomes d'oxygène,
- Le ou les radicaux hydrophobes -Hy de formule X étant liés au PLG:
o via une liaison covalente entre un carbonyle du radical hydrophobe -Hy et un atome d'azote porté par le PLG formant ainsi une fonction amide issue de la réaction d'une fonction amine portée par le PLG et une fonction acide portée par le précurseur -Hy' du radical hydrophobe -Hy, et
∘ via une liaison covalente entre un atome d'azote du radical hydrophobe -Hy et un carbonyle porté par le PLG formant ainsi une fonction amide issue de la réaction d'une fonction amine du précurseur -Hy' du radical hydrophobe -Hy et une fonction acide portée par le PLG,
- le ratio M entre le nombre de radicaux hydrophobes et le nombre d'unités glutamiques ou aspartiques étant compris entre 0 < M ≤ 0,5 ;
- lorsque plusieurs radicaux hydrophobes sont portés par un co-polyaminoacide alors ils sont identiques ou différents,
- le degré de polymérisation DP en unités glutamiques ou aspartiques pour les chaines PLG est compris entre 5 et 250 ;
- les fonctions acides carboxyliques libres étant sous forme de sel de cation alcalin choisi dans le groupe constitué par Na⁺ et K⁺.

18. Précurseur Hy' du radical hydrophobe -Hy de formule X' telle que définie ci-dessous : 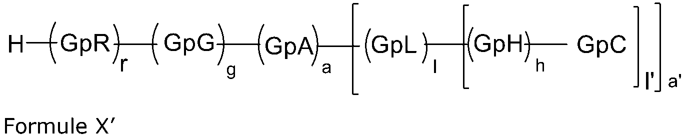 dans laquelle
GpR est choisi parmi les radicaux de formules VII, VII' ou VII" : 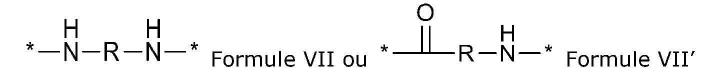 ou 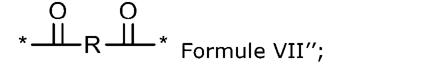
- GpG et GpH identiques ou différents sont choisis parmi les radicaux de formules XI ou XI': 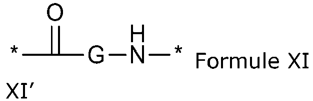 *
-NH-G-NH-* Formule
- GpA est choisi parmi les radicaux de formule VIII 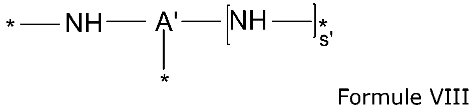 Dans laquelle A' est choisi parmi les radicaux de formule VIII',VIII" ou VIII‴ 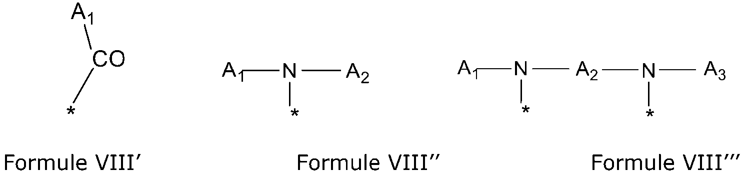
- -GpL est choisi parmi les radicaux de formule XII 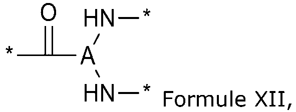
- GpC est un radical de formule IX : 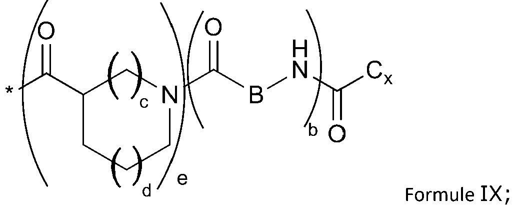
- les * indiquent les sites de rattachement des différents groupes liés par des fonctions amides ;
- a est un entier égal à 0 ou à 1 et a' = 1 si a = 0 et a' = 1, 2 ou 3 si a = 1 ;
- a' est un entier égal à 1, à 2 ou à 3 ;
- b est un entier égal à 0 ou à 1 ;
- c est un entier égal à 0 ou à 1, et si c est égal à 0 alors d est égal à 1 ou à 2;
- d est un entier égal à 0, à 1 ou à 2 ;
- e est un entier égal à 0 ou à 1 ;
- g est un entier égal à 0, à 1, à 2, à 3 à 4 à 5 ou à 6;
- h est un entier égal à 0, à 1, à 2, à 3 à 4 à 5 ou à 6, et au moins un des g, h ou l est différent de 0;
- l est un entier égal à 0 ou 1 et l' = 1 si l = 0 et l' = 2 si l = 1 ;
- r est un entier égal à 0, 1 ou à 2, et
- s'est un entier égal à 0 ou 1 ;
- et si e est différent de 0 alors au moins un des g, h ou l est différent de 0 ;
- et si a = 0 alors l = 0 ;
- A, A₁, A₂ et A₃ identiques ou différents sont des radicaux alkyles linéaires ou ramifiés comprenant de 1 à 8 atomes de carbone, et éventuellement substitués par un radical issu d'un cycle saturé, insaturé ou aromatique;
- B est un radical éther ou polyéther non substitué comprenant de 4 à 14 atomes de carbone et de 1 à 5 atomes d'oxygène ou un radical alkyle linéaire ou ramifié, éventuellement comprenant un noyau aromatique, comprenant de 1 à 9 atomes de carbone
- Cₓ est un radical alkyl monovalent linéaire ou ramifié éventuellement comprenant une partie cyclique, dans lequel x indique le nombre d'atomes de carbone et :
▪ Lorsque le radical hydrophobe -Hy porte 1 -GpC, alors 9 ≤ x ≤ 25,
▪ Lorsque le radical hydrophobe -Hy porte 2 -GpC, alors 9 ≤ x ≤ 15,
▪ Lorsque le radical hydrophobe -Hy porte 3 -GpC, alors 7 ≤ x ≤ 13,
▪ Lorsque le radical hydrophobe -Hy porte 4 -GpC, alors 7 ≤ x ≤ 11,
▪ Lorsque le radical hydrophobe -Hy porte au moins 5 -GpC alors, 6 ≤ x ≤ 11,
- G est un radical alkyle linéaire ou ramifié divalent de 1 à 8 atomes de carbone ledit radical alkyle portant une ou plusieurs fonction(s) acide carboxylique libre,
- R est un radical choisi dans le groupe constitué par un radical alkyle divalent, linéaire ou ramifié comprenant de 1 à 12 atomes de carbone, un radical alkyle divalent, linéaire ou ramifié comprenant de 1 à 12 atomes de carbone portant une ou plusieurs fonctions -CONH₂ ou un radical éther ou polyéther non substitué comprenant de 4 à 14 atomes de carbone et de 1 à 5 atomes d'oxygène,
- Le ou les radicaux hydrophobes -Hy de formule X étant liés au PLG:
o via une liaison covalente entre un carbonyle du radical hydrophobe -Hy et un atome d'azote porté par le PLG formant ainsi une fonction amide issue de la réaction d'une fonction amine portée par le PLG et une fonction acide portée par le précurseur -Hy' du radical hydrophobe -Hy, et
∘ via une liaison covalente entre un atome d'azote du radical hydrophobe -Hy et un carbonyle porté par le PLG formant ainsi une fonction amide issue de la réaction d'une fonction amine du précurseur -Hy' du radical hydrophobe -Hy et une fonction acide portée par le PLG,
- le ratio M entre le nombre de radicaux hydrophobes et le nombre d'unités glutamiques ou aspartiques étant compris entre 0 < M ≤ 0,5 ;
- lorsque plusieurs radicaux hydrophobes sont portés par un co-polyaminoacide alors ils sont identiques ou différents,
- les fonctions acides carboxyliques libres étant sous forme de sel de cation alcalin choisi dans le groupe constitué par Na⁺ et K⁺.

19. Utilisation d'espèces ioniques choisies dans le groupe des anions, des cations et/ou zwitterions pour améliorer la stabilité physico-chimique des compositions.
